# EUROPEAN PATENT SPECIFICATION

(11) **EP 2 734 618 B1**
(45) Date of publication and mention of the grant of the patent: **14.06.2017**
(21) Application number: 12746299.2
(22) Date of filing: 20.07.2012
(51) Int. Cl.: C12N 5/074, C12N 5/02, C12Q 1/68

(54) **PLURIPOTENT TENDON AND LIGAMENT PERIVASCULAR CELLS**
PLURIPOTENTE PERIVASKULÄRE SEHNEN- UND BÄNDERZELLEN
CELLULES PÉRIVASCULAIRES PLURIPOTENTES DE TENDON ET DE LIGAMENT

(30) Priority: 20.07.2011 EP 11174762
(43) Date of publication of application: 28.05.2014
(73) Proprietor: Paracelsus Medizinische Privatuniversität Salzburg - Privatstiftung, 5020 Salzburg (AT)
(72) Inventor: TEMPFER, Herbert, A-5061 Elsbethen (AT); BAUER, Hans-Christian, A-5411 Oberalm (AT); AIGNER, Ludwig, A-5061 Elsbethen (AT); RIVERA, Francisco, A-5020 Salzburg (AT); GEHWOLF, Renate, A-5020 Salzburg (AT)
(74) Representative: Vossius & Partner Patentanwälte Rechtsanwälte mbB
(86) International application number: PCT/EP2012/064304
(87) International publication number: WO 2013/011131

(56) References cited:
- WO-A1-2004/085630
- WO-A1-2008/080200
- WO-A1-2011/060135
- WO-A2-2004/094588
- WO-A2-2005/052140
- WO-A2-2008/156685
- WO-A2-2012/024573
- US-A1- 2008 213 231
- BI YANMING ET AL: "Identification of tendon stem/progenitor cells and the role of the extracellular matrix in their niche", NATURE MEDICINE, vol. 13, no. 10, October 2006 (2006-10), pages 1219-1227, XP002496557, ISSN: 1078-8956, DOI: 10.1038/NM1630 [retrieved on 2007-09-09] cited in the application
- SALINGCARNBORIBOON R ET AL: "Establishment of tendon-derived cell lines ehibiting pluripotent mesenchymal stem cell-lik property", EXPERIMENTAL CELL RESEARCH, vol. 287, 15 July 2003 (2003-07-15), pages 289-300, XP008072153, ISSN: 0014-4827, DOI: 10.1016/S0014-4827(03)00107-1 cited in the application
- TEMPFER H ET AL: "Perivascular cells of the supraspinatus tendon express both tendon and stem cell-related markers", HISTOCHEMISTRY AND CELL BIOLOGY, vol. 131, no. 6, 11 March 2009 (2009-03-11), pages 733-741, XP019714817, ISSN: 1432-119X cited in the application
- ZHANG JIANYING ET AL: "Characterization of differential properties of rabbit tendon stem cells and tenocytes", BMC MUSCULOSKELETAL DISORDERS, vol. 11, no. 1, 18 January 2010 (2010-01-18), page 10, XP021067322, ISSN: 1471-2474 cited in the application
- RUI YUN-FENG ET AL: "Isolation and Characterization of Multipotent Rat Tendon-Derived Stem Cells", TISSUE ENGINEERING PART A, vol. 16, no. 5, May 2010 (2010-05), pages 1549-1558, XP055041552, ISSN: 1937-3341, DOI: 10.1089/ten.tea.2009.0529
- CRISAN M ET AL: "A Perivascular Origin for Mesenchymal Stem Cells in Multiple Human Organs", CELL STEM CELL, vol. 3, no. 3, 11 September 2008 (2008-09-11), pages 301-313, XP002611378, ISSN: 1934-5909, DOI: 10.1016/J.STEM.2008.07.003 [retrieved on 2008-09-10] cited in the application
- ATARI M ET AL: "Isolation of pluripotent stem cells from human third molar dental pulp", HISTOLOGY AND HISTOPATHOLOGY, vol. 26, no. 8, August 2011 (2011-08), pages 1057-1070, XP008157255, ISSN: 1699-5848 cited in the application
- LEHNER C ET AL: "Tendons from non-diabetic humans and rats harbor a population of insulin-producing, pancreatic beta cell-like cells", HORMONE AND METABOLIC RESEARCH, vol. 44, no. 7, June 2012 (2012-06), pages 506-510, XP008157289, ISSN: 1439-4286
- TEMPFER H ET AL: "Human tendon perivascular cells are multipotent and express embryonic stem cell associated markers", JOURNAL OF TISSUE ENGINEERING AND REGENERATIVE MEDICINE, [Online] vol. 6, no. Suppl. 1, 40.02, 1 September 2012 (2012-09-01), page 249, XP008157294, 3rd World Congress of the Tissue-Engineering-and-Regenerative-Medici ne-International-Society (TERMIS; Vienna, Austria; 5-8 September 2012 ISSN: 1932-6254 Retrieved from the Internet: URL:http://www3.interscience.wiley.com/cgi -bin/issn?DESCRIPTOR=PRINTISSN&VALUE=1932- 6254>

### SEQUENCE LISTING

<110> Angewandte Biotechnologie GmbH
<120> Pluripotent tendon and ligament perivascular cells
<130> S3254 PCT S3
<150> EP11 17 4762.2
   <151> 2011-07-20
<160> 118
<170> BiSSAP 1.0
<210> 1
   <211> 22
   <212> DNA
   <213> artificial sequences
<220>
   <221> source
   <222> 1..22
   <223> /mol_type="DNA"
   /note="human sense primer hOct4"
   /organism="artificial sequences"
<400> 1
   acctggctaa gcttccaagg cc 22
<210> 2
   <211> 22
   <212> DNA
   <213> artificial sequences
<220>
   <221> source
   <222> 1..22
   <223> /mol_type="DNA"
   /note="human antisense primer hOct4"
   /organism="artificial sequences"
<400> 2
   tcgtttggct gaataccttc cc 22
<210> 3
   <211> 21
   <212> DNA
   <213> artificial sequences
<220>
   <221> source
   <222> 1..21
   <223> /mol_type="DNA"
   /note="human sense primer Sox2"
   /organism="artificial sequences"
<400> 3
   aagatgcaca actcggagat c 21
<210> 4
   <211> 21
   <212> DNA
   <213> artificial sequences
<220>
   <221> source
   <222> 1..21
   <223> /mol_type="DNA"
   /note="human antisense primer Sox2"
   /organism="artificial sequences"
<400> 4
   tatttataat ccgggtgctc c 21
<210> 5
   <211> 22
   <212> DNA
   <213> artificial sequences
<220>
   <221> source
   <222> 1..22
   <223> /mol_type="DNA"
   /note="human sense primer hNanog"
   /organism="artificial sequences"
<400> 5
   tcaggacagc cctgattctt cc 22
<210> 6
   <211> 18
   <212> DNA
   <213> artificial sequences
<220>
   <221> source
   <222> 1..18
   <223> /mol_type="DNA"
   /note="human antisense primer hNanog"
   /organism="artificial sequences"
<400> 6
   tcttgcatct gctggagg 18
<210> 7
   <211> 21
   <212> DNA
   <213> artificial sequences
<220>
   <221> source
   <222> 1..21
   <223> /mol_type="DNA"
   /note="human sense primer Klf-4"
   /organism="artificial sequences"
<400> 7
   agaccgagga gttcaacgat c 21
<210> 8
   <211> 21
   <212> DNA
   <213> artificial sequences
<220>
   <221> source
   <222> 1..21
   <223> /mol_type="DNA"
   /note="human antisense primer Klf-4"
   /organism="artificial sequences"
<400> 8
   ttgatgtccg ccaggttgaa g 21
<210> 9
   <211> 20
   <212> DNA
   <213> artificial sequences
<220>
   <221> source
   <222> 1..20
   <223> /mol_type="DNA"
   /note="human sense primer cMyc"
   /organism="artificial sequences"
<400> 9
   acaggaacta tgacctcgac 20
<210> 10
   <211> 20
   <212> DNA
   <213> artificial sequences
<220>
   <221> source
   <222> 1..20
   <223> /mol_type="DNA"
   /note="human antisense primer cMyc"
   /organism="artificial sequences"
<400> 10
   tacagtcctg gatgatgatg 20
<210> 11
   <211> 23
   <212> DNA
   <213> artificial sequences
<220>
   <221> source
   <222> 1..23
   <223> /mol_type="DNA"
   /note="human sense primer hNestin"
   /organism="artificial sequences"
<400> 11
   tctgaggaag tggggcaagg aat 23
<210> 12
   <211> 24
   <212> DNA
   <213> artificial sequences
<220>
   <221> source
   <222> 1..24
   <223> /mol_type="DNA"
   /note="human antisense primer hNestin"
   /organism="artificial sequences"
<400> 12
   ttaagagtgc tgctcctgag cagg 24
<210> 13
   <211> 22
   <212> DNA
   <213> artificial sequences
<220>
   <221> source
   <222> 1..22
   <223> /mol_type="DNA"
   /note="human sense primer Col1A1"
   /organism="artificial sequences"
<400> 13
   tcacctacca ctgcaagaac ag 22
<210> 14
   <211> 21
   <212> DNA
   <213> artificial sequences
<220>
   <221> source
   <222> 1..21
   <223> /mol_type="DNA"
   /note="human antisense primer Col1A1"
   /organism="artificial sequences"
<400> 14
   atggagggag tttacaggaa g 21
<210> 15
   <211> 19
   <212> DNA
   <213> artificial sequences
<220>
   <221> source
   <222> 1..19
   <223> /mol_type="DNA"
   /note="human sense primer Col3A1"
   /organism="artificial sequences"
<400> 15
   agtgccaatc ctttgaatg 19
<210> 16
   <211> 20
   <212> DNA
   <213> artificial sequences
<220>
   <221> source
   <222> 1..20
   <223> /mol_type="DNA"
   /note="human antisense primer Col3A1"
   /organism="artificial sequences"
<400> 16
   tatgtgatgt tctgggaagc 20
<210> 17
   <211> 21
   <212> DNA
   <213> artificial sequences
<220>
   <221> source
   <222> 1..21
   <223> /mol_type="DNA"
   /note="human sense primer hGalc"
   /organism="artificial sequences"
<400> 17
   tggatcttac agggttacag g 21
<210> 18
   <211> 21
   <212> DNA
   <213> artificial sequences
<220>
   <221> source
   <222> 1..21
   <223> /mol_type="DNA"
   /note="human antisense primer Galc"
   /organism="artificial sequences"
<400> 18
   acacaccagg taatgttaaa g 21
<210> 19
   <211> 23
   <212> DNA
   <213> artificial sequences
<220>
   <221> source
   <222> 1..23
   <223> /mol_type="DNA"
   /note="human sense primer hSCX"
   /organism="artificial sequences"
<400> 19
   tgcgcctggc ctccagctac atc 23
<210> 20
   <211> 23
   <212> DNA
   <213> artificial sequences
<220>
   <221> source
   <222> 1..23
   <223> /mol_type="DNA"
   /note="human antisense primer hSCX"
   /organism="artificial sequences"
<400> 20
   gttgctgagg cagaaggtgc aga 23
<210> 21
   <211> 24
   <212> DNA
   <213> artificial sequences
<220>
   <221> source
   <222> 1..24
   <223> /mol_type="DNA"
   /note="human sense primer hSOX9"
   /organism="artificial sequences"
<400> 21
   tcagcccact gacagacctt aatc 24
<210> 22
   <211> 23
   <212> DNA
   <213> artificial sequences
<220>
   <221> source
   <222> 1..23
   <223> /mol_type="DNA"
   /note="human antisense primer hSOX9"
   /organism="artificial sequences"
<400> 22
   aggtttgggg ctgggaggga aac 23
<210> 23
   <211> 22
   <212> DNA
   <213> artificial sequences
<220>
   <221> source
   <222> 1..22
   <223> /mol_type="DNA"
   /note="human sense primer INS1"
   /organism="artificial sequences"
<400> 23
   tcatagacca tcagcaagca gg 22
<210> 24
   <211> 21
   <212> DNA
   <213> artificial sequences
<220>
   <221> source
   <222> 1..21
   <223> /mol_type="DNA"
   /note="human antisense primer INS1"
   /organism="artificial sequences"
<400> 24
   tggtggactc agttgcagta g 21
<210> 25
   <211> 20
   <212> DNA
   <213> artificial sequences
<220>
   <221> source
   <222> 1..20
   <223> /mol_type="DNA"
   /note="rat sense primer ratOct4"
   /organism="artificial sequences"
<400> 25
   tggctaagct tccaggggcc 20
<210> 26
   <211> 21
   <212> DNA
   <213> artificial sequences
<220>
   <221> source
   <222> 1..21
   <223> /mol_type="DNA"
   /note="rat antisense primer ratOct4"
   /organism="artificial sequences"
<400> 26
   atggttgtct ggctgaacac c 21
<210> 27
   <211> 21
   <212> DNA
   <213> artificial sequences
<220>
   <221> source
   <222> 1..21
   <223> /mol_type="DNA"
   /note="rat sense primer Sox2"
   /organism="artificial sequences"
<400> 27
   aagatgcaca actcggagat c 21
<210> 28
   <211> 21
   <212> DNA
   <213> artificial sequences
<220>
   <221> source
   <222> 1..21
   <223> /mol_type="DNA"
   /note="rat antisense primer Sox2"
   /organism="artificial sequences"
<400> 28
   tatttataat ccgggtgctc c 21
<210> 29
   <211> 21
   <212> DNA
   <213> artificial sequences
<220>
   <221> source
   <222> 1..21
   <223> /mol_type="DNA"
   /note="rat sense primer ratNanog"
   /organism="artificial sequences"
<400> 29
   tcaagatagc cctgattctt c 21
<210> 30
   <211> 18
   <212> DNA
   <213> artificial sequences
<220>
   <221> source
   <222> 1..18
   <223> /mol_type="DNA"
   /note="rat antisense primer ratNanog"
   /organism="artificial sequences"
<400> 30
   tcttgcatct gctggagg 18
<210> 31
   <211> 21
   <212> DNA
   <213> artificial sequences
<220>
   <221> source
   <222> 1..21
   <223> /mol_type="DNA"
   /note="rat sense primer Klf-4"
   /organism="artificial sequences"
<400> 31
   agaccgagga gttcaacgat c 21
<210> 32
   <211> 21
   <212> DNA
   <213> artificial sequences
<220>
   <221> source
   <222> 1..21
   <223> /mol_type="DNA"
   /note="rat antisense primer Klf-4"
   /organism="artificial sequences"
<400> 32
   ttgatgtccg ccaggttgaa g 21
<210> 33
   <211> 20
   <212> DNA
   <213> artificial sequences
<220>
   <221> source
   <222> 1..20
   <223> /mol_type="DNA"
   /note="rat sense primer cMyc"
   /organism="artificial sequences"
<400> 33
   acaggaacta tgacctcgac 20
<210> 34
   <211> 20
   <212> DNA
   <213> artificial sequences
<220>
   <221> source
   <222> 1..20
   <223> /mol_type="DNA"
   /note="rat antisense primer cMyc"
   /organism="artificial sequences"
<400> 34
   tacagtcctg gatgatgatg 20
<210> 35
   <211> 21
   <212> DNA
   <213> artificial sequences
<220>
   <221> source
   <222> 1..21
<223> /mol_type="DNA"
   /note="rat sense primer rat_Nestin"
   /organism="artificial sequences"
<400> 35
   tgctgatgag gaagaaagtg g 21
<210> 36
   <211> 21
   <212> DNA
   <213> artificial sequences
<220>
   <221> source
   <222> 1..21
   <223> /mol_type="DNA"
   /note="rat antisense primer rat_Nestin"
   /organism="artificial sequences"
<400> 36
   accaatgtca aaggcatctc c 21
<210> 37
   <211> 22
   <212> DNA
   <213> artificial sequences
<220>
   <221> source
   <222> 1..22
   <223> /mol_type="DNA"
   /note="rat sense primer Col1A1"
   /organism="artificial sequences"
<400> 37
   tcacctacca ctgcaagaac ag 22
<210> 38
   <211> 21
   <212> DNA
   <213> artificial sequences
<220>
   <221> source
   <222> 1..21
   <223> /mol_type="DNA"
   /note="rat antisense primer Col1A1"
   /organism="artificial sequences"
<400> 38
   atggagggag tttacaggaa g 21
<210> 39
   <211> 19
   <212> DNA
   <213> artificial sequences
<220>
   <221> source
<222> 1..19
   <223> /mol_type="DNA"
   /note="rat sense primer Col3A1"
   /organism="artificial sequences"
<400> 39
   agtgccaatc ctttgaatg 19
<210> 40
   <211> 20
   <212> DNA
   <213> artificial sequences
<220>
   <221> source
   <222> 1..20
   <223> /mol_type="DNA"
   /note="rat antisense primer Col3A1"
   /organism="artificial sequences"
<400> 40
   tatgtgatgt tctgggaagc 20
<210> 41
   <211> 22
   <212> DNA
   <213> artificial sequences
<220>
   <221> source
   <222> 1..22
   <223> /mol_type="DNA"
   /note="rat sense primer ratGalc"
   /organism="artificial sequences"
<400> 41
   tgttcacact caccactctg ac 22
<210> 42
   <211> 22
   <212> DNA
   <213> artificial sequences
<220>
   <221> source
   <222> 1..22
   <223> /mol_type="DNA"
   /note="rat antisense primer ratGalc"
   /organism="artificial sequences"
<400> 42
   aggtcggctg taactctgta ag 22
<210> 43
   <211> 23
   <212> DNA
   <213> artificial sequences
<220>
   <221> source
   <222> 1..23
   <223> /mol_type="DNA"
   /note="rat sense primer SCX"
   /organism="artificial sequences"
<400> 43
   tgcgcctggc ctccagctac atc 23
<210> 44
   <211> 23
   <212> DNA
   <213> artificial sequences
<220>
   <221> source
   <222> 1..23
   <223> /mol_type="DNA"
   /note="rat antisense primer SCX"
   /organism="artificial sequences"
<400> 44
   gttgctgagg cagaaggtgc aga 23
<210> 45
   <211> 24
   <212> DNA
   <213> artificial sequences
<220>
   <221> source
   <222> 1..24
   <223> /mol_type="DNA"
   /note="rat sense primer SOX9"
   /organism="artificial sequences"
<400> 45
   tcagcccact gacagacctt aatc 24
<210> 46
   <211> 23
   <212> DNA
   <213> artificial sequences
<220>
   <221> source
   <222> 1..23
   <223> /mol_type="DNA"
   /note="rat antisense primer SOX9"
   /organism="artificial sequences"
<400> 46
   aggtttgggg ctgggaggga aac 23
<210> 47
   <211> 22
   <212> DNA
   <213> artificial sequences
<220>
   <221> source
   <222> 1..22
   <223> /mol_type="DNA"
   /note="rat sense primer INS1"
   /organism="artificial sequences"
<400> 47
   tcatagacca tcagcaagca gg 22
<210> 48
   <211> 21
   <212> DNA
   <213> artificial sequences
<220>
   <221> source
   <222> 1..21
   <223> /mol_type="DNA"
   /note="rat antisense primer INS1"
   /organism="artificial sequences"
<400> 48
   tggtggactc agttgcagta g 21
<210> 49
   <211> 5591
   <212> DNA
   <213> Homo sapiens
<220>
   <221> source
   <222> 1..5591
   <223> /mol_type="DNA"
   /note="Homo sapiens Nestin"
   /organism="Homo sapiens"
<400> 49
   gctactccca ccccgccccg ccccgtcatt gtccccgtcg gtctcttttc tcttccgtcc      60
   taaaagctct gcgagccgct cccttctccc ggtgccccgc gtctgtccat cctcagtggg     120
   tcagacgagc aggatggagg gctgcatggg ggaggagtcg tttcagatgt gggagctcaa     180
   tcggcgcctg gaggcctacc tggcccgggt caaggcgctg gaggagcaga atgagctgct     240
   cagcgcggag ctcggggggc tccgggcaca atccgcggac acctcctggc gggcgcatgc      300
   cgacgacgag ctggcggccc tgcgggccct cgttgaccaa cgctggcggg agaagcacgc     360
   ggccgaggtg gcgcgcgaca acctggctga agagctggag ggcgtggcag gccgatgcca     420
   gcagctgcgg ctggcccggg agcggacgac ggaggaggta gcccgcaacc ggcgcgccgt     480
   cgaggcagag aaatgcgccc gggcctggct gagtagccag gtggcagagc tggagcgcga     540
   gctagaggct ctacgcgtgg cgcacgagga ggagcgcgtc ggcctgaacg cgcaggctgc     600
   ctgtgccccc cgctgccccg cgccgccccg cgggcctccc gcgccggccc cggaggtaga     660
   ggagctggca aggcgactgg gcgaggcgtg gcgcggggca gtgcgcggct accaggagcg     720
   cgtggcacac atggagacgt cgctgggcca ggcccgcgag cggctgggcc gggcggtgca 780
   gggtgcccgc gagggccgcc tggagctgca gcagctccag gctgagcgcg gaggcctcct 840
   ggagcgcagg gcagcgttgg aacagaggtt ggagggccgc tggcaggagc ggctgcgggc 900
   tactgaaaag ttccagctgg ctgtggaggc cctggagcag gagaaacagg gcctacagag 960
   ccagatcgct caggtcctgg aaggtcggca gcagctggcg cacctcaaga tgtccctcag 1020
   cctggaggtg gccacgtaca ggaccctcct ggaggctgag aactcccggc tgcaaacacc 1080
   tggcggtggc tccaagactt ccctcagctt tcaggacccc aagctggagc tgcaattccc 1140
   taggacccca gagggccggc gtcttggatc tttgctccca gtcctgagcc caacttccct 1200
   cccctcaccc ttgcctgcta cccttgagac acctgtgcca gcctttctta agaaccaaga 1260
   attcctccag gcccgtaccc ctaccttggc cagcaccccc atccccccca cacctcaggc 1320
   accctctcct gctgtagatg cagagatcag agcccaggat gctcctctct ctctgctcca 1380
   gacacagggt gggaggaaac aggctccaga gcccctgcgg gctgaagcca gggtggccat 1440
   tcctgccagc gtcctgcctg gaccagagga gcctgggggc cagcggcaag aggccagtac 1500
   aggccagtcc ccagaggacc atgcctcctt ggcaccaccc ctcagccctg accactccag 1560
   tttagaggct aaggatggag aatccggtgg gtctagagtg ttcagcatat gccgagggga 1620
   aggtgaaggg caaatctggg ggttggtaga gaaagaaaca gccatagagg gcaaagtggt 1680
   aagcagcttg cagcaggaaa tatgggaaga agaggatcta aacaggaagg aaatccagga 1740
   ctcccaggtt cctttggaaa aagaaaccct gaagtctctg ggagaggaga ttcaagagtc 1800
   actgaagact ctggaaaacc agagccatga gacactagaa agggagaatc aagaatgtcc 1860
   gaggtcttta gaagaagact tagaaacact aaaaagtcta gaaaaggaaa ataaagagct 1920
   attaaaggat gtggaggtag tgagacctct agaaaaagag gctgtaggcc aacttaagcc 1980
   tacaggaaaa gaggacacac agacattgca atccctgcaa aaggagaatc aagaactaat 2040
   gaaatctctt gaaggtaatc tagagacatt tttatttcca ggaacggaaa atcaagaatt 2100
   agtaagttct ctgcaagaga acttagagtc attgacagct ctggaaaagg agaatcaaga 2160
   gccactgaga tctccagaag taggggatga ggaggcactg agacctctga caaaggagaa 2220
   tcaggaaccc ctgaggtctc ttgaagatga gaacaaagag gcctttagat ctctagaaaa 2280
   agagaaccag gagccactga agactctaga agaagaggac cagagtattg tgagacctct 2340
   agaaacagag aatcacaaat cactgaggtc tttagaagaa caggaccaag agacattgag 2400
   aactcttgaa aaagagactc aacagcgacg gaggtctcta ggggaacagg atcagatgac 2460
   attaagaccc ccagaaaaag tggatctaga accactgaag tctcttgacc aggagatagc 2520
   tagacctctt gaaaatgaga atcaagagtt cttaaagtca ctcaaagaag agagcgtaga 2580
   ggcagtaaaa tctttagaaa cagagatcct agaatcactg aagtctgcgg gacaagagaa 2640
   cctggaaaca ctgaaatctc cagaaactca agcaccactg tggactccag aagaaataaa 2700
   tcagggggca atgaatcctc tagaaaagga aattcaagaa ccactggagt ctgtggaagt 2760
   gaaccaagag acattcagac tcctggaaga ggagaatcag gaatcattga gatctctggg 2820
   agcatggaac ctggagaatt tgagatctcc agaggaggta gacaaggaaa gtcaaaggaa 2880
   tctggaagag gaagagaacc tgggaaaggg agagtaccaa gagtcactga ggtctctgga 2940
   ggaggaggga caggagctgc cgcagtctgc agatgtgcag aggtgggaag atacggtgga 3000
   gaaggaccaa gaactggctc aggaaagccc tcctgggatg gctggagtgg aaaatgagga 3060
   tgaggcagag ctgaatctga gggagcagga tggcttcact gggaaggagg aggtggtaga 3120
   gcagggagag ctgaatgcca cagaggaggt ctggatccca ggcgaggggc acccagagag 3180
   ccctgagccc aaagagcaga gaggcctggt tgagggagcc agtgtgaagg gaggggctga 3240
   gggcctccag gaccctgaag ggcaatcaca acaggtgggg gccccaggcc tccaggctcc 3300
   ccaggggctg ccagaggcga tagagcccct ggtggaagat gatgtggccc cagggggtga 3360
   ccaagcctcc ccagaggtca tgttggggtc agagcctgcc atgggtgagt ctgctgcggg 3420
   agctgagcca ggcccggggc agggggtggg agggctgggg gacccaggcc atctgaccag 3480
   ggaagaggtg atggaaccac ccctggaaga ggagagtttg gaggcaaaga gggttcaggg 3540
   cttggaaggg cctagaaagg acctagagga ggcaggtggt ctggggacag agttctccga 3600
   gctgcctggg aagagcagag acccttggga gcctcccagg gagggtaggg aggagtcaga 3660
   ggctgaggcc cccaggggag cagaggaggc gttccctgct gagaccctgg gccacactgg 3720
   aagtgatgcc ccttcacctt ggcctctggg gtcagaggaa gctgaggagg atgtaccacc 3780
   agtgctggtc tcccccagcc caacgtacac cccgatcctg gaagatgccc ctgggcctca 3840
   gcctcaggct gaagggagtc aggaggctag ctggggggtg caggggaggg ctgaagccct 3900
   ggggaaagta gagagcgagc aggaggagtt gggttctggg gagatccccg agggccccca 3960
   ggaggaaggg gaggagagca gagaagagag cgaggaggat gagctcgggg agacccttcc 4020
   agactccact cccctgggct tctacctcag gtcccccacc tcccccaggt gggaccccac 4080
   tggagagcag aggccacccc ctcaagggga gactggaaag gagggctggg atcctgctgt 4140
   cctggcttcc gagggccttg aggccccacc ctcagaaaag gaggaggggg aggagggaga 4200
   agaggagtgt ggccgtgact ctgacctgtc agaagaattt gaggacctgg ggactgaggc 4260
   accttttctt cctggggtcc ctggggaggt ggcagaacct ctgggccagg tgccccagct 4320
   gctactggat cctgcagcct gggatcgaga tggggagtcc gatgggtttg cagatgagga 4380
   agaaagtggg gaggagggag aggaggatca ggaggagggg agggagccag gggctgggcg 4440
   gtgggggcca gggtcttctg ttggcagcct ccaggccctg agtagctccc agagagggga 4500
   attcctggag tctgattctg tgagtgtcag tgtcccctgg gatgacagct tgaggggtgc 4560
   agtggctggt gcccccaaga ctgccctgga aacggagtcc caggacagtg ctgagccttc 4620
   tggctcagag gaagagtctg accctgtttc cttggagagg gaggacaaag tccctggccc 4680
   tctagagatc cccagtggga tggaggatgc aggcccaggg gcagacatca ttggtgttaa 4740
   tggccagggt cccaacttgg aggggaagtc acagcatgtg aatgggggag tgatgaacgg 4800
   gctggagcag tctgaggaag tggggcaagg aatgccgcta gtctctgagg gagaccgagg 4860
   gagccccttt caggaggagg aggggagtgc tctgaagacc tcttgggcag gggctcctgt 4920
   tcacctgggc cagggtcagt tcctgaagtt cactcagagg gaaggagata gagagtcctg 4980
   gtcctcaggg gaggactagg aaaagaccat ctgcccggca ctggggactt aggggtgcgg 5040
   ggaggggaag gacgcctcca agcccgctcc ctgctcagga gcagcactct taacttacga 5100
   tctcttgaca tatggtttct ggctgagagg cctggcccgc taaggtgaaa aggggtgtgg 5160
   caaaggagcc tactccaaga atggaggctg taggaatata acctcccacc ctgcaaaggg 5220
   aatctcttgc ctgctccatc tcataggcta agtcagctga atcccgatag tactaggtcc 5280
   ccttccctcc gcatcccgtc agctggaaaa ggcctgtggc ccagaggctt ctccaaaggg 5340
   agggtgacat gctggctttt gtgcccaagc tcaccagccc tgcgccacct cactgcagta 5400
   gtgcaccatc tcactgcagt agcacgccct cctgggccgt ctggcctgtg gctaatggag 5460
   gtgacggcac tcccatgtgc tgactccccc catccctgcc acgctgtggc cctgcctggc 5520
   tagtccctgc ctgaataaag taatgcctcc gcttcaaaaa aaaaaaaaaa aaaaaaaaaa 5580
   aaaaaaaaaa a 5591
<210> 50
   <211> 1621
   <212> PRT
   <213> Homo sapiens
<220>
   <221> SOURCE
   <222> 1..1621
   <223> /mol_type="protein"
   /note="Homo sapiens Nestin"
   /organism="Homo sapiens"
<400> 50
   Met Glu Gly Cys Met Gly Glu Glu Ser Phe Gln Met Trp Glu Leu Asn
   1               5                   10                  15
   Arg Arg Leu Glu Ala Tyr Leu Ala Arg Val Lys Ala Leu Glu Glu Gln
               20                  25                  30
   Asn Glu Leu Leu Ser Ala Glu Leu Gly Gly Leu Arg Ala Gln Ser Ala
           35                  40                  45
   Asp Thr Ser Trp Arg Ala His Ala Asp Asp Glu Leu Ala Ala Leu Arg
       50                  55                  60
   Ala Leu Val Asp Gln Arg Trp Arg Glu Lys His Ala Ala Glu Val Ala
   65                  70                  75                  80
   Arg Asp Asn Leu Ala Glu Glu Leu Glu Gly Val Ala Gly Arg Cys Gln
   85 90 95
   Gln Leu Arg Leu Ala Arg Glu Arg Thr Thr Glu Glu Val Ala Arg Asn
   100 105 110
   Arg Arg Ala Val Glu Ala Glu Lys Cys Ala Arg Ala Trp Leu Ser Ser
   115 120 125
   Gln Val Ala Glu Leu Glu Arg Glu Leu Glu Ala Leu Arg Val Ala His
   130 135 140
   Glu Glu Glu Arg Val Gly Leu Asn Ala Gln Ala Ala Cys Ala Pro Arg
   145 150 155 160
   Cys Pro Ala Pro Pro Arg Gly Pro Pro Ala Pro Ala Pro Glu Val Glu
   165 170 175
   Glu Leu Ala Arg Arg Leu Gly Glu Ala Trp Arg Gly Ala Val Arg Gly
   180 185 190
   Tyr Gln Glu Arg Val Ala His Met Glu Thr Ser Leu Gly Gln Ala Arg
   195 200 205
   Glu Arg Leu Gly Arg Ala Val Gln Gly Ala Arg Glu Gly Arg Leu Glu
   210 215 220
   Leu Gln Gln Leu Gln Ala Glu Arg Gly Gly Leu Leu Glu Arg Arg Ala
   225 230 235 240
   Ala Leu Glu Gln Arg Leu Glu Gly Arg Trp Gln Glu Arg Leu Arg Ala
   245 250 255
   Thr Glu Lys Phe Gln Leu Ala Val Glu Ala Leu Glu Gln Glu Lys Gln
   260 265 270
   Gly Leu Gln Ser Gln Ile Ala Gln Val Leu Glu Gly Arg Gln Gln Leu
   275 280 285
   Ala His Leu Lys Met Ser Leu Ser Leu Glu Val Ala Thr Tyr Arg Thr
   290 295 300
   Leu Leu Glu Ala Glu Asn Ser Arg Leu Gln Thr Pro Gly Gly Gly Ser
   305 310 315 320
   Lys Thr Ser Leu Ser Phe Gln Asp Pro Lys Leu Glu Leu Gln Phe Pro
   325 330 335
   Arg Thr Pro Glu Gly Arg Arg Leu Gly Ser Leu Leu Pro Val Leu Ser
   340 345 350
   Pro Thr Ser Leu Pro Ser Pro Leu Pro Ala Thr Leu Glu Thr Pro Val
   355 360 365
   Pro Ala Phe Leu Lys Asn Gln Glu Phe Leu Gln Ala Arg Thr Pro Thr
   370 375 380
   Leu Ala Ser Thr Pro Ile Pro Pro Thr Pro Gln Ala Pro Ser Pro Ala
   385 390 395 400
   Val Asp Ala Glu Ile Arg Ala Gln Asp Ala Pro Leu Ser Leu Leu Gln
   405 410 415
   Thr Gln Gly Gly Arg Lys Gln Ala Pro Glu Pro Leu Arg Ala Glu Ala
   420 425 430
   Arg Val Ala Ile Pro Ala Ser Val Leu Pro Gly Pro Glu Glu Pro Gly
   435 440 445
   Gly Gln Arg Gln Glu Ala Ser Thr Gly Gln Ser Pro Glu Asp His Ala
   450 455 460
   Ser Leu Ala Pro Pro Leu Ser Pro Asp His Ser Ser Leu Glu Ala Lys
   465 470 475 480
   Asp Gly Glu Ser Gly Gly Ser Arg Val Phe Ser Ile Cys Arg Gly Glu
   485 490 495
   Gly Glu Gly Gln Ile Trp Gly Leu Val Glu Lys Glu Thr Ala Ile Glu
   500 505 510
   Gly Lys Val Val Ser Ser Leu Gln Gln Glu Ile Trp Glu Glu Glu Asp
   515 520 525
   Leu Asn Arg Lys Glu Ile Gln Asp Ser Gln Val Pro Leu Glu Lys Glu
   530 535 540
   Thr Leu Lys Ser Leu Gly Glu Glu Ile Gln Glu Ser Leu Lys Thr Leu
   545 550 555 560
   Glu Asn Gln Ser His Glu Thr Leu Glu Arg Glu Asn Gln Glu Cys Pro
   565 570 575
   Arg Ser Leu Glu Glu Asp Leu Glu Thr Leu Lys Ser Leu Glu Lys Glu
                   580                      585                      590
   Asn Lys Glu Leu Leu Lys Asp Val Glu Val Val Arg Pro Leu Glu Lys
               595                     600                     605
   Glu Ala Val Gly Gln Leu Lys Pro Thr Gly Lys Glu Asp Thr Gln Thr
       610                     615                     620
   Leu Gln Ser Leu Gln Lys Glu Asn Gln Glu Leu Met Lys Ser Leu Glu
   625                     630                     635                     640
   Gly Asn Leu Glu Thr Phe Leu Phe Pro Gly Thr Glu Asn Gln Glu Leu
                       645                     650                     655
   Val Ser Ser Leu Gln Glu Asn Leu Glu Ser Leu Thr Ala Leu Glu Lys
                   660                     665                     670
   Glu Asn Gln Glu Pro Leu Arg Ser Pro Glu Val Gly Asp Glu Glu Ala
               675                     680                     685
   Leu Arg Pro Leu Thr Lys Glu Asn Gln Glu Pro Leu Arg Ser Leu Glu
       690                     695                     700
   Asp Glu Asn Lys Glu Ala Phe Arg Ser Leu Glu Lys Glu Asn Gln Glu
   705                     710                     715                     720
   Pro Leu Lys Thr Leu Glu Glu Glu Asp Gln Ser Ile Val Arg Pro Leu
                       725                     730                     735
   Glu Thr Glu Asn His Lys Ser Leu Arg Ser Leu Glu Glu Gln Asp Gln
                   740                     745                     750
   Glu Thr Leu Arg Thr Leu Glu Lys Glu Thr Gln Gln Arg Arg Arg Ser
               755                     760                     765
   Leu Gly Glu Gln Asp Gln Met Thr Leu Arg Pro Pro Glu Lys Val Asp
       770                     775                     780
   Leu Glu Pro Leu Lys Ser Leu Asp Gln Glu Ile Ala Arg Pro Leu Glu
   785                     790                     795                     800
   Asn Glu Asn Gln Glu Phe Leu Lys Ser Leu Lys Glu Glu Ser Val Glu
                       805                     810                     815
   Ala Val Lys Ser Leu Glu Thr Glu Ile Leu Glu Ser Leu Lys Ser Ala
                   820                     825                     830
   Gly Gln Glu Asn Leu Glu Thr Leu Lys Ser Pro Glu Thr Gln Ala Pro
               835                     840                     845
   Leu Trp Thr Pro Glu Glu Ile Asn Gln Gly Ala Met Asn Pro Leu Glu
       850                     855                     860
   Lys Glu Ile Gln Glu Pro Leu Glu Ser Val Glu Val Asn Gln Glu Thr
   865                     870                     875                     880
   Phe Arg Leu Leu Glu Glu Glu Asn Gln Glu Ser Leu Arg Ser Leu Gly
                       885                     890                     895
   Ala Trp Asn Leu Glu Asn Leu Arg Ser Pro Glu Glu Val Asp Lys Glu
                   900                     905                     910
   Ser Gln Arg Asn Leu Glu Glu Glu Glu Asn Leu Gly Lys Gly Glu Tyr
               915                     920                     925
   Gln Glu Ser Leu Arg Ser Leu Glu Glu Glu Gly Gln Glu Leu Pro Gln
       930                     935                     940
   Ser Ala Asp Val Gln Arg Trp Glu Asp Thr Val Glu Lys Asp Gln Glu
   945                     950                     955                     960
   Leu Ala Gln Glu Ser Pro Pro Gly Met Ala Gly Val Glu Asn Glu Asp
                       965                     970                     975
   Glu Ala Glu Leu Asn Leu Arg Glu Gln Asp Gly Phe Thr Gly Lys Glu
                   980                     985                     990
   Glu Val Val Glu Gln Gly Glu Leu Asn Ala Thr Glu Glu Val Trp Ile
               995                     1000                    1005
   Pro Gly Glu Gly His Pro Glu Ser Pro Glu Pro Lys Glu Gln Arg Gly
       1010                    1015                    1020
   Leu Val Glu Gly Ala Ser Val Lys Gly Gly Ala Glu Gly Leu Gln Asp
   1025                    1030                    1035                    1040
   Pro Glu Gly Gln Ser Gln Gln Val Gly Ala Pro Gly Leu Gln Ala Pro
                       1045                    1050                    1055
   Gln Gly Leu Pro Glu Ala Ile Glu Pro Leu Val Glu Asp Asp Val Ala
                   1060                    1065                    1070
   Pro Gly Gly Asp Gln Ala Ser Pro Glu Val Met Leu Gly Ser Glu Pro
               1075                    1080                    1085
   Ala Met Gly Glu Ser Ala Ala Gly Ala Glu Pro Gly Pro Gly Gln Gly
       1090                1095                1100
   Val Gly Gly Leu Gly Asp Pro Gly His Leu Thr Arg Glu Glu Val Met
   1105                1110                1115                1120
   Glu Pro Pro Leu Glu Glu Glu Ser Leu Glu Ala Lys Arg Val Gln Gly
                   1125                1130                1135
   Leu Glu Gly Pro Arg Lys Asp Leu Glu Glu Ala Gly Gly Leu Gly Thr
               1140                1145                1150
   Glu Phe Ser Glu Leu Pro Gly Lys Ser Arg Asp Pro Trp Glu Pro Pro
           1155                1160                1165
   Arg Glu Gly Arg Glu Glu Ser Glu Ala Glu Ala Pro Arg Gly Ala Glu
       1170                1175                1180
   Glu Ala Phe Pro Ala Glu Thr Leu Gly His Thr Gly Ser Asp Ala Pro
   1185                1190                1195                1200
   Ser Pro Trp Pro Leu Gly Ser Glu Glu Ala Glu Glu Asp Val Pro Pro
                   1205                1210                1215
   Val Leu Val Ser Pro Ser Pro Thr Tyr Thr Pro Ile Leu Glu Asp Ala
               1220                1225                1230
   Pro Gly Pro Gln Pro Gln Ala Glu Gly Ser Gln Glu Ala Ser Trp Gly
           1235                1240                1245
   Val Gln Gly Arg Ala Glu Ala Leu Gly Lys Val Glu Ser Glu Gln Glu
       1250                1255                1260
   Glu Leu Gly Ser Gly Glu Ile Pro Glu Gly Pro Gln Glu Glu Gly Glu
   1265                1270                1275                1280
   Glu Ser Arg Glu Glu Ser Glu Glu Asp Glu Leu Gly Glu Thr Leu Pro
                   1285                1290                1295
   Asp Ser Thr Pro Leu Gly Phe Tyr Leu Arg Ser Pro Thr Ser Pro Arg
               1300                1305                1310
   Trp Asp Pro Thr Gly Glu Gln Arg Pro Pro Pro Gln Gly Glu Thr Gly
           1315                1320                1325
   Lys Glu Gly Trp Asp Pro Ala Val Leu Ala Ser Glu Gly Leu Glu Ala
       1330                1335                1340
   Pro Pro Ser Glu Lys Glu Glu Gly Glu Glu Gly Glu Glu Glu Cys Gly
   1345                1350                1355                1360
   Arg Asp Ser Asp Leu Ser Glu Glu Phe Glu Asp Leu Gly Thr Glu Ala
                   1365                1370                1375
   Pro Phe Leu Pro Gly Val Pro Gly Glu Val Ala Glu Pro Leu Gly Gln
               1380                1385                1390
   Val Pro Gln Leu Leu Leu Asp Pro Ala Ala Trp Asp Arg Asp Gly Glu
           1395                1400                1405
   Ser Asp Gly Phe Ala Asp Glu Glu Glu Ser Gly Glu Glu Gly Glu Glu
       1410                1415                1420
   Asp Gln Glu Glu Gly Arg Glu Pro Gly Ala Gly Arg Trp Gly Pro Gly
   1425                1430                1435                1440
   Ser Ser Val Gly Ser Leu Gln Ala Leu Ser Ser Ser Gln Arg Gly Glu
                   1445                1450                1455
   Phe Leu Glu Ser Asp Ser Val Ser Val Ser Val Pro Trp Asp Asp Ser
               1460                1465                1470
   Leu Arg Gly Ala Val Ala Gly Ala Pro Lys Thr Ala Leu Glu Thr Glu
           1475                1480                1485
   Ser Gln Asp Ser Ala Glu Pro Ser Gly Ser Glu Glu Glu Ser Asp Pro
       1490                1495                1500
   Val Ser Leu Glu Arg Glu Asp Lys Val Pro Gly Pro Leu Glu Ile Pro
   1505                1510                1515                1520
   Ser Gly Met Glu Asp Ala Gly Pro Gly Ala Asp Ile Ile Gly Val Asn
                   1525                1530                1535
   Gly Gln Gly Pro Asn Leu Glu Gly Lys Ser Gln His Val Asn Gly Gly
               1540                1545                1550
   Val Met Asn Gly Leu Glu Gln Ser Glu Glu Val Gly Gln Gly Met Pro
           1555                1560                1565
   Leu Val Ser Glu Gly Asp Arg Gly Ser Pro Phe Gln Glu Glu Glu Gly
       1570                1575                1580
   Ser Ala Leu Lys Thr Ser Trp Ala Gly Ala Pro Val His Leu Gly Gln
   1585                1590                1595                1600
   Gly Gln Phe Leu Lys Phe Thr Gln Arg Glu Gly Asp Arg Glu Ser Trp
                   1605                1610                1615
   Ser Ser Gly Glu Asp
               1620
<210> 51
   <211> 5927
   <212> DNA
   <213> Homo sapiens
<220>
   <221> source
   <222> 1..5927
   <223> /mol_type="DNA"
   /note="Homo sapiens Col1A1"
   /organism="Homo sapiens"
<400> 51
   tcgtcggagc agacgggagt ttctcctcgg ggtcggagca ggaggcacgc ggagtgtgag     60
   gccacgcatg agcggacgct aaccccctcc ccagccacaa agagtctaca tgtctagggt    120
   ctagacatgt tcagctttgt ggacctccgg ctcctgctcc tcttagcggc caccgccctc    180
   ctgacgcacg gccaagagga aggccaagtc gagggccaag acgaagacat cccaccaatc    240
   acctgcgtac agaacggcct caggtaccat gaccgagacg tgtggaaacc cgagccctgc    300
   cggatctgcg tctgcgacaa cggcaaggtg ttgtgcgatg acgtgatctg tgacgagacc    360
   aagaactgcc ccggcgccga agtccccgag ggcgagtgct gtcccgtctg ccccgacggc    420
   tcagagtcac ccaccgacca agaaaccacc ggcgtcgagg gacccaaggg agacactggc    480
   ccccgaggcc caaggggacc cgcaggcccc cctggccgag atggcatccc tggacagcct    540
   ggacttcccg gaccccccgg accccccgga cctcccggac cccctggcct cggaggaaac    600
   tttgctcccc agctgtctta tggctatgat gagaaatcaa ccggaggaat ttccgtgcct    660
   ggccccatgg gtccctctgg tcctcgtggt ctccctggcc cccctggtgc acctggtccc    720
   caaggcttcc aaggtccccc tggtgagcct ggcgagcctg gagcttcagg tcccatgggt    780
   ccccgaggtc ccccaggtcc ccctggaaag aatggagatg atggggaagc tggaaaacct    840
   ggtcgtcctg gtgagcgtgg gcctcctggg cctcagggtg ctcgaggatt gcccggaaca    900
   gctggcctcc ctggaatgaa gggacacaga ggtttcagtg gtttggatgg tgccaaggga    960
   gatgctggtc ctgctggtcc taagggtgag cctggcagcc ctggtgaaaa tggagctcct   1020
   ggtcagatgg gcccccgtgg cctgcctggt gagagaggtc gccctggagc ccctggccct   1080
   gctggtgctc gtggaaatga tggtgctact ggtgctgccg ggccccctgg tcccaccggc   1140
   cccgctggtc ctcctggctt ccctggtgct gttggtgcta agggtgaagc tggtccccaa   1200
   gggccccgag gctctgaagg tccccagggt gtgcgtggtg agcctggccc ccctggccct   1260
   gctggtgctg ctggccctgc tggaaaccct ggtgctgatg gacagcctgg tgctaaaggt   1320
   gccaatggtg ctcctggtat tgctggtgct cctggcttcc ctggtgcccg aggcccctct 1380
   ggaccccagg gccccggcgg ccctcctggt cccaagggta acagcggtga acctggtgct 1440
   cctggcagca aaggagacac tggtgctaag ggagagcctg gccctgttgg tgttcaagga 1500
   ccccctggcc ctgctggaga ggaaggaaag cgaggagctc gaggtgaacc cggacccact 1560
   ggcctgcccg gaccccctgg cgagcgtggt ggacctggta gccgtggttt ccctggcgca 1620
   gatggtgttg ctggtcccaa gggtcccgct ggtgaacgtg gttctcctgg ccctgctggc 1680
   cccaaaggat ctcctggtga agctggtcgt cccggtgaag ctggtctgcc tggtgccaag 1740
   ggtctgactg gaagccctgg cagccctggt cctgatggca aaactggccc ccctggtccc 1800
   gccggtcaag atggtcgccc cggaccccca ggcccacctg gtgcccgtgg tcaggctggt 1860
   gtgatgggat tccctggacc taaaggtgct gctggagagc ccggcaaggc tggagagcga 1920
   ggtgttcccg gaccccctgg cgctgtcggt cctgctggca aagatggaga ggctggagct 1980
   cagggacccc ctggccctgc tggtcccgct ggcgagagag gtgaacaagg ccctgctggc 2040
   tcccccggat tccagggtct ccctggtcct gctggtcctc caggtgaagc aggcaaacct 2100
   ggtgaacagg gtgttcctgg agaccttggc gcccctggcc cctctggagc aagaggcgag 2160
   agaggtttcc ctggcgagcg tggtgtgcaa ggtccccctg gtcctgctgg tccccgaggg 2220
   gccaacggtg ctcccggcaa cgatggtgct aagggtgatg ctggtgcccc tggagctccc 2280
   ggtagccagg gcgcccctgg ccttcaggga atgcctggtg aacgtggtgc agctggtctt 2340
   ccagggccta agggtgacag aggtgatgct ggtcccaaag gtgctgatgg ctctcctggc 2400
   aaagatggcg tccgtggtct gactggcccc attggtcctc ctggccctgc tggtgcccct 2460
   ggtgacaagg gtgaaagtgg tcccagcggc cctgctggtc ccactggagc tcgtggtgcc 2520
   cccggagacc gtggtgagcc tggtcccccc ggccctgctg gctttgctgg cccccctggt 2580
   gctgacggcc aacctggtgc taaaggcgaa cctggtgatg ctggtgctaa aggcgatgct 2640
   ggtccccctg gccctgccgg acccgctgga ccccctggcc ccattggtaa tgttggtgct 2700
   cctggagcca aaggtgctcg cggcagcgct ggtccccctg gtgctactgg tttccctggt 2760
   gctgctggcc gagtcggtcc tcctggcccc tctggaaatg ctggaccccc tggccctcct 2820
   ggtcctgctg gcaaagaagg cggcaaaggt ccccgtggtg agactggccc tgctggacgt 2880
   cctggtgaag ttggtccccc tggtccccct ggccctgctg gcgagaaagg atcccctggt 2940
   gctgatggtc ctgctggtgc tcctggtact cccgggcctc aaggtattgc tggacagcgt 3000
   ggtgtggtcg gcctgcctgg tcagagagga gagagaggct tccctggtct tcctggcccc 3060
   tctggtgaac ctggcaaaca aggtccctct ggagcaagtg gtgaacgtgg tccccctggt 3120
   cccatgggcc cccctggatt ggctggaccc cctggtgaat ctggacgtga gggggctcct 3180
   ggtgccgaag gttcccctgg acgagacggt tctcctggcg ccaagggtga ccgtggtgag 3240
   accggccccg ctggaccccc tggtgctcct ggtgctcctg gtgcccctgg ccccgttggc 3300
   cctgctggca agagtggtga tcgtggtgag actggtcctg ctggtcccgc cggtcctgtc 3360
   ggccctgttg gcgcccgtgg ccccgccgga ccccaaggcc cccgtggtga caagggtgag 3420
   acaggcgaac agggcgacag aggcataaag ggtcaccgtg gcttctctgg cctccagggt 3480
   ccccctggcc ctcctggctc tcctggtgaa caaggtccct ctggagcctc tggtcctgct 3540
   ggtccccgag gtccccctgg ctctgctggt gctcctggca aagatggact caacggtctc 3600
   cctggcccca ttgggccccc tggtcctcgc ggtcgcactg gtgatgctgg tcctgttggt 3660
   ccccccggcc ctcctggacc tcctggtccc cctggtcctc ccagcgctgg tttcgacttc 3720
   agcttcctgc cccagccacc tcaagagaag gctcacgatg gtggccgcta ctaccgggct 3780
   gatgatgcca atgtggttcg tgaccgtgac ctcgaggtgg acaccaccct caagagcctg 3840
   agccagcaga tcgagaacat ccggagccca gagggcagcc gcaagaaccc cgcccgcacc 3900
   tgccgtgacc tcaagatgtg ccactctgac tggaagagtg gagagtactg gattgacccc 3960
   aaccaaggct gcaacctgga tgccatcaaa gtcttctgca acatggagac tggtgagacc 4020
   tgcgtgtacc ccactcagcc cagtgtggcc cagaagaact ggtacatcag caagaacccc 4080
   aaggacaaga ggcatgtctg gttcggcgag agcatgaccg atggattcca gttcgagtat 4140
   ggcggccagg gctccgaccc tgccgatgtg gccatccagc tgaccttcct gcgcctgatg 4200
   tccaccgagg cctcccagaa catcacctac cactgcaaga acagcgtggc ctacatggac 4260
   cagcagactg gcaacctcaa gaaggccctg ctcctccagg gctccaacga gatcgagatc 4320
   cgcgccgagg gcaacagccg cttcacctac agcgtcactg tcgatggctg cacgagtcac 4380
   accggagcct ggggcaagac agtgattgaa tacaaaacca ccaagacctc ccgcctgccc 4440
   atcatcgatg tggccccctt ggacgttggt gccccagacc aggaattcgg cttcgacgtt 4500
   ggccctgtct gcttcctgta aactccctcc atcccaacct ggctccctcc cacccaacca 4560
   actttccccc caacccggaa acagacaagc aacccaaact gaaccccctc aaaagccaaa 4620
   aaatgggaga caatttcaca tggactttgg aaaatatttt tttcctttgc attcatctct 4680
   caaacttagt ttttatcttt gaccaaccga acatgaccaa aaaccaaaag tgcattcaac 4740
   cttaccaaaa aaaaaaaaaa aaaaagaata aataaataac tttttaaaaa aggaagcttg 4800
   gtccacttgc ttgaagaccc atgcgggggt aagtcccttt ctgcccgttg ggcttatgaa 4860
   accccaatgc tgccctttct gctcctttct ccacaccccc cttggggcct cccctccact 4920
   ccttccccaa tctgtctccc cagaagacac aggaaacaat gtattgtctg cccagcaatc 4980
   aaaggcaatg ctcaaacacc caagtggccc ccaccctcag cccgctcctg cccgcccagc 5040
   acccccaggc cctgggggac ctggggttct cagactgcca aagaagcctt gccatctggc 5100
   gctcccatgg ctcttgcaac atctcccctt cgtttttgag ggggtcatgc cgggggagcc 5160
   accagcccct cactgggttc ggaggagagt caggaagggc cacgacaaag cagaaacatc 5220
   ggatttgggg aacgcgtgtc aatcccttgt gccgcagggc tgggcgggag agactgttct 5280
   gttccttgtg taactgtgtt gctgaaagac tacctcgttc ttgtcttgat gtgtcaccgg 5340
   ggcaactgcc tgggggcggg gatgggggca gggtggaagc ggctccccat tttataccaa 5400
   aggtgctaca tctatgtgat gggtggggtg gggagggaat cactggtgct atagaaattg 5460
   agatgccccc ccaggccagc aaatgttcct ttttgttcaa agtctatttt tattccttga 5520
   tatttttctt tttttttttt tttttttgtg gatggggact tgtgaatttt tctaaaggtg 5580
   ctatttaaca tgggaggaga gcgtgtgcgg ctccagccca gcccgctgct cactttccac 5640
   cctctctcca cctgcctctg gcttctcagg cctctgctct ccgacctctc tcctctgaaa 5700
   ccctcctcca cagctgcagc ccatcctccc ggctccctcc tagtctgtcc tgcgtcctct 5760
   gtccccgggt ttcagagaca acttcccaaa gcacaaagca gtttttcccc ctaggggtgg 5820
   gaggaagcaa aagactctgt acctattttg tatgtgtata ataatttgag atgtttttaa 5880
   ttattttgat tgctggaata aagcatgtgg aaatgaccca aacataa 5927
<210> 52
   <211> 1464
   <212> PRT
   <213> Homo sapiens
<220>
   <221> SOURCE
   <222> 1..1464
   <223> /mol_type="protein"
   /note="Homo sapiens Col1A1"
   /organism="Homo sapiens"
<400> 52
<210> 53
   <211> 5490
   <212> DNA
   <213> Homo sapiens
<220>
   <221> source
   <222> 1..5490
   <223> /mol_type="DNA"
   /note="Homo sapiens Col3A1"
   /organism="Homo sapiens"
<400> 53
   ggctgagttt tatgacgggc ccggtgctga agggcaggga acaacttgat ggtgctactt 60
   tgaactgctt ttcttttctc ctttttgcac aaagagtctc atgtctgata tttagacatg 120
   atgagctttg tgcaaaaggg gagctggcta cttctcgctc tgcttcatcc cactattatt 180
   ttggcacaac aggaagctgt tgaaggagga tgttcccatc ttggtcagtc ctatgcggat 240
   agagatgtct ggaagccaga accatgccaa atatgtgtct gtgactcagg atccgttctc 300
   tgcgatgaca taatatgtga cgatcaagaa ttagactgcc ccaacccaga aattccattt 360
   ggagaatgtt gtgcagtttg cccacagcct ccaactgctc ctactcgccc tcctaatggt 420
   caaggacctc aaggccccaa gggagatcca ggccctcctg gtattcctgg gagaaatggt 480
   gaccctggta ttccaggaca accagggtcc cctggttctc ctggcccccc tggaatctgt 540
   gaatcatgcc ctactggtcc tcagaactat tctccccagt atgattcata tgatgtcaag 600
   tctggagtag cagtaggagg actcgcaggc tatcctggac cagctggccc cccaggccct 660
   cccggtcccc ctggtacatc tggtcatcct ggttcccctg gatctccagg ataccaagga 720
   ccccctggtg aacctgggca agctggtcct tcaggccctc caggacctcc tggtgctata 780
   ggtccatctg gtcctgctgg aaaagatgga gaatcaggta gacccggacg acctggagag 840
   cgaggattgc ctggacctcc aggtatcaaa ggtccagctg ggatacctgg attccctggt 900
   atgaaaggac acagaggctt cgatggacga aatggagaaa agggtgaaac aggtgctcct 960
   ggattaaagg gtgaaaatgg tcttccaggc gaaaatggag ctcctggacc catgggtcca 1020
   agaggggctc ctggtgagcg aggacggcca ggacttcctg gggctgcagg tgctcggggt 1080
   aatgacggtg ctcgaggcag tgatggtcaa ccaggccctc ctggtcctcc tggaactgcc 1140
   ggattccctg gatcccctgg tgctaagggt gaagttggac ctgcagggtc tcctggttca 1200
   aatggtgccc ctggacaaag aggagaacct ggacctcagg gacacgctgg tgctcaaggt 1260
   cctcctggcc ctcctgggat taatggtagt cctggtggta aaggcgaaat gggtcccgct 1320
   ggcattcctg gagctcctgg actgatggga gcccggggtc ctccaggacc agccggtgct 1380
   aatggtgctc ctggactgcg aggtggtgca ggtgagcctg gtaagaatgg tgccaaagga 1440
   gagcccggac cacgtggtga acgcggtgag gctggtattc caggtgttcc aggagctaaa 1500
   ggcgaagatg gcaaggatgg atcacctgga gaacctggtg caaatgggct tccaggagct 1560
   gcaggagaaa ggggtgcccc tgggttccga ggacctgctg gaccaaatgg catcccagga 1620
   gaaaagggtc ctgctggaga gcgtggtgct ccaggccctg cagggcccag aggagctgct 1680
   ggagaacctg gcagagatgg cgtccctgga ggtccaggaa tgaggggcat gcccggaagt 1740
   ccaggaggac caggaagtga tgggaaacca gggcctcccg gaagtcaagg agaaagtggt 1800
   cgaccaggtc ctcctgggcc atctggtccc cgaggtcagc ctggtgtcat gggcttcccc 1860
   ggtcctaaag gaaatgatgg tgctcctggt aagaatggag aacgaggtgg ccctggagga 1920
   cctggccctc agggtcctcc tggaaagaat ggtgaaactg gacctcaggg acccccaggg 1980
   cctactgggc ctggtggtga caaaggagac acaggacccc ctggtccaca aggattacaa 2040
   ggcttgcctg gtacaggtgg tcctccagga gaaaatggaa aacctgggga accaggtcca 2100
   aagggtgatg ccggtgcacc tggagctcca ggaggcaagg gtgatgctgg tgcccctggt 2160
   gaacgtggac ctcctggatt ggcaggggcc ccaggactta gaggtggagc tggtccccct 2220
   ggtcccgaag gaggaaaggg tgctgctggt cctcctgggc cacctggtgc tgctggtact 2280
   cctggtctgc aaggaatgcc tggagaaaga ggaggtcttg gaagtcctgg tccaaagggt     2340
   gacaagggtg aaccaggcgg tccaggtgct gatggtgtcc cagggaaaga tggcccaagg     2400
   ggtcctactg gtcctattgg tcctcctggc ccagctggcc agcctggaga taagggtgaa     2460
   ggtggtgccc ccggacttcc aggtatagct ggacctcgtg gtagccctgg tgagagaggt     2520
   gaaactggcc ctccaggacc tgctggtttc cctggtgctc ctggacagaa tggtgaacct     2580
   ggtggtaaag gagaaagagg ggctccgggt gagaaaggtg aaggaggccc tcctggagtt     2640
   gcaggacccc ctggaggttc tggacctgct ggtcctcctg gtccccaagg tgtcaaaggt     2700
   gaacgtggca gtcctggtgg acctggtgct gctggcttcc ctggtgctcg tggtcttcct     2760
   ggtcctcctg gtagtaatgg taacccagga cccccaggtc ccagcggttc tccaggcaag     2820
   gatgggcccc caggtcctgc gggtaacact ggtgctcctg gcagccctgg agtgtctgga     2880
   ccaaaaggtg atgctggcca accaggagag aagggatcgc ctggtgccca gggcccacca     2940
   ggagctccag gcccacttgg gattgctggg atcactggag cacggggtct tgcaggacca     3000
   ccaggcatgc caggtcctag gggaagccct ggccctcagg gtgtcaaggg tgaaagtggg     3060
   aaaccaggag ctaacggtct cagtggagaa cgtggtcccc ctggacccca gggtcttcct     3120
   ggtctggctg gtacagctgg tgaacctgga agagatggaa accctggatc agatggtctt     3180
   ccaggccgag atggatctcc tggtggcaag ggtgatcgtg gtgaaaatgg ctctcctggt     3240
   gcccctggcg ctcctggtca tccaggccca cctggtcctg tcggtccagc tggaaagagt     3300
   ggtgacagag gagaaagtgg ccctgctggc cctgctggtg ctcccggtcc tgctggttcc     3360
   cgaggtgctc ctggtcctca aggcccacgt ggtgacaaag gtgaaacagg tgaacgtgga     3420
   gctgctggca tcaaaggaca tcgaggattc cctggtaatc caggtgcccc aggttctcca     3480
   ggccctgctg gtcagcaggg tgcaatcggc agtccaggac ctgcaggccc cagaggacct     3540
   gttggaccca gtggacctcc tggcaaagat ggaaccagtg gacatccagg tcccattgga     3600
   ccaccagggc ctcgaggtaa cagaggtgaa agaggatctg agggctcccc aggccaccca     3660
   gggcaaccag gccctcctgg acctcctggt gcccctggtc cttgctgtgg tggtgttgga     3720
   gccgctgcca ttgctgggat tggaggtgaa aaagctggcg gttttgcccc gtattatgga     3780
   gatgaaccaa tggatttcaa aatcaacacc gatgagatta tgacttcact caagtctgtt     3840
   aatggacaaa tagaaagcct cattagtcct gatggttctc gtaaaaaccc cgctagaaac     3900
   tgcagagacc tgaaattctg ccatcctgaa ctcaagagtg gagaatactg ggttgaccct     3960
   aaccaaggat gcaaattgga tgctatcaag gtattctgta atatggaaac tggggaaaca     4020
   tgcataagtg ccaatccttt gaatgttcca cggaaacact ggtggacaga ttctagtgct     4080
   gagaagaaac acgtttggtt tggagagtcc atggatggtg gttttcagtt tagctacggc     4140
   aatcctgaac ttcctgaaga tgtccttgat gtgcagctgg cattccttcg acttctctcc     4200
   agccgagctt cccagaacat cacatatcac tgcaaaaata gcattgcata catggatcag     4260
   gccagtggaa atgtaaagaa ggccctgaag ctgatggggt caaatgaagg tgaattcaag     4320
   gctgaaggaa atagcaaatt cacctacaca gttctggagg atggttgcac gaaacacact     4380
   ggggaatgga gcaaaacagt ctttgaatat cgaacacgca aggctgtgag actacctatt     4440
   gtagatattg caccctatga cattggtggt cctgatcaag aatttggtgt ggacgttggc     4500
   cctgtttgct ttttataaac caaactctat ctgaaatccc aacaaaaaaa atttaactcc     4560
   atatgtgttc ctcttgttct aatcttgtca accagtgcaa gtgaccgaca aaattccagt     4620
   tatttatttc caaaatgttt ggaaacagta taatttgaca aagaaaaatg atacttctct     4680
   ttttttgctg ttccaccaaa tacaattcaa atgctttttg ttttattttt ttaccaattc     4740
   caatttcaaa atgtctcaat ggtgctataa taaataaact tcaacactct ttatgataac     4800
   aacactgtgt tatattcttt gaatcctagc ccatctgcag agcaatgact gtgctcacca     4860
   gtaaaagata acctttcttt ctgaaatagt caaatacgaa attagaaaag ccctccctat     4920
   tttaactacc tcaactggtc agaaacacag attgtattct atgagtccca gaagatgaaa     4980
   aaaattttat acgttgataa aacttataaa tttcattgat taatctcctg gaagattggt     5040
   ttaaaaagaa aagtgtaatg caagaattta aagaaatatt tttaaagcca caattatttt     5100
   aatattggat atcaactgct tgtaaaggtg ctcctctttt ttcttgtcat tgctggtcaa     5160
   gattactaat atttgggaag gctttaaaga cgcatgttat ggtgctaatg tactttcact     5220
   tttaaactct agatcagaat tgttgacttg cattcagaac ataaatgcac aaaatctgta     5280
   catgtctccc atcagaaaga ttcattggca tgccacaggg gattctcctc cttcatcctg     5340
   taaaggtcaa caataaaaac caaattatgg ggctgctttt gtcacactag catagagaat     5400
   gtgttgaaat ttaactttgt aagcttgtat gtggttgttg atcttttttt tccttacaga     5460
   cacccataat aaaatatcat attaaaattc                                     5490
<210> 54
   <211> 1466
   <212> PRT
   <213> Homo sapiens
<220>
   <221> SOURCE
   <222> 1..1466
   <223> /mol_type="protein"
   /note="Homo sapiens Col3A1"
   /organism="Homo sapiens"
<400> 54
   Met Met Ser Phe Val Gln Lys Gly Ser Trp Leu Leu Leu Ala Leu Leu
   1               5                   10                  15
   His Pro Thr Ile Ile Leu Ala Gln Gln Glu Ala Val Glu Gly Gly Cys
                   20                  25                  30
   Ser His Leu Gly Gln Ser Tyr Ala Asp Arg Asp Val Trp Lys Pro Glu
           35                  40                  45
   Pro Cys Gln Ile Cys Val Cys Asp Ser Gly Ser Val Leu Cys Asp Asp
       50                  55                  60
   Ile Ile Cys Asp Asp Gln Glu Leu Asp Cys Pro Asn Pro Glu Ile Pro
   65                  70                  75                  80
   Phe Gly Glu Cys Cys Ala Val Cys Pro Gln Pro Pro Thr Ala Pro Thr
                   85                  90                  95
   Arg Pro Pro Asn Gly Gln Gly Pro Gln Gly Pro Lys Gly Asp Pro Gly
               100                 105                 110
   Pro Pro Gly Ile Pro Gly Arg Asn Gly Asp Pro Gly Ile Pro Gly Gln
           115                 120                 125
   Pro Gly Ser Pro Gly Ser Pro Gly Pro Pro Gly Ile Cys Glu Ser Cys
       130                 135                 140
   Pro Thr Gly Pro Gln Asn Tyr Ser Pro Gln Tyr Asp Ser Tyr Asp Val
   145                 150                 155                 160
   Lys Ser Gly Val Ala Val Gly Gly Leu Ala Gly Tyr Pro Gly Pro Ala
                   165                 170                 175
   Gly Pro Pro Gly Pro Pro Gly Pro Pro Gly Thr Ser Gly His Pro Gly
               180                 185                 190
   Ser Pro Gly Ser Pro Gly Tyr Gln Gly Pro Pro Gly Glu Pro Gly Gln
           195                 200                 205
   Ala Gly Pro Ser Gly Pro Pro Gly Pro Pro Gly Ala Ile Gly Pro Ser
       210                 215                 220
   Gly Pro Ala Gly Lys Asp Gly Glu Ser Gly Arg Pro Gly Arg Pro Gly
   225                 230                 235                 240
   Glu Arg Gly Leu Pro Gly Pro Pro Gly Ile Lys Gly Pro Ala Gly Ile
                   245                 250                 255
   Pro Gly Phe Pro Gly Met Lys Gly His Arg Gly Phe Asp Gly Arg Asn
               260                 265                 270
   Gly Glu Lys Gly Glu Thr Gly Ala Pro Gly Leu Lys Gly Glu Asn Gly
           275                 280                 285
   Leu Pro Gly Glu Asn Gly Ala Pro Gly Pro Met Gly Pro Arg Gly Ala
       290                 295                 300
   Pro Gly Glu Arg Gly Arg Pro Gly Leu Pro Gly Ala Ala Gly Ala Arg
   305                 310                 315                 320
   Gly Asn Asp Gly Ala Arg Gly Ser Asp Gly Gln Pro Gly Pro Pro Gly
                   325                 330                 335
   Pro Pro Gly Thr Ala Gly Phe Pro Gly Ser Pro Gly Ala Lys Gly Glu
               340                 345                 350
   Val Gly Pro Ala Gly Ser Pro Gly Ser Asn Gly Ala Pro Gly Gln Arg
           355                 360                 365
   Gly Glu Pro Gly Pro Gln Gly His Ala Gly Ala Gln Gly Pro Pro Gly
       370                 375                 380
   Pro Pro Gly Ile Asn Gly Ser Pro Gly Gly Lys Gly Glu Met Gly Pro
   385                 390                 395                 400
   Ala Gly Ile Pro Gly Ala Pro Gly Leu Met Gly Ala Arg Gly Pro Pro
                   405                 410                 415
   Gly Pro Ala Gly Ala Asn Gly Ala Pro Gly Leu Arg Gly Gly Ala Gly
               420                 425                 430
   Glu Pro Gly Lys Asn Gly Ala Lys Gly Glu Pro Gly Pro Arg Gly Glu
           435                 440                 445
   Arg Gly Glu Ala Gly Ile Pro Gly Val Pro Gly Ala Lys Gly Glu Asp
       450                 455                 460
   Gly Lys Asp Gly Ser Pro Gly Glu Pro Gly Ala Asn Gly Leu Pro Gly
   465                 470                 475                 480
   Ala Ala Gly Glu Arg Gly Ala Pro Gly Phe Arg Gly Pro Ala Gly Pro
                   485                 490                 495
   Asn Gly Ile Pro Gly Glu Lys Gly Pro Ala Gly Glu Arg Gly Ala Pro
               500                 505                 510
   Gly Pro Ala Gly Pro Arg Gly Ala Ala Gly Glu Pro Gly Arg Asp Gly
           515                 520                 525
   Val Pro Gly Gly Pro Gly Met Arg Gly Met Pro Gly Ser Pro Gly Gly
       530                 535                 540
   Pro Gly Ser Asp Gly Lys Pro Gly Pro Pro Gly Ser Gln Gly Glu Ser
   545                 550                 555                 560
   Gly Arg Pro Gly Pro Pro Gly Pro Ser Gly Pro Arg Gly Gln Pro Gly
                   565                 570                 575
   Val Met Gly Phe Pro Gly Pro Lys Gly Asn Asp Gly Ala Pro Gly Lys
               580                 585                 590
   Asn Gly Glu Arg Gly Gly Pro Gly Gly Pro Gly Pro Gln Gly Pro Pro
           595                 600                 605
   Gly Lys Asn Gly Glu Thr Gly Pro Gln Gly Pro Pro Gly Pro Thr Gly
       610                 615                 620
   Pro Gly Gly Asp Lys Gly Asp Thr Gly Pro Pro Gly Pro Gln Gly Leu
   625                 630                 635                 640
   Gln Gly Leu Pro Gly Thr Gly Gly Pro Pro Gly Glu Asn Gly Lys Pro
                   645                 650                 655
   Gly Glu Pro Gly Pro Lys Gly Asp Ala Gly Ala Pro Gly Ala Pro Gly
               660                 665                 670
   Gly Lys Gly Asp Ala Gly Ala Pro Gly Glu Arg Gly Pro Pro Gly Leu
           675                 680                 685
   Ala Gly Ala Pro Gly Leu Arg Gly Gly Ala Gly Pro Pro Gly Pro Glu
       690                 695                 700
   Gly Gly Lys Gly Ala Ala Gly Pro Pro Gly Pro Pro Gly Ala Ala Gly
   705                 710                 715                 720
   Thr Pro Gly Leu Gln Gly Met Pro Gly Glu Arg Gly Gly Leu Gly Ser
                   725                 730                 735
   Pro Gly Pro Lys Gly Asp Lys Gly Glu Pro Gly Gly Pro Gly Ala Asp
               740                 745                 750
   Gly Val Pro Gly Lys Asp Gly Pro Arg Gly Pro Thr Gly Pro Ile Gly
           755                 760                 765
   Pro Pro Gly Pro Ala Gly Gln Pro Gly Asp Lys Gly Glu Gly Gly Ala
       770                 775                 780
   Pro Gly Leu Pro Gly Ile Ala Gly Pro Arg Gly Ser Pro Gly Glu Arg
   785                 790                 795                 800
   Gly Glu Thr Gly Pro Pro Gly Pro Ala Gly Phe Pro Gly Ala Pro Gly
                   805                 810                 815
   Gln Asn Gly Glu Pro Gly Gly Lys Gly Glu Arg Gly Ala Pro Gly Glu
               820                 825                 830
   Lys Gly Glu Gly Gly Pro Pro Gly Val Ala Gly Pro Pro Gly Gly Ser
           835                 840                 845
   Gly Pro Ala Gly Pro Pro Gly Pro Gln Gly Val Lys Gly Glu Arg Gly
       850                 855                 860
   Ser Pro Gly Gly Pro Gly Ala Ala Gly Phe Pro Gly Ala Arg Gly Leu
   865                 870                 875                 880
   Pro Gly Pro Pro Gly Ser Asn Gly Asn Pro Gly Pro Pro Gly Pro Ser
                   885                 890                 895
   Gly Ser Pro Gly Lys Asp Gly Pro Pro Gly Pro Ala Gly Asn Thr Gly
               900                 905                 910
   Ala Pro Gly Ser Pro Gly Val Ser Gly Pro Lys Gly Asp Ala Gly Gln
           915                 920                 925
   Pro Gly Glu Lys Gly Ser Pro Gly Ala Gln Gly Pro Pro Gly Ala Pro
       930                 935                 940
   Gly Pro Leu Gly Ile Ala Gly Ile Thr Gly Ala Arg Gly Leu Ala Gly
   945                 950                 955                 960
   Pro Pro Gly Met Pro Gly Pro Arg Gly Ser Pro Gly Pro Gln Gly Val
                   965                 970                 975
   Lys Gly Glu Ser Gly Lys Pro Gly Ala Asn Gly Leu Ser Gly Glu Arg
               980                 985                 990
   Gly Pro Pro Gly Pro Gln Gly Leu Pro Gly Leu Ala Gly Thr Ala Gly
           995                 1000                1005
   Glu Pro Gly Arg Asp Gly Asn Pro Gly Ser Asp Gly Leu Pro Gly Arg
       1010                1015                1020
   Asp Gly Ser Pro Gly Gly Lys Gly Asp Arg Gly Glu Asn Gly Ser Pro
   1025                1030                1035                1040
   Gly Ala Pro Gly Ala Pro Gly His Pro Gly Pro Pro Gly Pro Val Gly
                   1045                1050                1055
   Pro Ala Gly Lys Ser Gly Asp Arg Gly Glu Ser Gly Pro Ala Gly Pro
               1060                1065                1070
   Ala Gly Ala Pro Gly Pro Ala Gly Ser Arg Gly Ala Pro Gly Pro Gln
           1075                1080                1085
   Gly Pro Arg Gly Asp Lys Gly Glu Thr Gly Glu Arg Gly Ala Ala Gly
       1090                1095                1100
   Ile Lys Gly His Arg Gly Phe Pro Gly Asn Pro Gly Ala Pro Gly Ser
   1105                1110                1115                1120
   Pro Gly Pro Ala Gly Gln Gln Gly Ala Ile Gly Ser Pro Gly Pro Ala
                   1125                1130                1135
   Gly Pro Arg Gly Pro Val Gly Pro Ser Gly Pro Pro Gly Lys Asp Gly
               1140                1145                1150
   Thr Ser Gly His Pro Gly Pro Ile Gly Pro Pro Gly Pro Arg Gly Asn
           1155                1160                1165
   Arg Gly Glu Arg Gly Ser Glu Gly Ser Pro Gly His Pro Gly Gln Pro
       1170                1175                1180
   Gly Pro Pro Gly Pro Pro Gly Ala Pro Gly Pro Cys Cys Gly Gly Val
   1185                1190                1195                1200
   Gly Ala Ala Ala Ile Ala Gly Ile Gly Gly Glu Lys Ala Gly Gly Phe
                   1205                1210                1215
   Ala Pro Tyr Tyr Gly Asp Glu Pro Met Asp Phe Lys Ile Asn Thr Asp
               1220                1225                1230
   Glu Ile Met Thr Ser Leu Lys Ser Val Asn Gly Gln Ile Glu Ser Leu
           1235                1240                1245
   Ile Ser Pro Asp Gly Ser Arg Lys Asn Pro Ala Arg Asn Cys Arg Asp
       1250                1255                1260
   Leu Lys Phe Cys His Pro Glu Leu Lys Ser Gly Glu Tyr Trp Val Asp
   1265                1270                1275                1280
   Pro Asn Gln Gly Cys Lys Leu Asp Ala Ile Lys Val Phe Cys Asn Met
                   1285                1290                1295
   Glu Thr Gly Glu Thr Cys Ile Ser Ala Asn Pro Leu Asn Val Pro Arg
               1300                1305                1310
   Lys His Trp Trp Thr Asp Ser Ser Ala Glu Lys Lys His Val Trp Phe
           1315                1320                1325
   Gly Glu Ser Met Asp Gly Gly Phe Gln Phe Ser Tyr Gly Asn Pro Glu
       1330                1335                1340
   Leu Pro Glu Asp Val Leu Asp Val Gln Leu Ala Phe Leu Arg Leu Leu
   1345                1350                1355                1360
   Ser Ser Arg Ala Ser Gln Asn Ile Thr Tyr His Cys Lys Asn Ser Ile
                   1365                1370                1375
   Ala Tyr Met Asp Gln Ala Ser Gly Asn Val Lys Lys Ala Leu Lys Leu
               1380                1385                1390
   Met Gly Ser Asn Glu Gly Glu Phe Lys Ala Glu Gly Asn Ser Lys Phe
           1395                1400                1405
   Thr Tyr Thr Val Leu Glu Asp Gly Cys Thr Lys His Thr Gly Glu Trp
       1410                1415                1420
   Ser Lys Thr Val Phe Glu Tyr Arg Thr Arg Lys Ala Val Arg Leu Pro
   1425                1430                1435                1440
   Ile Val Asp Ile Ala Pro Tyr Asp Ile Gly Gly Pro Asp Gln Glu Phe
                   1445                1450                1455
   Gly Val Asp Val Gly Pro Val Cys Phe Leu
               1460                1465
<210> 55
   <211> 1411
   <212> DNA
   <213> Homo sapiens
<220>
   <221> source
   <222> 1..1411
   <223> /mol_type="DNA"
   /note="Homo sapiens Oct4/POU5F1"
   /organism="Homo sapiens"
<400> 55
   ccttcgcaag ccctcatttc accaggcccc cggcttgggg cgccttcctt ccccatggcg      60
   ggacacctgg cttcggattt cgccttctcg ccccctccag gtggtggagg tgatgggcca     120
   ggggggccgg agccgggctg ggttgatcct cggacctggc taagcttcca aggccctcct     180
   ggagggccag gaatcgggcc gggggttggg ccaggctctg aggtgtgggg gattccccca     240
   tgccccccgc cgtatgagtt ctgtgggggg atggcgtact gtgggcccca ggttggagtg     300
   gggctagtgc cccaaggcgg cttggagacc tctcagcctg agggcgaagc aggagtcggg     360
   gtggagagca actccgatgg ggcctccccg gagccctgca ccgtcacccc tggtgccgtg     420
   aagctggaga aggagaagct ggagcaaaac ccggaggagt cccaggacat caaagctctg     480
   cagaaagaac tcgagcaatt tgccaagctc ctgaagcaga agaggatcac cctgggatat     540
   acacaggccg atgtggggct caccctgggg gttctatttg ggaaggtatt cagccaaacg     600
   accatctgcc gctttgaggc tctgcagctt agcttcaaga acatgtgtaa gctgcggccc     660
   ttgctgcaga agtgggtgga ggaagctgac aacaatgaaa atcttcagga gatatgcaaa     720
   gcagaaaccc tcgtgcaggc ccgaaagaga aagcgaacca gtatcgagaa ccgagtgaga     780
   ggcaacctgg agaatttgtt cctgcagtgc ccgaaaccca cactgcagca gatcagccac     840
   atcgcccagc agcttgggct cgagaaggat gtggtccgag tgtggttctg taaccggcgc     900
   cagaagggca agcgatcaag cagcgactat gcacaacgag aggattttga ggctgctggg     960
   tctcctttct cagggggacc agtgtccttt cctctggccc cagggcccca ttttggtacc   1020
   ccaggctatg ggagccctca cttcactgca ctgtactcct cggtcccttt ccctgagggg    1080
   gaagcctttc cccctgtctc cgtcaccact ctgggctctc ccatgcattc aaactgaggt    1140
   gcctgccctt ctaggaatgg gggacagggg gaggggagga gctagggaaa gaaaacctgg    1200
   agtttgtgcc agggtttttg ggattaagtt cttcattcac taaggaagga attgggaaca    1260
   caaagggtgg gggcagggga gtttggggca actggttgga gggaaggtga agttcaatga    1320
   tgctcttgat tttaatccca catcatgtat cacttttttc ttaaataaag aagcctggga    1380
   cacagtagat agacacactt aaaaaaaaaa a                                   1411
<210> 56
   <211> 360
   <212> PRT
   <213> Homo sapiens
<220>
   <221> SOURCE
   <222> 1..360
   <223> /mol_type="protein"
   /note="Homo sapiens Oct4/POU5F1"
   /organism="Homo sapiens"
<400> 56
   Met Ala Gly His Leu Ala Ser Asp Phe Ala Phe Ser Pro Pro Pro Gly
   1               5                   10                  15
   Gly Gly Gly Asp Gly Pro Gly Gly Pro Glu Pro Gly Trp Val Asp Pro
               20                  25                  30
   Arg Thr Trp Leu Ser Phe Gln Gly Pro Pro Gly Gly Pro Gly Ile Gly
           35                  40                  45
   Pro Gly Val Gly Pro Gly Ser Glu Val Trp Gly Ile Pro Pro Cys Pro
       50                  55                  60
   Pro Pro Tyr Glu Phe Cys Gly Gly Met Ala Tyr Cys Gly Pro Gln Val
   65                  70                  75                  80
   Gly Val Gly Leu Val Pro Gln Gly Gly Leu Glu Thr Ser Gln Pro Glu
                   85                  90                  95
   Gly Glu Ala Gly Val Gly Val Glu Ser Asn Ser Asp Gly Ala Ser Pro
               100                 105                 110
   Glu Pro Cys Thr Val Thr Pro Gly Ala Val Lys Leu Glu Lys Glu Lys
           115                 120                 125
   Leu Glu Gln Asn Pro Glu Glu Ser Gln Asp Ile Lys Ala Leu Gln Lys
       130                 135                 140
   Glu Leu Glu Gln Phe Ala Lys Leu Leu Lys Gln Lys Arg Ile Thr Leu
   145                 150                 155                 160
   Gly Tyr Thr Gln Ala Asp Val Gly Leu Thr Leu Gly Val Leu Phe Gly
                   165                 170                 175
   Lys Val Phe Ser Gln Thr Thr Ile Cys Arg Phe Glu Ala Leu Gln Leu
               180                 185                 190
   Ser Phe Lys Asn Met Cys Lys Leu Arg Pro Leu Leu Gln Lys Trp Val
           195                 200                 205
   Glu Glu Ala Asp Asn Asn Glu Asn Leu Gln Glu Ile Cys Lys Ala Glu
       210                 215                 220
   Thr Leu Val Gln Ala Arg Lys Arg Lys Arg Thr Ser Ile Glu Asn Arg
   225                 230                 235                 240
   Val Arg Gly Asn Leu Glu Asn Leu Phe Leu Gln Cys Pro Lys Pro Thr
                   245                 250                 255
   Leu Gln Gln Ile Ser His Ile Ala Gln Gln Leu Gly Leu Glu Lys Asp
               260                 265                 270
   Val Val Arg Val Trp Phe Cys Asn Arg Arg Gln Lys Gly Lys Arg Ser
           275                 280                 285
   Ser Ser Asp Tyr Ala Gln Arg Glu Asp Phe Glu Ala Ala Gly Ser Pro
       290                 295                 300
   Phe Ser Gly Gly Pro Val Ser Phe Pro Leu Ala Pro Gly Pro His Phe
   305                 310                 315                 320
   Gly Thr Pro Gly Tyr Gly Ser Pro His Phe Thr Ala Leu Tyr Ser Ser
                   325                 330                 335
   Val Pro Phe Pro Glu Gly Glu Ala Phe Pro Pro Val Ser Val Thr Thr
               340                 345                 350
   Leu Gly Ser Pro Met His Ser Asn
           355                 360
<210> 57
   <211> 3897
   <212> DNA
   <213> Homo sapiens
<220>
   <221> source
   <222> 1..3897
   <223> /mol_type="DNA"
   /note="Homo sapiens Gale"
   /organism="Homo sapiens"
<400> 57
   actcaaaatg gcggcggcgg cgtcagcatc agcggcctcc tgcccgtatc tatcgtggcg 60
   gcgacgggac ccgcctccct gggcgccgga gtcatgtgac ccacacaatg gctgagtggc 120
   tactctcggc ttcctggcaa cgccgagcga aagctatgac tgcggccgcg ggttcggcgg 180
   gccgcgccgc ggtgcccttg ctgctgtgtg cgctgctggc gcccggcggc gcgtacgtgc 240
   tcgacgactc cgacgggctg ggccgggagt tcgacggcat cggcgcggtc agcggcggcg 300
   gggcaacctc ccgacttcta gtaaattacc cagagcccta tcgttctcag atattggatt 360
   atctctttaa gccgaatttt ggtgcctctt tgcatatttt aaaagtggaa ataggtggtg 420
   atgggcagac aacagacggc actgagccct cccacatgca ttatgcacta gatgagaatt 480
   atttccgagg atacgagtgg tggttgatga aagaagctaa gaagaggaat cccaatatta 540
   cactcattgg gttgccatgg tcattccctg gatggctggg aaaaggtttc gactggcctt 600
   atgtcaatct tcagctgact gcctattatg tcgtgacctg gattgtgggc gccaagcgtt 660
   accatgattt ggacattgat tatattggaa tttggaatga gaggtcatat aatgccaatt 720
   atattaagat attaagaaaa atgctgaatt atcaaggtct ccagcgagtg aaaatcatag 780
   caagtgataa tctctgggag tccatctctg catccatgct ccttgatgcc gaactcttca 840
   aggtggttga tgttataggg gctcattatc ctggaaccca ttcagcaaaa gatgcaaagt 900
   tgactgggaa gaagctttgg tcttctgaag actttagcac tttaaatagt gacatgggtg 960
   caggctgctg ggtcgcattt ttaaatcaga attatatcaa tggctatatg acttccacaa 1020
   tcgcatggaa tttagtggct agttactatg aacagttgcc ttatgggaga tgcgggttga 1080
   tgacggccca ggagccatgg agtgggcact acgtggtaga atctcctgtc tgggtatcag 1140
   ctcataccac tcagtttact caacctggct ggtattacct gaagacagtt ggccatttag 1200
   agaaaggagg aagctacgta gctctgactg atggcttagg gaacctcacc atcatcattg 1260
   aaaccatgag tcataaacat tctaagtgca tacggccatt tcttccttat ttcaatgtgt 1320
   cacaacaatt tgccaccttt gttcttaagg gatcttttag tgaaatacca gagctacagg 1380
   tatggtatac caaacttgga aaaacatccg aaagatttct ttttaagcag ctggattctc 1440
   tatggctcct tgacagcgat ggcagtttca cactgagcct gcatgaagat gagctgttca 1500
   cactcaccac tctcaccact ggtcgcaaag gcagctaccc gcttcctcca aaatcccagc 1560
   ccttcccaag tacctataag gatgatttca atgttgatta cccatttttt agtgaagctc 1620
   caaactttgc tgatcaaact ggtgtatttg aatattttac aaatattgaa gaccctggcg 1680
   agcatcactt cacgctacgc caagttctca accagagacc cattacgtgg gctgccgatg 1740
   catccaacac aatcagtatt ataggagact acaactggac caatctgact ataaagtgtg 1800
   atgtatacat agagacccct gacacaggag gtgtgttcat tgcaggaaga gtaaataaag 1860
   gtggtatttt gattagaagt gccagaggaa ttttcttctg gatttttgca aatggatctt 1920
   acagggttac aggtgattta gctggatgga ttatatatgc tttaggacgt gttgaagtta 1980
   cagcaaaaaa atggtataca ctcacgttaa ctattaaggg tcatttcacc tctggcatgc 2040
   tgaatgacaa gtctctgtgg acagacatcc ctgtgaattt tccaaagaat ggctgggctg 2100
   caattggaac tcactccttt gaatttgcac agtttgacaa ctttcttgtg gaagccacac 2160
   gctaatactt aacagggcat catagaatac tctggatttt cttcccttct ttttggtttt 2220
   ggttcagagc caattcttgt ttcattggaa cagtatatga ggcttttgag actaaaaata 2280
   atgaagagta aaaggggaga gaaatttatt tttaatttac cctgtggaag attttattag 2340
   aattaattcc aaggggaaaa ctggtgaatc tttaacatta cctggtgtgt tccctaacat 2400
   tcaaactgtg cattggccat acccttagga gtggtttgag tagtacagac ctcgaagcct 2460
   tgctgctaac actgaggtag ctctcttcat cttatttgca agcggtcctg tagatggcag 2520
   taacttgatc atcactgaga tgtatttatg catgctgacc gtgtgtccaa gtgagccagt 2580
   gtcttcatca caagatgatg ctgccataat agaaagctga agaacactag aagtagcttt 2640
   ttgaaaacca cttcaacctg ttatgcttta tgctctaaaa agtatttttt tattttcctt 2700
   tttaagatga tacttttgaa atgcaggata tgatgagtgg gatgatttta aaaatgcctc 2760
   tttaataaac tacctctaac actatttctg cagtaataga tattagcaga ttaattgggt 2820
   tatttgcatt atttaatttt tttgattcca agttttggtc ttgtaaccac tataactctc 2880
   tgtgaacgtt tttccaggtg gctggaagaa ggaagaaaac ctgatatagc caatgctgtt 2940
   gtagtcgttt cctcagcctc atctcactgt gctgtggtct gtcctcacat gtgcactggt 3000
   aacagactca cacagctgat gaatgctttt ctctccttat gtgtggaagg aggggagcac 3060
   ttagacattt gctaactccc agaattggat catctcctaa gatgtactta ctttttaaag 3120
   tccaaatatg tttatattta aatatacgtg agcgtgttca tcatgttgta tgatttatac 3180
   taagcattaa tgtggctcta tgtagcaaat cagttattca tgtaggtaaa gtaaatctag 3240
   aattatttat aagaattact cattgaacta attctactat ttaggaattt gtaagagtct 3300
   aacataggct tagctacagt gaagttttgc attgcttttg aagacaagaa gataagtgct 3360
   agaataaata agattacaga gaaaattttt tgttaaaacc aagtgatttc cagctgatgt 3420
   atctaatatt ttttaaaacg aacattatag aggtgtaatt tatttacaat aaaatgttcc 3480
   tactttaaat atacaattca gtgagttttg ataaattgat atacccatgt aaccaacact 3540
   ccagtcaagc ttcagaatat ttccatcacc ccagaaggtt ctcttgtata cctgctcagt 3600
   cagttccttt cactcccaat tgttggcagc cattgatagg aattctatca ctataggtta 3660
   gttttctttg ttccagaaca tcatgaaagc ggcgtcatgt actgtgtatt cttatgaatg 3720
   gtttctttcc atcagcataa tgatttgaga tttgtccatg ttgtgtgatt cagtggtttg 3780
   ttccttctta tttctgaaga gttttccatt gtatgaatat accacaattt gtttcctccc 3840
   caccagtttc tgatactaca attaaaactg tctacattta caaaaaaaaa aaaaaaa 3897
<210> 58
   <211> 685
   <212> PRT
   <213> Homo sapiens
<220>
   <221> SOURCE
   <222> 1..685
   <223> /mol_type="protein"
   /note="Homo sapiens Galc"
   /organism="Homo sapiens"
<400> 58
<210> 59
   <211> 606
   <212> DNA
   <213> Homo sapiens
<220>
   <221> source
   <222> 1..606
   <223> /mol_type="DNA"
   /note="Homo sapiens Scleraxis A and Scleraxis B"
   /organism="Homo sapiens"
<400> 59
   atgtccttcg ccacgctgcg cccggcgccg ccgggccgct acctgtaccc cgaggtgagc      60
   ccgctgtcgg aggacgagga ccgcggcagc gacagctcgg gctccgacga gaaaccctgt     120
   cgcgtgcacg cggcgcgctg cggcctccag ggcgcccggc ggagggcggg gggccggcgg     180
   gccgggggcg gggggccagg gggccggcca ggccgtgagc cccggcagcg gcacacggcg     240
   aacgcgcgcg agcgagaccg caccaacagc gtgaacacgg ccttcacggc gctgcgcacg     300
   ctgatcccca ccgagcccgc cgaccgcaag ctctccaaga ttgagacgct gcgcctggcc     360
   tccagctaca tctcgcacct gggcaacgtg ctgctggcgg gcgaggcctg cggcgacgga     420
   cagccctgcc actccgggcc cgccttcttc cacgcggcgc gcgccggcag cccccccgcc     480
   ccgcccccgc cgcctcccgc ccgcgacggc gagaacaccc agcccaaaca gatctgcacc     540
   ttctgcctca gcaaccagag aaagttgagc aaggaccgcg acagaaagac agcgattcgc     600
   agttag                                                                606
<210> 60
   <211> 201
   <212> PRT
   <213> Homo sapiens
<220>
   <221> SOURCE
   <222> 1..201
   <223> /mol_type="protein"
   /note="Homo sapiens Scleraxis A and Scleraxis B"
   /organism="Homo sapiens"
<400> 60
   Met Ser Phe Ala Thr Leu Arg Pro Ala Pro Pro Gly Arg Tyr Leu Tyr
   1               5                   10                  15
   Pro Glu Val Ser Pro Leu Ser Glu Asp Glu Asp Arg Gly Ser Asp Ser
               20                  25                  30
   Ser Gly Ser Asp Glu Lys Pro Cys Arg Val His Ala Ala Arg Cys Gly
           35                  40                  45
   Leu Gln Gly Ala Arg Arg Arg Ala Gly Gly Arg Arg Ala Gly Gly Gly
       50                  55                  60
   Gly Pro Gly Gly Arg Pro Gly Arg Glu Pro Arg Gln Arg His Thr Ala
   65                  70                  75                  80
   Asn Ala Arg Glu Arg Asp Arg Thr Asn Ser Val Asn Thr Ala Phe Thr
                   85                  90                  95
   Ala Leu Arg Thr Leu Ile Pro Thr Glu Pro Ala Asp Arg Lys Leu Ser
               100                 105                 110
   Lys Ile Glu Thr Leu Arg Leu Ala Ser Ser Tyr Ile Ser His Leu Gly
           115                 120                 125
   Asn Val Leu Leu Ala Gly Glu Ala Cys Gly Asp Gly Gln Pro Cys His
       130                 135                 140
   Ser Gly Pro Ala Phe Phe His Ala Ala Arg Ala Gly Ser Pro Pro Pro
   145                 150                 155                 160
   Pro Pro Pro Pro Pro Pro Ala Arg Asp Gly Glu Asn Thr Gln Pro Lys
                   165                 170                 175
   Gln Ile Cys Thr Phe Cys Leu Ser Asn Gln Arg Lys Leu Ser Lys Asp
               180                 185                 190
   Arg Asp Arg Lys Thr Ala Ile Arg Ser
           195                 200
<210> 61
   <211> 3963
   <212> DNA
   <213> Homo sapiens
<220>
   <221> source
   <222> 1..3963
   <223> /mol_type="DNA"
   /note="Homo sapiens Sox-9"
   /organism="Homo sapiens"
<400> 61
   ggagagccga aagcggagct cgaaactgac tggaaacttc agtggcgcgg agactcgcca      60
   gtttcaaccc cggaaacttt tctttgcagg aggagaagag aaggggtgca agcgccccca     120
   cttttgctct ttttcctccc ctcctcctcc tctccaattc gcctcccccc acttggagcg     180
   ggcagctgtg aactggccac cccgcgcctt cctaagtgct cgccgcggta gccggccgac      240
   gcgccagctt ccccgggagc cgcttgctcc gcatccgggc agccgagggg agaggagccc     300
   gcgcctcgag tccccgagcc gccgcggctt ctcgcctttc ccggccacca gccccctgcc     360
   ccgggcccgc gtatgaatct cctggacccc ttcatgaaga tgaccgacga gcaggagaag     420
   ggcctgtccg gcgcccccag ccccaccatg tccgaggact ccgcgggctc gccctgcccg     480
   tcgggctccg gctcggacac cgagaacacg cggccccagg agaacacgtt ccccaagggc     540
   gagcccgatc tgaagaagga gagcgaggag gacaagttcc ccgtgtgcat ccgcgaggcg     600
   gtcagccagg tgctcaaagg ctacgactgg acgctggtgc ccatgccggt gcgcgtcaac     660
   ggctccagca agaacaagcc gcacgtcaag cggcccatga acgccttcat ggtgtgggcg     720
   caggcggcgc gcaggaagct cgcggaccag tacccgcact tgcacaacgc cgagctcagc     780
   aagacgctgg gcaagctctg gagacttctg aacgagagcg agaagcggcc cttcgtggag     840
   gaggcggagc ggctgcgcgt gcagcacaag aaggaccacc cggattacaa gtaccagccg     900
   cggcggagga agtcggtgaa gaacgggcag gcggaggcag aggaggccac ggagcagacg     960
   cacatctccc ccaacgccat cttcaaggcg ctgcaggccg actcgccaca ctcctcctcc    1020
   ggcatgagcg aggtgcactc ccccggcgag cactcggggc aatcccaggg cccaccgacc    1080
   ccacccacca cccccaaaac cgacgtgcag ccgggcaagg ctgacctgaa gcgagagggg    1140
   cgccccttgc cagagggggg cagacagccc cctatcgact tccgcgacgt ggacatcggc    1200
   gagctgagca gcgacgtcat ctccaacatc gagaccttcg atgtcaacga gtttgaccag    1260
   tacctgccgc ccaacggcca cccgggggtg ccggccacgc acggccaggt cacctacacg    1320
   ggcagctacg gcatcagcag caccgcggcc accccggcga gcgcgggcca cgtgtggatg    1380
   tccaagcagc aggcgccgcc gccacccccg cagcagcccc cacaggcccc gccggccccg     1440
   caggcgcccc cgcagccgca ggcggcgccc ccacagcagc cggcggcacc cccgcagcag    1500
   ccacaggcgc acacgctgac cacgctgagc agcgagccgg gccagtccca gcgaacgcac    1560
   atcaagacgg agcagctgag ccccagccac tacagcgagc agcagcagca ctcgccccaa    1620
   cagatcgcct acagcccctt caacctccca cactacagcc cctcctaccc gcccatcacc    1680
   cgctcacagt acgactacac cgaccaccag aactccagct cctactacag ccacgcggca 1740
   ggccagggca ccggcctcta ctccaccttc acctacatga accccgctca gcgccccatg 1800
   tacaccccca tcgccgacac ctctggggtc ccttccatcc cgcagaccca cagcccccag 1860
   cactgggaac aacccgtcta cacacagctc actcgacctt gaggaggcct cccacgaagg 1920
   gcgaagatgg ccgagatgat cctaaaaata accgaagaaa gagaggacca accagaattc 1980
   cctttggaca tttgtgtttt tttgtttttt tattttgttt tgttttttct tcttcttctt 2040
   cttccttaaa gacatttaag ctaaaggcaa ctcgtaccca aatttccaag acacaaacat 2100
   gacctatcca agcgcattac ccacttgtgg ccaatcagtg gccaggccaa ccttggctaa 2160
   atggagcagc gaaatcaacg agaaactgga ctttttaaac cctcttcaga gcaagcgtgg 2220
   aggatgatgg agaatcgtgt gatcagtgtg ctaaatctct ctgcctgttt ggactttgta 2280
   attatttttt tagcagtaat taaagaaaaa agtcctctgt gaggaatatt ctctatttta 2340
   aatattttta gtatgtactg tgtatgattc attaccattt tgaggggatt tatacatatt 2400
   tttagataaa attaaatgct cttatttttc caacagctaa actactctta gttgaacagt 2460
   gtgccctagc ttttcttgca accagagtat ttttgtacag atttgctttc tcttacaaaa 2520
   agaaaaaaaa aatcctgttg tattaacatt taaaaacaga attgtgttat gtgatcagtt 2580
   ttgggggtta actttgctta attcctcagg ctttgcgatt taaggaggag ctgccttaaa 2640
   aaaaaataaa ggccttattt tgcaattatg ggagtaaaca atagtctaga gaagcatttg 2700
   gtaagcttta tcatatatat attttttaaa gaagagaaaa acaccttgag ccttaaaacg 2760
   gtgctgctgg gaaacatttg cactctttta gtgcatttcc tcctgccttt gcttgttcac 2820
   tgcagtctta agaaagaggt aaaaggcaag caaaggagat gaaatctgtt ctgggaatgt 2880
   ttcagcagcc aataagtgcc cgagcacact gcccccggtt gcctgcctgg gccccatgtg 2940
   gaaggcagat gcctgctcgc tctgtcacct gtgcctctca gaacaccagc agttaacctt 3000
   caagacattc cacttgctaa aattatttat tttgtaagga gaggttttaa ttaaaacaaa 3060
   aaaaaattct tttttttttt tttttccaat tttaccttct ttaaaatagg ttgttggagc 3120
   tttcctcaaa gggtatggtc atctgttgtt aaattatgtt cttaactgta accagttttt 3180
   ttttatttat ctctttaatc tttttttatt attaaaagca agtttctttg tattcctcac 3240
   cctagatttg tataaatgcc tttttgtcca tccctttttt ctttgttgtt tttgttgaaa 3300
   acaaactgga aacttgtttc tttttttgta taaatgagag attgcaaatg tagtgtatca 3360
   ctgagtcatt tgcagtgttt tctgccacag acctttgggc tgccttatat tgtgtgtgtg 3420
   tgtgggtgtg tgtgtgtttt gacacaaaaa caatgcaagc atgtgtcatc catatttctc 3480
   tgcatcttct cttggagtga gggaggctac ctggagggga tcagcccact gacagacctt 3540
   aatcttaatt actgctgtgg ctagagagtt tgaggattgc tttttaaaaa agacagcaaa 3600
   cttttttttt tatttaaaaa aagatatatt aacagtttta gaagtcagta gaataaaatc     3660
   ttaaagcact cataatatgg catccttcaa tttctgtata aaagcagatc tttttaaaaa     3720
   gatacttctg taacttaaga aacctggcat ttaaatcata ttttgtcttt aggtaaaagc     3780
   tttggtttgt gttcgtgttt tgtttgtttc acttgtttcc ctcccagccc caaacctttt     3840
   gttctctccg tgaaacttac ctttcccttt ttctttctct tttttttttt tgtatattat     3900
   tgtttacaat aaatatacat tgcattaaaa agaaaaaaaa aaaaaaaaaa aaaaaaaaaa     3960
   aaa                                                                   3963
<210> 62
   <211> 509
   <212> PRT
   <213> Homo sapiens
<220>
   <221> SOURCE
   <222> 1..509
   <223> /mol_type="protein"
   /note="Homo sapiens Sox-9"
   /organism="Homo sapiens"
<400> 62
<210> 63
   <211> 2379
   <212> DNA
   <213> Homo sapiens
<220>
   <221> source
   <222> 1..2379
   <223> /mol_type="DNA"
   /note="Homo sapiens c-Myc"
   /organism="Homo sapiens"
<400> 63
   gacccccgag ctgtgctgct cgcggccgcc accgccgggc cccggccgtc cctggctccc      60
   ctcctgcctc gagaagggca gggcttctca gaggcttggc gggaaaaaga acggagggag     120
   ggatcgcgct gagtataaaa gccggttttc ggggctttat ctaactcgct gtagtaattc     180
   cagcgagagg cagagggagc gagcgggcgg ccggctaggg tggaagagcc gggcgagcag     240
   agctgcgctg cgggcgtcct gggaagggag atccggagcg aatagggggc ttcgcctctg     300
   gcccagccct cccgctgatc ccccagccag cggtccgcaa cccttgccgc atccacgaaa     360
   ctttgcccat agcagcgggc gggcactttg cactggaact tacaacaccc gagcaaggac     420
   gcgactctcc cgacgcgggg aggctattct gcccatttgg ggacacttcc ccgccgctgc     480
   caggacccgc ttctctgaaa ggctctcctt gcagctgctt agacgctgga tttttttcgg     540
   gtagtggaaa accagcagcc tcccgcgacg atgcccctca acgttagctt caccaacagg     600
   aactatgacc tcgactacga ctcggtgcag ccgtatttct actgcgacga ggaggagaac 660
   ttctaccagc agcagcagca gagcgagctg cagcccccgg cgcccagcga ggatatctgg 720
   aagaaattcg agctgctgcc caccccgccc ctgtccccta gccgccgctc cgggctctgc 780
   tcgccctcct acgttgcggt cacacccttc tcccttcggg gagacaacga cggcggtggc 840
   gggagcttct ccacggccga ccagctggag atggtgaccg agctgctggg aggagacatg 900
   gtgaaccaga gtttcatctg cgacccggac gacgagacct tcatcaaaaa catcatcatc 960
   caggactgta tgtggagcgg cttctcggcc gccgccaagc tcgtctcaga gaagctggcc 1020
   tcctaccagg ctgcgcgcaa agacagcggc agcccgaacc ccgcccgcgg ccacagcgtc 1080
   tgctccacct ccagcttgta cctgcaggat ctgagcgccg ccgcctcaga gtgcatcgac 1140
   ccctcggtgg tcttccccta ccctctcaac gacagcagct cgcccaagtc ctgcgcctcg 1200
   caagactcca gcgccttctc tccgtcctcg gattctctgc tctcctcgac ggagtcctcc 1260
   ccgcagggca gccccgagcc cctggtgctc catgaggaga caccgcccac caccagcagc 1320
   gactctgagg aggaacaaga agatgaggaa gaaatcgatg ttgtttctgt ggaaaagagg 1380
   caggctcctg gcaaaaggtc agagtctgga tcaccttctg ctggaggcca cagcaaacct 1440
   cctcacagcc cactggtcct caagaggtgc cacgtctcca cacatcagca caactacgca 1500
   gcgcctccct ccactcggaa ggactatcct gctgccaaga gggtcaagtt ggacagtgtc 1560
   agagtcctga gacagatcag caacaaccga aaatgcacca gccccaggtc ctcggacacc 1620
   gaggagaatg tcaagaggcg aacacacaac gtcttggagc gccagaggag gaacgagcta 1680
   aaacggagct tttttgccct gcgtgaccag atcccggagt tggaaaacaa tgaaaaggcc 1740
   cccaaggtag ttatccttaa aaaagccaca gcatacatcc tgtccgtcca agcagaggag 1800
   caaaagctca tttctgaaga ggacttgttg cggaaacgac gagaacagtt gaaacacaaa 1860
   cttgaacagc tacggaactc ttgtgcgtaa ggaaaagtaa ggaaaacgat tccttctaac 1920
   agaaatgtcc tgagcaatca cctatgaact tgtttcaaat gcatgatcaa atgcaacctc 1980
   acaaccttgg ctgagtcttg agactgaaag atttagccat aatgtaaact gcctcaaatt 2040
   ggactttggg cataaaagaa cttttttatg cttaccatct tttttttttc tttaacagat 2100
   ttgtatttaa gaattgtttt taaaaaattt taagatttac acaatgtttc tctgtaaata 2160
   ttgccattaa atgtaaataa ctttaataaa acgtttatag cagttacaca gaatttcaat 2220
   cctagtatat agtacctagt attataggta ctataaaccc taattttttt tatttaagta 2280
   cattttgctt tttaaagttg atttttttct attgttttta gaaaaaataa aataactggc 2340
   aaatatatca ttgagccaaa tcttaaaaaa aaaaaaaaa 2379
<210> 64
   <211> 454
   <212> PRT
   <213> Homo sapiens
<220>
   <221> SOURCE
   <222> 1..454
   <223> /mol_type="protein"
   /note="Homo sapiens c-Myc"
   /organism="Homo sapiens"
<400> 64
   Met Asp Phe Phe Arg Val Val Glu Asn Gln Gln Pro Pro Ala Thr Met
   1               5                   10                  15
   Pro Leu Asn Val Ser Phe Thr Asn Arg Asn Tyr Asp Leu Asp Tyr Asp
               20                  25                  30
   Ser Val Gln Pro Tyr Phe Tyr Cys Asp Glu Glu Glu Asn Phe Tyr Gln
           35                  40                  45
   Gln Gln Gln Gln Ser Glu Leu Gln Pro Pro Ala Pro Ser Glu Asp Ile
       50                  55                  60
   Trp Lys Lys Phe Glu Leu Leu Pro Thr Pro Pro Leu Ser Pro Ser Arg
   65                  70                  75                  80
   Arg Ser Gly Leu Cys Ser Pro Ser Tyr Val Ala Val Thr Pro Phe Ser
                   85                  90                  95
   Leu Arg Gly Asp Asn Asp Gly Gly Gly Gly Ser Phe Ser Thr Ala Asp
               100                 105                 110
   Gln Leu Glu Met Val Thr Glu Leu Leu Gly Gly Asp Met Val Asn Gln
           115                 120                 125
   Ser Phe Ile Cys Asp Pro Asp Asp Glu Thr Phe Ile Lys Asn Ile Ile
       130                 135                 140
   Ile Gln Asp Cys Met Trp Ser Gly Phe Ser Ala Ala Ala Lys Leu Val
   145                 150                 155                 160
   Ser Glu Lys Leu Ala Ser Tyr Gln Ala Ala Arg Lys Asp Ser Gly Ser
                   165                 170                 175
   Pro Asn Pro Ala Arg Gly His Ser Val Cys Ser Thr Ser Ser Leu Tyr
               180                 185                 190
   Leu Gln Asp Leu Ser Ala Ala Ala Ser Glu Cys Ile Asp Pro Ser Val
           195                 200                 205
   Val Phe Pro Tyr Pro Leu Asn Asp Ser Ser Ser Pro Lys Ser Cys Ala
       210                 215                 220
   Ser Gln Asp Ser Ser Ala Phe Ser Pro Ser Ser Asp Ser Leu Leu Ser
   225                 230                 235                 240
   Ser Thr Glu Ser Ser Pro Gln Gly Ser Pro Glu Pro Leu Val Leu His
                   245                 250                 255
   Glu Glu Thr Pro Pro Thr Thr Ser Ser Asp Ser Glu Glu Glu Gln Glu
               260                 265                 270
   Asp Glu Glu Glu Ile Asp Val Val Ser Val Glu Lys Arg Gln Ala Pro
           275                 280                 285
   Gly Lys Arg Ser Glu Ser Gly Ser Pro Ser Ala Gly Gly His Ser Lys
       290                 295                 300
   Pro Pro His Ser Pro Leu Val Leu Lys Arg Cys His Val Ser Thr His
   305                 310                 315                 320
   Gln His Asn Tyr Ala Ala Pro Pro Ser Thr Arg Lys Asp Tyr Pro Ala
                   325                 330                 335
   Ala Lys Arg Val Lys Leu Asp Ser Val Arg Val Leu Arg Gln Ile Ser
               340                 345                 350
   Asn Asn Arg Lys Cys Thr Ser Pro Arg Ser Ser Asp Thr Glu Glu Asn
           355                 360                 365
   Val Lys Arg Arg Thr His Asn Val Leu Glu Arg Gln Arg Arg Asn Glu
       370                 375                 380
   Leu Lys Arg Ser Phe Phe Ala Leu Arg Asp Gln Ile Pro Glu Leu Glu
   385                 390                 395                 400
   Asn Asn Glu Lys Ala Pro Lys Val Val Ile Leu Lys Lys Ala Thr Ala
                       405                     410                 415
   Tyr Ile Leu Ser Val Gln Ala Glu Glu Gln Lys Leu Ile Ser Glu Glu
                   420                     425                 430
   Asp Leu Leu Arg Lys Arg Arg Glu Gln Leu Lys His Lys Leu Glu Gln
               435                     440                 445
   Leu Arg Asn Ser Cys Ala
       450
<210> 65
   <211> 2949
   <212> DNA
   <213> Homo sapiens
<220>
   <221> source
   <222> 1..2949
   <223> /mol_type="DNA"
   /note="Homo sapiens Klf-4"
   /organism="Homo sapiens"
<400> 65
   agtttcccga ccagagagaa cgaacgtgtc tgcgggcgcg cggggagcag aggcggtggc      60
   gggcggcggc ggcaccggga gccgccgagt gaccctcccc cgcccctctg gccccccacc     120
   ctcccacccg cccgtggccc gcgcccatgg ccgcgcgcgc tccacacaac tcaccggagt     180
   ccgcgccttg cgccgccgac cagttcgcag ctccgcgcca cggcagccag tctcacctgg     240
   cggcaccgcc cgcccaccgc cccggccaca gcccctgcgc ccacggcagc actcgaggcg     300
   accgcgacag tggtggggga cgctgctgag tggaagagag cgcagcccgg ccaccggacc     360
   tacttactcg ccttgctgat tgtctatttt tgcgtttaca acttttctaa gaacttttgt     420
   atacaaagga actttttaaa aaagacgctt ccaagttata tttaatccaa agaagaagga     480
   tctcggccaa tttggggttt tgggttttgg cttcgtttct tctcttcgtt gactttgggg     540
   ttcaggtgcc ccagctgctt cgggctgccg aggaccttct gggcccccac attaatgagg     600
   cagccacctg gcgagtctga catggctgtc agcgacgcgc tgctcccatc tttctccacg     660
   ttcgcgtctg gcccggcggg aagggagaag acactgcgtc aagcaggtgc cccgaataac     720
   cgctggcggg aggagctctc ccacatgaag cgacttcccc cagtgcttcc cggccgcccc     780
   tatgacctgg cggcggcgac cgtggccaca gacctggaga gcggcggagc cggtgcggct     840
   tgcggcggta gcaacctggc gcccctacct cggagagaga ccgaggagtt caacgatctc     900
   ctggacctgg actttattct ctccaattcg ctgacccatc ctccggagtc agtggccgcc     960
   accgtgtcct cgtcagcgtc agcctcctct tcgtcgtcgc cgtcgagcag cggccctgcc    1020
   agcgcgccct ccacctgcag cttcacctat ccgatccggg ccgggaacga cccgggcgtg    1080
   gcgccgggcg gcacgggcgg aggcctcctc tatggcaggg agtccgctcc ccctccgacg    1140
   gctcccttca acctggcgga catcaacgac gtgagcccct cgggcggctt cgtggccgag    1200
   ctcctgcggc cagaattgga cccggtgtac attccgccgc agcagccgca gccgccaggt    1260
   ggcgggctga tgggcaagtt cgtgctgaag gcgtcgctga gcgcccctgg cagcgagtac 1320
   ggcagcccgt cggtcatcag cgtcagcaaa ggcagccctg acggcagcca cccggtggtg 1380
   gtggcgccct acaacggcgg gccgccgcgc acgtgcccca agatcaagca ggaggcggtc 1440
   tcttcgtgca cccacttggg cgctggaccc cctctcagca atggccaccg gccggctgca 1500
   cacgacttcc ccctggggcg gcagctcccc agcaggacta ccccgaccct gggtcttgag 1560
   gaagtgctga gcagcaggga ctgtcaccct gccctgccgc ttcctcccgg cttccatccc 1620
   cacccggggc ccaattaccc atccttcctg cccgatcaga tgcagccgca agtcccgccg 1680
   ctccattacc aagagctcat gccacccggt tcctgcatgc cagaggagcc caagccaaag 1740
   aggggaagac gatcgtggcc ccggaaaagg accgccaccc acacttgtga ttacgcgggc 1800
   tgcggcaaaa cctacacaaa gagttcccat ctcaaggcac acctgcgaac ccacacaggt 1860
   gagaaacctt accactgtga ctgggacggc tgtggatgga aattcgcccg ctcagatgaa 1920
   ctgaccaggc actaccgtaa acacacgggg caccgcccgt tccagtgcca aaaatgcgac 1980
   cgagcatttt ccaggtcgga ccacctcgcc ttacacatga agaggcattt ttaaatccca 2040
   gacagtggat atgacccaca ctgccagaag agaattcagt attttttact tttcacactg 2100
   tcttcccgat gagggaagga gcccagccag aaagcactac aatcatggtc aagttcccaa 2160
   ctgagtcatc ttgtgagtgg ataatcagga aaaatgagga atccaaaaga caaaaatcaa 2220
   agaacagatg gggtctgtga ctggatcttc tatcattcca attctaaatc cgacttgaat 2280
   attcctggac ttacaaaatg ccaagggggt gactggaagt tgtggatatc agggtataaa 2340
   ttatatccgt gagttggggg agggaagacc agaattccct tgaattgtgt attgatgcaa 2400
   tataagcata aaagatcacc ttgtattctc tttaccttct aaaagccatt attatgatgt 2460
   tagaagaaga ggaagaaatt caggtacaga aaacatgttt aaatagccta aatgatggtg 2520
   cttggtgagt cttggttcta aaggtaccaa acaaggaagc caaagttttc aaactgctgc 2580
   atactttgac aaggaaaatc tatatttgtc ttccgatcaa catttatgac ctaagtcagg 2640
   taatatacct ggtttacttc tttagcattt ttatgcagac agtctgttat gcactgtggt 2700
   ttcagatgtg caataatttg tacaatggtt tattcccaag tatgccttaa gcagaacaaa 2760
   tgtgtttttc tatatagttc cttgccttaa taaatatgta atataaattt aagcaaacgt 2820
   ctattttgta tatttgtaaa ctacaaagta aaatgaacat tttgtggagt ttgtattttg 2880
   catactcaag gtgagaatta agttttaaat aaacctataa tattttatct gaaaaaaaaa 2940
   aaaaaaaaa 2949
<210> 66
   <211> 478
   <212> PRT
   <213> Homo sapiens
<220>
   <221> SOURCE
   <222> 1..478
   <223> /mol_type="protein"
   /note="Homo sapiens Klf-4"
   /organism="Homo sapiens"
<400> 66
   Met Arg Gln Pro Pro Gly Glu Ser Asp Met Ala Val Ser Asp Ala Leu
   1 5 10 15
   Leu Pro Ser Phe Ser Thr Phe Ala Ser Gly Pro Ala Gly Arg Glu Lys
   20 25 30
   Thr Leu Arg Gln Ala Gly Ala Pro Asn Asn Arg Trp Arg Glu Glu Leu
   35 40 45
   Ser His Met Lys Arg Leu Pro Pro Val Leu Pro Gly Arg Pro Tyr Asp
   50 55 60
   Leu Ala Ala Ala Thr Val Ala Thr Asp Leu Glu Ser Gly Gly Ala Gly
   65 70 75 80
   Ala Ala Cys Gly Gly Ser Asn Leu Ala Pro Leu Pro Arg Arg Glu Thr
   85 90 95
   Glu Glu Phe Asn Asp Leu Leu Asp Leu Asp Phe Ile Leu Ser Asn Ser
   100 105 110
   Leu Thr His Pro Pro Glu Ser Val Ala Ala Thr Val Ser Ser Ser Ala
   115 120 125
   Ser Ala Ser Ser Ser Ser Ser Pro Ser Ser Ser Gly Pro Ala Ser Ala
   130 135 140
   Pro Ser Thr Cys Ser Phe Thr Tyr Pro Ile Arg Ala Gly Asn Asp Pro
   145 150 155 160
   Gly Val Ala Pro Gly Gly Thr Gly Gly Gly Leu Leu Tyr Gly Arg Glu
   165 170 175
   Ser Ala Pro Pro Pro Thr Ala Pro Phe Asn Leu Ala Asp Ile Asn Asp
   180 185 190
   Val Ser Pro Ser Gly Gly Phe Val Ala Glu Leu Leu Arg Pro Glu Leu
   195 200 205
   Asp Val Tyr Ile Pro Pro Gln Gln Pro Gln Pro Pro Gly Gly Gly Leu
   210 215 220
   Met Gly Lys Phe Val Leu Lys Ala Ser Leu Ser Ala Pro Gly Ser Glu
   225 230 235 240
   Tyr Gly Ser Pro Ser Val Ile Ser Val Ser Lys Gly Ser Pro Asp Gly
   245 250 255
   Ser His Pro Val Val Val Ala Pro Tyr Asn Gly Gly Pro Pro Arg Thr
   260 265 270
   Cys Pro Lys Ile Lys Gln Glu Ala Val Ser Ser Cys Thr His Leu Gly
   275 280 285
   Ala Gly Pro Pro Leu Ser Asn Gly His Arg Pro Ala Ala His Asp Phe
   290 295 300
   Pro Leu Gly Arg Gln Leu Pro Ser Arg Thr Thr Pro Thr Leu Gly Leu
   305 310 315 320
   Glu Glu Val Leu Ser Ser Arg Asp Cys His Pro Ala Leu Pro Leu Pro
   325 330 335
   Pro Gly Phe His Pro His Pro Gly Pro Asn Tyr Pro Ser Phe Leu Pro
   340 345 350
   Asp Gln Met Gln Pro Gln Val Pro Pro Leu His Tyr Gln Glu Leu Met
   355 360 365
   Pro Pro Gly Ser Cys Met Pro Glu Glu Pro Lys Pro Lys Arg Gly Arg
   370 375 380
   Arg Ser Trp Pro Arg Lys Arg Thr Ala Thr His Thr Cys Asp Tyr Ala
   385 390 395 400
   Gly Cys Gly Lys Thr Tyr Thr Lys Ser Ser His Leu Lys Ala His Leu
   405 410 415
   Arg Thr His Thr Gly Glu Lys Pro Tyr His Cys Asp Trp Asp Gly Cys
   420 425 430
   Gly Trp Lys Phe Ala Arg Ser Asp Glu Leu Thr Arg His Tyr Arg Lys
           435                     440                 445
   His Thr Gly His Arg Pro Phe Gln Cys Gln Lys Cys Asp Arg Ala Phe
       450                 455                 460
   Ser Arg Ser Asp His Leu Ala Leu His Met Lys Arg His Phe
   465                 470                 475
<210> 67
   <211> 2520
   <212> DNA
   <213> Homo sapiens
<220>
   <221> source
   <222> 1..2520
   <223> /mol_type="DNA"
   /note="Homo sapiens Sox-2"
   /organism="Homo sapiens"
<400> 67
   ggatggttgt ctattaactt gttcaaaaaa gtatcaggag ttgtcaaggc agagaagaga     60
   gtgtttgcaa aagggggaaa gtagtttgct gcctctttaa gactaggact gagagaaaga    120
   agaggagaga gaaagaaagg gagagaagtt tgagccccag gcttaagcct ttccaaaaaa    180
   taataataac aatcatcggc ggcggcagga tcggccagag gaggagggaa gcgctttttt     240
   tgatcctgat tccagtttgc ctctctcttt ttttccccca aattattctt cgcctgattt     300
   tcctcgcgga gccctgcgct cccgacaccc ccgcccgcct cccctcctcc tctccccccg    360
   cccgcgggcc ccccaaagtc ccggccgggc cgagggtcgg cggccgccgg cgggccgggc    420
   ccgcgcacag cgcccgcatg tacaacatga tggagacgga gctgaagccg ccgggcccgc    480
   agcaaacttc ggggggcggc ggcggcaact ccaccgcggc ggcggccggc ggcaaccaga    540
   aaaacagccc ggaccgcgtc aagcggccca tgaatgcctt catggtgtgg tcccgcgggc    600
   agcggcgcaa gatggcccag gagaacccca agatgcacaa ctcggagatc agcaagcgcc    660
   tgggcgccga gtggaaactt ttgtcggaga cggagaagcg gccgttcatc gacgaggcta    720
   agcggctgcg agcgctgcac atgaaggagc acccggatta taaataccgg ccccggcgga    780
   aaaccaagac gctcatgaag aaggataagt acacgctgcc cggcgggctg ctggcccccg    840
   gcggcaatag catggcgagc ggggtcgggg tgggcgccgg cctgggcgcg ggcgtgaacc    900
   agcgcatgga cagttacgcg cacatgaacg gctggagcaa cggcagctac agcatgatgc    960
   aggaccagct gggctacccg cagcacccgg gcctcaatgc gcacggcgca gcgcagatgc   1020
   agcccatgca ccgctacgac gtgagcgccc tgcagtacaa ctccatgacc agctcgcaga   1080
   cctacatgaa cggctcgccc acctacagca tgtcctactc gcagcagggc acccctggca   1140
   tggctcttgg ctccatgggt tcggtggtca agtccgaggc cagctccagc ccccctgtgg   1200
   ttacctcttc ctcccactcc agggcgccct gccaggccgg ggacctccgg gacatgatca   1260
   gcatgtatct ccccggcgcc gaggtgccgg aacccgccgc ccccagcaga cttcacatgt   1320
   cccagcacta ccagagcggc ccggtgcccg gcacggccat taacggcaca ctgcccctct 1380
   cacacatgtg agggccggac agcgaactgg aggggggaga aattttcaaa gaaaaacgag 1440
   ggaaatggga ggggtgcaaa agaggagagt aagaaacagc atggagaaaa cccggtacgc 1500
   tcaaaaagaa aaaggaaaaa aaaaaatccc atcacccaca gcaaatgaca gctgcaaaag 1560
   agaacaccaa tcccatccac actcacgcaa aaaccgcgat gccgacaaga aaacttttat 1620
   gagagagatc ctggacttct tttgggggga ctatttttgt acagagaaaa cctggggagg 1680
   gtggggaggg cgggggaatg gaccttgtat agatctggag gaaagaaagc tacgaaaaac 1740
   tttttaaaag ttctagtggt acggtaggag ctttgcagga agtttgcaaa agtctttacc 1800
   aataatattt agagctagtc tccaagcgac gaaaaaaatg ttttaatatt tgcaagcaac 1860
   ttttgtacag tatttatcga gataaacatg gcaatcaaaa tgtccattgt ttataagctg 1920
   agaatttgcc aatatttttc aaggagaggc ttcttgctga attttgattc tgcagctgaa 1980
   atttaggaca gttgcaaacg tgaaaagaag aaaattattc aaatttggac attttaattg 2040
   tttaaaaatt gtacaaaagg aaaaaattag aataagtact ggcgaaccat ctctgtggtc 2100
   ttgtttaaaa agggcaaaag ttttagactg tactaaattt tataacttac tgttaaaagc 2160
   aaaaatggcc atgcaggttg acaccgttgg taatttataa tagcttttgt tcgatcccaa 2220
   ctttccattt tgttcagata aaaaaaacca tgaaattact gtgtttgaaa tattttctta 2280
   tggtttgtaa tatttctgta aatttattgt gatattttaa ggttttcccc cctttatttt 2340
   ccgtagttgt attttaaaag attcggctct gtattatttg aatcagtctg ccgagaatcc 2400
   atgtatatat ttgaactaat atcatcctta taacaggtac attttcaact taagttttta 2460
   ctccattatg cacagtttga gataaataaa tttttgaaat atggacactg aaaaaaaaaa 2520
<210> 68
   <211> 317
   <212> PRT
   <213> Homo sapiens
<220>
   <221> SOURCE
   <222> 1..317
   <223> /mol_type="protein"
   /note="Homo sapiens Sox-2"
   /organism="Homo sapiens"
<400> 68
<210> 69
   <211> 2098
   <212> DNA
   <213> Homo sapiens
<220>
   <221> source
   <222> 1..2098
   <223> /mol_type="DNA"
   /note="Homo sapiens NANOG"
   /organism="Homo sapiens"
<400> 69
   attataaatc tagagactcc aggattttaa cgttctgctg gactgagctg gttgcctcat      60
   gttattatgc aggcaactca ctttatccca atttcttgat acttttcctt ctggaggtcc     120
   tatttctcta acatcttcca gaaaagtctt aaagctgcct taaccttttt tccagtccac     180
   ctcttaaatt ttttcctcct cttcctctat actaacatga gtgtggatcc agcttgtccc     240
   caaagcttgc cttgctttga agcatccgac tgtaaagaat cttcacctat gcctgtgatt     300
   tgtgggcctg aagaaaacta tccatccttg caaatgtctt ctgctgagat gcctcacacg     360
   gagactgtct ctcctcttcc ttcctccatg gatctgctta ttcaggacag ccctgattct     420
   tccaccagtc ccaaaggcaa acaacccact tctgcagaga agagtgtcgc aaaaaaggaa     480
   gacaaggtcc cggtcaagaa acagaagacc agaactgtgt tctcttccac ccagctgtgt     540
   gtactcaatg atagatttca gagacagaaa tacctcagcc tccagcagat gcaagaactc 600
   tccaacatcc tgaacctcag ctacaaacag gtgaagacct ggttccagaa ccagagaatg 660
   aaatctaaga ggtggcagaa aaacaactgg ccgaagaata gcaatggtgt gacgcagaag 720
   gcctcagcac ctacctaccc cagcctttac tcttcctacc accagggatg cctggtgaac 780
   ccgactggga accttccaat gtggagcaac cagacctgga acaattcaac ctggagcaac 840
   cagacccaga acatccagtc ctggagcaac cactcctgga acactcagac ctggtgcacc 900
   caatcctgga acaatcaggc ctggaacagt cccttctata actgtggaga ggaatctctg 960
   cagtcctgca tgcagttcca gccaaattct cctgccagtg acttggaggc tgccttggaa 1020
   gctgctgggg aaggccttaa tgtaatacag cagaccacta ggtattttag tactccacaa 1080
   accatggatt tattcctaaa ctactccatg aacatgcaac ctgaagacgt gtgaagatga 1140
   gtgaaactga tattactcaa tttcagtctg gacactggct gaatccttcc tctcccctcc 1200
   tcccatccct cataggattt ttcttgtttg gaaaccacgt gttctggttt ccatgatgcc 1260
   catccagtca atctcatgga gggtggagta tggttggagc ctaatcagcg aggtttcttt 1320
   tttttttttt ttcctattgg atcttcctgg agaaaatact tttttttttt ttttttttga 1380
   aacggagtct tgctctgtcg cccaggctgg agtgcagtgg cgcggtcttg gctcactgca 1440
   agctccgtct cccgggttca cgccattctc ctgcctcagc ctcccgagca gctgggacta 1500
   caggcgcccg ccacctcgcc cggctaatat tttgtatttt tagtagagac ggggtttcac 1560
   tgtgttagcc aggatggtct cgatctcctg accttgtgat ccacccgcct cggcctccct 1620
   aacagctggg atttacaggc gtgagccacc gcgccctgcc tagaaaagac attttaataa 1680
   ccttggctgc cgtctctggc tatagataag tagatctaat actagtttgg atatctttag 1740
   ggtttagaat ctaacctcaa gaataagaaa tacaagtaca aattggtgat gaagatgtat 1800
   tcgtattgtt tgggattggg aggctttgct tattttttaa aaactattga ggtaaagggt 1860
   taagctgtaa catacttaat tgatttctta ccgtttttgg ctctgttttg ctatatcccc 1920
   taatttgttg gttgtgctaa tctttgtaga aagaggtctc gtatttgctg catcgtaatg 1980
   acatgagtac tgctttagtt ggtttaagtt caaatgaatg aaacaactat ttttccttta 2040
   gttgatttta ccctgatttc accgagtgtt tcaatgagta aatatacagc ttaaacat 2098
<210> 70
   <211> 305
   <212> PRT
   <213> Homo sapiens
<220>
   <221> SOURCE
   <222> 1..305
   <223> /mol_type="protein"
   /note="Homo sapiens NANOG"
   /organism="Homo sapiens"
<400> 70
   Met Ser Val Asp Pro Ala Cys Pro Gln Ser Leu Pro Cys Phe Glu Ala
   1               5                   10                  15
   Ser Asp Cys Lys Glu Ser Ser Pro Met Pro Val Ile Cys Gly Pro Glu
               20                  25                  30
   Glu Asn Tyr Pro Ser Leu Gln Met Ser Ser Ala Glu Met Pro His Thr
           35                  40                  45
   Glu Thr Val Ser Pro Leu Pro Ser Ser Met Asp Leu Leu Ile Gln Asp
       50                  55                  60
   Ser Pro Asp Ser Ser Thr Ser Pro Lys Gly Lys Gln Pro Thr Ser Ala
   65                  70                  75                  80
   Glu Lys Ser Val Ala Lys Lys Glu Asp Lys Val Pro Val Lys Lys Gln
                   85                  90                  95
   Lys Thr Arg Thr Val Phe Ser Ser Thr Gln Leu Cys Val Leu Asn Asp
               100                 105                 110
   Arg Phe Gln Arg Gln Lys Tyr Leu Ser Leu Gln Gln Met Gln Glu Leu
           115                 120                 125
   Ser Asn Ile Leu Asn Leu Ser Tyr Lys Gln Val Lys Thr Trp Phe Gln
       130                 135                 140
   Asn Gln Arg Met Lys Ser Lys Arg Trp Gln Lys Asn Asn Trp Pro Lys
   145                 150                 155                 160
   Asn Ser Asn Gly Val Thr Gln Lys Ala Ser Ala Pro Thr Tyr Pro Ser
                   165                 170                 175
   Leu Tyr Ser Ser Tyr His Gln Gly Cys Leu Val Asn Pro Thr Gly Asn
               180                 185                 190
   Leu Pro Met Trp Ser Asn Gln Thr Trp Asn Asn Ser Thr Trp Ser Asn
           195                 200                 205
   Gln Thr Gln Asn Ile Gln Ser Trp Ser Asn His Ser Trp Asn Thr Gln
       210                 215                 220
   Thr Trp Cys Thr Gln Ser Trp Asn Asn Gln Ala Trp Asn Ser Pro Phe
   225                 230                 235                 240
   Tyr Asn Cys Gly Glu Glu Ser Leu Gln Ser Cys Met Gln Phe Gln Pro
                   245                 250                 255
   Asn Ser Pro Ala Ser Asp Leu Glu Ala Ala Leu Glu Ala Ala Gly Glu
               260                 265                 270
   Gly Leu Asn Val Ile Gln Gln Thr Thr Arg Tyr Phe Ser Thr Pro Gln
           275                 280                 285
   Thr Met Asp Leu Phe Leu Asn Tyr Ser Met Asn Met Gln Pro Glu Asp
       290                 295                 300
   Val
   305
<210> 71
   <211> 469
   <212> DNA
   <213> Homo sapiens
<220>
   <221> source
   <222> 1..469
   <223> /mol_type="DNA"
   /note="Homo sapiens Insulin"
   /organism="Homo sapiens"
<400> 71
   agccctccag gacaggctgc atcagaagag gccatcaagc agatcactgt ccttctgcca      60
   tggccctgtg gatgcgcctc ctgcccctgc tggcgctgct ggccctctgg ggacctgacc     120
   cagccgcagc ctttgtgaac caacacctgt gcggctcaca cctggtggaa gctctctacc     180
   tagtgtgcgg ggaacgaggc ttcttctaca cacccaagac ccgccgggag gcagaggacc     240
   tgcaggtggg gcaggtggag ctgggcgggg gccctggtgc aggcagcctg cagcccttgg     300
   ccctggaggg gtccctgcag aagcgtggca ttgtggaaca atgctgtacc agcatctgct     360
   ccctctacca gctggagaac tactgcaact agacgcagcc cgcaggcagc cccacacccg     420
   ccgcctcctg caccgagaga gatggaataa agcccttgaa ccagcaaaa                 469
<210> 72
   <211> 110
   <212> PRT
   <213> Homo sapiens
<220>
   <221> SOURCE
   <222> 1..110
   <223> /mol_type="protein"
   /note="Homo sapiens Insulin"
   /organism="Homo sapiens"
<400> 72
<210> 73
   <211> 1310
   <212> DNA
   <213> Homo sapiens
<220>
   <221> source
   <222> 1..1310
   <223> /mol_type="DNA"
   /note="Homo sapiens GAPDH"
   /organism="Homo sapiens"
<400> 73
   aaattgagcc cgcagcctcc cgcttcgctc tctgctcctc ctgttcgaca gtcagccgca      60
   tcttcttttg cgtcgccagc cgagccacat cgctcagaca ccatggggaa ggtgaaggtc     120
   ggagtcaacg gatttggtcg tattgggcgc ctggtcacca gggctgcttt taactctggt     180
   aaagtggata ttgttgccat caatgacccc ttcattgacc tcaactacat ggtttacatg     240
   ttccaatatg attccaccca tggcaaattc catggcaccg tcaaggctga gaacgggaag     300
   cttgtcatca atggaaatcc catcaccatc ttccaggagc gagatccctc caaaatcaag     360
   tggggcgatg ctggcgctga gtacgtcgtg gagtccactg gcgtcttcac caccatggag     420
   aaggctgggg ctcatttgca ggggggagcc aaaagggtca tcatctctgc cccctctgct     480
   gatgccccca tgttcgtcat gggtgtgaac catgagaagt atgacaacag cctcaagatc     540
   atcagcaatg cctcctgcac caccaactgc ttagcacccc tggccaaggt catccatgac     600
   aactttggta tcgtggaagg actcatgacc acagtccatg ccatcactgc cacccagaag     660
   actgtggatg gcccctccgg gaaactgtgg cgtgatggcc gcggggctct ccagaacatc     720
   atccctgcct ctactggcgc tgccaaggct gtgggcaagg tcatccctga gctgaacggg     780
   aagctcactg gcatggcctt ccgtgtcccc actgccaacg tgtcagtggt ggacctgacc     840
   tgccgtctag aaaaacctgc caaatatgat gacatcaaga aggtggtgaa gcaggcgtcg     900
   gagggccccc tcaagggcat cctgggctac actgagcacc aggtggtctc ctctgacttc     960
   aacagcgaca cccactcctc cacctttgac gctggggctg gcattgccct caacgaccac    1020
   tttgtcaagc tcatttcctg gtatgacaac gaatttggct acagcaacag ggtggtggac    1080
   ctcatggccc acatggcctc caaggagtaa gacccctgga ccaccagccc cagcaagagc    1140
   acaagaggaa gagagagacc ctcactgctg gggagtccct gccacactca gtcccccacc    1200
   acactgaatc tcccctcctc acagttgcca tgtagacccc ttgaagaggg gaggggccta    1260
   gggagccgca ccttgtcatg taccatcaat aaagtaccct gtgctcaacc               1310
<210> 74
   <211> 335
   <212> PRT
   <213> Homo sapiens
<220>
   <221> SOURCE
   <222> 1..335
   <223> /mol_type="protein"
   /note="Homo sapiens GAPDH"
   /organism="Homo sapiens"
<400> 74
   Met Gly Lys Val Lys Val Gly Val Asn Gly Phe Gly Arg Ile Gly Arg
   1               5                   10                  15
   Leu Val Thr Arg Ala Ala Phe Asn Ser Gly Lys Val Asp Ile Val Ala
               20                  25                  30
   Ile Asn Asp Pro Phe Ile Asp Leu Asn Tyr Met Val Tyr Met Phe Gln
           35                  40                  45
   Tyr Asp Ser Thr His Gly Lys Phe His Gly Thr Val Lys Ala Glu Asn
       50                  55                  60
   Gly Lys Leu Val Ile Asn Gly Asn Pro Ile Thr Ile Phe Gln Glu Arg
   65                  70                  75                  80
   Asp Pro Ser Lys Ile Lys Trp Gly Asp Ala Gly Ala Glu Tyr Val Val
                   85                  90                  95
   Glu Ser Thr Gly Val Phe Thr Thr Met Glu Lys Ala Gly Ala His Leu
               100                 105                 110
   Gln Gly Gly Ala Lys Arg Val Ile Ile Ser Ala Pro Ser Ala Asp Ala
           115                 120                 125
   Pro Met Phe Val Met Gly Val Asn His Glu Lys Tyr Asp Asn Ser Leu
       130                 135                 140
   Lys Ile Ile Ser Asn Ala Ser Cys Thr Thr Asn Cys Leu Ala Pro Leu
   145                 150                 155                 160
   Ala Lys Val Ile His Asp Asn Phe Gly Ile Val Glu Gly Leu Met Thr
                   165                 170                 175
   Thr Val His Ala Ile Thr Ala Thr Gln Lys Thr Val Asp Gly Pro Ser
               180                 185                 190
   Gly Lys Leu Trp Arg Asp Gly Arg Gly Ala Leu Gln Asn Ile Ile Pro
           195                 200                 205
   Ala Ser Thr Gly Ala Ala Lys Ala Val Gly Lys Val Ile Pro Glu Leu
       210                 215                 220
   Asn Gly Lys Leu Thr Gly Met Ala Phe Arg Val Pro Thr Ala Asn Val
   225                 230                 235                 240
   Ser Val Val Asp Leu Thr Cys Arg Leu Glu Lys Pro Ala Lys Tyr Asp
                   245                 250                 255
   Asp Ile Lys Lys Val Val Lys Gln Ala Ser Glu Gly Pro Leu Lys Gly
               260                 265                 270
   Ile Leu Gly Tyr Thr Glu His Gln Val Val Ser Ser Asp Phe Asn Ser
           275                 280                 285
   Asp Thr His Ser Ser Thr Phe Asp Ala Gly Ala Gly Ile Ala Leu Asn
       290                 295                 300
   Asp His Phe Val Lys Leu Ile Ser Trp Tyr Asp Asn Glu Phe Gly Tyr
   305                 310                 315                 320
   Ser Asn Arg Val Val Asp Leu Met Ala His Met Ala Ser Lys Glu
                   325                 330                 335
<210> 75
   <211> 5946
   <212> DNA
   <213> Rattus norvegicus
<220>
   <221> source
   <222> 1..5946
   <223> /mol_type="DNA"
   /note="Rattus norvegicus Nestin"
   /organism="Rattus norvegicus"
<400> 75
   tgctggagtt ctccgcttcc gctgggtcac tgtcgccgct acttcttttc aacccctaaa    60
   agctccacgg gccactccct tctctagtgc tccacgtccg cttgccctcg ggggccagac   120
   cagcgacatg gagggttgcg tcggggaaga atcttttcag atgtgggagc tcaatcgacg   180
   cctggaggcc tacctgaccc gggtcaagac gctagaagag caaaaccagc tgctcagcgc   240
   cgagcttggg ggactccggg cgcagtccgg agacacctcc tggagagccc gagccgatga   300
   cgagctggca tccctgcgga tcctcgtcga tcagcgctgg cgggagaagc tcgaggctga   360
   ggtgcagcgc gacaaccttg cggaagagct ggagagcgtg gcgggccggt gccagcaggt   420
   gcggcttgct cgggagcgga ccgtccagga ggccgcctgc agccggcgcg cactcgaggc   480
   ggagaagaat gcgcggggct ggctgagcac ccaggcggcc gagctggagc gggagttaga   540
   ggctctgcga gccgcgcacg aggaggagcg cgcacacctg aacgcccagg ccgcctgtgc   600
   gcctcgccgg ccccccgcac cgccccaccg gatccccggt ccggcccccg aagtcgagga   660
   tctggccagg cgactaggcg aagtgtggcg cggggcggtg cgtgactacc aggagcgcgt 720
   ggctcacatg gaaagctcgc tgggtcaggc acgcgagcgg ctgagccaag ccgtgcgggg 780
   cgctcgggag tgtcgcttag aggtgcaaca gctgcaggct gatcgcgaca gcctccagga 840
   gcgcagagaa gcgctggaac agagattgga aggccgctgg caggaccggc tgcaggccac 900
   tgataagttc cagctggctg tggaagccct ggagcaggag aagcaaggtc tacagagtca 960
   gatcgctcag atcctggaag gtgggcagca actggcacac ctcaagatgt cccttagtct 1020
   ggaggtggct acatacagga ctctgctgga ggctgagaac tctcggttgc agacacctgg 1080
   acgaggttcc caggcttctc ttggctttct ggaccccaag ctgaagccga atttccttgg 1140
   gataccagag gaccagtacc tgggatctgt gctccctgcc ctcagcccca catccttccc 1200
   ttcccccttg cctaataccc ttgagactcc tgtgacagcc ttcctgaaga ctcaggagtt 1260
   ccttcaggcc agaaccccca ccttggccag cactcccatc ccacctatat ctgaggctcc 1320
   ctgtcctcca aatgcagagg tgagagccca ggaggtccct ctttctctgc tccagacaca 1380
   ggctccagag cccctttggc tgaaggccac agtgcctagt tcttctgcta tcctcccaga 1440
   actagaggaa cctgggggca agcagcaggg tcacttccct gatgatctga cctccttagc 1500
   cacaaacctc aaccctcacc accctacttt agaggctaaa gatggagaat ccagtgagtc 1560
   tagagtttct agcatattcc aggaagatga ggggcaaatc tgggaactgg tagagaaaga 1620
   agcagatata gaggtaaaag tagaaaacag ctcagcccag aaaacacaag aaagtggtct 1680
   ggacacagaa gaaacccagg attcccaggg acctttgcag aaggaaacac tgaaggctct 1740
   aggagaggag ccactgatgt ctctgaaaat ccagaactat gagacagcag ggaaagagaa 1800
   ttgcaattct tctacagaag gccacctggg aacactagaa ggcccagaaa aagaaaagca 1860
   aataccacta aagtctttag aagaaaagaa tgtagagtca gagaaaactc tagaaaatgg 1920
   ggttcctgta ctatctgagc ttttaggaaa agaagacaca agaacagagg atcaagaatt 1980
   aatgtctcct aaaggtacac taaagagatt ttcatctcta ggaaaggaaa gtcaagaagt 2040
   agtgaggcct tcaaaagagg ggaacctaga atcatggaca gcttttaaag aggagagcca 2100
   acacccactg ggatttccag gagctgagga ccagatgctt gagagactgg tagagaaaga 2160
   ggatcagagc ttcccaaggt ctccagagga agaggaccag gaggcatgta gacctctgca 2220
   gaaagagaat caggaaccac tagggtatga agaagcagag ggccagatac ttgagagact 2280
   gatagaaaaa gagagtcagg agtccctgag gtctccagaa gaagaggacc aggaggcagg 2340
   tagatctctg cagaaagaga atcaggagcc actagggtat gaagaagcag aggaccagat 2400
   gcttgagaga ctgatagaaa aagagagtca ggagtccctg aagtctccag aagaaaacca 2460
   gaggattggg aagcctctag aaagagagaa tcagaaatct ctgaggtatc ttgaagaaaa 2520
   ccaggagact tttgtaccac tagaaagcag gaaccagagg ccactgagat ctctagaagt 2580
   agaagaggag gagcagagaa ttgtgaaacc tctagaaaaa gtgagtcagg attccctcgg 2640
   atctctagca gaagagaatg tgcagccact gaggtatctg gaagaagatg actgcataaa 2700
   taagagcctt ctagaagaca agactcacaa gtccttgggg tctcttgaag atagaaatgg 2760
   ggatagcatt attataccac aagaaagtga gacccaggtt tcattgaggc ctccagaaga 2820
   ggaggaccag aggattgtga accatctaga aaaagaaagt caggagttct cgaggtcttc 2880
   agaagaagaa gagcaggtga tggagagatc tctagaagga gagaaccatg aatcactgag 2940
   ttctgtagaa aaagaggacc agatggttga gagccaacta gagaaagaga gtcaggactc 3000
   agggaagtct cttgaagatg agagccagga gacctttgga cctctggaaa aagagaatgc 3060
   agagtccctg agatctctag caggacagga ccaagaggaa cagaagcttg aacaagagac 3120
   ccaacaaaca ctgagggctg tagggaatga gcagatggca gtgagcccac cagaaaaggt 3180
   ggatccagag ttaccgaagc ctcttggaaa tgaccaggaa atagctagat ctcttggaaa 3240
   agagaatcaa gagtcactag tgtcactgaa agaaaaaggt atagagacag tgaagtcttt 3300
   agaaacagag atcatagaac cactggagac tgcagaagag gacctggaaa gaaggaagtc 3360
   tatagatact caggagccat tgtggtctac tgaagtggct agagagacag tagaacctcc 3420
   agaagatgag cccccaggat cgctagggtc tgtggatgag aaccgagaga cactgacatc 3480
   ccttgaaaag gagagtcaag aactgagctc tctgggcaag tggaacgtag agaccagggt 3540
   agaggacagt cagcagtgcc tgcaagtaga agagggtctg caggaggaac agcaccaaga 3600
   gtctctgaga gaggtgaagc aggagctgcc tagctctgga aatcaacagc ggtgggagga 3660
   tgtggtggag ggcaaagcag tgggtcagga agcacctctg gcaaccacag gagtgggaac 3720
   tgaggataag gcagagttgc atctgagggg gcaaggtgga gaggaagaag ctgcagcaga    3780
   gggagagctg ttgcaggata ttgtggggga ggcctggagt ctggggagct ctgagcccaa    3840
   ggagcagagg gtccctgctg aggccctcga caacctggaa ggaggggcct tagaggtccc 3900
   agttgctcag tcaatgccag aggtgacaga gcgagatgag gatagagccc aagcaggtga 3960
   acaagactcc atagaggtga cccttgggtt agaggctgcc agaactggac tggaactcga 4020
   gcaggaagtg gtagggctag aggacccaag gcattttgcc agggaggagg ccattccccc 4080
   atccctgggg gaggaaagtg tgaaggcaaa gatagctcag ggcttggaag ggcctggaaa 4140
   ggaaccaaaa gaggcaggtg ctctggactc ggggatcctt gaattgccca agactagcag 4200
   cgaggctctg gaatgccagg gccatgaaga gtctgagtcc atggagggct gggaagaaga 4260
   ggaggcctca ctggagactt cagatcatga gggcagtgat gcccctcagc ccaggccccc 4320
   agaaacagaa gaagatgagg gtgcacaggc agcactgaca gcccctggtc ccaagctctt 4380
   ggaaccctgt tcacccatcc caatcctgac agatgcccat gagctgcagc cccaggctga 4440
   ggggatccag gaggctggct ggcagccaga agctgggtct gaagcactag aaagggtaga      4500
   aaatgagcca gagtttggtc ttggggagat cccggagggc ctccaggatt gggaagaggg      4560
   cagagaagaa agcgaggcag atgatctagg ggaaactctc cctgactcta ctcccctggg      4620
   cctctacctg aggtcccctg cttctccaaa gtgggatctg gctggagaac agaggctttc      4680
   ccctcaaggg gatgccggga aggaagactg gggtcctgct gtccccgctg cccagggcct       4740
   cagtggtcca ccggaagagg aggaggagca aggccatggc tctgacctat catctgagga      4800
   gtttgaggac ctagggactg aggcctctct tcttccaggg gttcccaagg aggtggcaga      4860
   tcacgtgggc caagtgcccc cggtactgca gcctgcatgc tgggatcagg gtggggaatc      4920
   tgatgggttt gctgatgagg aagaaagtgg ggaggaggga gaggaagaag atgctgatga      4980
   ggaaggagca gagtcaggag ctcagtggtg ggggtcaggg gcctctggtg gaggctgcaa       5040
   ggtccaggat attgcccaaa gaggagaccc ggtacaggag tctgtgggtg tcagtggtct      5100
   ctgggatgat ggcttgagag gtgctgcagc taatgttcct gccctagaga tggtatctca      5160
   ggacagtgct gagccttctg ggtcagagga gtctgagtct gcttccttgg agggggagga       5220
   aggtcaagtg actgaccatt tagatgctcc ccaggaggtg accagcatgg tcccgggggt      5280
   aggagatgcc tttgacattg gtggccagag ccccaacttg gactcagaac aagtgaatgg      5340
   gaaaatggag aatggactag aacaggctga ggggcaggtg gtcctggatg gggacgagga      5400
   tcaagaactc ctattacagg gacaggaggt gggtgctcta aaggttcctt tggtagcatc      5460
   tcctgtgcat ctaggcccaa gccagcccct gaagttcact ctgagtgggg tagatgggga      5520
   ttcctggtcc tcaggggaag actagaaact gcccctctgg ctctgaggat gtactggtgg      5580
   ggatgtccct ccctgctctg ggtgaccact cttagctttg ataacttgac ccatggtatt      5640
   tgtcctggag agttgtggct gggctgagca agggaggtga gatcctcctg aaggctcagg      5700
   agttccaggc ctatagttct accccctctt tcttctgtgg ctcacctgct ggaagaggcc      5760
   tgggcccaga gctttcccac aaggctgttc tggccacagc ttgctagcct tgcctaccac      5820
   ctgcacaagg tctggtctgg tgtatgacca ggggagctga gggcagcatt tatctgaccc      5880
   ttcatctcag cctgctgaga gcttgttcct ctcttcctcc ctgaataaag ccgtatccct      5940
   acctac                                                                 5946
<210> 76
   <211> 1805
   <212> PRT
   <213> Rattus norvegicus
<220>
   <221> SOURCE
   <222> 1..1805
   <223> /mol_type="protein"
   /note="Rattus norvegicus Nestin"
   /organism="Rattus norvegicus"
<400> 76
   Met Glu Gly Cys Val Gly Glu Glu Ser Phe Gln Met Trp Glu Leu Asn
   1 5 10 15
   Arg Arg Leu Glu Ala Tyr Leu Thr Arg Val Lys Thr Leu Glu Glu Gln
   20 25 30
   Asn Gln Leu Leu Ser Ala Glu Leu Gly Gly Leu Arg Ala Gln Ser Gly
   35 40 45
   Asp Thr Ser Trp Arg Ala Arg Ala Asp Asp Glu Leu Ala Ser Leu Arg
   50 55 60
   Ile Leu Val Asp Gln Arg Trp Arg Glu Lys Leu Glu Ala Glu Val Gln
   65 70 75 80
   Arg Asp Asn Leu Ala Glu Glu Leu Glu Ser Val Ala Gly Arg Cys Gln
   85 90 95
   Gln Val Arg Leu Ala Arg Glu Arg Thr Val Gln Glu Ala Ala Cys Ser
   100 105 110
   Arg Arg Ala Leu Glu Ala Glu Lys Asn Ala Arg Gly Trp Leu Ser Thr
   115 120 125
   Gln Ala Ala Glu Leu Glu Arg Glu Leu Glu Ala Leu Arg Ala Ala His
   130 135 140
   Glu Glu Glu Arg Ala His Leu Asn Ala Gln Ala Ala Cys Ala Pro Arg
   145 150 155 160
   Arg Pro Pro Ala Pro Pro His Arg Ile Pro Gly Pro Ala Pro Glu Val
   165 170 175
   Glu Asp Leu Ala Arg Arg Leu Gly Glu Val Trp Arg Gly Ala Val Arg
   180 185 190
   Asp Tyr Gln Glu Arg Val Ala His Met Glu Ser Ser Leu Gly Gln Ala
   195 200 205
   Arg Glu Arg Leu Ser Gln Ala Val Arg Gly Ala Arg Glu Cys Arg Leu
   210 215 220
   Glu Val Gln Gln Leu Gln Ala Asp Arg Asp Ser Leu Gln Glu Arg Arg
   225 230 235 240
   Glu Ala Leu Glu Gln Arg Leu Glu Gly Arg Trp Gln Asp Arg Leu Gln
   245 250 255
   Ala Thr Asp Lys Phe Gln Leu Ala Val Glu Ala Leu Glu Gln Glu Lys
   260 265 270
   Gln Gly Leu Gln Ser Gln Ile Ala Gln Ile Leu Glu Gly Gly Gln Gln
   275 280 285
   Leu Ala His Leu Lys Met Ser Leu Ser Leu Glu Val Ala Thr Tyr Arg
   290 295 300
   Thr Leu Leu Glu Ala Glu Asn Ser Arg Leu Gln Thr Pro Gly Arg Gly
   305 310 315 320
   Ser Gln Ala Ser Leu Gly Phe Leu Asp Pro Lys Leu Lys Pro Asn Phe
   325 330 335
   Leu Gly Ile Pro Glu Asp Gln Tyr Leu Gly Ser Val Leu Pro Ala Leu
   340 345 350
   Ser Pro Thr Ser Phe Pro Ser Pro Leu Pro Asn Thr Leu Glu Thr Pro
   355 360 365
   Val Thr Ala Phe Leu Lys Thr Gln Glu Phe Leu Gln Ala Arg Thr Pro
   370 375 380
   Thr Leu Ala Ser Thr Pro Ile Pro Pro Ile Ser Glu Ala Pro Cys Pro
   385 390 395 400
   Pro Asn Ala Glu Val Arg Ala Gln Glu Val Pro Leu Ser Leu Leu Gln
   405 410 415
   Thr Gln Ala Pro Glu Pro Leu Trp Leu Lys Ala Thr Val Pro Ser Ser
   420 425 430
   Ser Ala Ile Leu Pro Glu Leu Glu Glu Pro Gly Gly Lys Gln Gln Gly
   435 440 445
   His Phe Pro Asp Asp Leu Thr Ser Leu Ala Thr Asn Leu Asn Pro His
   450 455 460
   His Pro Thr Leu Glu Ala Lys Asp Gly Glu Ser Ser Glu Ser Arg Val
   465 470 475 480
   Ser Ser Ile Phe Gln Glu Asp Glu Gly Gln Ile Trp Glu Leu Val Glu
                   485                 490                 495
   Lys Glu Ala Asp Ile Glu Val Lys Val Glu Asn Ser Ser Ala Gln Lys
               500                 505                 510
   Thr Gln Glu Ser Gly Leu Asp Thr Glu Glu Thr Gln Asp Ser Gln Gly
           515                 520                 525
   Pro Leu Gln Lys Glu Thr Leu Lys Ala Leu Gly Glu Glu Pro Leu Met
       530                 535                 540
   Ser Leu Lys Ile Gln Asn Tyr Glu Thr Ala Gly Lys Glu Asn Cys Asn
   545                 550                 555                 560
   Ser Ser Thr Glu Gly His Leu Gly Thr Leu Glu Gly Pro Glu Lys Glu
                   565                 570                 575
   Lys Gln Ile Pro Leu Lys Ser Leu Glu Glu Lys Asn Val Glu Ser Glu
               580                 585                 590
   Lys Thr Leu Glu Asn Gly Val Pro Val Leu Ser Glu Leu Leu Gly Lys
           595                 600                 605
   Glu Asp Thr Arg Thr Glu Asp Gln Glu Leu Met Ser Pro Lys Gly Thr
       610                 615                 620
   Leu Lys Arg Phe Ser Ser Leu Gly Lys Glu Ser Gln Glu Val Val Arg
   625                 630                 635                 640
   Pro Ser Lys Glu Gly Asn Leu Glu Ser Trp Thr Ala Phe Lys Glu Glu
                   645                 650                 655
   Ser Gln His Pro Leu Gly Phe Pro Gly Ala Glu Asp Gln Met Leu Glu
               660                 665                 670
   Arg Leu Val Glu Lys Glu Asp Gln Ser Phe Pro Arg Ser Pro Glu Glu
           675                 680                 685
   Glu Asp Gln Glu Ala Cys Arg Pro Leu Gln Lys Glu Asn Gln Glu Pro
       690                 695                 700
   Leu Gly Tyr Glu Glu Ala Glu Gly Gln Ile Leu Glu Arg Leu Ile Glu
   705                 710                 715                 720
   Lys Glu Ser Gln Glu Ser Leu Arg Ser Pro Glu Glu Glu Asp Gln Glu
                   725                 730                 735
   Ala Gly Arg Ser Leu Gln Lys Glu Asn Gln Glu Pro Leu Gly Tyr Glu
               740                 745                 750
   Glu Ala Glu Asp Gln Met Leu Glu Arg Leu Ile Glu Lys Glu Ser Gln
           755                 760                 765
   Glu Ser Leu Lys Ser Pro Glu Glu Asn Gln Arg Ile Gly Lys Pro Leu
       770                 775                 780
   Glu Arg Glu Asn Gln Lys Ser Leu Arg Tyr Leu Glu Glu Asn Gln Glu
   785                 790                 795                 800
   Thr Phe Val Pro Leu Glu Ser Arg Asn Gln Arg Pro Leu Arg Ser Leu
                   805                 810                 815
   Glu Val Glu Glu Glu Glu Gln Arg Ile Val Lys Pro Leu Glu Lys Val
               820                 825                 830
   Ser Gln Asp Ser Leu Gly Ser Leu Ala Glu Glu Asn Val Gln Pro Leu
           835                 840                 845
   Arg Tyr Leu Glu Glu Asp Asp Cys Ile Asn Lys Ser Leu Leu Glu Asp
       850                 855                 860
   Lys Thr His Lys Ser Leu Gly Ser Leu Glu Asp Arg Asn Gly Asp Ser
   865                 870                 875                 880
   Ile Ile Ile Pro Gln Glu Ser Glu Thr Gln Val Ser Leu Arg Pro Pro
                   885                 890                 895
   Glu Glu Glu Asp Gln Arg Ile Val Asn His Leu Glu Lys Glu Ser Gln
               900                 905                 910
   Glu Phe Ser Arg Ser Ser Glu Glu Glu Glu Gln Val Met Glu Arg Ser
           915                 920                 925
   Leu Glu Gly Glu Asn His Glu Ser Leu Ser Ser Val Glu Lys Glu Asp
       930                 935                 940
   Gln Met Val Glu Ser Gln Leu Glu Lys Glu Ser Gln Asp Ser Gly Lys
   945                 950                 955                 960
   Ser Leu Glu Asp Glu Ser Gln Glu Thr Phe Gly Pro Leu Glu Lys Glu
                   965                 970                 975
   Asn Ala Glu Ser Leu Arg Ser Leu Ala Gly Gln Asp Gln Glu Glu Gln
   980 985 990
   Lys Leu Glu Gln Glu Thr Gln Gln Thr Leu Arg Ala Val Gly Asn Glu
   995 1000 1005
   Gln Met Ala Val Ser Pro Pro Glu Lys Val Asp Pro Glu Leu Pro Lys
   1010 1015 1020
   Pro Leu Gly Asn Asp Gln Glu Ile Ala Arg Ser Leu Gly Lys Glu Asn
   1025 1030 1035 1040
   Gln Glu Ser Leu Val Ser Leu Lys Glu Lys Gly Ile Glu Thr Val Lys
   1045 1050 1055
   Ser Leu Glu Thr Glu Ile Ile Glu Pro Leu Glu Thr Ala Glu Glu Asp
   1060 1065 1070
   Leu Glu Arg Arg Lys Ser Ile Asp Thr Gln Glu Pro Leu Trp Ser Thr
   1075 1080 1085
   Glu Val Ala Arg Glu Thr Val Glu Pro Pro Glu Asp Glu Pro Pro Gly
   1090 1095 1100
   Ser Leu Gly Ser Val Asp Glu Asn Arg Glu Thr Leu Thr Ser Leu Glu
   1105 1110 1115 1120
   Lys Glu Ser Gln Glu Leu Ser Ser Leu Gly Lys Trp Asn Val Glu Thr
   1125 1130 1135
   Arg Val Glu Asp Ser Gln Gln Cys Leu Gln Val Glu Glu Gly Leu Gln
   1140 1145 1150
   Glu Glu Gln His Gln Glu Ser Leu Arg Glu Val Lys Gln Glu Leu Pro
   1155 1160 1165
   Ser Ser Gly Asn Gln Gln Arg Trp Glu Asp Val Val Glu Gly Lys Ala
   1170 1175 1180
   Val Gly Gln Glu Ala Pro Leu Ala Thr Thr Gly Val Gly Thr Glu Asp
   1185 1190 1195 1200
   Lys Ala Glu Leu His Leu Arg Gly Gln Gly Gly Glu Glu Glu Ala Ala
   1205 1210 1215
   Ala Glu Gly Glu Leu Leu Gln Asp Ile Val Gly Glu Ala Trp Ser Leu
   1220 1225 1230
   Gly Ser Ser Glu Pro Lys Glu Gln Arg Val Pro Ala Glu Ala Leu Asp
   1235 1240 1245
   Asn Leu Glu Gly Gly Ala Leu Glu Val Pro Val Ala Gln Ser Met Pro
   1250 1255 1260
   Glu Val Thr Glu Arg Asp Glu Asp Arg Ala Gln Ala Gly Glu Gln Asp
   1265 1270 1275 1280
   Ser Ile Glu Val Thr Leu Gly Leu Glu Ala Ala Arg Thr Gly Leu Glu
   1285 1290 1295
   Leu Glu Gln Glu Val Val Gly Leu Glu Asp Pro Arg His Phe Ala Arg
   1300 1305 1310
   Glu Glu Ala Ile Pro Pro Ser Leu Gly Glu Glu Ser Val Lys Ala Lys
   1315 1320 1325
   Ile Ala Gln Gly Leu Glu Gly Pro Gly Lys Glu Pro Lys Glu Ala Gly
   1330 1335 1340
   Ala Leu Asp Ser Gly Ile Leu Glu Leu Pro Lys Thr Ser Ser Glu Ala
   1345 1350 1355 1360
   Leu Glu Cys Gln Gly His Glu Glu Ser Glu Ser Met Glu Gly Trp Glu
   1365 1370 1375
   Glu Glu Glu Ala Ser Leu Glu Thr Ser Asp His Glu Gly Ser Asp Ala
   1380 1385 1390
   Pro Gln Pro Arg Pro Pro Glu Thr Glu Glu Asp Glu Gly Ala Gln Ala
   1395 1400 1405
   Ala Leu Thr Ala Pro Gly Pro Lys Leu Leu Glu Pro Cys Ser Pro Ile
   1410 1415 1420
   Pro Ile Leu Thr Asp Ala His Glu Leu Gln Pro Gln Ala Glu Gly Ile
   1425 1430 1435 1440
   Gln Glu Ala Gly Trp Gln Pro Glu Ala Gly Ser Glu Ala Leu Glu Arg
   1445 1450 1455
   Val Glu Asn Glu Pro Glu Phe Gly Leu Gly Glu Ile Pro Glu Gly Leu
   1460 1465 1470
   Gln Asp Trp Glu Glu Gly Arg Glu Glu Ser Glu Ala Asp Asp Leu Gly
   1475 1480 1485
   Glu Thr Leu Pro Asp Ser Thr Pro Leu Gly Leu Tyr Leu Arg Ser Pro
   1490 1495 1500
   Ala Ser Pro Lys Trp Asp Leu Ala Gly Glu Gln Arg Leu Ser Pro Gln
   1505 1510 1515 1520
   Gly Asp Ala Gly Lys Glu Asp Trp Gly Pro Ala Val Pro Ala Ala Gln
   1525 1530 1535
   Gly Leu Ser Gly Pro Pro Glu Glu Glu Glu Glu Gln Gly His Gly Ser
   1540 1545 1550
   Asp Leu Ser Ser Glu Glu Phe Glu Asp Leu Gly Thr Glu Ala Ser Leu
   1555 1560 1565
   Leu Pro Gly Val Pro Lys Glu Val Ala Asp His Val Gly Gln Val Pro
   1570 1575 1580
   Pro Val Leu Gln Pro Ala Cys Trp Asp Gln Gly Gly Glu Ser Asp Gly
   1585 1590 1595 1600
   Phe Ala Asp Glu Glu Glu Ser Gly Glu Glu Gly Glu Glu Glu Asp Ala
   1605 1610 1615
   Asp Glu Glu Gly Ala Glu Ser Gly Ala Gln Trp Trp Gly Ser Gly Ala
   1620 1625 1630
   Ser Gly Gly Gly Cys Lys Val Gln Asp Ile Ala Gln Arg Gly Asp Pro
   1635 1640 1645
   Val Gln Glu Ser Val Gly Val Ser Gly Leu Trp Asp Asp Gly Leu Arg
   1650 1655 1660
   Gly Ala Ala Ala Asn Val Pro Ala Leu Glu Met Val Ser Gln Asp Ser
   1665 1670 1675 1680
   Ala Glu Pro Ser Gly Ser Glu Glu Ser Glu Ser Ala Ser Leu Glu Gly
   1685 1690 1695
   Glu Glu Gly Gln Val Thr Asp His Leu Asp Ala Pro Gln Glu Val Thr
   1700 1705 1710
   Ser Met Val Pro Gly Val Gly Asp Ala Phe Asp Ile Gly Gly Gln Ser
   1715 1720 1725
   Pro Asn Leu Asp Ser Glu Gln Val Asn Gly Lys Met Glu Asn Gly Leu
   1730 1735 1740
   Glu Gln Ala Glu Gly Gln Val Val Leu Asp Gly Asp Glu Asp Gln Glu
   1745 1750 1755 1760
   Leu Leu Leu Gln Gly Gln Glu Val Gly Ala Leu Lys Val Pro Leu Val
   1765 1770 1775
   Ala Ser Pro Val His Leu Gly Pro Ser Gln Pro Leu Lys Phe Thr Leu
   1780 1785 1790
   Ser Gly Val Asp Gly Asp Ser Trp Ser Ser Gly Glu Asp
   1795 1800 1805
<210> 77
   <211> 5843
   <212> DNA
   <213> Rattus norvegicus
<220>
   <221> source
   <222> 1..5843
   <223> /mol_type="DNA"
   /note="Rattus norvegicus Col1A1"
   /organism="Rattus norvegicus"
<400> 77
   cgggagtttc acctccggac ggagcaggag gcacacggag tgaggccacg catgagccga      60
   agctaacccc ccaccccagc cgcaaagagt ctacatgtct agggtctaga catgttcagc     120
   tttgtggacc tccggctcct gctcctctta ggggccactg ccctcctgac gcatggccaa     180
   gaagacatcc ctgaagtcag ctgcatacac aatggcctaa gggtccctaa tggtgagacg     240
   tggaaacctg atgtatgctt gatctgtatc tgccacaatg gcacggctgt gtgcgatggc     300
   gtgctatgca aagaagactt ggactgtccc aacccccaaa aacgggaggg cgagtgctgt 360
   cctttctgcc cagaagaata tgtatcacca gacgcagaag tcataggagt cgagggaccc 420
   aagggagacc ctggccccca aggcccacgg ggacctgttg gcccccctgg acaagatggc 480
   atccctggac agcctggact tcctggtcct cctggtcccc ccggcccccc cggaccccct 540
   ggtcttggag gaaactttgc ttcccagatg tcctatggct atgatgagaa atcagctgga 600
   gtttccgtgc ctggccccat gggtccttct ggtcctcgtg gtctccctgg cccccctggt 660
   gcacctggtc ctcaaggttt ccaaggcccc cctggtgaac ctggcgagcc tggcgcttca 720
   ggtccaatgg gtccccgagg tccccctggc cctcctggca agaacggaga tgatggggaa 780
   gctggtaagc ctggccgccc tggtgagcgt ggacctcctg gacctcaggg tgctcgtgga 840
   ttgcctggaa cagctggcct ccccggaatg aagggacaca gaggtttcag tggtttggat 900
   ggtgccaaag gagatactgg tcctgctggt cctaagggag agcctggcag tcctggtgaa 960
   aatggagctc ccggccagat gggtccccga ggtctgcctg gtgagagagg tcgccctgga 1020
   ccccctggca gtgctggtgc tcgtggtaac gatggtgctg tcggtgcagc tgggcccccc 1080
   ggtcccaccg gccctactgg ccctcctggc ttccctggtg cagctggtgc taagggtgaa 1140
   gctggtcccc agggagcccg aggctctgaa ggtccccagg gtgtgcgtgg tgagcccgga 1200
   ccccctggcc ctgctggtgc tgctggtcct gctggaaacc ctggtgctga tggacaacct 1260
   ggtgctaaag gtgccaatgg tgctcctggt attgctggtg ctcctggctt ccctggtgcc 1320
   cgaggcccct ctggacctca gggccccagc ggcgctcctg gtcccaaagg caacagtggt 1380
   gaacctggtg cccctggcaa caaaggagac actggtgcca aaggagaacc cggccctgct 1440
   ggagttcaag gtccccctgg ccctgctgga gaagaaggaa aacgaggagc ccgtggtgag 1500
   cctggaccta gcggcctgcc cggacctcct ggcgagcgcg gtggacctgg tagccgtggt 1560
   ttccctggtg ctgatggtgt tgctggcccc aagggtcctg ctggtgaacg tggttctcct 1620
   ggccctgctg gtcccaaagg ttctcctggt gaagctggtc gccctggtga agctggtctc 1680
   cctggtgcca agggtctcac tggcagtcct ggcagccctg gtcctgatgg caaaaccggc 1740
   ccccctggtc ccgctggtca agatggtcgc cctggacccg caggtcctcc tggagcccgt 1800
   ggacaggctg gtgtgatggg attccctgga cctaagggta ctgctggaga acctggaaag 1860
   gctggagaac gaggtgtccc cggaccccct ggcgctgttg gtcccgctgg caaagatggc 1920
   gaagctggag ctcaaggagc ccccggccct gctggtcctg ctggtgagag aggtgaacaa 1980
   ggtcccgctg gctcccctgg attccagggt cttcctggtc ccgctggtcc tcctggtgaa 2040
   gcaggcaagc ctggtgaaca gggtgttcct ggagaccttg gtgcccctgg accctctggc 2100
   gcaagaggcg agagaggttt ccctggtgaa cgtggtgtac aaggtccccc aggtcctgct 2160
   ggtccccgag gaaacaatgg tgcccccggc aacgatggtg ccaagggtga tactggtgcc 2220
   cccggagctc ctggtagcca gggtgccccc ggtcttcagg gaatgcctgg tgaacgtggt 2280
   gcagctggtc ttccaggtcc taagggtgac agaggtgatg ctggtcccaa aggtgctgat 2340
   ggttctcctg gcaaagatgg cgtccgtggt ctgactggtc ccattggtcc tcctggccct 2400
   gctggtgccc ctggtgacaa gggtgaaact ggtcccagtg gtcctgctgg ccccaccgga 2460
   gcccgtggtg cccccggaga ccgtggtgag cctggtcccc ctggtcctgc tggcttcgct 2520
   ggcccccctg gtgctgatgg ccaacctggt gcgaaaggtg aacctggtga tactggtgtg 2580
   aaaggtgacg ctggtcctcc tggccctgct ggtcccgctg gaccccctgg ccccattggt 2640
   aacgttggtg ctcctggacc caaaggttct cgtggtgctg ctggtccccc tggtgctact 2700
   ggtttccctg gtgctgctgg tcgtgttggt ccccctggtc cctctggaaa tgctggaccc 2760
   cctggccctc ccggtcccgt tggcaaagaa gggggcaaag gtccccgtgg tgagactggt 2820
   cccgctggac gtcctggtga agttggtccc ccaggtcccc ctggccctgc tggtgagaaa 2880
   ggatctcctg gtgctgatgg acctgctggc tctcctggta cccctggacc tcagggtatt 2940
   gctggacagc gtggtgtggt cggtcttccc ggtcagagag gagaaagagg cttccctggt 3000
   cttcctggac cctctggtga acccggcaaa caaggtcctt ctggagcaag tggtgaacgt 3060
   ggtccccctg gccctatggg cccccctgga ttggctggcc cccctggtga atctggtcgt 3120
   gagggatccc ctggtgctga aggctcccct ggaagagatg gtgctcctgg tgccaagggt 3180
   gaccgtggtg agactggccc tgctggcccc cctggtgctc ctggtgctcc tggtgctccc 3240
   ggccctgttg gtcctgctgg caagaatggc gaccgtggtg agactggtcc tgctggtcct 3300
   gctggtccca ttggccctgc tggtgcccgt ggtcctgctg gaccccaagg cccccgtggt 3360
   gacaagggtg agacaggcga acaaggtgac agaggcataa agggtcatcg tggcttctct 3420
   ggtctccagg gtcctcctgg ctctcctggc tctcctggtg aacaaggccc ctctggagct 3480
   tctggtcctg caggtccccg gggtccccct ggctctgctg gttctcctgg caaagatgga 3540
   ctcaacggtc tccctggccc cattggtccc cctggtcctc gaggtcgcac tggcgatagt 3600
   ggtcctgctg gtccccccgg acctcctgga ccccctggcc ctcccggtcc tcccagcggt 3660
   ggttatgact tcagcttcct gcctcagcca cctcaagaga agtctcaaga tggtggccgt 3720
   tactaccggg ccgatgatgc caacgtggtc cgtgaccgtg accttgaggt ggacactacc 3780
   ctcaagagcc tgagccagca gattgagaac atccgcagcc ctgagggcag ccgcaagaac 3840
   cccgcccgca catgccgtga cctcaagatg tgccactctg actggaagag cggagagtac 3900
   tggatcgacc ctaaccaagg ctgcaacctg gatgccatca aggtctactg caacatggag 3960
   acaggtcaga cctgtgtgtt ccccactcag ccctctgtgc ctcagaagaa ctggtacatc 4020
   agcccaaacc ccaaggagaa gaagcatgtc tggtttggag agagcatgac cgatggattc 4080
   cagttcgagt atggaagcga aggttccgat cctgccgatg tcgctatcca gctgaccttc 4140
   ctgcgcctga tgtccaccga ggcctcccag aacatcacct atcactgcaa gaacagcgta 4200
   gcctacatgg accaacagac tggcaacctc aagaagtccc tgctcctcca gggctccaac 4260
   gagatcgagc tcaggggcga aggcaacagt cgattcacct acagcacgct tgtggatggc 4320
   tgcacgagtc acaccggaac ttggggcaag acagtcatcg aatacaaaac caccaagacc 4380
   tcccgcctgc ccatcatcga tgtggctccc ttggacattg gtgccccaga ccaggaattc 4440
   ggaatggaca ttggccctgc ctgcttcgtg taaactccct ccatcccaat ctggttccct 4500
   cccacccagc ccactttccc ccaaccctgg aaacagacca acaacccaaa ctcaatttcc 4560
   ccaaaagcca aaaattggga gacaatttca catggacttt ggaaaacatt tttttccttt 4620
   gcattcatct ctcaaactta gtttttatct ttgaccaact gaacgtgacc aaaaaccaaa 4680
   agtgcattca accttaccaa aaagaaaaaa aaataagaat aaataaataa ctttttaaaa 4740
   aaggaagctt ggtcctcttg cttgaagacc tatgtgggta taagtccctt tctgcccact 4800
   gggcttatga taccccaaat gctgcctttt ctgttccttt ctccaccccc tcttggggcc 4860
   tctcctccat tgctccccaa atttaagtct cccccaaaga cacaggaaat aatgcattgt 4920
   ctgcccagcc agcaaaggca atgctgaatc gtcccaccag cccctcaacc cccagcctac 4980
   ttccctaccc agcaccttca aatcctgccg ggacatgggg ttctcggact attgaaggag 5040
   cctaaccatc tggcatctcc atggcctctg caacaaatcc ccacacacac tttgtttttg 5100
   agggcctgtg ctgggggagc cacctgcccc tcgcaggggt ttggagccag gcagggtcac 5160
   agcagactgg aaacatcggc cacacatgtg caggctgggt gggagagact gttctgttcc 5220
   ttgtgtaatt gtgttgctga aagactacct cgttcttgtc tttgtgtgtc accggggcaa 5280
   ctgtgtgggg gcggggatgg gggcagggtg gcagcgcgcc cagtttggta tcaaaggtgc 5340
   tacatctctg tgaaggggtg gggtgggaag gaatttctgg tgctatagaa tctgagatgc 5400
   tcccctagac cagcaaatgt tccttttgtt caaagtattt ttttattctt ttttttttaa     5460
   tggataggga cttgtgtgaa ttttcttttc ctgacggtgc tatttaacaa gggaggagag     5520
   agtgccaact ccagcctgct ctctctctac ccccctcttc actcttccag ctcctgggcc 5580
   tatctgatga tctctctctc ttctgaaacc ctcccctctt gctgctgctc cctaccctca 5640
   gcttctctct ctctctgtcc tgcatcaggg tttcagagca ccattttcca aagcacaaag 5700
   cagtttttat ccctggggtg ggaggaagca agagactctg tacctatttt gtatgtgtat 5760
   aataatttga gatgttttta attattttga ttgctggaat aaagcatgtg gaaatgaccc 5820
   aacgcaaaaa aaaaaaaaaa aaa 5843
<210> 78
<211> 1453
   <212> PRT
   <213> Rattus norvegicus
<220>
   <221> SOURCE
   <222> 1..1453
   <223> /mol_type="protein"
   /note="Rattus norvegicus Col1A1"
   /organism="Rattus norvegicus"
<400> 78
   Met Phe Ser Phe Val Asp Leu Arg Leu Leu Leu Leu Leu Gly Ala Thr
   1               5                   10                  15
   Ala Leu Leu Thr His Gly Gln Glu Asp Ile Pro Glu Val Ser Cys Ile
               20                  25                  30
   His Asn Gly Leu Arg Val Pro Asn Gly Glu Thr Trp Lys Pro Asp Val
           35                  40                  45
   Cys Leu Ile Cys Ile Cys His Asn Gly Thr Ala Val Cys Asp Gly Val
       50                  55                  60
   Leu Cys Lys Glu Asp Leu Asp Cys Pro Asn Pro Gln Lys Arg Glu Gly
   65                  70                  75                  80
   Glu Cys Cys Pro Phe Cys Pro Glu Glu Tyr Val Ser Pro Asp Ala Glu
                   85                  90                  95
   Val Ile Gly Val Glu Gly Pro Lys Gly Asp Pro Gly Pro Gln Gly Pro
               100                 105                 110
   Arg Gly Pro Val Gly Pro Pro Gly Gln Asp Gly Ile Pro Gly Gln Pro
           115                 120                 125
   Gly Leu Pro Gly Pro Pro Gly Pro Pro Gly Pro Pro Gly Pro Pro Gly
       130                 135                 140
   Leu Gly Gly Asn Phe Ala Ser Gln Met Ser Tyr Gly Tyr Asp Glu Lys
   145                 150                 155                 160
   Ser Ala Gly Val Ser Val Pro Gly Pro Met Gly Pro Ser Gly Pro Arg
                   165                 170                 175
   Gly Leu Pro Gly Pro Pro Gly Ala Pro Gly Pro Gln Gly Phe Gln Gly
               180                 185                 190
   Pro Pro Gly Glu Pro Gly Glu Pro Gly Ala Ser Gly Pro Met Gly Pro
           195                 200                 205
   Arg Gly Pro Pro Gly Pro Pro Gly Lys Asn Gly Asp Asp Gly Glu Ala
       210                 215                 220
   Gly Lys Pro Gly Arg Pro Gly Glu Arg Gly Pro Pro Gly Pro Gln Gly
   225                 230                 235                 240
   Ala Arg Gly Leu Pro Gly Thr Ala Gly Leu Pro Gly Met Lys Gly His
                   245                 250                 255
   Arg Gly Phe Ser Gly Leu Asp Gly Ala Lys Gly Asp Thr Gly Pro Ala
               260                 265                 270
   Gly Pro Lys Gly Glu Pro Gly Ser Pro Gly Glu Asn Gly Ala Pro Gly
           275                 280                 285
   Gln Met Gly Pro Arg Gly Leu Pro Gly Glu Arg Gly Arg Pro Gly Pro
       290                 295                 300
   Pro Gly Ser Ala Gly Ala Arg Gly Asn Asp Gly Ala Val Gly Ala Ala
   305                 310                 315                 320
   Gly Pro Pro Gly Pro Thr Gly Pro Thr Gly Pro Pro Gly Phe Pro Gly
                   325                 330                 335
   Ala Ala Gly Ala Lys Gly Glu Ala Gly Pro Gln Gly Ala Arg Gly Ser
               340                 345                 350
   Glu Gly Pro Gln Gly Val Arg Gly Glu Pro Gly Pro Pro Gly Pro Ala
           355                 360                 365
   Gly Ala Ala Gly Pro Ala Gly Asn Pro Gly Ala Asp Gly Gln Pro Gly
       370                 375                 380
   Ala Lys Gly Ala Asn Gly Ala Pro Gly Ile Ala Gly Ala Pro Gly Phe
   385                 390                 395                 400
   Pro Gly Ala Arg Gly Pro Ser Gly Pro Gln Gly Pro Ser Gly Ala Pro
                   405                 410                 415
   Gly Pro Lys Gly Asn Ser Gly Glu Pro Gly Ala Pro Gly Asn Lys Gly
               420                 425                 430
   Asp Thr Gly Ala Lys Gly Glu Pro Gly Pro Ala Gly Val Gln Gly Pro
           435                 440                 445
   Pro Gly Pro Ala Gly Glu Glu Gly Lys Arg Gly Ala Arg Gly Glu Pro
       450                 455                 460
   Gly Pro Ser Gly Leu Pro Gly Pro Pro Gly Glu Arg Gly Gly Pro Gly
   465                 470                 475                 480
   Ser Arg Gly Phe Pro Gly Ala Asp Gly Val Ala Gly Pro Lys Gly Pro
                   485                 490                 495
   Ala Gly Glu Arg Gly Ser Pro Gly Pro Ala Gly Pro Lys Gly Ser Pro
               500                 505                 510
   Gly Glu Ala Gly Arg Pro Gly Glu Ala Gly Leu Pro Gly Ala Lys Gly
           515                 520                 525
   Leu Thr Gly Ser Pro Gly Ser Pro Gly Pro Asp Gly Lys Thr Gly Pro
       530                 535                 540
   Pro Gly Pro Ala Gly Gln Asp Gly Arg Pro Gly Pro Ala Gly Pro Pro
   545                 550                 555                 560
   Gly Ala Arg Gly Gln Ala Gly Val Met Gly Phe Pro Gly Pro Lys Gly
                   565                 570                 575
   Thr Ala Gly Glu Pro Gly Lys Ala Gly Glu Arg Gly Val Pro Gly Pro
               580                 585                 590
   Pro Gly Ala Val Gly Pro Ala Gly Lys Asp Gly Glu Ala Gly Ala Gln
           595                 600                 605
   Gly Ala Pro Gly Pro Ala Gly Pro Ala Gly Glu Arg Gly Glu Gln Gly
       610                 615                 620
   Pro Ala Gly Ser Pro Gly Phe Gln Gly Leu Pro Gly Pro Ala Gly Pro
   625                 630                 635                 640
   Pro Gly Glu Ala Gly Lys Pro Gly Glu Gln Gly Val Pro Gly Asp Leu
                   645                 650                 655
   Gly Ala Pro Gly Pro Ser Gly Ala Arg Gly Glu Arg Gly Phe Pro Gly
               660                 665                 670
   Glu Arg Gly Val Gln Gly Pro Pro Gly Pro Ala Gly Pro Arg Gly Asn
           675                 680                 685
   Asn Gly Ala Pro Gly Asn Asp Gly Ala Lys Gly Asp Thr Gly Ala Pro
       690                 695                 700
   Gly Ala Pro Gly Ser Gln Gly Ala Pro Gly Leu Gln Gly Met Pro Gly
   705                 710                 715                 720
   Glu Arg Gly Ala Ala Gly Leu Pro Gly Pro Lys Gly Asp Arg Gly Asp
                   725                 730                 735
   Ala Gly Pro Lys Gly Ala Asp Gly Ser Pro Gly Lys Asp Gly Val Arg
               740                 745                 750
   Gly Leu Thr Gly Pro Ile Gly Pro Pro Gly Pro Ala Gly Ala Pro Gly
           755                 760                 765
   Asp Lys Gly Glu Thr Gly Pro Ser Gly Pro Ala Gly Pro Thr Gly Ala
       770                 775                 780
   Arg Gly Ala Pro Gly Asp Arg Gly Glu Pro Gly Pro Pro Gly Pro Ala
   785                 790                 795                 800
   Gly Phe Ala Gly Pro Pro Gly Ala Asp Gly Gln Pro Gly Ala Lys Gly
                   805                 810                 815
   Glu Pro Gly Asp Thr Gly Val Lys Gly Asp Ala Gly Pro Pro Gly Pro
               820                 825                 830
   Ala Gly Pro Ala Gly Pro Pro Gly Pro Ile Gly Asn Val Gly Ala Pro
           835                 840                 845
   Gly Pro Lys Gly Ser Arg Gly Ala Ala Gly Pro Pro Gly Ala Thr Gly
       850                 855                 860
   Phe Pro Gly Ala Ala Gly Arg Val Gly Pro Pro Gly Pro Ser Gly Asn
   865                 870                 875                 880
   Ala Gly Pro Pro Gly Pro Pro Gly Pro Val Gly Lys Glu Gly Gly Lys
                   885                 890                 895
   Gly Pro Arg Gly Glu Thr Gly Pro Ala Gly Arg Pro Gly Glu Val Gly
               900                 905                 910
   Pro Pro Gly Pro Pro Gly Pro Ala Gly Glu Lys Gly Ser Pro Gly Ala
   915 920 925
   Asp Gly Pro Ala Gly Ser Pro Gly Thr Pro Gly Pro Gln Gly Ile Ala
   930 935 940
   Gly Gln Arg Gly Val Val Gly Leu Pro Gly Gln Arg Gly Glu Arg Gly
   945 950 955 960
   Phe Pro Gly Leu Pro Gly Pro Ser Gly Glu Pro Gly Lys Gln Gly Pro
   965 970 975
   Ser Gly Ala Ser Gly Glu Arg Gly Pro Pro Gly Pro Met Gly Pro Pro
   980 985 990
   Gly Leu Ala Gly Pro Pro Gly Glu Ser Gly Arg Glu Gly Ser Pro Gly
   995 1000 1005
   Ala Glu Gly Ser Pro Gly Arg Asp Gly Ala Pro Gly Ala Lys Gly Asp
   1010 1015 1020
   Arg Gly Glu Thr Gly Pro Ala Gly Pro Pro Gly Ala Pro Gly Ala Pro
   1025 1030 1035 1040
   Gly Ala Pro Gly Pro Val Gly Pro Ala Gly Lys Asn Gly Asp Arg Gly
   1045 1050 1055
   Glu Thr Gly Pro Ala Gly Pro Ala Gly Pro Ile Gly Pro Ala Gly Ala
   1060 1065 1070
   Arg Gly Pro Ala Gly Pro Gln Gly Pro Arg Gly Asp Lys Gly Glu Thr
   1075 1080 1085
   Gly Glu Gln Gly Asp Arg Gly Ile Lys Gly His Arg Gly Phe Ser Gly
   1090 1095 1100
   Leu Gln Gly Pro Pro Gly Ser Pro Gly Ser Pro Gly Glu Gln Gly Pro
   1105 1110 1115 1120
   Ser Gly Ala Ser Gly Pro Ala Gly Pro Arg Gly Pro Pro Gly Ser Ala
   1125 1130 1135
   Gly Ser Pro Gly Lys Asp Gly Leu Asn Gly Leu Pro Gly Pro Ile Gly
   1140 1145 1150
   Pro Pro Gly Pro Arg Gly Arg Thr Gly Asp Ser Gly Pro Ala Gly Pro
   1155 1160 1165
   Pro Gly Pro Pro Gly Pro Pro Gly Pro Pro Gly Pro Pro Ser Gly Gly
   1170 1175 1180
   Tyr Asp Phe Ser Phe Leu Pro Gln Pro Pro Gln Glu Lys Ser Gln Asp
   1185 1190 1195 1200
   Gly Gly Arg Tyr Tyr Arg Ala Asp Asp Ala Asn Val Val Arg Asp Arg
   1205 1210 1215
   Asp Leu Glu Val Asp Thr Thr Leu Lys Ser Leu Ser Gln Gln Ile Glu
   1220 1225 1230
   Asn Ile Arg Ser Pro Glu Gly Ser Arg Lys Asn Pro Ala Arg Thr Cys
   1235 1240 1245
   Arg Asp Leu Lys Met Cys His Ser Asp Trp Lys Ser Gly Glu Tyr Trp
   1250 1255 1260
   Ile Asp Pro Asn Gln Gly Cys Asn Leu Asp Ala Ile Lys Val Tyr Cys
   1265 1270 1275 1280
   Asn Met Glu Thr Gly Gln Thr Cys Val Phe Pro Thr Gln Pro Ser Val
   1285 1290 1295
   Pro Gln Lys Asn Trp Tyr Ile Ser Pro Asn Pro Lys Glu Lys Lys His
   1300 1305 1310
   Val Trp Phe Gly Glu Ser Met Thr Asp Gly Phe Gln Phe Glu Tyr Gly
   1315 1320 1325
   Ser Glu Gly Ser Asp Pro Ala Asp Val Ala Ile Gln Leu Thr Phe Leu
   1330 1335 1340
   Arg Leu Met Ser Thr Glu Ala Ser Gln Asn Ile Thr Tyr His Cys Lys
   1345 1350 1355 1360
   Asn Ser Val Ala Tyr Met Asp Gln Gln Thr Gly Asn Leu Lys Lys Ser
   1365 1370 1375
   Leu Leu Leu Gln Gly Ser Asn Glu Ile Glu Leu Arg Gly Glu Gly Asn
   1380 1385 1390
   Ser Arg Phe Thr Tyr Ser Thr Leu Val Asp Gly Cys Thr Ser His Thr
   1395 1400 1405
   Gly Thr Trp Gly Lys Thr Val Ile Glu Tyr Lys Thr Thr Lys Thr Ser
       1410                1415                1420
   Arg Leu Pro Ile Ile Asp Val Ala Pro Leu Asp Ile Gly Ala Pro Asp
   1425                1430                1435                1440
   Gln Glu Phe Gly Met Asp Ile Gly Pro Ala Cys Phe Val
                   1445                1450
<210> 79
   <211> 4792
   <212> DNA
   <213> Rattus norvegicus
<220>
   <221> source
   <222> 1..4792
   <223> /mol_type="DNA"
   /note="Rattus norvegicus Co13A1"
   /organism="Rattus norvegicus"
<400> 79
   acgggcccgg tgctgaaggg cagggaacaa ctgatggtgc tactgtgagc tgcttcttcc      60
   tctctcctct tttgcacaaa gagtctcatg tctgatattt agacatgatg agctttgtgc     120
   aatgtgggac ctggtttctt ctcaccctgc ttcacccctc tcttattttg gcacagcagt     180
   ccaatgtaga tgaattggga tgcaactacc ttggtcagtc ctatgagtct agagatgtct     240
   ggaagccaga accatgtcaa atatgtgtct gcgactcggg atctgtcctc tgtgatgaca     300
   taatgtgtga tgatgagcca ctagactgcc ccaacccaga gatcccattt ggagaatgtt     360
   gtgcaatatg tccacagcct tctacacctg ctcctgtcat tcctgatggt aatagacctc     420
   aaggccccaa gggagatcca ggccctcctg gcattcctgg gagaaatggc gaccctggtc     480
   ttccaggaca accagggctc cctggtcctc ctggctcccc tggaatctgt gaatcatgtc     540
   caactggtgg ccagaattat tctccccaat tcgactcata tgatgtcaag tctggagtcg     600
   gaggaatggg tggctatcct ggaccagctg gtccaccagg ccctccagga ccccctggtt     660
   cctctggaca tcctggatct cctggttctc ctggatacca aggtcctcct ggtgaaccgg     720
   gtcaagctgg tcctgcaggc cctccaggac ctcctggtgc tattggtcca tctggccctg     780
   caggaaagga tggagagtca ggaagacccg gacgacctgg agagcgtgga ctgcctggac     840
   ctccaggtat caaaggccca gctggtatcc ctggattccc gggtatgaaa ggacacagag     900
   gctttgatgg acgcaatgga gagaagggtg aaaccggtgc tcctggattg aagggtgaaa     960
   acggtcttcc gggagacaac ggagctcctg gccccatggg tcccagaggg gctcctggtg    1020
   agcgaggacg accaggtctt cctggagccg caggtgctcg aggcaatgat ggtgctcggg    1080
   gcagtgacgg gcaaccaggt ccccctggtc ctcctggaac tgcaggattc cctggatctc    1140
   ctggtgctaa gggtgaggtt ggacctgcag gatcccctgg ctcaaatggc tctccaggac    1200
   aaagagggga acctggacca caaggacacg ctggtgctca aggacctcct ggccctcctg    1260
   gcaataatgg cagtcctggt gggaaaggtg aaatgggtcc agctggcatt cctggagctc    1320
   ctggactcct aggagctagg ggtccccctg gaccagcagg agctaatggt gcaccgggac 1380
   aacgaggtcc ttcaggcgaa ccgggcaaga atggagcaaa gggagagcca ggagctcgcg 1440
   gtgaacgggg cgaagctggt tccccaggga ttccaggacc taagggcgaa gacggcaaag 1500
   atgggtcacc aggagaacct ggtgcaaatg gagttccagg aaacccagga gaaaggggtg 1560
   cccctggctt ccgaggacct gcagggccaa atggcgcccc aggagaaaag ggtcctgctg 1620
   gagagcgtgg tggcccaggt cctgcaggac ctagaggagt agctggagaa cctggccgag 1680
   atggaacccc tggaggtcca gggatacggg gtatgcccgg aagtccaggt ggaccaggca 1740
   atgatgggaa accaggacct cctggaagtc aaggagaaag tggtcgccct ggtcctcctg 1800
   gcccatctgg ccccagaggt cagcctggtg tcatgggttt ccctggtcct aaaggaaatg 1860
   atggtgcacc tggcaaaaac ggagaacggg gtggccctgg gggtcctggc cttccaggtc 1920
   ctgctggaaa gaatggggag actggacctc agggtccccc aggacctact ggcgcacctg 1980
   gtgacaaggg agacgctgga ccccctggtc cacaaggatt acaaggcata cctggtacca 2040
   gtggtcctcc aggagaaaat ggaaaaccag gagaaccagg tccaaagggt gaagctggag 2100
   cacctggagt tcctggaggc aagggtgatt ctggtgcccc tggagaacgt ggaccccctg 2160
   gaaccgcagg aacccctggg cttagaggtg gagctggacc ccctgggcct gagggaggaa 2220
   agggccctgc tggtccccct ggtccccctg gtacttctgg tcctcctggt ctgcaaggga 2280
   tgccaggaga gagaggaggt cctgggagtc ctggcccaaa gggtgaaaag ggtgagccag 2340
   gcggtgcggg tgctgatgga gttccaggaa aggatgggcc gagaggtcct gctggtccta 2400
   ttggtccccc tggcccagct ggtcagcctg gagataaggg tgaaggtggt gcccctggac 2460
   ttccaggtat agctggacct cgaggtggcc ctggtgagag aggtgaacat ggccctccag 2520
   gacctgctgg cttcccgggt gctcctgggc agaatggtga accaggtgct aaaggagaaa 2580
   gaggtgcccc cggagagaaa ggtgaaggag gccctcctgg tgccgcagga ccccctggtg 2640
   gttctggacc tgctggtcct cctgggcctc aaggtgtaaa gggtgaacgg ggcagtcctg 2700
   gcggtcctgg tgctgctggc tttcccggtg gccgtggtct tcctggtcct cccggcaaca 2760
   atggtaatcc agggccccca ggacccagtg gtgctcctgg caaggatggc cctccaggtc 2820
   ctgcaggtaa cagtggttct cctggaaacc ctggagtagc tggaccaaaa ggtgatgctg 2880
   gccaaccagg agagaagggg ccacctggtg ctcagggtcc tccaggatct ccaggtccac 2940
   ttggaattgc agggctaact ggagcacgag gtcttgctgg accaccaggc atgccaggtc 3000
   cacggggtag ccctggacct cagggtatca agggtgaaag tggaaagcca ggagccagtg 3060
   gccataatgg ggaacgtggt cctcctggac cccaaggtct tcctggtcag cctggtacag 3120
   ctggtgagcc cggaagggat ggaaaccctg gatcagatgg tcagccaggt cgagacggat 3180
   ctcctggtgg caagggtgat cgtggtgaaa atggttctcc tggtgcccca ggcgctcctg 3240
   gtcatccagg accacctggt cctgttggtc catctggaaa aaatggtgac agaggagaaa 3300
   cgggtcctgc tggtccttct ggtgctccag gtcctgctgg agctcggggt gctcctggtc 3360
   cccaaggtcc acgaggtgac aaaggtgaaa ctggtgaacg tggctctaat ggcatcaaag 3420
   gacatcgagg attccctggc aatccaggtc ccccaggttc tcccggtgct gctggacacc 3480
   agggtgcagt tggtagtcca ggccctgcag gtcccagagg cccagttggt ccacatggac 3540
   ctcctggaaa agatggatca agtggacatc caggtcccat tggaccacca gggcctcgag 3600
   gtaacagagg tgaaagagga tctgagggct cgcccggtca ccctggacag ccaggacccc 3660
   ctggaccccc tggtgcccct ggtccctgct gtggtggcgg tgctgccatt gctggagttg 3720
   gaggtgaaaa gtctggcggc ttttcaccat attacgggga tgatccaatg gatttcaaga 3780
   tcaacactga ggagatcatg tcttcactca agtctgttaa cggacaaata gagagtctta 3840
   tcagccctga tggttctaga aaaaaccctg ctcggaattg cagagacctg aaattctgcc 3900
   accctgaact caagagcgga gaatactggg ttgatcctaa ccaaggctgc aagatggatg 3960
   ctataaaagt gttctgtaat atggaaaccg gagaaacatg cataaatgcc agtcccatga 4020
   ctgtcccgcg gaagcactgg tggacagatg ctggtgctga gaagaaacat gtttggtttg 4080
   gagaatctat gaatggtggc tttcagttca gctatggcaa tcctgatctt cctgaagatg 4140
   tccttgatgt acagctggcc ttcctcagac ttctttccag ccgggcctcc cagaacatta 4200
   cataccactg caaaaacagc attgcctaca tggatcaggc caatggcaat gtaaagaagt 4260
   ctctgaagct gatgggatcc aatgagggag aattcaaggc tgaaggaaat agcaaattca 4320
   cttacacagt tctagaggat ggctgcacta aacacactgg tgaatggagc aagacagtct 4380
   ttgaatatca aacacgcaag gccatgagac tccccatcat agatatcgca ccctatgaca 4440
   ttggtggtcc tgatcaagaa tttggtgtgg acattggccc tgtttgcttt ttataagcca 4500
   aactctctga aaccccagca aaacaaaaac cacatccatg tgttcctctt gctttaatct 4560
   cgtcaaccag tgaaagtgac caactaaatt ccagttattt atttccaaac ttttggaaaa 4620
   agcataattt gacaaaaata gaatacaatt tttttgctgt ttcacccacc aatacagttc 4680
   aaatgctttt gttttatttt tttaccaatt ccaacttcat aatgtctcaa tggtgctata 4740
   ataaataaac atcaacactt ttatgataaa aaaaaaaaaa aaaaaaaaaa aa 4792
<210> 80
   <211> 1463
   <212> PRT
   <213> Rattus norvegicus
<220>
   <221> SOURCE
   <222> 1..1463
   <223> /mol_type="protein"
   /note="Rattus norvegicus Co13A1"
   /organism="Rattus norvegicus"
<400> 80
   Met Met Ser Phe Val Gln Cys Gly Thr Trp Phe Leu Leu Thr Leu Leu
   1               5                   10                  15
   His Pro Ser Leu Ile Leu Ala Gln Gln Ser Asn Val Asp Glu Leu Gly
               20                  25                  30
   Cys Asn Tyr Leu Gly Gln Ser Tyr Glu Ser Arg Asp Val Trp Lys Pro
           35                  40                  45
   Glu Pro Cys Gln Ile Cys Val Cys Asp Ser Gly Ser Val Leu Cys Asp
       50                  55                  60
   Asp Ile Met Cys Asp Asp Glu Pro Leu Asp Cys Pro Asn Pro Glu Ile
   65                  70                  75                  80
   Pro Phe Gly Glu Cys Cys Ala Ile Cys Pro Gln Pro Ser Thr Pro Ala
                   85                  90                  95
   Pro Val Ile Pro Asp Gly Asn Arg Pro Gln Gly Pro Lys Gly Asp Pro
               100                 105                 110
   Gly Pro Pro Gly Ile Pro Gly Arg Asn Gly Asp Pro Gly Leu Pro Gly
           115                 120                 125
   Gln Pro Gly Leu Pro Gly Pro Pro Gly Ser Pro Gly Ile Cys Glu Ser
       130                 135                 140
   Cys Pro Thr Gly Gly Gln Asn Tyr Ser Pro Gln Phe Asp Ser Tyr Asp
   145                 150                 155                 160
   Val Lys Ser Gly Val Gly Gly Met Gly Gly Tyr Pro Gly Pro Ala Gly
                   165                 170                 175
   Pro Pro Gly Pro Pro Gly Pro Pro Gly Ser Ser Gly His Pro Gly Ser
               180                 185                 190
   Pro Gly Ser Pro Gly Tyr Gln Gly Pro Pro Gly Glu Pro Gly Gln Ala
           195                 200                 205
   Gly Pro Ala Gly Pro Pro Gly Pro Pro Gly Ala Ile Gly Pro Ser Gly
       210                 215                 220
   Pro Ala Gly Lys Asp Gly Glu Ser Gly Arg Pro Gly Arg Pro Gly Glu
   225                 230                 235                 240
   Arg Gly Leu Pro Gly Pro Pro Gly Ile Lys Gly Pro Ala Gly Ile Pro
                   245                 250                 255
   Gly Phe Pro Gly Met Lys Gly His Arg Gly Phe Asp Gly Arg Asn Gly
               260                 265                 270
   Glu Lys Gly Glu Thr Gly Ala Pro Gly Leu Lys Gly Glu Asn Gly Leu
           275                 280                 285
   Pro Gly Asp Asn Gly Ala Pro Gly Pro Met Gly Pro Arg Gly Ala Pro
       290                 295                 300
   Gly Glu Arg Gly Arg Pro Gly Leu Pro Gly Ala Ala Gly Ala Arg Gly
   305                 310                 315                 320
   Asn Asp Gly Ala Arg Gly Ser Asp Gly Gln Pro Gly Pro Pro Gly Pro
                   325                 330                 335
   Pro Gly Thr Ala Gly Phe Pro Gly Ser Pro Gly Ala Lys Gly Glu Val
               340                 345                 350
   Gly Pro Ala Gly Ser Pro Gly Ser Asn Gly Ser Pro Gly Gln Arg Gly
           355                 360                 365
   Glu Pro Gly Pro Gln Gly His Ala Gly Ala Gln Gly Pro Pro Gly Pro
       370                 375                 380
   Pro Gly Asn Asn Gly Ser Pro Gly Gly Lys Gly Glu Met Gly Pro Ala
   385                 390                 395                 400
   Gly Ile Pro Gly Ala Pro Gly Leu Leu Gly Ala Arg Gly Pro Pro Gly
                   405                 410                 415
   Pro Ala Gly Ala Asn Gly Ala Pro Gly Gln Arg Gly Pro Ser Gly Glu
               420                 425                 430
   Pro Gly Lys Asn Gly Ala Lys Gly Glu Pro Gly Ala Arg Gly Glu Arg
           435                 440                 445
   Gly Glu Ala Gly Ser Pro Gly Ile Pro Gly Pro Lys Gly Glu Asp Gly
       450                 455                 460
   Lys Asp Gly Ser Pro Gly Glu Pro Gly Ala Asn Gly Val Pro Gly Asn
   465                 470                 475                 480
   Pro Gly Glu Arg Gly Ala Pro Gly Phe Arg Gly Pro Ala Gly Pro Asn
                   485                 490                 495
   Gly Ala Pro Gly Glu Lys Gly Pro Ala Gly Glu Arg Gly Gly Pro Gly
               500                 505                 510
   Pro Ala Gly Pro Arg Gly Val Ala Gly Glu Pro Gly Arg Asp Gly Thr
           515                 520                 525
   Pro Gly Gly Pro Gly Ile Arg Gly Met Pro Gly Ser Pro Gly Gly Pro
       530                 535                 540
   Gly Asn Asp Gly Lys Pro Gly Pro Pro Gly Ser Gln Gly Glu Ser Gly
   545                 550                 555                 560
   Arg Pro Gly Pro Pro Gly Pro Ser Gly Pro Arg Gly Gln Pro Gly Val
                   565                 570                 575
   Met Gly Phe Pro Gly Pro Lys Gly Asn Asp Gly Ala Pro Gly Lys Asn
               580                 585                 590
   Gly Glu Arg Gly Gly Pro Gly Gly Pro Gly Leu Pro Gly Pro Ala Gly
           595                 600                 605
   Lys Asn Gly Glu Thr Gly Pro Gln Gly Pro Pro Gly Pro Thr Gly Ala
       610                 615                 620
   Pro Gly Asp Lys Gly Asp Ala Gly Pro Pro Gly Pro Gln Gly Leu Gln
   625                 630                 635                 640
   Gly Ile Pro Gly Thr Ser Gly Pro Pro Gly Glu Asn Gly Lys Pro Gly
                   645                 650                 655
   Glu Pro Gly Pro Lys Gly Glu Ala Gly Ala Pro Gly Val Pro Gly Gly
               660                 665                 670
   Lys Gly Asp Ser Gly Ala Pro Gly Glu Arg Gly Pro Pro Gly Thr Ala
           675                 680                 685
   Gly Thr Pro Gly Leu Arg Gly Gly Ala Gly Pro Pro Gly Pro Glu Gly
       690                 695                 700
   Gly Lys Gly Pro Ala Gly Pro Pro Gly Pro Pro Gly Thr Ser Gly Pro
   705                 710                 715                 720
   Pro Gly Leu Gln Gly Met Pro Gly Glu Arg Gly Gly Pro Gly Ser Pro
                   725                 730                 735
   Gly Pro Lys Gly Glu Lys Gly Glu Pro Gly Gly Ala Gly Ala Asp Gly
               740                 745                 750
   Val Pro Gly Lys Asp Gly Pro Arg Gly Pro Ala Gly Pro Ile Gly Pro
           755                 760                 765
   Pro Gly Pro Ala Gly Gln Pro Gly Asp Lys Gly Glu Gly Gly Ala Pro
       770                 775                 780
   Gly Leu Pro Gly Ile Ala Gly Pro Arg Gly Gly Pro Gly Glu Arg Gly
   785                 790                 795                 800
   Glu His Gly Pro Pro Gly Pro Ala Gly Phe Pro Gly Ala Pro Gly Gln
                   805                 810                 815
   Asn Gly Glu Pro Gly Ala Lys Gly Glu Arg Gly Ala Pro Gly Glu Lys
               820                 825                 830
   Gly Glu Gly Gly Pro Pro Gly Ala Ala Gly Pro Pro Gly Gly Ser Gly
           835                 840                 845
   Pro Ala Gly Pro Pro Gly Pro Gln Gly Val Lys Gly Glu Arg Gly Ser
       850                 855                 860
   Pro Gly Gly Pro Gly Ala Ala Gly Phe Pro Gly Gly Arg Gly Leu Pro
   865                 870                 875                 880
   Gly Pro Pro Gly Asn Asn Gly Asn Pro Gly Pro Pro Gly Pro Ser Gly
                   885                 890                 895
   Ala Pro Gly Lys Asp Gly Pro Pro Gly Pro Ala Gly Asn Ser Gly Ser
               900                 905                 910
   Pro Gly Asn Pro Gly Val Ala Gly Pro Lys Gly Asp Ala Gly Gln Pro
           915                 920                 925
   Gly Glu Lys Gly Pro Pro Gly Ala Gln Gly Pro Pro Gly Ser Pro Gly
       930                 935                 940
   Pro Leu Gly Ile Ala Gly Leu Thr Gly Ala Arg Gly Leu Ala Gly Pro
   945                 950                 955                 960
   Pro Gly Met Pro Gly Pro Arg Gly Ser Pro Gly Pro Gln Gly Ile Lys
                   965                 970                 975
   Gly Glu Ser Gly Lys Pro Gly Ala Ser Gly His Asn Gly Glu Arg Gly
   980 985 990
   Pro Pro Gly Pro Gln Gly Leu Pro Gly Gln Pro Gly Thr Ala Gly Glu
   995 1000 1005
   Pro Gly Arg Asp Gly Asn Pro Gly Ser Asp Gly Gln Pro Gly Arg Asp
   1010 1015 1020
   Gly Ser Pro Gly Gly Lys Gly Asp Arg Gly Glu Asn Gly Ser Pro Gly
   1025 1030 1035 1040
   Ala Pro Gly Ala Pro Gly His Pro Gly Pro Pro Gly Pro Val Gly Pro
   1045 1050 1055
   Ser Gly Lys Asn Gly Asp Arg Gly Glu Thr Gly Pro Ala Gly Pro Ser
   1060 1065 1070
   Gly Ala Pro Gly Pro Ala Gly Ala Arg Gly Ala Pro Gly Pro Gln Gly
   1075 1080 1085
   Pro Arg Gly Asp Lys Gly Glu Thr Gly Glu Arg Gly Ser Asn Gly Ile
   1090 1095 1100
   Lys Gly His Arg Gly Phe Pro Gly Asn Pro Gly Pro Pro Gly Ser Pro
   1105 1110 1115 1120
   Gly Ala Ala Gly His Gln Gly Ala Val Gly Ser Pro Gly Pro Ala Gly
   1125 1130 1135
   Pro Arg Gly Pro Val Gly Pro His Gly Pro Pro Gly Lys Asp Gly Ser
   1140 1145 1150
   Ser Gly His Pro Gly Pro Ile Gly Pro Pro Gly Pro Arg Gly Asn Arg
   1155 1160 1165
   Gly Glu Arg Gly Ser Glu Gly Ser Pro Gly His Pro Gly Gln Pro Gly
   1170 1175 1180
   Pro Pro Gly Pro Pro Gly Ala Pro Gly Pro Cys Cys Gly Gly Gly Ala
   1185 1190 1195 1200
   Ala Ile Ala Gly Val Gly Gly Glu Lys Ser Gly Gly Phe Ser Pro Tyr
   1205 1210 1215
   Tyr Gly Asp Asp Pro Met Asp Phe Lys Ile Asn Thr Glu Glu Ile Met
   1220 1225 1230
   Ser Ser Leu Lys Ser Val Asn Gly Gln Ile Glu Ser Leu Ile Ser Pro
   1235 1240 1245
   Asp Gly Ser Arg Lys Asn Pro Ala Arg Asn Cys Arg Asp Leu Lys Phe
   1250 1255 1260
   Cys His Pro Glu Leu Lys Ser Gly Glu Tyr Trp Val Asp Pro Asn Gln
   1265 1270 1275 1280
   Gly Cys Lys Met Asp Ala Ile Lys Val Phe Cys Asn Met Glu Thr Gly
   1285 1290 1295
   Glu Thr Cys Ile Asn Ala Ser Pro Met Thr Val Pro Arg Lys His Trp
   1300 1305 1310
   Trp Thr Asp Ala Gly Ala Glu Lys Lys His Val Trp Phe Gly Glu Ser
   1315 1320 1325
   Met Asn Gly Gly Phe Gln Phe Ser Tyr Gly Asn Pro Asp Leu Pro Glu
   1330 1335 1340
   Asp Val Leu Asp Val Gln Leu Ala Phe Leu Arg Leu Leu Ser Ser Arg
   1345 1350 1355 1360
   Ala Ser Gln Asn Ile Thr Tyr His Cys Lys Asn Ser Ile Ala Tyr Met
   1365 1370 1375
   Asp Gln Ala Asn Gly Asn Val Lys Lys Ser Leu Lys Leu Met Gly Ser
   1380 1385 1390
   Asn Glu Gly Glu Phe Lys Ala Glu Gly Asn Ser Lys Phe Thr Tyr Thr
   1395 1400 1405
   Val Leu Glu Asp Gly Cys Thr Lys His Thr Gly Glu Trp Ser Lys Thr
   1410 1415 1420
   Val Phe Glu Tyr Gln Thr Arg Lys Ala Met Arg Leu Pro Ile Ile Asp
   1425 1430 1435 1440
   Ile Ala Pro Tyr Asp Ile Gly Gly Pro Asp Gln Glu Phe Gly Val Asp
   1445 1450 1455
   Ile Gly Pro Val Cys Phe Leu
   1460
<210> 81
   <211> 1388
   <212> DNA
   <213> Rattus norvegicus
<220>
   <221> source
   <222> 1..1388
   <223> /mol_type="DNA"
   /note="Rattus norvegicus Oct-4/POU5F1"
   /organism="Rattus norvegicus"
<400> 81
   ggggagggag agaggtgcaa acagtcccta ggtgagtcgt ccttccacca ggcccccggc      60
   tcggggagcc caccttcccc atggctggac acctggcttc agacttcgcc ttctcacccc     120
   cacctggtgg gggtgatggg tcagcagggc tggagccggg ctgggtggac cctcgaacct     180
   ggctaagctt ccaggggcct ccaagtgggc ctggaatcgg accaggttca gaggtgctgg     240
   ggatctcccc gtgtccccca gcatacgagt tctgtggagg gatggcatac tgtggacctc     300
   aggttggact gggcctagtc ccccaagttg gcgtggagac tctgcagccc gagggccagg     360
   caggagcacg agtggagagc aactcggagg gagcctcctc tgggccctgt actgcccgcc     420
   ccagcgccgt gaagttggag aaggtggaac ctagtcccga ggagtcccag gatatgaaag     480
   ccctgcagaa ggagctagag cagtttgcca agctgctgaa acagaagagg atcaccttgg     540
   ggtacaccca ggccgacgtg gggctcaccc tgggcgttct ctttggaaag gtgttcagcc     600
   agacaaccat ctgccgcttc gaggccctgc agctcagcct taagaacatg tgtaagctgc     660
   ggcccctgct ggagaagtgg gtggaggaag ctgacaacaa cgagaacctt caggagatat     720
   gcaaatcgga gaccctggtg caggcccgga agagaaagcg gactagcatt gagaaccgtg     780
   tgaggtggaa cctggagaac atgtttctgc agtgcccgaa gccctccctg cagcagatca     840
   ctagcattgc caagcagctt gggctggaga gggatgtggt tcgagtgtgg ttctgtaacc     900
   ggcgccagaa ggggaaaaga tcgagcattg aatattccca acgagaagag tatgaggccg     960
   cggggaaacc tttcccaggg ggggctgtgt cctttcctct gcccccaggc ccccactttg     1020
   gtgctccagg ctatgggagc ccccatttca ccacactcta ctcggtccct tttcctgagg    1080
   gcgaggcctt tccctctgtt cctgtcactg ctctgggctc tcccatgcat tcaaactgag    1140
   gcaccagccc tccctgggga tgaggtgagc caaggcaagg aagggaggta gacaaagaga    1200
   acctgaagct ttggggccta gattctttta ctggggagga attgaaagca caaacagggg    1260
   tggttggggg ggcggggggg gagagatgaa gttcagtgac gctgttgatc aggaacccgt    1320
   ccgaagttct taagttctta aataaagtct gggatacaca gtaaaaaaaa aaaaaaaaaa    1380
   aaaaaaaa                                                             1388
<210> 82
   <211> 352
   <212> PRT
   <213> Rattus norvegicus
<220>
   <221> SOURCE
   <222> 1..352
   <223> /mol_type="protein"
   /note="Rattus norvegicus Oct-4"
   /organism="Rattus norvegicus"
<400> 82
   Met Ala Gly His Leu Ala Ser Asp Phe Ala Phe Ser Pro Pro Pro Gly
   1               5                   10                  15
   Gly Gly Asp Gly Ser Ala Gly Leu Glu Pro Gly Trp Val Asp Pro Arg
               20                  25                  30
   Thr Trp Leu Ser Phe Gln Gly Pro Pro Ser Gly Pro Gly Ile Gly Pro
           35                  40                  45
   Gly Ser Glu Val Leu Gly Ile Ser Pro Cys Pro Pro Ala Tyr Glu Phe
       50                  55                  60
   Cys Gly Gly Met Ala Tyr Cys Gly Pro Gln Val Gly Leu Gly Leu Val
   65                  70                  75                  80
   Pro Gln Val Gly Val Glu Thr Leu Gln Pro Glu Gly Gln Ala Gly Ala
                   85                  90                  95
   Arg Val Glu Ser Asn Ser Glu Gly Ala Ser Ser Gly Pro Cys Thr Ala
               100                 105                 110
   Arg Pro Ser Ala Val Lys Leu Glu Lys Val Glu Pro Ser Pro Glu Glu
           115                 120                 125
   Ser Gln Asp Met Lys Ala Leu Gln Lys Glu Leu Glu Gln Phe Ala Lys
       130                 135                 140
   Leu Leu Lys Gln Lys Arg Ile Thr Leu Gly Tyr Thr Gln Ala Asp Val
   145                 150                 155                 160
   Gly Leu Thr Leu Gly Val Leu Phe Gly Lys Val Phe Ser Gln Thr Thr
                   165                 170                 175
   Ile Cys Arg Phe Glu Ala Leu Gln Leu Ser Leu Lys Asn Met Cys Lys
               180                 185                 190
   Leu Arg Pro Leu Leu Glu Lys Trp Val Glu Glu Ala Asp Asn Asn Glu
           195                 200                 205
   Asn Leu Gln Glu Ile Cys Lys Ser Glu Thr Leu Val Gln Ala Arg Lys
       210                 215                 220
   Arg Lys Arg Thr Ser Ile Glu Asn Arg Val Arg Trp Asn Leu Glu Asn
   225                 230                 235                 240
   Met Phe Leu Gln Cys Pro Lys Pro Ser Leu Gln Gln Ile Thr Ser Ile
                   245                 250                 255
   Ala Lys Gln Leu Gly Leu Glu Arg Asp Val Val Arg Val Trp Phe Cys
               260                 265                 270
   Asn Arg Arg Gln Lys Gly Lys Arg Ser Ser Ile Glu Tyr Ser Gln Arg
           275                 280                 285
   Glu Glu Tyr Glu Ala Ala Gly Lys Pro Phe Pro Gly Gly Ala Val Ser
       290                 295                 300
   Phe Pro Leu Pro Pro Gly Pro His Phe Gly Ala Pro Gly Tyr Gly Ser
   305                 310                 315                 320
   Pro His Phe Thr Thr Leu Tyr Ser Val Pro Phe Pro Glu Gly Glu Ala
                   325                 330                 335
   Phe Pro Ser Val Pro Val Thr Ala Leu Gly Ser Pro Met His Ser Asn
               340                 345                 350
<210> 83
   <211> 2057
   <212> DNA
   <213> Rattus norvegicus
<220>
   <221> source
   <222> 1..2057
   <223> /mol_type="DNA"
   /note="Rattus norvegicus Gale"
   /organism="Rattus norvegicus"
<400> 83
   atgtaacagc cgacctaagg cttcccagca acgccaagca aaagtcatga ccgccgccgc      60
   gggctcggcg agccgtgtcg cggtgccctt gttgttgtgc gcgatgctgg cgcccagcgg     120
   cgcctacgag ctagacgact ccgacgggct gggcctggag ttcgatggca tcggcgcagt     180
   cagcggcggc ggggcaacct cccggcttct agtaaattac ccagagccct atcgttctga     240
   aatactggat tatctattta agccaaactt cggtgcctct ctgcatattc taaaagtgga     300
   aataggtggc gatggacaaa caacagacgg cactgaaccc tcccacatgc actatgagct     360
   agatgagaat tatttccgag gctatgagtg gtggctaatg aaggaagcta agaagaggaa     420
   cccaaatatt atacttatgg ggttgccatg gtcattccct ggatggctgg gcaaaggctt     480
   cagttggcct tatgtaaatc ttcagctgac tgccttctat attgtgagat ggattttggg     540
   tgccaagcat tatcatgacc tggacattga ttatattggg atatggaatg aaaggccgtt     600
   tgatgccaac tatataaagg aattaagaaa aatgcttgat tatgaaggtc tccagcgagt     660
   gagaatcata gcaagtgata atctctggga gccaatttcc tcttctgtgc tgcttgacca     720
   ggagctctgg aaggtggttg atgttatagg agctcattat cccggaacct acacagtctg     780
   gaatgcaaag atgtcaggga agaagctttg gtcgtcggaa gactttagca ctgtcaacag     840
   taatgttggc gcaggctgtt ggggtcgcat attgaaccag aattacatca acggcaatat     900
   gaccgccaca attgcttgga atttggtggc tagttactat gaagaattgc cctatggacg     960
   aagtggattg atgacagccc aggagccgtg gagtggacat tatgtagtag catctcctat    1020
   ctgggtatca gctcatacaa ctcaatttac tcaaccaggc tggtattacc tgaagacagt    1080
   tggccatcta gagaaaggag gaagctatgt agctctgact gatggcttgg ggaatttcac    1140
   catcatcgtt gaaactatga gtcgtcaaca ttctatgtgt atacggccat atctaccgta    1200
   ttataatgtc tcacgtcagc tggccacctt cattctaaag gggtctttga aagaaataca    1260
   agagctgcag gtgtggtaca ccaagcttgg aacaacacca gagaaacttc attttaaaca    1320
   actggaaact ctatggctcc ttgacggcag cggcagtttc tcactggaac tggaggagga    1380
   tgagatgttc acactcacca ctctgaccac agggcacaaa ggcagctacc gtccccctcc    1440
   caagtcacag cccttcccaa ccagctataa ggatgatttc aacgttgagt acccattatt    1500
   tagtgaagct ccgaattttg ctgatcagac cggcgtgttc gagtactaca cgaataacga    1560
   agaccttgag catcgcttca cactgcgcca agttctcaac caacgaccta ttacctgggc    1620
   cgcggatgcc tccagcacga tcagtgtcat aggcgatcac cactggagca acatgactgt    1680
   gcagtgtgat gtttacatag agactcctcg gactggcggt gtgttcattg ctggaagagt 1740
   gaataaaggt ggtatcttga tcagaactgc ttcaggagtt ttcttctgga tttttgcaaa 1800
   tggctcttac agagttacag ccgacctggg tgggtggata acatatgctt caggacatgc 1860
   tgacgttaca gcaaagcgat ggtatacact caccttaggc attaagggtt atctcgcctc 1920
   tggtatgttg aacggcaaga tcctgtggga aaatgtgccc gtgaaatacc cagggcatgg 1980
   ctgggctgcg atcggaacgc ataccttcga gtttgcgcag tttgacaact ttcatgtgga 2040
   agctgctcgc taatatt 2057
<210> 84
   <211> 668
   <212> PRT
   <213> Rattus norvegicus
<220>
   <221> SOURCE
   <222> 1..668
   <223> /mol_type="protein"
   /note="Rattus norvegicus Gale"
   /organism="Rattus norvegicus"
<400> 84
   Met Thr Ala Ala Ala Gly Ser Ala Ser Arg Val Ala Val Pro Leu Leu
   1 5 10 15
   Leu Cys Ala Met Leu Ala Pro Ser Gly Ala Tyr Glu Leu Asp Asp Ser
   20 25 30
   Asp Gly Leu Gly Leu Glu Phe Asp Gly Ile Gly Ala Val Ser Gly Gly
   35 40 45
   Gly Ala Thr Ser Arg Leu Leu Val Asn Tyr Pro Glu Pro Tyr Arg Ser
   50 55 60
   Glu Ile Leu Asp Tyr Leu Phe Lys Pro Asn Phe Gly Ala Ser Leu His
   65 70 75 80
   Ile Leu Lys Val Glu Ile Gly Gly Asp Gly Gln Thr Thr Asp Gly Thr
   85 90 95
   Glu Pro Ser His Met His Tyr Glu Leu Asp Glu Asn Tyr Phe Arg Gly
   100 105 110
   Tyr Glu Trp Trp Leu Met Lys Glu Ala Lys Lys Arg Asn Pro Asn Ile
   115 120 125
   Ile Leu Met Gly Leu Pro Trp Ser Phe Pro Gly Trp Leu Gly Lys Gly
   130 135 140
   Phe Ser Trp Pro Tyr Val Asn Leu Gln Leu Thr Ala Phe Tyr Ile Val
   145 150 155 160
   Arg Trp Ile Leu Gly Ala Lys His Tyr His Asp Leu Asp Ile Asp Tyr
   165 170 175
   Ile Gly Ile Trp Asn Glu Arg Pro Phe Asp Ala Asn Tyr Ile Lys Glu
   180 185 190
   Leu Arg Lys Met Leu Asp Tyr Glu Gly Leu Gln Arg Val Arg Ile Ile
   195 200 205
   Ala Ser Asp Asn Leu Trp Glu Pro Ile Ser Ser Ser Val Leu Leu Asp
   210 215 220
   Gln Glu Leu Trp Lys Val Val Asp Val Ile Gly Ala His Tyr Pro Gly
   225 230 235 240
   Thr Tyr Thr Val Trp Asn Ala Lys Met Ser Gly Lys Lys Leu Trp Ser
   245 250 255
   Ser Glu Asp Phe Ser Thr Val Asn Ser Asn Val Gly Ala Gly Cys Trp
   260 265 270
   Gly Arg Ile Leu Asn Gln Asn Tyr Ile Asn Gly Asn Met Thr Ala Thr
           275                     280                 285
   Ile Ala Trp Asn Leu Val Ala Ser Tyr Tyr Glu Glu Leu Pro Tyr Gly
       290                     295                 300
   Arg Ser Gly Leu Met Thr Ala Gln Glu Pro Trp Ser Gly His Tyr Val
   305                 310                 315                 320
   Val Ala Ser Pro Ile Trp Val Ser Ala His Thr Thr Gln Phe Thr Gln
                   325                 330                 335
   Pro Gly Trp Tyr Tyr Leu Lys Thr Val Gly His Leu Glu Lys Gly Gly
               340                 345                 350
   Ser Tyr Val Ala Leu Thr Asp Gly Leu Gly Asn Phe Thr Ile Ile Val
           355                 360                 365
   Glu Thr Met Ser Arg Gln His Ser Met Cys Ile Arg Pro Tyr Leu Pro
       370                 375                 380
   Tyr Tyr Asn Val Ser Arg Gln Leu Ala Thr Phe Ile Leu Lys Gly Ser
   385                 390                 395                 400
   Leu Lys Glu Ile Gln Glu Leu Gln Val Trp Tyr Thr Lys Leu Gly Thr
                   405                 410                 415
   Thr Pro Glu Lys Leu His Phe Lys Gln Leu Glu Thr Leu Trp Leu Leu
               420                 425                 430
   Asp Gly Ser Gly Ser Phe Ser Leu Glu Leu Glu Glu Asp Glu Met Phe
           435                 440                 445
   Thr Leu Thr Thr Leu Thr Thr Gly His Lys Gly Ser Tyr Arg Pro Pro
       450                 455                 460
   Pro Lys Ser Gln Pro Phe Pro Thr Ser Tyr Lys Asp Asp Phe Asn Val
   465                 470                 475                 480
   Glu Tyr Pro Leu Phe Ser Glu Ala Pro Asn Phe Ala Asp Gln Thr Gly
                   485                 490                 495
   Val Phe Glu Tyr Tyr Thr Asn Asn Glu Asp Leu Glu His Arg Phe Thr
               500                 505                 510
   Leu Arg Gln Val Leu Asn Gln Arg Pro Ile Thr Trp Ala Ala Asp Ala
           515                 520                 525
   Ser Ser Thr Ile Ser Val Ile Gly Asp His His Trp Ser Asn Met Thr
       530                 535                 540
   Val Gln Cys Asp Val Tyr Ile Glu Thr Pro Arg Thr Gly Gly Val Phe
   545                 550                 555                 560
   Ile Ala Gly Arg Val Asn Lys Gly Gly Ile Leu Ile Arg Thr Ala Ser
                   565                 570                 575
   Gly Val Phe Phe Trp Ile Phe Ala Asn Gly Ser Tyr Arg Val Thr Ala
               580                 585                 590
   Asp Leu Gly Gly Trp Ile Thr Tyr Ala Ser Gly His Ala Asp Val Thr
           595                 600                 605
   Ala Lys Arg Trp Tyr Thr Leu Thr Leu Gly Ile Lys Gly Tyr Leu Ala
       610                 615                 620
   Ser Gly Met Leu Asn Gly Lys Ile Leu Trp Glu Asn Val Pro Val Lys
   625                 630                 635                 640
   Tyr Pro Gly His Gly Trp Ala Ala Ile Gly Thr His Thr Phe Glu Phe
                   645                 650                 655
   Ala Gln Phe Asp Asn Phe His Val Glu Ala Ala Arg
               660                 665
<210> 85
   <211> 1134
   <212> DNA
   <213> Rattus norvegicus
<220>
   <221> source
   <222> 1..1134
   <223> /mol_type="DNA"
   /note="Rattus norvegicus Scleraxis"
   /organism="Rattus norvegicus"
<400> 85
   gatccgctgg cggccccact ccagtccgaa cacatgtgcc cgcctcgccc ggcagccacc      60
   accgcggggc gcagcggaga cccctggccc ggcggcctgt ggggacctaa agaggcggca     120
   tgagcagcgc accgtggagc tgacgccgcg ccccctgccc ggcccatgtc cttcgccatg     180
   ctgcgttcag cgccaccgcc gggccgctac ctgtaccctg aggtgagccc gctgtccgag     240
   gatgaggacc gcggaagcga gagctcgggc tccgacgaaa aaccctgtcg tgttcatgct     300
   gcgcgctgcg gcctccaggg cgcccggcgg cgggcaggag gacggagggc cgcgggtagt     360
   gggccaggac ccggggggcg gccaggccgc gagccccggc agcggcacac agcgaatgcg     420
   cgcgagcggg accgcaccaa cagcgtgaac acggccttca ctgcgctgcg cacactcatc     480
   ccgaccgagc cagcagatcg caagctctcc aagattgaga cgctgcgcct ggcctccagc     540
   tacatttctc acctgggcaa cgtgctactg gtgggtgaag cctgcggtga cgggcagccc     600
   tgccactcgg gacccgcttt cttccacagc ggtcgtgcgg gcagcccact accaccgcca     660
   ccgccgccgc ccccactacc actggccaga gacggcggcg agaacaccca gcccaaacag     720
   atctgcacct tctgcctcag caaccagaga aagttgagca aagaccgtga cagaaagacg     780
   gcgattcgaa gttagaagga ggagggtcca cagcagccaa gaggtgatgc cactagtgaa     840
   ggtggaagcc actgaagagt catggagagc cccaccttgg acctgatctg agcacaggtc     900
   cccaagagcg cgctcaccag gccagacctg ccccagctct gagcagggcc tgtgaacaga     960
   gagatggaca ggcgggctat ggcttgactc taagtagctg aacacttgta cccaggccca    1020
   ctggaacttt ccatgctggc tctctacctg ggccaagtgt tggcatcttg tgtctttaat    1080
   atgataatat aaagtctgga aattttgtat aattaaagtg aaacagtatc ttcc          1134
<210> 86
   <211> 209
   <212> PRT
   <213> Rattus norvegicus
<220>
   <221> SOURCE
   <222> 1..209
   <223> /mol_type="protein"
   /note="Rattus norvegicus Scleraxis"
   /organism="Rattus norvegicus"
<400> 86
   Met Ser Phe Ala Met Leu Arg Ser Ala Pro Pro Pro Gly Arg Tyr Leu
   1               5                   10                  15
   Tyr Pro Glu Val Ser Pro Leu Ser Glu Asp Glu Asp Arg Gly Ser Glu
               20                  25                  30
   Ser Ser Gly Ser Asp Glu Lys Pro Cys Arg Val His Ala Ala Arg Cys
           35                  40                  45
   Gly Leu Gln Gly Ala Arg Arg Arg Ala Gly Gly Arg Arg Ala Ala Gly
       50                  55                  60
   Ser Gly Pro Gly Pro Gly Gly Arg Pro Gly Arg Glu Pro Arg Gln Arg
   65                  70                  75                  80
   His Thr Ala Asn Ala Arg Glu Arg Asp Arg Thr Asn Ser Val Asn Thr
                   85                  90                  95
   Ala Phe Thr Ala Leu Arg Thr Leu Ile Pro Thr Glu Pro Ala Asp Arg
               100                 105                 110
   Lys Leu Ser Lys Ile Glu Thr Leu Arg Leu Ala Ser Ser Tyr Ile Ser
           115                 120                 125
   His Leu Gly Asn Val Leu Leu Val Gly Glu Ala Cys Gly Asp Gly Gln
       130                 135                 140
   Pro Cys His Ser Gly Pro Ala Phe Phe His Ser Gly Arg Ala Gly Ser
   145                 150                 155                 160
   Pro Leu Pro Pro Pro Pro Pro Pro Pro Pro Leu Pro Leu Ala Arg Asp
                   165                 170                 175
   Gly Gly Glu Asn Thr Gln Pro Lys Gln Ile Cys Thr Phe Cys Leu Ser
               180                 185                 190
   Asn Gln Arg Lys Leu Ser Lys Asp Arg Asp Arg Lys Thr Ala Ile Arg
           195                 200                 205
   Ser
<210> 87
   <211> 885
   <212> DNA
   <213> Rattus norvegicus
<220>
   <221> source
   <222> 1..885
   <223> /mol_type="DNA"
   /note="Rattus norvegicus Sox-9"
   /organism="Rattus norvegicus"
<400> 87
   atgaagtcac tccctcccac ctcctccagg cgcctccccc cacctgatgg agccctgtgt 60
   ctaagaagag actctgggct gggagccgga gagctgagag cgttgctcgg aactgtctgg 120
   aaacttcagt gggagcgaca actttaccag tttcggtcca ggaacttttc tttgcaaaag 180
   agacggggtg caagtagccc tggtttcgtt ctctgttttc cctccctcct cctccgctcc 240
   gattcgcctt ccccgggttt ggagcgggca actgagcact ggccacccag cgcctctgct 300
   aagtgctcgc cgccgcagcc gggtgacgcg ccaacctccc cgggagccgt tcgctcggcg 360
   tccgcgtccg ggcagctgag ggaagaggag ccccagcagc cgcggcttct ctcctttccc 420
   ggccacccgc cccctgcccc gggctcgcgt atgaatctcc tggacccctt catgaagatg 480
   accgacgagc aggagaaggg cttgtctggc gcccccagcc ccaccatgtc ggaggactcg 540
   gctggttcgc cctgcccctc gggctcaggc tccgacacgg agaacacacg gccccaggag 600
   aacacgttcc ccaagggcga gccggatctg aagaaggaga gcgaggaaga taaattccca 660
   gtgtgcatcc gcgaggcggt cagccaggtg ctgaagggct acgactggac cctggtgccc 720
   atgccggtgc gcgtcaacgg ctccagcaag aacaagccac acgtcaagcg gcccatgaac 780
   gccttcatgg tgtgggcgca ggctgcgcgc aggaagctgg cagaccagta cccgcatctg 840
   cacaacgcgg agctcagcaa gactctgggc aagctctgga ggtga 885
<210> 88
   <211> 294
   <212> PRT
   <213> Rattus norvegicus
<220>
   <221> SOURCE
   <222> 1..294
   <223> /mol_type="protein"
   /note="Rattus norvegicus Sox-9"
   /organism="Rattus norvegicus"
<400> 88
   Met Lys Ser Leu Pro Pro Thr Ser Ser Arg Arg Leu Pro Pro Pro Asp
   1               5                   10                  15
   Gly Ala Leu Cys Leu Arg Arg Asp Ser Gly Leu Gly Ala Gly Glu Leu
               20                  25                  30
   Arg Ala Leu Leu Gly Thr Val Trp Lys Leu Gln Trp Glu Arg Gln Leu
           35                  40                  45
   Tyr Gln Phe Arg Ser Arg Asn Phe Ser Leu Gln Lys Arg Arg Gly Ala
       50                  55                  60
   Ser Ser Pro Gly Phe Val Leu Cys Phe Pro Ser Leu Leu Leu Arg Ser
   65                  70                  75                  80
   Asp Ser Pro Ser Pro Gly Leu Glu Arg Ala Thr Glu His Trp Pro Pro
                   85                  90                  95
   Ser Ala Ser Ala Lys Cys Ser Pro Pro Gln Pro Gly Asp Ala Pro Thr
               100                 105                 110
   Ser Pro Gly Ala Val Arg Ser Ala Ser Ala Ser Gly Gln Leu Arg Glu
           115                 120                 125
   Glu Glu Pro Gln Gln Pro Arg Leu Leu Ser Phe Pro Gly His Pro Pro
       130                 135                 140
   Pro Ala Pro Gly Ser Arg Met Asn Leu Leu Asp Pro Phe Met Lys Met
   145                 150                 155                 160
   Thr Asp Glu Gln Glu Lys Gly Leu Ser Gly Ala Pro Ser Pro Thr Met
                   165                 170                 175
   Ser Glu Asp Ser Ala Gly Ser Pro Cys Pro Ser Gly Ser Gly Ser Asp
               180                 185                 190
   Thr Glu Asn Thr Arg Pro Gln Glu Asn Thr Phe Pro Lys Gly Glu Pro
           195                 200                 205
   Asp Leu Lys Lys Glu Ser Glu Glu Asp Lys Phe Pro Val Cys Ile Arg
       210                 215                 220
   Glu Ala Val Ser Gln Val Leu Lys Gly Tyr Asp Trp Thr Leu Val Pro
   225                 230                 235                 240
   Met Pro Val Arg Val Asn Gly Ser Ser Lys Asn Lys Pro His Val Lys
                   245                 250                 255
   Arg Pro Met Asn Ala Phe Met Val Trp Ala Gln Ala Ala Arg Arg Lys
               260                 265                 270
   Leu Ala Asp Gln Tyr Pro His Leu His Asn Ala Glu Leu Ser Lys Thr
           275                 280                 285
   Leu Gly Lys Leu Trp Arg
       290
<210> 89
   <211> 2355
   <212> DNA
   <213> Rattus norvegicus
<220>
   <221> source
   <222> 1..2355
   <223> /mol_type="DNA"
   /note="Rattus norvegicus c-Myc"
   /organism="Rattus norvegicus"

<210> 90
   <211> 453
   <212> PRT
   <213> Rattus norvegicus
<220>
   <221> SOURCE
   <222> 1..453
   <223> /mol_type="protein"
   /note="Rattus norvegicus c-Myc"
   /organism="Rattus norvegicus"
<400> 90
   Met Asn Phe Leu Trp Glu Val Glu Asn Pro Thr Val Thr Thr Met Pro
   1               5                   10                  15
   Leu Asn Val Ser Phe Ala Asn Arg Asn Tyr Asp Leu Asp Tyr Asp Ser
               20                  25                  30
   Val Gln Pro Tyr Phe Ile Cys Asp Glu Glu Glu Asn Phe Tyr His Gln
           35                  40                  45
   Gln Gln Gln Ser Glu Leu Gln Pro Pro Ala Pro Ser Glu Asp Ile Trp
       50                  55                  60
   Lys Lys Phe Glu Leu Leu Pro Thr Pro Pro Leu Ser Pro Ser Arg Arg
   65                  70                  75                  80
   Ser Gly Leu Cys Ser Pro Ser Tyr Val Ala Val Ala Thr Ser Phe Ser
                   85                  90                  95
   Pro Arg Glu Asp Asp Asp Gly Gly Gly Gly Asn Phe Ser Thr Ala Asp
               100                 105                 110
   Gln Leu Glu Met Met Thr Glu Leu Leu Gly Gly Asp Met Val Asn Gln
           115                 120                 125
   Ser Phe Ile Cys Asp Pro Asp Asp Glu Thr Phe Ile Lys Asn Ile Ile
       130                 135                 140
   Ile Gln Asp Cys Met Trp Ser Gly Phe Ser Ala Ala Ala Lys Leu Val
   145                 150                 155                 160
   Ser Glu Lys Leu Ala Ser Tyr Gln Ala Ala Arg Lys Asp Ser Thr Ser
                   165                 170                 175
   Leu Ser Pro Ala Arg Gly His Ser Val Cys Ser Thr Ser Ser Leu Tyr
               180                 185                 190
   Leu Gln Asp Leu Thr Ala Ala Ala Ser Glu Cys Ile Asp Pro Ser Val
           195                 200                 205
   Val Phe Pro Tyr Pro Leu Asn Asp Ser Ser Ser Pro Lys Ser Cys Thr
       210                 215                 220
   Ser Ser Asp Ser Thr Ala Phe Ser Ser Ser Ser Asp Ser Leu Leu Ser
   225                 230                 235                 240
   Ser Glu Ser Ser Pro Arg Ala Thr Pro Glu Pro Leu Val Leu His Glu
                   245                 250                 255
   Glu Thr Pro Pro Thr Thr Ser Ser Asp Ser Glu Glu Glu Gln Asp Asp
               260                 265                 270
   Glu Glu Glu Ile Asp Val Val Ser Val Glu Lys Arg Gln Pro Pro Ala
           275                 280                 285
   Lys Arg Ser Glu Ser Gly Ser Ser Pro Ser Arg Gly His Ser Lys Pro
       290                 295                 300
   Pro His Ser Pro Leu Val Leu Lys Arg Cys His Val Ser Thr His Gln
   305                 310                 315                 320
   His Asn Tyr Ala Ala Pro Pro Ser Thr Arg Lys Asp Tyr Pro Ala Ala
                   325                 330                 335
   Lys Arg Ala Lys Leu Asp Ser Gly Arg Val Leu Lys Gln Ile Ser Asn
               340                 345                 350
   Asn Arg Lys Cys Ser Ser Pro Arg Ser Ser Asp Thr Glu Glu Asn Asp
           355                 360                 365
   Lys Arg Arg Thr His Asn Val Leu Glu Arg Gln Arg Arg Asn Glu Leu
       370                 375                 380
   Lys Arg Ser Phe Phe Ala Leu Arg Asp Gln Ile Pro Glu Leu Glu Asn
   385                 390                 395                 400
   Asn Glu Lys Ala Pro Lys Val Val Ile Leu Lys Lys Ala Thr Ala Tyr
                   405                 410                 415
   Ile Leu Ser Val Gln Ala Asp Glu His Lys Leu Ile Ser Glu Lys Asp
               420                 425                 430
   Leu Leu Arg Lys Arg Arg Glu Gln Leu Lys His Lys Leu Glu Gln Leu
           435                 440                 445
   Arg Asn Ser Gly Ala
       450
<210> 91
   <211> 2393
   <212> DNA
   <213> Rattus norvegicus
<220>
   <221> source
   <222> 1..2393
   <223> /mol_type="DNA"
   /note="Rattus norvegicus Klf-4"
   /organism="Rattus norvegicus"
<400> 91
   atcttcgttg acttcggggt ttgggtaccc ctctctcttc ttcggactcc ggaggacctt        60
   ctgggccccc acattaatga ggcagccacc tggcgagtct gacatggctg tcagcgacgc       120
   tctgctcccg tccttctcca cgttcgcgtc cggcccggcg ggaagggaga agacactgcg       180
   tccagcaggt gccccgacta accgttggcg agaggaactc tctcacatga agcgacttcc       240
   cccacttccc ggccgcccct acgacctggc ggcgacggtg gccacagacc tggaaagtgg       300
   tggagctggt gcagcttgca gcagtaacaa cccggcccta ccccggaggg agaccgagga       360
   gttcaacgat ctcctggacc tagactttat cctttccaac tcgctatccc accaggaatc       420
   ggtggccgcc accgtgacca cctcggcgtc agcttcatcc tcgtcttccc cagctagcag       480
   cggccctgcc agcgcgccct ccacctgcag cttcagctat ccgatccggg ccgggggtga       540
   cccgggcgtg gctgcgggca acacaggtgg agggctcctc tacagccgag aatctgcgcc       600
   acctcccacg gcccccttca acctggcgga catcaatgac gtgagcccct cgggcggctt       660
   cgtggctgag ctcctgcggc cggagttgga cccagtatac attccgccac agcagcctca 720
   gccgccaggt ggcgggctga tgggcaagtt tgtgctgaag gcgtctctga gcacccctgg 780
   cagcgagtac accagccctt cggtcatcag tgttagcaaa ggaagcccag acggcagcca 840
   ccctgtggta gtggcgccct acagcggtgg cccgccgcgt atgtgcccca agattaagca 900
   agaggccgtc ccgtcctgca cggtcagccg gtccctagag gcccacttga gcgctggacc 960
   ccagctcagc aacggccaca ggcccaacac acacgacttc cccctggggc ggcagctccc 1020
   caccaggact acccctacac tgagtcccga ggaactgctg aacagcaggg actgtcaccc 1080
   tggcctgcct cttcccccag gattccatcc ccatccgggg cccagctacc ctcctttcct 1140
   gccagaccag atgcagtcgc aagtcccctc tctccattat caagagctca tgccaccggg 1200
   atcctgcctg ccagaggagc ccaagccaaa gaggggaaga aggtcttggc cccggaaaag 1260
   aacagccacc cacacttgtg actatgcagg ctgtggcaaa acctatacga agagttctca 1320
   tctcaaggca cacctgcgaa ctcacacagg cgagaaacct taccactgtg actgggacgg 1380
   ctgtgggtgg aaattcgccc gctcagatga actgaccagg cactaccgca aacacaccgg 1440
   gcaccggccc tttcagtgcc agaagtgcga cagggccttt tccaggtcgg accaccttgc 1500
   cttacacatg aagaggcact tttaaattcc acatcgtgga catgacccac actgccagga 1560
   gagagttcag tatttttttt taacctttca cactgtcttc ccacgagggg aggagcccag 1620
   ctggcaagcg ctacaatcat ggtcaagttc ccagcaagtc agcttgtgaa tggataatca 1680
   ggagaaagga agagtccaag ggacaaaaga aaagaaaaga aaaaaatact aaaaaacaaa 1740
   caaacaaaaa aaaaaaacaa aagaaaaaaa tcacagaaca gatggggtct gagactggat 1800
   cttctatcat tccaatacca aatccgactt gaacaagact ggacttacaa aatgccaagg 1860
   ggtgactgga agtttgtgga tatcagggta tacattaaat cagtgacctg gggggaggga 1920
   agaccagagt tcccttgaat tgtgcttcaa tgatgcaata tacatggaaa gaccaccttg 1980
   tatgctcttt gccttctaaa aagccattat gacgtcagag gaagaggaag caattcaggt 2040
   acagaacgtg ttctaatagc ctaaacgatg gtgcttggtg agtcgtggtt ctaaaggtac 2100
   caaacggggg agccaaagtt ctccaactgc tgcatacttt gacaaggaaa atctattttt 2160
   gtcttccgat ctacatttat gacctaagtc aggtaaataa gcctggttta tttctgtaac 2220
   attttttatg cagacagtct gttatgcact gtggtttcag atgtgcaata atttgtacaa 2280
   tggtttattc ccaagtatgc ctttaagcag aacaaatgtg tttttctata tagttccttg 2340
   ccttaataaa tatgtaatat aaatttaaaa aaaaaaaaaa aaaaaaaaaa aaa 2393
<210> 92
   <211> 482
   <212> PRT
   <213> Rattus norvegicus
<220>
   <221> SOURCE
   <222> 1..482
   <223> /mol_type="protein"
   /note="Rattus norvegicus Klf4-9"
   /organism="Rattus norvegicus"
<400> 92
   Met Arg Gln Pro Pro Gly Glu Ser Asp Met Ala Val Ser Asp Ala Leu
   1 5 10 15
   Leu Pro Ser Phe Ser Thr Phe Ala Ser Gly Pro Ala Gly Arg Glu Lys
   20 25 30
   Thr Leu Arg Pro Ala Gly Ala Pro Thr Asn Arg Trp Arg Glu Glu Leu
   35 40 45
   Ser His Met Lys Arg Leu Pro Pro Leu Pro Gly Arg Pro Tyr Asp Leu
   50 55 60
   Ala Ala Thr Val Ala Thr Asp Leu Glu Ser Gly Gly Ala Gly Ala Ala
   65 70 75 80
   Cys Ser Ser Asn Asn Pro Ala Leu Pro Arg Arg Glu Thr Glu Glu Phe
   85 90 95
   Asn Asp Leu Leu Asp Leu Asp Phe Ile Leu Ser Asn Ser Leu Ser His
   100 105 110
   Gln Glu Ser Val Ala Ala Thr Val Thr Thr Ser Ala Ser Ala Ser Ser
   115 120 125
   Ser Ser Ser Pro Ala Ser Ser Gly Pro Ala Ser Ala Pro Ser Thr Cys
   130 135 140
   Ser Phe Ser Tyr Pro Ile Arg Ala Gly Gly Asp Pro Gly Val Ala Ala
   145 150 155 160
   Gly Asn Thr Gly Gly Gly Leu Leu Tyr Ser Arg Glu Ser Ala Pro Pro
   165 170 175
   Pro Thr Ala Pro Phe Asn Leu Ala Asp Ile Asn Asp Val Ser Pro Ser
   180 185 190
   Gly Gly Phe Val Ala Glu Leu Leu Arg Pro Glu Leu Asp Pro Val Tyr
   195 200 205
   Ile Pro Pro Gln Gln Pro Gln Pro Pro Gly Gly Gly Leu Met Gly Lys
   210 215 220
   Phe Val Leu Lys Ala Ser Leu Ser Thr Pro Gly Ser Glu Tyr Thr Ser
   225 230 235 240
   Pro Ser Val Ile Ser Val Ser Lys Gly Ser Pro Asp Gly Ser His Pro
   245 250 255
   Val Val Val Ala Pro Tyr Ser Gly Gly Pro Pro Arg Met Cys Pro Lys
   260 265 270
   Ile Lys Gln Glu Ala Val Pro Ser Cys Thr Val Ser Arg Ser Leu Glu
   275 280 285
   Ala His Leu Ser Ala Gly Pro Gln Leu Ser Asn Gly His Arg Pro Asn
   290 295 300
   Thr His Asp Phe Pro Leu Gly Arg Gln Leu Pro Thr Arg Thr Thr Pro
   305 310 315 320
   Thr Leu Ser Pro Glu Glu Leu Leu Asn Ser Arg Asp Cys His Pro Gly
   325 330 335
   Leu Pro Leu Pro Pro Gly Phe His Pro His Pro Gly Pro Ser Tyr Pro
   340 345 350
   Pro Phe Leu Pro Asp Gln Met Gln Ser Gln Val Pro Ser Leu His Tyr
   355 360 365
   Gln Glu Leu Met Pro Pro Gly Ser Cys Leu Pro Glu Glu Pro Lys Pro
   370 375 380
   Lys Arg Gly Arg Arg Ser Trp Pro Arg Lys Arg Thr Ala Thr His Thr
   385 390 395 400
   Cys Asp Tyr Ala Gly Cys Gly Lys Thr Tyr Thr Lys Ser Ser His Leu
   405 410 415
   Lys Ala His Leu Arg Thr His Thr Gly Glu Lys Pro Tyr His Cys Asp
   420 425 430
   Trp Asp Gly Cys Gly Trp Lys Phe Ala Arg Ser Asp Glu Leu Thr Arg
   435 440 445
   His Tyr Arg Lys His Thr Gly His Arg Pro Phe Gln Cys Gln Lys Cys
   450 455 460
   Asp Arg Ala Phe Ser Arg Ser Asp His Leu Ala Leu His Met Lys Arg
   465 470 475 480
   His Phe
<210> 93
   <211> 2323
   <212> DNA
   <213> Rattus norvegicus
<220>
   <221> source
   <222> 1..2323
   <223> /mol_type="DNA"
   /note="Rattus norvegicus Sox-2"
   /organism="Rattus norvegicus"
<400> 93
   gtgtttgcaa aaagggaaaa gtactttgct gcctctttaa gactagggct gggagaaaga      60
   agaggagaga aaaagaaagg agagaagttt ggagcccgag gcttaagcct ttccaaaaac     120
   taatcacaac aatcgcggcg gcccgaggag gagagcgact gttttttcat cccaattgca    180
   cttcgcccgt ctcgagctcc gcttcccccc aactattctc cgccagatct ccgcgcaagg     240
   ccgtgcacgc cgacgacccc gcccgcggcc cctgcatccc ggcccccgcg cgcggccccc     300
   gcagtcccgg ccgggccgag ggtcggcggc cgccggcggg ccgcgcccgc gcccagcgcc     360
   cgcatgtata acatgatgga gacggagctg aagccgccgg gccctcagca agcttcgggg     420
   ggcggcggcg gaggaggcaa cgccacggcg gcggcgaccg gcggcaacca gaagaacagc     480
   ccggaccgcg tcaagaggcc catgaatgcc ttcatggtgt ggtcccgggg gcagcggcgt     540
   aagatggccc aggagaaccc caagatgcac aactcggaga tcagcaagcg cctgggcgcc     600
   gagtggaaac ttttgtcgga gaccgagaag cggccgttca tcgacgaggc caagcggctg     660
   cgcgctctgc acatgaagga gcacccggat tataaatacc ggccgcggcg gaaaaccaag     720
   acgctcatga agaaggataa gtacacgctt cccggaggct tgctggcccc cggcgggaac     780
   agcatggcga gcggggttgg ggtgggcgcc ggcctgggtg cgggcgtgaa ccagcgcatg     840
   gacagctacg cgcacatgaa cggctggagc aacggcagct acagcatgat gcaggagcag     900
   ctgggctacc cgcagcaccc gggcctcaac gctcacggcg cggcacagat gcagccgatg     960
   caccgctacg acgtcagcgc cctgcagtac aactccatga ccagctcgca gacctacatg    1020
   aacggctcgc ccacctacag catgtcctac tcgcagcagg gcacccccgg tatggcgctg    1080
   ggctccatgg gctctgtggt caagtccgag gccagttcca gcccccccgt ggttacctct    1140
   tcctcccact ccagggcgcc ctgccaggcc ggggacctcc gggacatgat cagcatgtac    1200
   ctccccggcg ccgaggtgcc ggagcccgct gcgcccagta gactgcacat ggcccagcac    1260
   taccagagcg gcccggtgcc cggcacggcc attaacggca cactgcccct gtcgcacatg    1320
   tgagggccgg accgcgaact ggagaagggg agagattttt caaaaagata caagggaatt    1380
   gggaggggtg caaaagagga gagtaagaaa aatctgaatg ctcaaaagga aaaaaaaaat    1440
   ctcattaccc gcagcaaaat gacagctgcg gaaaaaaacc accaatccca tccaaattaa    1500
   cgcaaaaacc gtgatgccga ctagaaaact tttatgagag atctggagga aaaaaactac    1560
   gcaaaacttt tttttaaagt tctagtggta cgttaggcgc ttcgcaggga gttctcaaaa    1620
   gtctttacca gtaatattta gaactagact ccgggcgatg aaaaaagttt taatatttgc    1680
   aagcaacttt tgtacagtat ttatcgagat aaacatggca atcaaatgtc cattgtttat    1740
   aagctgagaa tttgccaata tttttcgagg aaagggttct tgctgggttt tgattctgca    1800
   gcttaaatta aggaccgtta cagacaagga aggaatttat tcggatttga acgttttagt    1860
   tttaaaattg tacaaaagga aaacatgaga gcaagtactg gcaagaccat tttcgtggtc    1920
   ttgtttaggg caaacgttct agattgtact aaatttttaa cttactgtta aaggcaaaaa    1980
   aaaaatgtcc atgcaggttg atatcgttgg taatttataa tagcttttgt tcaatcccac    2040
   ccttttcatt ttgttcacat aaaaatatgg aaattactgt gtttgaaata ttttcttatg    2100
   gtttgtaata tttctgtaaa ttgtgatatt ttaaggtttt ttcccccttt tattttccgt    2160
   agttgtattt taaaagattc ggctgttatt ggaaccaggc tgccgagaat ccatgtatat    2220
   atttgaacta ataccatcct tataacagtt acgtttccaa cttaagtttt tactccatta    2280
   tgcacagttt gagataaata aatttttgaa atatggacac tga                      2323
<210> 94
   <211> 319
   <212> PRT
   <213> Rattus norvegicus
<220>
   <221> SOURCE
   <222> 1..319
   <223> /mol_type="protein"
   /note="Rattus norvegicus Sox-2"
   /organism="Rattus norvegicus"
<400> 94
   Met Tyr Asn Met Met Glu Thr Glu Leu Lys Pro Pro Gly Pro Gln Gln
   1               5                   10                  15
   Ala Ser Gly Gly Gly Gly Gly Gly Gly Asn Ala Thr Ala Ala Ala Thr
               20                  25                  30
   Gly Gly Asn Gln Lys Asn Ser Pro Asp Arg Val Lys Arg Pro Met Asn
           35                  40                  45
   Ala Phe Met Val Trp Ser Arg Gly Gln Arg Arg Lys Met Ala Gln Glu
       50                  55                  60
   Asn Pro Lys Met His Asn Ser Glu Ile Ser Lys Arg Leu Gly Ala Glu
   65                  70                  75                  80
   Trp Lys Leu Leu Ser Glu Thr Glu Lys Arg Pro Phe Ile Asp Glu Ala
                   85                  90                  95
   Lys Arg Leu Arg Ala Leu His Met Lys Glu His Pro Asp Tyr Lys Tyr
               100                 105                 110
   Arg Pro Arg Arg Lys Thr Lys Thr Leu Met Lys Lys Asp Lys Tyr Thr
           115                 120                 125
   Leu Pro Gly Gly Leu Leu Ala Pro Gly Gly Asn Ser Met Ala Ser Gly
       130                 135                 140
   Val Gly Val Gly Ala Gly Leu Gly Ala Gly Val Asn Gln Arg Met Asp
   145                 150                 155                 160
   Ser Tyr Ala His Met Asn Gly Trp Ser Asn Gly Ser Tyr Ser Met Met
                   165                 170                 175
   Gln Glu Gln Leu Gly Tyr Pro Gln His Pro Gly Leu Asn Ala His Gly
               180                 185                 190
   Ala Ala Gln Met Gln Pro Met His Arg Tyr Asp Val Ser Ala Leu Gln
           195                 200                 205
   Tyr Asn Ser Met Thr Ser Ser Gln Thr Tyr Met Asn Gly Ser Pro Thr
       210                 215                 220
   Tyr Ser Met Ser Tyr Ser Gln Gln Gly Thr Pro Gly Met Ala Leu Gly
   225                 230                 235                 240
   Ser Met Gly Ser Val Val Lys Ser Glu Ala Ser Ser Ser Pro Pro Val
                   245                 250                 255
   Val Thr Ser Ser Ser His Ser Arg Ala Pro Cys Gln Ala Gly Asp Leu
               260                 265                 270
   Arg Asp Met Ile Ser Met Tyr Leu Pro Gly Ala Glu Val Pro Glu Pro
           275                 280                 285
   Ala Ala Pro Ser Arg Leu His Met Ala Gln His Tyr Gln Ser Gly Pro
       290                 295                 300
   Val Pro Gly Thr Ala Ile Asn Gly Thr Leu Pro Leu Ser His Met
   305                 310                 315
<210> 95
   <211> 2358
   <212> DNA
   <213> Rattus norvegicus
<220>
   <221> source
   <222> 1..2358
   <223> /mol_type="DNA"
   /note="Rattus norvegicus NANOG"
   /organism="Rattus norvegicus"
<400> 95
   tcagataggc tgatttcgag tctttctctt ttgtgggaag accgaggctc gcttcttttt      60
   ggcttgttga ctcttttaca tctggacatt taactcttac ttttaagatc tttccctcta     120
   gacactgagt tttaaagtct taactttttg gttgttaaaa actttttttt ttttaaagtc     180
   ccttcccttg ccgttgggct gacatgagcg tggatctttc tggtccccac agtctgccta     240
   gttgtgagga agcatcgaac tctggggatt cctcgccgat gcctgccgtt catcttcctg     300
   aggaaaatta ttcttgctta caagtgtctg ctactgagat gctctgcaca gagactgcct     360
   ctcctccgcc ttcctctggg gacctacctc ttcaagatag ccctgattct tctagcaatc     420
   ccaagctaaa gctgtctggt cccgaggctg acgagggccc tgagaagaaa gaagagaaca     480
   aggtcctcac caagaagcag aagatgcgga ctgtgttctc tcaggcccag ttgtgtgcac     540
   tcaaggatag gtttcagagg caaaggtacc tcagcctcca gcagatgcaa gatctctcta     600
   ccattctgaa cctgagctat aagcaggtga agacctggtt ccaaaaccaa agaatgaagt     660
   gcaagaggtg gcagaaaaac caatggttga agactagcaa cggcctgact cagaagggct 720
   cagcgccggt ggagtatccc agcatccatt gcagctattc tcagggctat ctgatgaacg 780
   cgtctggaaa ccttccagta tggggcagtc agacctggac caacccaact tggaacaacc 840
   agacctggac caacccaacc tggagcaacc agacctggac caacccaact tggagcaacc 900
   aggcctggag cactcagtcc tggtgtactc aggcctggaa cagccagact tggaacgctg 960
   ctccgctcca taacttcggg gaggactccc tgcagcctta tgtgccgttg cagcaaaact 1020
   tctccgccag tgatttggag gcgaatttgg aagccactag ggaaagccag gcgcatttta 1080
   gtaccccgca agccttggaa ttgttcctga actactccgt gaattctcca ggcgaaatat 1140
   gaggtttaca caacaactgg gcttaaagtc agggcagggc cagggtcagc tttcttcctt 1200
   cttccaaaga gttttatatt gttcttattt tttttttaat tattattttg tttttgtttt 1260
   ttgtttatca aggtagggtt tctctgtgtg gttctggctg tcctggaatt cactctgtag 1320
   accaggctgg cctcgtactc agagatctgc ctacttttgc ctcctgaagg ctagggctaa 1380
   agattttcta aagattttca tagtttttat ttttttaatt attatctgtt ttcatgtttg 1440
   tgtttttttg ttttgttttt gtttttgttt ttgtttatca agatagggtt tctctgtgtg 1500
   gctctagtag tcccggaaac tggctctgta gaccaggctg tccttgaact cagaaatctg 1560
   cctttgcctc cggactgcgg ggactaaagg cagtatataa ccacctggca cattgttttt 1620
   atttttattc ttttggtgcc agaaagcaaa cctaggactt tgagctgggc acccactcaa 1680
   ccactgagct ctgtttgcga cccccgtgtt ggctgcattt gtctgagctg ggtaacttgt 1740
   ctttttttcc gtgttaacga tgggcttcgg agacagtgca ctatacactc tatcctcccc 1800
   caggtctcac acacccaccc tactccatac caacccaggc ttgtctgtct tttttttttt 1860
   tggagctgag gactgaaccc agggccttgc gctttctagg caagcgctct acctctgagc 1920
   taaatcccca acccttgtct gtctttttag aagcttgggt cttggtgtgc actgtgtatc 1980
   gttttgaggg gtgaggttta aaagtataca aattataaag attcatgcag atatggtggc 2040
   tcctctcaag gacgagacag aaggatcacc agtttgaggc tatctcagat ataaaataag 2100
   ttcaagacca gcctgtacta tgtctaaata gtaagacagc atctcaacaa aataataaaa 2160
   ctaaggtaag gagataaaag taaagtctca acaaaataca agatctcgcc tgttacagtt 2220
   ctttgatttc ctccgtgtct ttgcagttcc gccaagaggc ttctatgtta atatctgtag 2280
   aaagatgttt atatttgact gtaccatgat aaaccagtgc cagctggact agtttaaata 2340
   aaacactaat tttaccca 2358
<210> 96
   <211> 312
   <212> PRT
   <213> Rattus norvegicus
<220>
   <221> SOURCE
   <222> 1..312
   <223> /mol_type="protein"
   /note="Rattus norvegicus NANOG"
   /organism="Rattus norvegicus"
<400> 96
   Met Ser Val Asp Leu Ser Gly Pro His Ser Leu Pro Ser Cys Glu Glu
   1               5                   10                  15
   Ala Ser Asn Ser Gly Asp Ser Ser Pro Met Pro Ala Val His Leu Pro
               20                  25                  30
   Glu Glu Asn Tyr Ser Cys Leu Gln Val Ser Ala Thr Glu Met Leu Cys
           35                  40                  45
   Thr Glu Thr Ala Ser Pro Pro Pro Ser Ser Gly Asp Leu Pro Leu Gln
       50                  55                  60
   Asp Ser Pro Asp Ser Ser Ser Asn Pro Lys Leu Lys Leu Ser Gly Pro
   65                  70                  75                  80
   Glu Ala Asp Glu Gly Pro Glu Lys Lys Glu Glu Asn Lys Val Leu Thr
                   85                  90                  95
   Lys Lys Gln Lys Met Arg Thr Val Phe Ser Gln Ala Gln Leu Cys Ala
               100                 105                 110
   Leu Lys Asp Arg Phe Gln Arg Gln Arg Tyr Leu Ser Leu Gln Gln Met
           115                 120                 125
   Gln Asp Leu Ser Thr Ile Leu Asn Leu Ser Tyr Lys Gln Val Lys Thr
       130                 135                 140
   Trp Phe Gln Asn Gln Arg Met Lys Cys Lys Arg Trp Gln Lys Asn Gln
   145                 150                 155                 160
   Trp Leu Lys Thr Ser Asn Gly Leu Thr Gln Lys Gly Ser Ala Pro Val
                   165                 170                 175
   Glu Tyr Pro Ser Ile His Cys Ser Tyr Ser Gln Gly Tyr Leu Met Asn
               180                 185                 190
   Ala Ser Gly Asn Leu Pro Val Trp Gly Ser Gln Thr Trp Thr Asn Pro
           195                 200                 205
   Thr Trp Asn Asn Gln Thr Trp Thr Asn Pro Thr Trp Ser Asn Gln Thr
       210                 215                 220
   Trp Thr Asn Pro Thr Trp Ser Asn Gln Ala Trp Ser Thr Gln Ser Trp
   225                 230                 235                 240
   Cys Thr Gln Ala Trp Asn Ser Gln Thr Trp Asn Ala Ala Pro Leu His
                   245                 250                 255
   Asn Phe Gly Glu Asp Ser Leu Gln Pro Tyr Val Pro Leu Gln Gln Asn
               260                 265                 270
   Phe Ser Ala Ser Asp Leu Glu Ala Asn Leu Glu Ala Thr Arg Glu Ser
           275                 280                 285
   Gln Ala His Phe Ser Thr Pro Gln Ala Leu Glu Leu Phe Leu Asn Tyr
       290                 295                 300
   Ser Val Asn Ser Pro Gly Glu Ile
   305                 310
<210> 97
   <211> 463
   <212> DNA
   <213> Rattus norvegicus
<220>
   <221> source
   <222> 1..463
   <223> /mol_type="DNA"
   /note="Rattus norvegicus Insulin"
   /organism="Rattus norvegicus"
<400> 97
   aaccctaagt gaccagctac aatcatagac catcagcaag caggtcattg ttccaacatg      60
   gccctgtgga tgcgcttcct gcccctgctg gccctgctcg tcctctggga gcccaagcct     120
   gcccaggctt ttgtcaaaca gcacctttgt ggtcctcacc tggtggaggc tctgtacctg     180
   gtgtgtgggg aacgtggttt cttctacaca cccaagtccc gtcgtgaagt ggaggacccg     240
   caagtgccac aactggagct gggtggaggc ccggaggccg gggatcttca gaccttggca     300
   ctggaggttg cccggcagaa gcgtggcatt gtggatcagt gctgcaccag catctgctcc     360
   ctctaccaac tggagaacta ctgcaactga gtccaccact ccccgcccac ccctctgcaa     420
   tgaataaagc ctttgaatga gcaccaaaaa aaaaaaaaaa aaa                       463
<210> 98
   <211> 110
   <212> PRT
   <213> Rattus norvegicus
<220>
   <221> SOURCE
   <222> 1..110
   <223> /mol_type="protein"
   /note="Rattus norvegicus Insulin"
   /organism="Rattus norvegicus"
<400> 98
   Met Ala Leu Trp Met Arg Phe Leu Pro Leu Leu Ala Leu Leu Val Leu
   1               5                   10                  15
   Trp Glu Pro Lys Pro Ala Gln Ala Phe Val Lys Gln His Leu Cys Gly
               20                  25                  30
   Pro His Leu Val Glu Ala Leu Tyr Leu Val Cys Gly Glu Arg Gly Phe
           35                  40                  45
   Phe Tyr Thr Pro Lys Ser Arg Arg Glu Val Glu Asp Pro Gln Val Pro
       50                  55                  60
   Gln Leu Glu Leu Gly Gly Gly Pro Glu Ala Gly Asp Leu Gln Thr Leu
   65                  70                  75                  80
   Ala Leu Glu Val Ala Arg Gln Lys Arg Gly Ile Val Asp Gln Cys Cys
                   85                  90                  95
   Thr Ser Ile Cys Ser Leu Tyr Gln Leu Glu Asn Tyr Cys Asn
               100                 105                 110
<210> 99
   <211> 1307
   <212> DNA
   <213> Rattus norvegicus
<220>
   <221> source
   <222> 1..1307
   <223> /mol_type="DNA"
   /note="Rattus norvegicus GAPDH"
   /organism="Rattus norvegicus"
<400> 99
   gggggctctc tgctcctccc tgttctagag acagccgcat cttcttgtgc agtgccagcc     60
   tcgtctcata gacaagatgg tgaaggtcgg tgtgaacgga tttggccgta tcggacgcct    120
   ggttaccagg gctgccttct cttgtgacaa agtggacatt gttgccatca acgacccctt    180
   cattgacctc aactacatgg tctacatgtt ccagtatgac tctacccacg gcaagttcaa    240
   cggcacagtc aaggctgaga atgggaagct ggtcatcaac gggaaaccca tcaccatctt    300
   ccaggagcga gatcccgcta acatcaaatg gggtgatgct ggtgctgagt atgtcgtgga    360
   gtctactggc gtcttcacca ccatggagaa ggctggggct cacctgaagg gtggggccaa    420
   aagggtcatc atctccgccc cttccgctga tgcccccatg tttgtgatgg gtgtgaacca    480
   cgagaaatat gacaactccc tcaagattgt cagcaatgca tcctgcacca ccaactgctt    540
   agcccccctg gccaaggtca tccatgacaa ctttggcatc gtggaagggc tcatgaccac    600
   agtccatgcc atcactgcca ctcagaagac tgtggatggc ccctctggaa agctgtggcg    660
   tgatggccgt ggggcagccc agaacatcat ccctgcatcc actggtgctg ccaaggctgt    720
   gggcaaggtc atcccagagc tgaacgggaa gctcactggc atggccttcc gtgttcctac    780
   ccccaatgta tccgttgtgg atctgacatg ccgcctggag aaacctgcca agtatgatga    840
   catcaagaag gtggtgaagc aggcggccga gggcccacta aagggcatcc tgggctacac    900
   tgaggaccag gttgtctcct gtgacttcaa cagcaactcc cattcttcca cctttgatgc    960
   tggggctggc attgctctca atgacaactt tgtgaagctc atttcctggt atgacaatga   1020
   atatggctac agcaacaggg tggtggacct catggcctac atggcctcca aggagtaaga   1080
   aaccctggac cacccagccc agcaaggata ctgagagcaa gagagaggcc ctcagttgct   1140
   gaggagtccc catcccaact cagcccccaa cactgagcat ctccctcaca attccatccc   1200
   agaccccata acaacaggag gggcctgggg agccctccct tctctcgaat accatcaata   1260
   aagttcgctg caccctcaaa aaaaaaaaaa aaaaaaaaaa aaaaaaa                 1307
<210> 100
   <211> 323
   <212> PRT
   <213> Rattus norvegicus
<220>
   <221> SOURCE
   <222> 1..323
   <223> /mol_type="protein"
   /note="Rattus norvegicus GAPDH"
   /organism="Rattus norvegicus"
<400> 100
   Met Val Lys Val Gly Val Asn Gly Phe Gly Arg Ile Gly Arg Leu Val
   1               5                   10                  15
   Thr Arg Ala Ala Phe Ser Cys Asp Lys Val Asp Ile Val Ala Ile Asn
               20                  25                  30
   Asp Pro Phe Ile Asp Leu Asn Tyr Met Val Tyr Met Phe Gln Tyr Asp
           35                  40                  45
   Ser Thr His Gly Lys Phe Asn Gly Thr Val Lys Ala Glu Asn Gly Lys
       50                  55                  60
   Leu Val Ile Asn Gly Lys Pro Ile Thr Ile Phe Gln Glu Arg Asp Pro
   65                  70                  75                  80
   Ala Asn Ile Lys Trp Gly Asp Ala Gly Ala Glu Tyr Val Val Glu Ser
                   85                  90                  95
   Thr Gly Val Phe Thr Thr Met Glu Lys Ala Gly Ala His Leu Lys Gly
               100                 105                 110
   Gly Ala Lys Arg Val Ile Ile Ser Ala Pro Ser Ala Asp Ala Pro Met
           115                 120                 125
   Phe Val Met Gly Val Asn His Glu Lys Tyr Asp Asn Ser Leu Lys Ile
       130                 135                 140
   Val Ser Asn Ala Ser Cys Thr Thr Asn Cys Leu Ala Pro Leu Ala Lys
   145                 150                 155                 160
   Val Ile His Asp Asn Phe Gly Ile Val Glu Gly Leu Met Thr Thr Val
                   165                 170                 175
   His Ala Ile Thr Ala Thr Gln Lys Thr Val Asp Gly Pro Ser Gly Lys
               180                 185                 190
   Leu Trp Arg Asp Gly Arg Gly Ala Ala Gln Asn Ile Ile Pro Ala Ser
           195                 200                 205
   Thr Gly Ala Ala Lys Ala Val Gly Lys Val Ile Pro Glu Leu Asn Gly
       210                 215                 220
   Lys Leu Thr Gly Met Ala Phe Arg Val Pro Thr Pro Asn Val Ser Val
   225                 230                 235                 240
   Val Asp Leu Thr Cys Arg Leu Glu Lys Pro Ala Lys Tyr Asp Asp Ile
                   245                 250                 255
   Lys Lys Val Val Lys Gln Ala Ala Glu Gly Pro Leu Lys Gly Ile Leu
               260                 265                 270
   Gly Tyr Thr Glu Asp Gln Val Val Ser Cys Asp Phe Asn Ser Asn Ser
           275                 280                 285
   His Ser Ser Thr Phe Asp Ala Gly Ala Gly Ile Ala Leu Asn Asp Asn
       290                 295                 300
   Phe Val Lys Leu Ile Ser Trp Tyr Asp Asn Glu Tyr Gly Tyr Ser Asn
   305                 310                 315                 320
   Arg Val Val
<210> 101
   <211> 1575
   <212> DNA
   <213> Homo sapiens
<220>
   <221> source
   <222> 1..1575
   <223> /mol_type="DNA"
   /note="Homo sapiens Glut-2 (SLC2A2)"
   /organism="Homo sapiens"
<400> 101
   atgacagaag ataaggtcac tgggaccctg gttttcactg tcatcactgc tgtgctgggt      60
   tccttccagt ttggatatga cattggtgtg atcaatgcac ctcaacaggt aataatatct     120
   cactatagac atgttttggg tgttccactg gatgaccgaa aagctatcaa caactatgtt     180
   atcaacagta cagatgaact gcccacaatc tcatactcaa tgaacccaaa accaacccct     240
   tgggctgagg aagagactgt ggcagctgct caactaatca ccatgctctg gtccctgtct     300
   gtatccagct ttgcagttgg tggaatgact gcatcattct ttggtgggtg gcttggggac     360
   acacttggaa gaatcaaagc catgttagta gcaaacattc tgtcattagt tggagctctc     420
   ttgatggggt tttcaaaatt gggaccatct catatactta taattgctgg aagaagcata     480
   tcaggactat attgtgggct aatttcaggc ctggttccta tgtatatcgg tgaaattgct     540
   ccaaccgctc tcaggggagc acttggcact tttcatcagc tggccatcgt cacgggcatt     600
   cttattagtc agattattgg tcttgaattt atcttgggca attatgatct gtggcacatc     660
   ctgcttggcc tgtctggtgt gcgagccatc cttcagtctc tgctactctt tttctgtcca     720
   gaaagcccca gataccttta catcaagtta gatgaggaag tcaaagcaaa acaaagcttg     780
   aaaagactca gaggatatga tgatgtcacc aaagatatta atgaaatgag aaaagaaaga     840
   gaagaagcat cgagtgagca gaaagtctct ataattcagc tcttcaccaa ttccagctac     900
   cgacagccta ttctagtggc actgatgctg catgtggctc agcaattttc cggaatcaat     960
   ggcatttttt actactcaac cagcattttt cagacggctg gtatcagcaa acctgtttat    1020
   gcaaccattg gagttggcgc tgtaaacatg gttttcactg ctgtctctgt attccttgtg    1080
   gagaaggcag ggcgacgttc tctctttcta attggaatga gtgggatgtt tgtttgtgcc    1140
   atcttcatgt cagtgggact tgtgctgctg aataagttct cttggatgag ttatgtgagc    1200
   atgatagcca tcttcctctt tgtcagcttc tttgaaattg ggccaggccc gatcccctgg    1260
   ttcatggtgg ctgagttttt cagtcaagga ccacgtcctg ctgctttagc aatagctgca    1320
   ttcagcaatt ggacctgcaa tttcattgta gctctgtgtt tccagtacat tgcggacttc    1380
   tgtggacctt atgtgttttt cctctttgct ggagtgctcc tggcctttac cctgttcaca    1440
   ttttttaaag ttccagaaac caaaggaaag tcttttgagg aaattgctgc agaattccaa    1500
   aagaagagtg gctcagccca caggccaaaa gctgctgtag aaatgaaatt cctaggagct    1560
   acagagactg tgtaa                                                     1575
<210> 102
   <211> 524
   <212> PRT
   <213> Homo sapiens
<220>
   <221> SOURCE
   <222> 1..524
   <223> /mol_type="protein"
   /note="Homo sapiens Glut-2 (SLC2A2)"
   /organism="Homo sapiens"
<400> 102
<210> 103
   <211> 1569
   <212> DNA
   <213> Rattus norvegicus
<220>
   <221> source
   <222> 1..1569
   <223> /mol_type="DNA"
   /note="Rattus norvegicus Glut-2 (SLC2A2)"
   /organism="Rattus norvegicus"
<400> 103
<210> 104
   <211> 522
   <212> PRT
   <213> Rattus norvegicus
<220>
   <221> SOURCE
   <222> 1..522
   <223> /mol_type="protein"
   /note="Rattus norvegicus (SLC2A2)"
   /organism="Rattus norvegicus"
<400> 104
   Met Ser Glu Asp Lys Ile Thr Gly Thr Leu Ala Phe Thr Val Phe Thr
   1               5                   10                  15
   Ala Val Leu Gly Ser Phe Gln Phe Gly Tyr Asp Ile Gly Val Ile Asn
               20                  25                  30
   Ala Pro Gln Glu Val Ile Ile Ser His Tyr Arg His Val Leu Gly Val
           35                  40                  45
   Pro Leu Asp Asp Arg Arg Ala Thr Ile Asn Tyr Asp Ile Asn Gly Thr
       50                  55                  60
   Asp Thr Pro Leu Ile Val Thr Pro Ala His Thr Thr Pro Asp Ala Trp
   65                  70                  75                  80
   Glu Glu Glu Thr Glu Gly Ser Ala His Ile Val Thr Met Leu Trp Ser
                   85                  90                  95
   Leu Ser Val Ser Ser Phe Ala Val Gly Gly Met Val Ala Ser Phe Phe
               100                 105                 110
   Gly Gly Trp Leu Gly Asp Lys Leu Gly Arg Ile Lys Ala Met Leu Ala
           115                 120                 125
   Ala Asn Ser Leu Ser Leu Thr Gly Ala Leu Leu Met Gly Cys Ser Lys
       130                 135                 140
   Phe Gly Pro Ala His Ala Leu Ile Ile Ala Gly Arg Ser Val Ser Gly
   145                 150                 155                 160
   Leu Tyr Cys Gly Leu Ile Ser Gly Leu Val Pro Met Tyr Ile Gly Glu
                   165                 170                 175
   Ile Ala Pro Thr Thr Leu Arg Gly Ala Leu Gly Thr Leu His Gln Leu
               180                 185                 190
   Ala Leu Val Thr Gly Ile Leu Ile Ser Gln Ile Ala Gly Leu Ser Phe
           195                 200                 205
   Ile Leu Gly Asn Gln Asp Tyr Trp His Ile Leu Leu Gly Leu Ser Ala
       210                 215                 220
   Val Pro Ala Leu Leu Gln Cys Leu Leu Leu Leu Phe Cys Pro Glu Ser
   225                 230                 235                 240
   Pro Arg Tyr Leu Tyr Leu Lys Leu Glu Glu Glu Val Arg Ala Lys Lys
                   245                 250                 255
   Ser Leu Lys Arg Leu Arg Gly Thr Glu Asp Val Thr Lys Asp Ile Asn
               260                 265                 270
   Glu Met Arg Lys Glu Lys Glu Glu Ala Ser Thr Glu Gln Lys Val Ser
           275                 280                 285
   Val Ile Gln Leu Phe Thr Asp Pro Asn Tyr Arg Gln Pro Ile Val Val
       290                 295                 300
   Ala Leu Met Leu His Leu Ala Gln Gln Phe Ser Gly Ile Asn Gly Ile
   305                 310                 315                 320
   Phe Tyr Tyr Ser Thr Ser Ile Phe Gln Thr Ala Gly Ile Ser Gln Pro
                   325                 330                 335
   Val Tyr Ala Thr Ile Gly Val Gly Ala Ile Asn Met Ile Phe Thr Ala
               340                 345                 350
   Val Ser Val Leu Leu Val Glu Lys Ala Gly Arg Arg Thr Leu Phe Leu
           355                 360                 365
   Ala Gly Met Ile Gly Met Phe Phe Cys Ala Val Phe Met Ser Leu Gly
       370                 375                 380
   Leu Val Leu Leu Asp Lys Phe Thr Trp Met Ser Tyr Val Ser Met Thr
   385                 390                 395                 400
   Ala Ile Phe Leu Phe Val Ser Phe Phe Glu Ile Gly Pro Gly Pro Ile
                   405                 410                 415
   Pro Trp Phe Met Val Ala Glu Phe Phe Ser Gln Gly Pro Arg Pro Thr
               420                     425                 430
   Ala Leu Ala Leu Ala Ala Phe Ser Asn Trp Val Cys Asn Phe Ile Ile
           435                     440                 445
   Ala Leu Cys Phe Gln Tyr Ile Ala Asp Phe Leu Gly Pro Tyr Val Phe
       450                     455                 460
   Phe Leu Phe Ala Gly Val Val Leu Ile Phe Thr Leu Phe Thr Phe Phe
   465                     470                 475                     480
   Lys Val Pro Glu Thr Lys Gly Lys Ser Phe Asp Glu Ile Ala Ala Glu
                   485                     490                 495
   Phe Arg Lys Lys Ser Gly Ser Ala Pro Pro Arg Lys Ala Ala Val Gln
               500                     505                 510
   Met Glu Phe Leu Gly Ser Ser Glu Thr Val
           515                     520
<210> 105
   <211> 1769
   <212> DNA
   <213> Homo sapiens
<220>
   <221> source
   <222> 1..1769
   <223> /mol_type="DNA"
   /note="beta-III-tubulin"
   /organism="Homo sapiens"
<400> 105
   gacatcagcc gatgcgaagg gcggggccgc ggctataaga gcgcgcggcc gcggtccccg 60
   accctcagca gccagcccgg cccgcccgcg cccgtccgca gccgcccgcc agacgcgccc 120
   agtatgaggg agatcgtgca catccaggcc ggccagtgcg gcaaccagat cggggccaag 180
   ttctgggaag tcatcagtga tgagcatggc atcgacccca gcggcaacta cgtgggcgac 240
   tcggacttgc agctggagcg gatcagcgtc tactacaacg aggcctcttc tcacaagtac 300
   gtgcctcgag ccattctggt ggacctggaa cccggaacca tggacagtgt ccgctcaggg 360
   gcctttggac atctcttcag gcctgacaat ttcatctttg gtcagagtgg ggccggcaac 420
   aactgggcca agggtcacta cacggagggg gcggagctgg tggattcggt cctggatgtg 480
   gtgcggaagg agtgtgaaaa ctgcgactgc ctgcagggct tccagctgac ccactcgctg 540
   gggggcggca cgggctccgg catgggcacg ttgctcatca gcaaggtgcg tgaggagtat 600
   cccgaccgca tcatgaacac cttcagcgtc gtgccctcac ccaaggtgtc agacacggtg 660
   gtggagccct acaacgccac gctgtccatc caccagctgg tggagaacac ggatgagacc 720
   tactgcatcg acaacgaggc gctctacgac atctgcttcc gcaccctcaa gctggccacg 780
   cccacctacg gggacctcaa ccacctggta tcggccacca tgagcggagt caccacctcc 840
   ttgcgcttcc cgggccagct caacgctgac ctgcgcaagc tggccgtcaa catggtgccc 900
   ttcccgcgcc tgcacttctt catgcccggc ttcgcccccc tcacagcccg gggcagccag 960
   cagtaccggg ccctgaccgt gcccgagctc acccagcaga tgttcgatgc caagaacatg 1020
   atggccgcct gcgacccgcg ccacggccgc tacctgacgg tggccaccgt gttccggggc 1080
   cgcatgtcca tgaaggaggt ggacgagcag atgctggcca tccagagcaa gaacagcagc 1140
   tacttcgtgg agtggatccc caacaacgtg aaggtggccg tgtgtgacat cccgccccgc 1200
   ggcctcaaga tgtcctccac cttcatcggg aacagcacgg ccatccagga gctgttcaag 1260
   cgcatctccg agcagttcac ggccatgttc cggcgcaagg ccttcctgca ctggtacacg 1320
   ggcgagggca tggacgagat ggagttcacc gaggccgaga gcaacatgaa cgacctggtg 1380
   tccgagtacc agcagtacca ggacgccacg gccgaggaag agggcgagat gtacgaagac 1440
   gacgaggagg agtcggaggc ccagggcccc aagtgaagct gctcgcagct ggagtgagag 1500
   gcaggtggcg gccggggccg aagccagcag tgtctaaacc cccggagcca tcttgctgcc 1560
   gacaccctgc tttcccctcg ccctagggct cccttgccgc cctcctgcag tatttatggc 1620
   ctcgtcctcc ccacctaggc cacgtgtgag ctgctcctgt ctctgtctta ttgcagctcc 1680
   aggcctgacg ttttacggtt ttgtttttta ctggtttgtg tttatatttt cggggatact 1740
   taataaatct attgctgtca gataccctt 1769
<210> 106
   <211> 450
   <212> PRT
   <213> Homo sapiens
<220>
   <221> SOURCE
   <222> 1..450
   <223> /mol_type="protein"
   /note="beta-III-tubulin"
   /organism="Homo sapiens"
<400> 106
<210> 107
   <211> 1704
   <212> DNA
   <213> Rattus norvegicus
<220>
   <221> source
   <222> 1..1704
   <223> /mol_type="DNA"
   /note="beta-III-tubulin"
   /organism="Rattus norvegicus"
<400> 107
   atcctccgca gcccgctgtc cgcctgcctc ttcgtctcta gccgagtgaa gtcagcatga 60
   gggagatcgt gcacatccag gccggccagt gcggcaacca gataggggcc aagttctggg 120
   aggtcatcag tgacgagcat ggcatagacc ccagcggcaa ctatgtgggg gactcggacc 180
   tgcagctgga acgcatcagt gtctactaca atgaggcctc ctctcacaag tatgtgccca 240
   gagccattct ggtggacctg gagcctggaa ccatggacag cgttcggtct ggggcctttg 300
   gacacctatt caggcctgac aactttatct tcggtcagag tggtgctggc aacaactggg 360
   ccaaagggca ctatacagag ggcgccgagc tggtggactc agtcctggat gtcgtgagga 420
   aagaatgtga gaattgtgac tgcctgcagg gcttccagct cactcactca ctgggtgggg 480
   gcacgggctc aggcatgggc acactgctca tcagcaaagt gcgtgaggag taccctgacc 540
   gcatcatgaa caccttcagc gtggtgccct cacccaaagt gtcagacacc gtggtagagc 600
   cctacaacgc caccctgtcc atccaccagc tagtggagaa cacggatgag acctactgca 660
   tcgacaatga agccctctac gacatctgct tccgcaccct caagctggct acacccacct 720
   acggggacct caaccacctt gtgtctgcta ccatgagcgg agtcaccacc tcccttcgat 780
   tccctggtca gctcaatgcc gacctccgca agctagctgt caacatggtg cccttccccc 840
   gcttgcattt cttcatgccc ggctttgccc cactaacagc ccggggcagc caacagtacc 900
   gtgccctgac ggtgcctgag ctcacccagc agatgttcga cgccaagaac atgatggctg 960
   cctgtgaccc acgccacggc cgctacctga ccgtggccac cgtcttccgt gggcgcatgt 1020
   ccatgaagga ggtggatgag cagatgctgg ccattcagag taagaacagt agttacttcg 1080
   tggagtggat ccccaacaac gtcaaggtag cggtgtgtga catcccgccc cgagggctca 1140
   agatgtcgtc caccttcatc ggcaacagca cggccatcca ggagctgttc aagcgcatct 1200
   ccgagcagtt tacggccatg ttccggcgca aggccttcct ccactggtac acgggcgaag 1260
   gcatggatga gatggagttc accgaggccg agagcaacat gaacgacctg gtgtctgagt 1320
   accagcagta ccaggacgcc actgccgagg aggaggggga gatgtatgaa gatgacgacg 1380
   aggagtcgga agcccagggg cccaagtgaa gttgcttgca gctggggtgt ggggccaagt 1440
   ggcagccagg gccaagacaa gcagcatctg tcccccagag ccatctagct actgacactg 1500
   cccccagctt accttcctac cagctcttca gggcacccta gggctcccgg gttaaagtcc 1560
   ttcagtattt atggccatcc cccaccccac gtgagtccac ttggctctgt cctccccgtt 1620
   ttagccacct ttgtatttat gttgcttttg tgtctgtttt attatggctc caggcctgat 1680
   gttttatgtt tttttttttt ttta 1704
<210> 108
   <211> 450
   <212> PRT
   <213> Rattus norvegicus
<220>
   <221> SOURCE
   <222> 1..450
   <223> /mol_type="protein"
   /note="beta-III-tubulin"
   /organism="Rattus norvegicus"
<400> 108
   Met Arg Glu Ile Val His Ile Gln Ala Gly Gln Cys Gly Asn Gln Ile
   1               5                   10                  15
   Gly Ala Lys Phe Trp Glu Val Ile Ser Asp Glu His Gly Ile Asp Pro
               20                  25                  30
   Ser Gly Asn Tyr Val Gly Asp Ser Asp Leu Gln Leu Glu Arg Ile Ser
           35                  40                  45
   Val Tyr Tyr Asn Glu Ala Ser Ser His Lys Tyr Val Pro Arg Ala Ile
       50                  55                  60
   Leu Val Asp Leu Glu Pro Gly Thr Met Asp Ser Val Arg Ser Gly Ala
   65                  70                  75                  80
   Phe Gly His Leu Phe Arg Pro Asp Asn Phe Ile Phe Gly Gln Ser Gly
                   85                  90                  95
   Ala Gly Asn Asn Trp Ala Lys Gly His Tyr Thr Glu Gly Ala Glu Leu
               100                 105                 110
   Val Asp Ser Val Leu Asp Val Val Arg Lys Glu Cys Glu Asn Cys Asp
           115                 120                 125
   Cys Leu Gln Gly Phe Gln Leu Thr His Ser Leu Gly Gly Gly Thr Gly
       130                 135                 140
   Ser Gly Met Gly Thr Leu Leu Ile Ser Lys Val Arg Glu Glu Tyr Pro
   145                 150                 155                 160
   Asp Arg Ile Met Asn Thr Phe Ser Val Val Pro Ser Pro Lys Val Ser
                   165                 170                 175
   Asp Thr Val Val Glu Pro Tyr Asn Ala Thr Leu Ser Ile His Gln Leu
               180                 185                 190
   Val Glu Asn Thr Asp Glu Thr Tyr Cys Ile Asp Asn Glu Ala Leu Tyr
           195                 200                 205
   Asp Ile Cys Phe Arg Thr Leu Lys Leu Ala Thr Pro Thr Tyr Gly Asp
       210                 215                 220
   Leu Asn His Leu Val Ser Ala Thr Met Ser Gly Val Thr Thr Ser Leu
   225                 230                 235                 240
   Arg Phe Pro Gly Gln Leu Asn Ala Asp Leu Arg Lys Leu Ala Val Asn
                   245                 250                 255
   Met Val Pro Phe Pro Arg Leu His Phe Phe Met Pro Gly Phe Ala Pro
               260                 265                 270
   Leu Thr Ala Arg Gly Ser Gln Gln Tyr Arg Ala Leu Thr Val Pro Glu
           275                 280                 285
   Leu Thr Gln Gln Met Phe Asp Ala Lys Asn Met Met Ala Ala Cys Asp
       290                 295                 300
   Pro Arg His Gly Arg Tyr Leu Thr Val Ala Thr Val Phe Arg Gly Arg
   305                 310                 315                 320
   Met Ser Met Lys Glu Val Asp Glu Gln Met Leu Ala Ile Gln Ser Lys
                   325                 330                 335
   Asn Ser Ser Tyr Phe Val Glu Trp Ile Pro Asn Asn Val Lys Val Ala
               340                 345                 350
   Val Cys Asp Ile Pro Pro Arg Gly Leu Lys Met Ser Ser Thr Phe Ile
           355                 360                 365
   Gly Asn Ser Thr Ala Ile Gln Glu Leu Phe Lys Arg Ile Ser Glu Gln
       370                 375                 380
   Phe Thr Ala Met Phe Arg Arg Lys Ala Phe Leu His Trp Tyr Thr Gly
   385                 390                 395                 400
   Glu Gly Met Asp Glu Met Glu Phe Thr Glu Ala Glu Ser Asn Met Asn
                   405                 410                 415
   Asp Leu Val Ser Glu Tyr Gln Gln Tyr Gln Asp Ala Thr Ala Glu Glu
               420                 425                 430
   Glu Gly Glu Met Tyr Glu Asp Asp Asp Glu Glu Ser Glu Ala Gln Gly
           435                 440                 445
   Pro Lys
       450
<210> 109
   <211> 1691
   <212> DNA
   <213> Homo sapiens
<220>
   <221> source
   <222> 1..1691
   <223> /mol_type="DNA"
   /note="GAP43 isoform 2"
   /organism="Homo sapiens"
<400> 109
   tccgagaaag agacggagag agagggaaga aaaagacaga tagatagata ttggggggaa 60
   ggagaaaaaa ggagaagaga gggaagagag gacagcggag agagagcacc agagagagag 120
   ggagagagag agagagcgct agagagaggg agcgagcatg tgcgatgagc aatagctgtg 180
   gaccttacag ttgctgctaa ctgccctggt gtgtgtgagg gagagagagg gagggaggga 240
   gagagagcgc gctagcgcga gagagcgagt gagcaagcga gcagaaaaga ggtggagagg 300
   gggggaataa gaaagagaga gaaggaaagg agagaaggca ggaagaaggc aagggacgag 360
   acaaccatgc tgtgctgtat gagaagaacc aaacaggttg aaaaaaatga tgacgaccaa 420
   aagattgaac aagatggtat caaaccagaa gataaagctc ataaggccgc aaccaaaatt 480
   caggctagct tccgtggaca cataacaagg aaaaagctca aaggagagaa gaaggatgat 540
   gtccaagctg ctgaggctga agctaataag aaggatgaag cccctgttgc cgatggggtg 600
   gagaagaagg gagaaggcac cactactgcc gaagcagccc cagccactgg ctccaagcct 660
   gatgagcccg gcaaagcagg agaaactcct tccgaggaga agaaggggga gggtgatgct 720
   gccacagagc aggcagcccc ccaggctcct gcatcctcag aggagaaggc cggctcagct 780
   gagacagaaa gtgccactaa agcttccact gataactcgc cgtcctccaa ggctgaagat 840
   gccccagcca aggaggagcc taaacaagcc gatgtgcctg ctgctgtcac tgctgctgct 900
   gccaccaccc ctgccgcaga ggatgctgct gccaaggcaa cagcccagcc tccaacggag 960
   actggggaga gcagccaagc tgaagagaac atagaagctg tagatgaaac caaacctaag 1020
   gaaagtgccc ggcaggacga gggtaaagaa gaggaacctg aggctgacca agaacatgcc 1080
   tgaactctaa gaaatggctt tccacatccc caccctcccc tctcctgagc ctgtctctcc 1140
   ctaccctctt ctcagctcca ctctgaagtc ccttcctgtc ctgctcacgt ctgtgagtct 1200
   gtcctttccc acccactagc cctctttctc tctgtgtggc aaacatttaa aaaaaaaaaa 1260
   aaaaagcagg aaagatccca agtcaaacag tgtggcttaa acattttttg tttcttggtg 1320
   ttgttatggc aagtttttgg taatgatgat tcaatcattt tgggaaattc ttgcactgta 1380
   tccaagttat ttgatctggt gcgtgtggcc ctgtgggagt ccactttcct ctctctctct 1440
   ctctctgttc caagtgtgtg tgcaatgttc cgttcatctg aggagtccaa aatatcgagt 1500
   gaattcaaaa tcatttttgt tttcctcctt ttcaatgtga tggaatgaac aaaaaggaaa 1560
   aaattcaaaa aacccagttt gttttaaaaa taaataaata aagcaaatgt gccaattagc 1620
   gtaaacttgc ggctctaagg ctcctttttc aacccgaata ttaataaatc atgagagtaa 1680
   tcaaggtcca a 1691
<210> 110
<211> 238
   <212> PRT
   <213> Homo sapiens
<220>
   <221> SOURCE
   <222> 1..238
   <223> /mol_type="protein"
   /note="GAP43 isoform 2"
   /organism="Homo sapiens"
<400> 110
   Met Leu Cys Cys Met Arg Arg Thr Lys Gln Val Glu Lys Asn Asp Asp
   1               5                   10                  15
   Asp Gln Lys Ile Glu Gln Asp Gly Ile Lys Pro Glu Asp Lys Ala His
               20                  25                  30
   Lys Ala Ala Thr Lys Ile Gln Ala Ser Phe Arg Gly His Ile Thr Arg
           35                  40                  45
   Lys Lys Leu Lys Gly Glu Lys Lys Asp Asp Val Gln Ala Ala Glu Ala
       50                  55                  60
   Glu Ala Asn Lys Lys Asp Glu Ala Pro Val Ala Asp Gly Val Glu Lys
   65                  70                  75                  80
   Lys Gly Glu Gly Thr Thr Thr Ala Glu Ala Ala Pro Ala Thr Gly Ser
                   85                  90                  95
   Lys Pro Asp Glu Pro Gly Lys Ala Gly Glu Thr Pro Ser Glu Glu Lys
               100                 105                 110
   Lys Gly Glu Gly Asp Ala Ala Thr Glu Gln Ala Ala Pro Gln Ala Pro
           115                 120                 125
   Ala Ser Ser Glu Glu Lys Ala Gly Ser Ala Glu Thr Glu Ser Ala Thr
       130                 135                 140
   Lys Ala Ser Thr Asp Asn Ser Pro Ser Ser Lys Ala Glu Asp Ala Pro
   145                 150                 155                 160
   Ala Lys Glu Glu Pro Lys Gln Ala Asp Val Pro Ala Ala Val Thr Ala
                   165                 170                 175
   Ala Ala Ala Thr Thr Pro Ala Ala Glu Asp Ala Ala Ala Lys Ala Thr
               180                 185                 190
   Ala Gln Pro Pro Thr Glu Thr Gly Glu Ser Ser Gln Ala Glu Glu Asn
           195                 200                 205
   Ile Glu Ala Val Asp Glu Thr Lys Pro Lys Glu Ser Ala Arg Gln Asp
       210                 215                 220
   Glu Gly Lys Glu Glu Glu Pro Glu Ala Asp Gln Glu His Ala
   225                 230                 235
<210> 111
   <211> 1901
   <212> DNA
   <213> Homo sapiens
<220>
   <221> source
   <222> 1..1901
   <223> /mol_type="DNA"
   /note="GAP43 isoform 1"
   /organism="Homo sapiens"
<400> 111
   acagttgctg ctaactgccc tggtgtgtgt gagggagaga gagggaggga gggagagaga    60
   gcgcgctagc gcgagagagc gagtgagcaa gcgagcagaa aagaggtgga gaggggggga    120
   ataagaaaga gagagaagga aaggagagaa ggcaggaaga aggcaaggga cgagacaacc    180
   atgctgtgct gtatgagaag aaccaaacag aattaaaagg gaacctggtc tctgggttgt    240
   tttcaacatc tcaagtgtga attttccctg tcaaaatctt cacaaggaaa atgagtcaca    300
   gcatcacctg ggtgacgagg tcataacacc tcagcccttg cttaaaaaat tttatttcta    360
   cttttctatt gtaaagagat ctcaaaacag gaagataaaa ttggactgac agctctacag    420
   cctagtcttt tagacagtga actaggccag cattggcaga cactggcgat gacaaagtcc    480
   tgctctgaat tatgccaccc cgcactccac tttttacctt gcctgggagg cttgaggaaa    540
   aatcttcaga gagcagttcg acctagtcct tattcacttg gcttcttgac tttctggatt    600
   tcaagggttg aaaaaaatga tgacgaccaa aagattgaac aagatggtat caaaccagaa    660
   gataaagctc ataaggccgc aaccaaaatt caggctagct tccgtggaca cataacaagg    720
   aaaaagctca aaggagagaa gaaggatgat gtccaagctg ctgaggctga agctaataag    780
   aaggatgaag cccctgttgc cgatggggtg gagaagaagg gagaaggcac cactactgcc    840
   gaagcagccc cagccactgg ctccaagcct gatgagcccg gcaaagcagg agaaactcct    900
   tccgaggaga agaaggggga gggtgatgct gccacagagc aggcagcccc ccaggctcct    960
   gcatcctcag aggagaaggc cggctcagct gagacagaaa gtgccactaa agcttccact   1020
   gataactcgc cgtcctccaa ggctgaagat gccccagcca aggaggagcc taaacaagcc   1080
   gatgtgcctg ctgctgtcac tgctgctgct gccaccaccc ctgccgcaga ggatgctgct   1140
   gccaaggcaa cagcccagcc tccaacggag actggggaga gcagccaagc tgaagagaac   1200
   atagaagctg tagatgaaac caaacctaag gaaagtgccc ggcaggacga gggtaaagaa   1260
   gaggaacctg aggctgacca agaacatgcc tgaactctaa gaaatggctt tccacatccc   1320
   caccctcccc tctcctgagc ctgtctctcc ctaccctctt ctcagctcca ctctgaagtc   1380
   ccttcctgtc ctgctcacgt ctgtgagtct gtcctttccc acccactagc cctctttctc   1440
   tctgtgtggc aaacatttaa aaaaaaaaaa aaaaagcagg aaagatccca agtcaaacag   1500
   tgtggcttaa acattttttg tttcttggtg ttgttatggc aagtttttgg taatgatgat    1560
   tcaatcattt tgggaaattc ttgcactgta tccaagttat ttgatctggt gcgtgtggcc   1620
   ctgtgggagt ccactttcct ctctctctct ctctctgttc caagtgtgtg tgcaatgttc   1680
   cgttcatctg aggagtccaa aatatcgagt gaattcaaaa tcatttttgt tttcctcctt   1740
   ttcaatgtga tggaatgaac aaaaaggaaa aaattcaaaa aacccagttt gttttaaaaa   1800
   taaataaata aagcaaatgt gccaattagc gtaaacttgc ggctctaagg ctcctttttc    1860
   aacccgaata ttaataaatc atgagagtaa tcaaggtcca a                        1901
<210> 112
   <211> 274
   <212> PRT
   <213> Homo sapiens
<220>
   <221> SOURCE
   <222> 1..274
   <223> /mol_type="protein"
   /note="GAP43 isoform 1"
   /organism="Homo sapiens"
<400> 112
   Met Thr Lys Ser Cys Ser Glu Leu Cys His Pro Ala Leu His Phe Leu
   1               5                   10                  15
   Pro Cys Leu Gly Gly Leu Arg Lys Asn Leu Gln Arg Ala Val Arg Pro
               20                  25                  30
   Ser Pro Tyr Ser Leu Gly Phe Leu Thr Phe Trp Ile Ser Arg Val Glu
           35                  40                  45
   Lys Asn Asp Asp Asp Gln Lys Ile Glu Gln Asp Gly Ile Lys Pro Glu
       50                  55                  60
   Asp Lys Ala His Lys Ala Ala Thr Lys Ile Gln Ala Ser Phe Arg Gly
   65                  70                  75                  80
   His Ile Thr Arg Lys Lys Leu Lys Gly Glu Lys Lys Asp Asp Val Gln
                   85                  90                  95
   Ala Ala Glu Ala Glu Ala Asn Lys Lys Asp Glu Ala Pro Val Ala Asp
               100                 105                 110
   Gly Val Glu Lys Lys Gly Glu Gly Thr Thr Thr Ala Glu Ala Ala Pro
           115                 120                 125
   Ala Thr Gly Ser Lys Pro Asp Glu Pro Gly Lys Ala Gly Glu Thr Pro
       130                 135                 140
   Ser Glu Glu Lys Lys Gly Glu Gly Asp Ala Ala Thr Glu Gln Ala Ala
   145                 150                 155                 160
   Pro Gln Ala Pro Ala Ser Ser Glu Glu Lys Ala Gly Ser Ala Glu Thr
                   165                 170                 175
   Glu Ser Ala Thr Lys Ala Ser Thr Asp Asn Ser Pro Ser Ser Lys Ala
               180                 185                 190
   Glu Asp Ala Pro Ala Lys Glu Glu Pro Lys Gln Ala Asp Val Pro Ala
           195                 200                 205
   Ala Val Thr Ala Ala Ala Ala Thr Thr Pro Ala Ala Glu Asp Ala Ala
       210                 215                 220
   Ala Lys Ala Thr Ala Gln Pro Pro Thr Glu Thr Gly Glu Ser Ser Gln
   225                 230                 235                 240
   Ala Glu Glu Asn Ile Glu Ala Val Asp Glu Thr Lys Pro Lys Glu Ser
                   245                 250                 255
   Ala Arg Gln Asp Glu Gly Lys Glu Glu Glu Pro Glu Ala Asp Gln Glu
               260                 265                 270
   His Ala
<210> 113
   <211> 1481
   <212> DNA
   <213> Rattus norvegicus
<220>
   <221> source
   <222> 1..1481
   <223> /mol_type="DNA"
   /note="GAP43"
   /organism="Rattus norvegicus"
<400> 113
   ctgatcgctg tagaccttac agttgctgct aactgccctg gtgtgtgtga gggagagaga        60
   gggagagaga gggagaggga gggagggagg gagggagagc gcgctagcgc gagagagcga       120
   gtgagcaagc gagcagaaaa gaggtggaga gggggggaat aaggaagaga ggaggaaagg       180
   agagaaggca ggaagaaggc aggggaagat accaccatgc tgtgctgtat gagaagaacc       240
   aaacaggttg aaaagaatga tgaggaccaa aagattgaac aagatggtgt caaaccggag       300
   gataaggctc ataaggctgc aaccaaaatt caggctagct tccgtggaca cataacaagg       360
   aaaaagctca aagacgagaa gaagggtgat gcaccagctg ctgaggccga ggccaaggag       420
   aaggatgatg ctcccgttgc tgatggtgtg gagaagaagg agggagatgg ctctgctact       480
   accgatgcag ccccagccac cagccctaag gctgaggagc ccagcaaggc aggagatgca       540
   ccttctgagg agaagaaggg cgaaggggat gcggcccctt cagaggaaaa ggccggctca       600
   gctgagacag aaagtgctgc taaagctacc actgataact cgccgtcctc caaggccgaa       660
   gatggcccag ccaaggagga gcctaaacaa gccgatgtgc ctgctgctgt cactgatgct       720
   gctgccacca cccctgctgc agaggatgct gccaaggcag cgcagcctcc aacggagact       780
   gcagaaagca gccaagctga ggaggagaaa gaagctgtag atgaagccaa acctaaggaa       840
   agtgcccgac aggatgaggg taaagaagac cccgaggctg accaagaaca tgcctgaact       900
   ttaagaaacg gctttccacg ttgcccccac ctgaaccctg tctctcctgc cctttctcag       960
   atccactctg aagtttcctc tcctgtcctg ctcacgtgtg tgagcctgtc ctctcctacc      1020
   tatgagccct ctctctctgt gtggcaaaca ttaaaaaaaa aaaaaagcag gaaagatccc      1080
   aagtccaaca gtgtggctta aacatttgtt tcttggtgtt gttatggcga gtttttggta      1140
   atgatgatgc agtcatcttg ggaaattctt gcactgtacc ccggtttttt gatctggtgc      1200
   gtgtggccct gtgggagtcc actttcctct ctatttctct ctgttccaag tgtgtgtgtg      1260
   caatgttccg ttctgaggag tccaaaatat taagtgaatt caaaaaacca tttctgtttc      1320
   ctcattttca atgtgatgga atgaacaaaa aggttaaaaa aattaaaaaa aaaacggttt      1380
   gttttaaaaa taaataaata aagcaaatgt gccaattagc gtaacttaag gctgtgaggc      1440
   tcctttttca atctgaatat taataaatca tgagagtaat c                          1481
<210> 114
   <211> 226
   <212> PRT
   <213> Rattus norvegicus
<220>
   <221> SOURCE
   <222> 1..226
   <223> /mol_type="protein"
   /note="GAP43"
   /organism="Rattus norvegicus"
<400> 114
   Met Leu Cys Cys Met Arg Arg Thr Lys Gln Val Glu Lys Asn Asp Glu
   1               5                   10                  15
   Asp Gln Lys Ile Glu Gln Asp Gly Val Lys Pro Glu Asp Lys Ala His
               20                  25                  30
   Lys Ala Ala Thr Lys Ile Gln Ala Ser Phe Arg Gly His Ile Thr Arg
           35                  40                  45
   Lys Lys Leu Lys Asp Glu Lys Lys Gly Asp Ala Pro Ala Ala Glu Ala
       50                  55                  60
   Glu Ala Lys Glu Lys Asp Asp Ala Pro Val Ala Asp Gly Val Glu Lys
   65                  70                  75                  80
   Lys Glu Gly Asp Gly Ser Ala Thr Thr Asp Ala Ala Pro Ala Thr Ser
                   85                  90                  95
   Pro Lys Ala Glu Glu Pro Ser Lys Ala Gly Asp Ala Pro Ser Glu Glu
               100                 105                 110
   Lys Lys Gly Glu Gly Asp Ala Ala Pro Ser Glu Glu Lys Ala Gly Ser
           115                 120                 125
   Ala Glu Thr Glu Ser Ala Ala Lys Ala Thr Thr Asp Asn Ser Pro Ser
       130                 135                 140
   Ser Lys Ala Glu Asp Gly Pro Ala Lys Glu Glu Pro Lys Gln Ala Asp
   145                 150                 155                 160
   Val Pro Ala Ala Val Thr Asp Ala Ala Ala Thr Thr Pro Ala Ala Glu
                   165                 170                 175
   Asp Ala Ala Lys Ala Ala Gln Pro Pro Thr Glu Thr Ala Glu Ser Ser
               180                 185                 190
   Gln Ala Glu Glu Glu Lys Glu Ala Val Asp Glu Ala Lys Pro Lys Glu
           195                 200                 205
   Ser Ala Arg Gln Asp Glu Gly Lys Glu Asp Pro Glu Ala Asp Gln Glu
       210                 215                 220
   His Ala
   225
<210> 115
   <211> 3081
   <212> DNA
   <213> Homo sapiens
<220>
   <221> source
   <222> 1..3081
   <223> /mol_type="DNA"
   /note="GFAP"
   /organism="Homo sapiens"
<400> 115
   agtctagccc actccttcat aaagccctcg catcccagga gcgagcagag ccagagcagg      60
   atggagagga gacgcatcac ctccgctgct cgccgctcct acgtctcctc aggggagatg     120
   atggtggggg gcctggctcc tggccgccgt ctgggtcctg gcacccgcct ctccctggct     180
   cgaatgcccc ctccactccc gacccgggtg gatttctccc tggctggggc actcaatgct     240
   ggcttcaagg agacccgggc cagtgagcgg gcagagatga tggagctcaa tgaccgcttt     300
   gccagctaca tcgagaaggt tcgcttcctg gaacagcaaa acaaggcgct ggctgctgag     360
   ctgaaccagc tgcgggccaa ggagcccacc aagctggcag acgtctacca ggctgagctg     420
   cgagagctgc ggctgcggct cgatcaactc accgccaaca gcgcccggct ggaggttgag     480
   agggacaatc tggcacagga cctggccact gtgaggcaga agctccagga tgaaaccaac     540
   ctgaggctgg aagccgagaa caacctggct gcctatagac aggaagcaga tgaagccacc     600
   ctggcccgtc tggatctgga gaggaagatt gagtcgctgg aggaggagat ccggttcttg     660
   aggaagatcc acgaggagga ggttcgggaa ctccaggagc agctggcccg acagcaggtc     720
   catgtggagc ttgacgtggc caagccagac ctcaccgcag ccctgaaaga gatccgcacg 780
   cagtatgagg caatggcgtc cagcaacatg catgaagccg aagagtggta ccgctccaag 840
   tttgcagacc tgacagacgc tgctgcccgc aacgcggagc tgctccgcca ggccaagcac 900
   gaagccaacg actaccggcg ccagttgcag tccttgacct gcgacctgga gtctctgcgc 960
   ggcacgaacg agtccctgga gaggcagatg cgcgagcagg aggagcggca cgtgcgggag 1020
   gcggccagtt atcaggaggc gctggcgcgg ctggaggaag aggggcagag cctcaaggac 1080
   gagatggccc gccacttgca ggagtaccag gacctgctca atgtcaagct ggccctggac 1140
   atcgagatcg ccacctacag gaagctgcta gagggcgagg agaaccggat caccattccc 1200
   gtgcagacct tctccaacct gcagattcga gaaaccagcc tggacaccaa gtctgtgtca 1260
   gaaggccacc tcaagaggaa catcgtggtg aagaccgtgg agatgcggga tggagaggtc 1320
   attaaggagt ccaagcagga gcacaaggat gtgatgtgag gcaggaccca cctggtggcc 1380
   tctgccccgt ctcatgaggg gcccgagcag aagcaggata gttgctccgc ctctgctggc 1440
   acatttcccc agacctgagc tccccaccac cccagctgct cccctccctc ctctgtccct 1500
   aggtcagctt gctgccctag gctccgtcag tatcaggcct gccagacggc acccacccag 1560
   cacccagcaa ctccaactaa caagaaactc acccccaagg ggcagtctgg aggggcatgg 1620
   ccagcagctt gcgttagaat gaggaggaag gagagaaggg gaggagggcg gggggcacct 1680
   actacatcgc cctccacatc cctgattcct gttgttatgg aaactgttgc cagagatgga 1740
   ggttctctcg gagtatctgg gaactgtgcc tttgagtttc ctcaggctgc tggaggaaaa 1800
   ctgagactca gacaggaaag ggaaggcccc acagacaagg tagccctggc cagaggcttg 1860
   ttttgtcttt tggtttttat gaggtgggat atccctatgc tgcctaggct gaccttgaac 1920
   tcctgggctc aagcagtcta cccacctcag cctcctgtgt agctgggatt atagattgga 1980
   gccaccatgc ccagctcaga gggttgttct cctagactga ccctgatcag tctaagatgg 2040
   gtggggacgt cctgccacct ggggcagtca cctgcccaga tcccagaagg acctcctgag 2100
   cgatgactca agtgtctcag tccacctgag ctgccatcca gggatgccat ctgtgggcac 2160
   gctgtgggca ggtgggagct tgattctcag cacttggggg atctgttgtg tacgtggaga 2220
   gggatgaggt gctgggaggg atagaggggg gctgcctggc ccccagctgt gggtacagag 2280
   aggtcaagcc caggaggact gccccgtgca gactggaggg gacgctggta gagatggagg 2340
   aggaggcaat tgggatggcg ctaggcatac aagtaggggt tgtgggtgac cagttgcact 2400
   tggcctctgg attgtgggaa ttaaggaagt gactcatcct cttgaagatg ctgaaacagg 2460
   agagaaaggg gatgtatcca tgggggcagg gcatgacttt gtcccatttc taaaggcctc 2520
   ttccttgctg tgtcatacca ggccgcccca gcctctgagc ccctgggact gctgcttctt 2580
   aaccccagta agccactgcc acacgtctga ccctctccac cccatagtga ccggctgctt     2640
   ttccctaagc caagggcctc ttgcggtccc ttcttactca cacacaaaat gtacccagta     2700
   ttctaggtag tgccctattt tacaattgta aaactgaggc acgagcaaag tgaagacact     2760
   ggctcatatt cctgcagcct ggaggccggg tgctcagggc tgacacgtcc accccagtgc     2820
   acccactctg ctttgactga gcagactggt gagcagactg gtgggatctg tgcccagaga     2880
   tgggactggg agggcccact tcagggttct cctctcccct ctaaggccga agaagggtcc     2940
   ttccctctcc ccaagacttg gtgtcctttc cctccactcc ttcctgccac ctgctgctgc     3000
   tgctgctgct aatcttcagg gcactgctgc tgcctttagt cgctgaggaa aaataaagac     3060
   aaatgctgcg cccttcccca g                                               3081
<210> 116
   <211> 432
   <212> PRT
   <213> Homo sapiens
<220>
   <221> SOURCE
   <222> 1..432
   <223> /mol_type="protein"
   /note="GFAP"
   /organism="Homo sapiens"
<400> 116
<210> 117
   <211> 1318
   <212> DNA
   <213> Rattus norvegicus
<220>
   <221> source
   <222> 1..1318
   <223> /mol_type="DNA"
   /note="GFAP"
   /organism="Rattus norvegicus".
<400> 117
   caggagccag cagaagcagg gcaagatgga gcggagacgt atcacctctg cacgccgctc      60
   ctatgcctcc tccgagacga tggtcagggg ccatggtcct accagacacc tgggtaccat     120
   tccgcgcctc tccctgtctc gaatgacgcc tccactccct gccagggtag acttctccct     180
   ggccggggcg ctcaatgccg gcttcaaaga gactcgggcc agcgagcgtg cggagatgat     240
   ggagctcaat gaccgctttg ctagctacat cgagaaggtc cgcttcctgg aacagcaaaa     300
   caaggcgctg gcagctgagc tgaaccagct tcgagccaag gagcccacca aactggctga     360
   cgtttaccag gcagaacttc gggagctgcg gctgcgtctg gaccagctta ctaccaacag     420
   tgcccggctg gaggtggaga gggacaatct cacacaggac ctcggcaccc tgaggcagaa     480
   gctccaagat gaaaccaacc tgaggctgga ggcggagaac aacctggctg tgtacagaca     540
   ggaggcggat gaagccacct tggctcgtgt ggatctggag aggaaggttg agtcgctgga     600
   ggaggagatc cagttcttga ggaagatcca tgaggaggaa gttcgagaac tccaggagca     660
   gctggcccag cagcaggtcc acgtggagat ggatgtggcc aagccagacc tcacagcggc     720
   tctgagagag attcgcactc agtacgaggc agtggccacc agtaacatgc aagaaacaga     780
   agagtggtat cggtccaagt ttgcagacct cacagacgtt gcttcccgca acgcagagct     840
   gctccgccag gccaagcacg aggctaatga ctatcgccgc caactgcagg ccttgacctg     900
   cgaccttgag tccttgcgcg gcacgaacga gtccttggag aggcaaatgc gcgaacagga     960
   ggagcgccac gcgcgggagt cggcgagtta ccaggaggca ctcgctcggc tggaggagga    1020
   gggccaaagc ctcaaggagg agatggcccg ccacctgcag gagtaccagg atctactcaa    1080
   cgttaagcta gccctggaca tcgagatcgc cacctacagg aaattgctgg agggcgaaga    1140
   aaaccgcatc accattcctg tacagacttt ctccaacctc cagatccgag aaaccagcct    1200
   ggacaccaaa tctgtgtcag aaggccacct caagaggaac atcgtggtaa agacggtgga    1260
   gatgcgggat ggcgaggtca ttaaggagtc gaagcaggag cacaaggatg tgatgctg      1318
<210> 118
   <211> 430
   <212> PRT
   <213> Rattus norvegicus
<220>
   <221> SOURCE
   <222> 1..430
   <223> /mol_type="protein"
   /note="GFAP"
   /organism="Rattus norvegicus"
<400> 118
   Met Glu Arg Arg Arg Ile Thr Ser Ala Arg Arg Ser Tyr Ala Ser Ser
   1               5                   10                  15
   Glu Thr Met Val Arg Gly His Gly Pro Thr Arg His Leu Gly Thr Ile
               20                  25                  30
   Pro Arg Leu Ser Leu Ser Arg Met Thr Pro Pro Leu Pro Ala Arg Val
           35                  40                  45
   Asp Phe Ser Leu Ala Gly Ala Leu Asn Ala Gly Phe Lys Glu Thr Arg
       50                  55                  60
   Ala Ser Glu Arg Ala Glu Met Met Glu Leu Asn Asp Arg Phe Ala Ser
   65                  70                  75                  80
   Tyr Ile Glu Lys Val Arg Phe Leu Glu Gln Gln Asn Lys Ala Leu Ala
                   85                  90                  95
   Ala Glu Leu Asn Gln Leu Arg Ala Lys Glu Pro Thr Lys Leu Ala Asp
               100                 105                 110
   Val Tyr Gln Ala Glu Leu Arg Glu Leu Arg Leu Arg Leu Asp Gln Leu
           115                 120                 125
   Thr Thr Asn Ser Ala Arg Leu Glu Val Glu Arg Asp Asn Leu Thr Gln
       130                 135                 140
   Asp Leu Gly Thr Leu Arg Gln Lys Leu Gln Asp Glu Thr Asn Leu Arg
   145                 150                 155                 160
   Leu Glu Ala Glu Asn Asn Leu Ala Val Tyr Arg Gln Glu Ala Asp Glu
                   165                 170                 175
   Ala Thr Leu Ala Arg Val Asp Leu Glu Arg Lys Val Glu Ser Leu Glu
               180                 185                 190
   Glu Glu Ile Gln Phe Leu Arg Lys Ile His Glu Glu Glu Val Arg Glu
           195                 200                 205
   Leu Gln Glu Gln Leu Ala Gln Gln Gln Val His Val Glu Met Asp Val
       210                 215                 220
   Ala Lys Pro Asp Leu Thr Ala Ala Leu Arg Glu Ile Arg Thr Gln Tyr
   225                 230                 235                 240
   Glu Ala Val Ala Thr Ser Asn Met Gln Glu Thr Glu Glu Trp Tyr Arg
                           245                     250                     255
   Ser Lys Phe Ala Asp Leu Thr Asp Val Ala Ser Arg Asn Ala Glu Leu
                   260                     265                     270
   Leu Arg Gln Ala Lys His Glu Ala Asn Asp Tyr Arg Arg Gln Leu Gln
               275                     280                     285
   Ala Leu Thr Cys Asp Leu Glu Ser Leu Arg Gly Thr Asn Glu Ser Leu
       290                     295                     300
   Glu Arg Gln Met Arg Glu Gln Glu Glu Arg His Ala Arg Glu Ser Ala
   305                     310                     315                     320
   Ser Tyr Gln Glu Ala Leu Ala Arg Leu Glu Glu Glu Gly Gln Ser Leu
                       325                     330                     335
   Lys Glu Glu Met Ala Arg His Leu Gln Glu Tyr Gln Asp Leu Leu Asn
                   340                     345                     350
   Val Lys Leu Ala Leu Asp Ile Glu Ile Ala Thr Tyr Arg Lys Leu Leu
               355                     360                     365
   Glu Gly Glu Glu Asn Arg Ile Thr Ile Pro Val Gln Thr Phe Ser Asn
       370                     375                     380
   Leu Gln Ile Arg Glu Thr Ser Leu Asp Thr Lys Ser Val Ser Glu Gly
   385                     390                     395                     400
   His Leu Lys Arg Asn Ile Val Val Lys Thr Val Glu Met Arg Asp Gly
                       405                     410                     415
   Glu Val Ile Lys Glu Ser Lys Gln Glu His Lys Asp Val Met
                   420                     425                     430

## Description

The present invention relates to methods for the generation of pluripotent cells derived from tendon perivascular cells. Pluripotent cells can also be derived from ligament perivascular cells. These methods may involve the formation of tenospheres (i.e. cellular aggregates containing pluripotent cells derived from tendon and/or ligament). Accordingly, also methods for generating tenospheres are subject of the present disclosure. Furthermore, the present disclosure concerns pluripotent cells and/or tenospheres which can be obtained by the herein provided methods as well as methods for differentiating these pluripotent cells and/or tenospheres into a desired cell type.
The possibility of generating pluripotent cells by reprogramming somatic tissue, i.e. the technology to produce induced pluripotent stem cells (IPS), presents a revolution in stem cell biology and conceivably in regenerative medicine. So far, the induction of pluripotency requires either genetic manipulation of somatic cells such as overexpression of the stemness associated genes Oct3/4, Sox2, Klf4, c-myc, nanog, and/or treatment with small molecule compounds affecting pluripotency associated signaling pathways such as TGF-beta. The IPS technology is still accompanied with concerns that present major limitations in its translational aspects such as i) the process of generating iPS is still time consuming and thus not applicable for therapies for acute interventions, and ii) the currently available IPS technology generates cells with a yet unsolved tumor risk [1]. Hence, the identification of cells in adult somatic tissue that would generate pluripotent cells within a short period of time and with low tumorigenicity represents a yet unmet need in stem cell research and regenerative / translational medicine.
In the art, a certain kind of bone marrow stem cell has been identified which express markers of pluripotent stem cells (PSC) such as SSEA-1, Oct-4 and Rex-1; see Kucia (2006) 20, 857-869. This rare non-hematopoietic stem cell population has been termed "very small embryonic-like (VSEL)" stem cells. In a later publication in 2009, an increase in the number of circulating cells expressing the VSEL stem cell phenotype was found in stroke patients and this increase is said to have potential prognostic value in stroke patients; see Paczkowska (2009) Stroke 40, 1237-1244. Furthermore, Zuba-Surma (2010) report on a potential therapeutic utility of VSEL stem cells treated with a cardiogenic medium for cardiac repair; see Zuba-Surma (2010) Journal of Cellular and Molecular Medicine, epublication ahead of print (DOI: 10.1111/j.1582-4934.2010.01126.x). Yiming (2010) discloses that bone-marrow derived VSEL stem cells express markers for pancreatic development and are mobilized into peripheral tissues after pancreatic damage. Thus, the VSEL stem cells may, according to the authors of this document, contribute to beta-cell regeneration and may transiently improve the function of damaged pancreas; see Yiming (2010) Transplantation 89, 677-685. In sum, these documents report on the existence of a certain cell population in bone marrow which express pluripotent markers; however, a method allowing the preparation of pluripotent cells wherein tendon or ligament perivascular cells are cultivated and spheres containing pluripotent cells are formed is not disclosed in the prior art.
Thus, the technical problem underlying the present invention is the provision of means and methods for obtaining pluripotent stem cells.
The technical problem is solved by provision of the embodiments characterized in the claims.

Accordingly, the present invention relates to a method for the in vitro generation of pluripotent cells (Oct4+; nanog+; Sox2+; Klf4+; c-myc+), said method comprising
a) providing a population of tendon perivascular cells (TPC) or of ligament perivascular cells;
b) cultivating the tendon perivascular cells or ligament perivascular cells of a) in a medium for a time sufficient for a tenosphere to form;
c) recovering pluripotent cells (Oct4+; nanog+; Sox2+; Klf4+; c-myc+) from the tenosphere.

The tenospheres (i.e. cellular aggregates containing pluripotent cells derived from tendon and/or ligament) provided herein are specific and particular cellular structures that can be obtained by the herein described methods, in particular if tendon or ligament perivascular cells are used as starting material. Such tenospheres comprise pluripotent cells (Oct-4+, Nanog+, Sox-2+, c-myc+ and, optionally, Klf4+) which can easily be isolated and purified from the tenospheres and further cultivated or differentiated in a desired cell type as explained below and demonstrated in the appended examples. The present invention solves the above identified technical problem since, as documented herein below and in the appended examples, it was surprisingly found that tendon perivascular cells and/or ligament perivascular cells are an excellent source to be used in the generation of pluripotent cells and tenospheres.

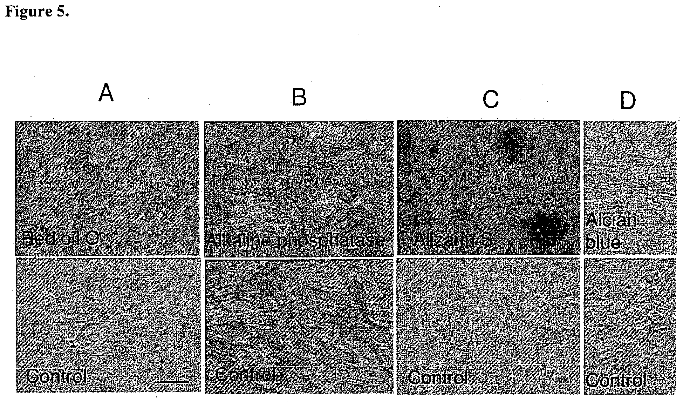

The present invention provides the following advantages:
- the tendon and/or ligament perivascular cells used in the methods for generating pluripotent cells can be easily obtained by biopsies throughout the entire lifespan of humans
- the methods of the present invention allow the cultivation and enrichment of pluripotent cells by formation of tenospheres (cellular aggregates containing pluripotent cells)
- the pluripotent cells provided herein have indeed pluripotent characteristics. The pluripotent capacity is reflected in the expression of pluripotent markers (Oct4-4+, Nanog+, Sox-2+, c-myc+ and, optionally, Klf4+). Further, it is shown herein that the pluripotent-like cells are capable of differentiating into cells of all three germ layers.
- the pluripotent cells retain their pluripotent capacity. These cells can be passaged more than 20 times and retain their pluripotent capacity (i.e. the pluripotent cells were differentiated to mesenchymal stem cells which were capable of further differentiation). In contrast, mesenchymal stem cells obtained from bone marrow or adipose tissue lost after about 6 to 7 passages the capacity to differentiate (i.e. these cells became sensecent).
- the pluripotent cells express Glut2 at the cell surface; this allows purification and enrichment of the pluripotent cells
- herein provided pluripotent cells did not induce the formation of tumors in an animal model (i.e. they do not have the side-effects reported for iPS cells)
- tenospheres containing pluripotent cells are not adherent (i.e. they are free floating in the medium); thus, the tenospheres can easily be purified from the medium
- the pluripotent cells or tenospheres containing same can be used for the generation of mesenchymal stem cells with a particularly high capacity to differentiate into a desired cell type: pluripotent cells/tenospheres are cultivated in the presence of serum which leads to adherent cells/cell clusters and the formation of "true" mesenchymal stem cells. These mesenchymal stem cells are capable of differentiating to osteoblasts and adipocytes at a particularly high rate; see Figure 5. About 95 % of the mesenchymal stem cells derived from the herein provided pluripotent cells differentiate to adipocytes and osteoblasts. In contrast, less than 60 % of bone marrow mesenchymal stem cells obtained by conventional means differentiate into adipocytes and less than 40 % of of bone marrow mesenchymal stem cells differentiate to osteoblasts.
- the protocols provided herein for the generation of pluripotent cells / tenospheres lead to the formation of a cell population within the tenospheres comprising various cell types, such as mesenchymal stem and progenitor cells and neuronal stem cells. This is advantageous as the mesenchymal stem cells "automatically" (i.e. no additional measures are needed) induce the differentiation of neuronal stem and progenitor cells to oligodendrocyte precursor cells
- the isolation of vessels from tendon tissue is easier than from any other tissue (such as brain, muscle or skin), due to the high content of collagens, which can be removed by collagenase digestion. Following digestion, the complex of vascular and perivascular cells remains intact and can easily be isolated for further treatment.

Typically, most organs and tissues such as brain or bone marrow house their stem and progenitor cells closely associated to the vasculature. The cellular and extracellular microenvironment that accommodates stem and progenitor cells has been recognized as a specialized stem cell and perivascular associated niche. For example, the perivascular area represents the stem cell niche of mesenchymal stem cells in various tissues, such as skeletal muscle, bone marrow, placenta and umbilical cord, and neural stem cells in the brain [5,6,7]. The perivascular area has been widely discussed to be a potential niche for mesenchymal stem cells (MSCs) in various tissues, such as bone marrow, skeletal muscle and adipose tissue [8,14,15,16]. Mesenchymal stem cells are mulipotent, but not pluripotent, i.e. these cells have a predetermined fate to differentiate into a specific cell type of the mesenchymal lineage. Though the perivascular area represents the stem cell niche of certain multipotent stem cells in various tissues, the perivascular area has not been proposed in the art as a source for potential pluripotent cells. Also cells in the tendon perivascular area have merely been described to express markers known to be specific for multipotent (not pluripotent) mesenchymal, hematopoietic and neural stem cells; Tempfer (2009) found that such cells merely express markers known to be specific for mesenchymal, hematopoietic and neural stem cells, such as smooth muscle actin (SMA), CD133, Nestin and Musashi 1; see Tempfer (2009) Histochem Cell Biol 131, 733-741 [8]. In the present invention a cell population was unexpectedly identified in the tendon perivascular niche which shows a characteristic expression pattern of pluripotent markers (Oct-4+, Nanog+, Sox-2+, c-myc+ and, optionally, Klf4+).
Zhou (2010) and Zhang (2010) have investigated the cell types of tendons per se (i.e. these documents are not concerned with the tendon perivascular niche). These documents describe that tendons not only contain tenocytes, the resident cells of tendons, but also a certain type of stem cells, referred to as tendon stem/progenitor cells (TSPCs) which, inter alia, express nucleostamin, Oct-4 and SSEA-4; see Zhou (2010), Aging Cell 9, 911-915 and Zhang (2010), BMC Musculoskeletal Disorders 11:10. However, Zhou (2010) and Zhang (2010) do not describe that these cells express pluripotent markers, Nanog+, Sox-2+, c-myc+ and, Klf4+. This lack of pluripotent markers is in line with the finding that TSPCs appear to have a determined fate since they merely form tendon-like, fibrocartilage-like and bone-like tissues as described in Zhang (2010); i.e. these cells are not pluripotent as Zhang does not describe that they are capable of differentiating into cells of all three germ layers.
In sum, the prior art does not disclose any cells in tendons or in the vicinity of tendons which express pluripotent markers (Oct-4+, Nanog+, Sox-2+, c-myc+ and, optionally, Klf4+). Therefore, the findings in the prior art support that neither tendons nor cells in the tendon perivascular area would not have been expected to be a suitable source for the generation of pluripotent cells. Usually, stem cells are thought to occur in proliferative tissue which is capable of regeneration. Tendon or ligament is not such a proliferative tissue and does not regenerate well.
Against all odds, it is demonstrated herein that a population of tendon perivascular cells express embryonic-stem cell-associated/pluripotency markers such as Oct-4, Nanog, Sox-2, c-myc and, optionally, Klf4. No such characteristic expression could be detected in the perivascular area of other tissues or organs, such as rat brain, human subcutaneous adipose tissue and human periosteumeum; see Example 1. The data provided in the appended Examples show that the perivascular area of human tendons is a niche for stem cells expressing the markers Oct-4, Nanog, c-myc, and SOX2 on protein level detected by IHC and Oct-4, Nanog, Sox-2, c-myc and Klf4 on mRNA level detected by RT-PCT. The expression of these marker genes is associated with pluripotency as shown herein. ,It is believed that this characteristic expression pattern is one reason why tendon perivascular cells are a particularly suitable source for the pluripotent cells (Oct-4+, Nanog+, Sox-2+, c-myc+ and, optionally, Klf4+) and tenospheres generated in accordance with the present invention.

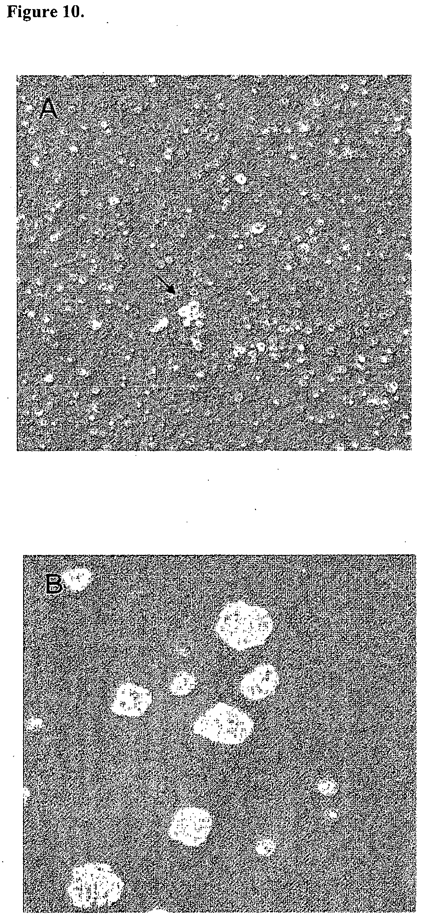

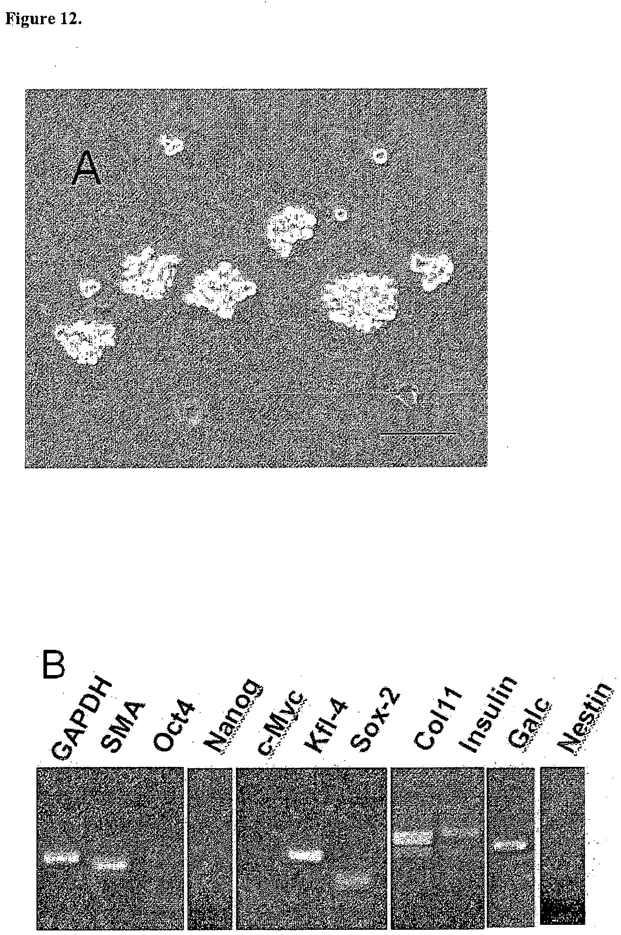

The prior art summarized below reports on certain multipotent (not pluripotent) cells; however, none of these documents provides a hint to tendon perivascular cells as a potential source for pluripotent stem cells.
A cell type Oct4+SSEA-1+Sca-1+lin-CD45- referred to as "very small embryonic like cells" in the art as discussed above may have a multipotent capacity. Yet, these cells are, in contrast to the pluripotent cells provided herein, derived from murine and human bone marrow. VSEL stem cells express Oct-4, are multipotent and do not form tumors in vivo [2]. However, the phenotype of pluripotent cells achieved under ES-cell specific culture conditions as described herein points towards fundamental differences between the herein provided pluripotent cells and VSEL cells. Smukler (2011) Cell Stem Cell 8, 281-293, describes that the adult mammalian pancrease contains a small population of insulin+ multipotent stem cell. A population of blood-derived stem cells described by Romangnani et al. (2007) [20] expressing Oct-4, Nanog and Sox 2 are not considered to be the direct source of the pluripotent cells described herein: no spheres and, accordingly, cells with a phenotype comparable to the herein provided tenospheres and pluripotent cells were obtained when culturing both human Buffy-coats; see Example 3 and Figure 10. A stem cell type displaying certain similarities to the pluripotent cells described herein coexpressing Oct-4 and Nestin was recently found in the human endometrium, with capacity to differentiate towards osteoblasts, chondrocytes, neurons and insulin producing cells. However, the art is silent on the niche of these cells and this document does also not even speculate that tendon perivascular cells might be a potential source for pluripotent cells [21]. Further, the present invention provides for the first time methods allowing the formation of tenospheres containing pluripotent cells, whereby tendon or ligament perivascular cells are used as "starting material" (ligament is a tissue that is structurally similar to tendon). By the formation of tenospheres, the present invention provides means for the preparation and enrichment of pluripotent cells. It was found herein that not only tendon samples but also ligament samples can advantageously be used to generate pluripotent cells and tenospheres containing such cells; see Example 5 and Figure 12.

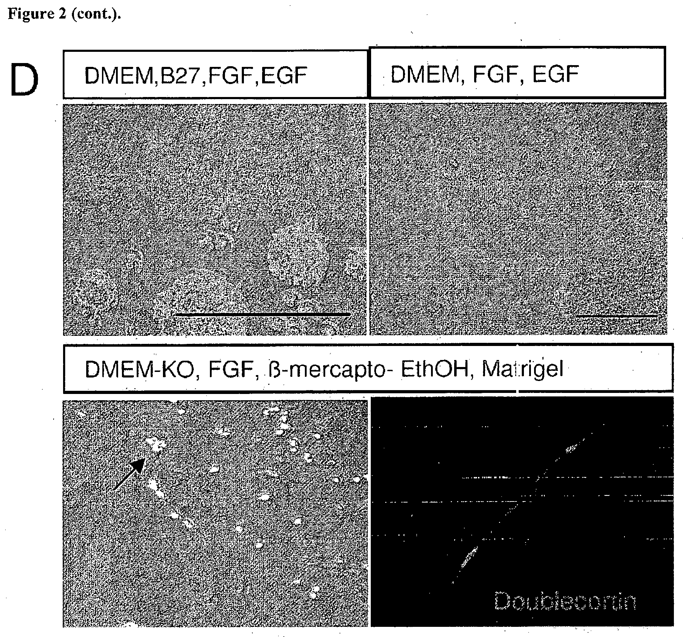

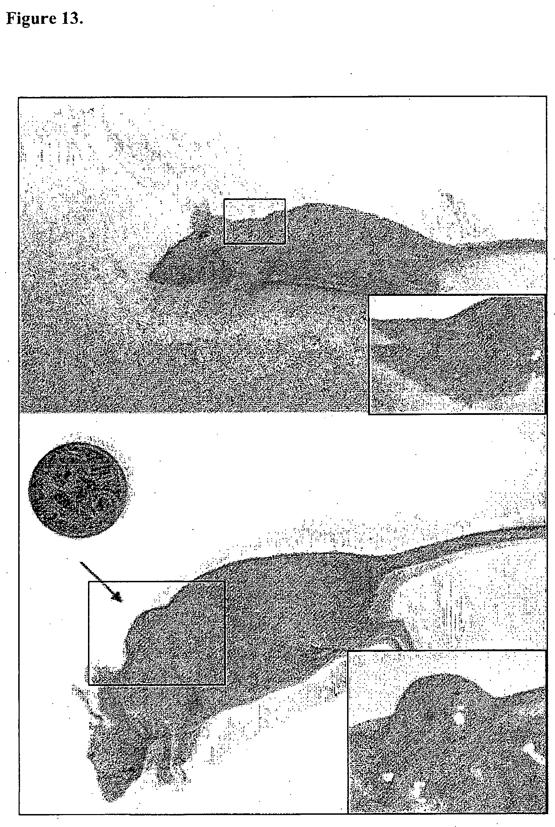

It was also found that perivascular cells derived from tissues or organs other than the tendon or ligament, like the periosteumeum or adipose tissue did not give rise to any kind of spheres containing pluripotent cells, let alone spheres resembling the tenospheres provided herein, even if such perivascular cells were cultured under the very same identical conditions as disclosed herein in respect of tendon perivascular cells or ligament perivascular cells. Accordingly, no pluripotent cells could be obtained from such periosteumeum or adipose perivascular cells. Thus, it has been found that tendon perivascular cells or ligament perivascular cells are a suitable starting material for the generation of pluripotent cells, whereas perivascular cells of other tissues are not suitable for this purpose. Ligament and tendon samples are an advantageous source of pluripotent cells, as such samples can be easily obtained by biopsies throughout the entire lifespan of an animal, preferably a human being. It has been found herein that also samples obtained from elderly patients are a suitable source for the generation of pluripotent cells in accordance with the present invention.
It has been found herein for the first time that tendon perivascular cells express embryonic stem cell like characteristics in vivo and are capable of retaining a remarkably high differentiation potential in vitro. TPCs were directly isolated from microvessels to examine their stem cell capacity. Further, it has been found herein that digestion (i.e. removal of collagen) of tendon or ligament samples in the presence of serum is advantageous in that it promotes the formation of tenospheres containing pluripotent cells. In the absence of serum, less tenospheres and more mesenchymal stem cells form.
Another surprising finding was that a typical medium routinely used in the art for maintenance or induction of embryonic stem cells, namely Dulbeccos Modified Eagle Medium (DMEM) serum knockout medium, is not advantageous for generating or maintaining pluripotent cells derived from tendon perivascular cells. As demonstrated in appended Example 1, use of this medium resulted in the expression of neural markers (such as doublecortin and GFAP) and the adherence of cells to the plastic dish; however, the tendon perivascular cells did not give rise to floating tenospheres comprising pluripotent cells. At most, aggregates or clusters of cells were formed; see Fig. 2D. However, these aggregated cells/cluster cells did not express the markers Oct-4, Nanog, Sox-2, c-myc and, optionally, Klf4 characteristic for the pluripotent cells generated in accordance with the present invention and comprised in the tenospheres obtained by the herein provided methods. Whereas almost all the aggregated cells/cluster cells expressed c-myc, only about 10 % of the cells expressed Sox-2 and none of the cells expressed Oct-4, nanog or Klf-4. Thus, these cells differ essentially from the pluripotent cells provided in the present invention.
In contrast to the use of Dulbeccos Modified Eagle Medium (DMEM) alone, it was found that Dulbeccos Modified Eagle Medium (DMEM) or Neurobasal medium (NB) if supplemented with B27 and, optionally, supplemented with FGF and/or EGF, leads to the formation of more tenospheres which comprise pluripotent cells (Oct-4+, Nanog+, Sox-2+, c-myc+ and, optionally, Klf4+) derived from perivascular tendon cells. The use of B27 supplement in context of induction or maintencance of pluripotency is particularly counterintuitive, since this B27 supplement is commonly used to induce the formation of multipotent, neural-restricted stem cells. Also EGF and/or FGF are routinely used for inducing the formation of lineage restricted stem cells.
Another crucial finding of the present invention was that an initial high seeding density is advantageous for the formation of tenospheres and, accordingly, for the generation of pluripotent cells. Therefore, it is preferred that the methods of the present invention are performed under high cell density conditions as defined and described below. An influence of the seeding density has not been described in the generation of "spheres" for example from bone marrow VSEL stem cells. Kucia (loc. cit.) report that a certain percentage of purified VSEL stem cells are able to form spheres that resemble embryoid bodies if plated over S2C12 murine sarcoma feeder layer. However, the culture conditions described in Kucia (loc. cit.) are entirely different from those provided herein for the generation of pluripotent cells derived from tendon perivascular cells and/or ligament perivascular cells.
Furthermore, it has been found that the change of the medium in an interval of two weeks or more is beneficial for the formation of tenospheres and, accordingly, for the generation of pluripotent cells. The change of the medium at such large intervals is unusual in the art and yet another indicator that the present invention is not obvious.
As mentioned above, the iPS technology which is presently used for the generation of pluripotent stem cells derived from somatic tissue, is not only disadvantageous in that it requires genetic manipulation of somatic cells but is also associated with a yet unsolved tumor risk, whereby this tumor risk may be associated with the use of viral vectors for overexpression of pluripotent marker genes like Oct-4, Sox-2, Klf4, c-myc and nanog. The present invention avoids the use of such genetic manipulation and is also advantageous in that the pluripotent cells provided herein do not lead to the generation of tumors as shown in Example 1 and Figure 13. The lacking capacity of pluripotent cells derived from tendon perivascular cells to form tumors following implantation indicates fundamental differences between these cells and iPS cells. This finding may be in line with the fact that there are no reports of primary tendon or ligament tumors.

As demonstrated herein, the pluripotent cells can easily be differentiated into a desired cell type. Exemplary protocols for differentiation into cells of the neural lineage, into mesenchymal cells and endodermal cells are described below and have also been employed in the appended Example 1 and Example 2.
In sum, it is shown in the Examples that pluripotent cells derived from tendon perivascular cells are capable of differentiating towards the neural lineage, giving rise to glial cells expressing GFAP or GalC. This may be in line with the fact that tendon cells (however, not tendon perivascular cells) express neural cell associated markers in vivo, such as Nestin, Musashi 1, nicotinic and muscarinic (M2) ACh receptors, substance P and neurokinin-1 receptor in vivo [8,9,10].
However, and unexpectedly, it was also found herein that pluripotent cells provided in accordance with the present invention are also capable of differentiating into cells of two other lineageas, namely mesenchymal cells and endodermal cells. It is shown herein that pluripotent cells could also be successfully induced to differentiate towards Mesenchymal cells, identified as osteoblasts and adipocytes. This may be in consistence with previous reports where it was shown that tendon derived stem cells (however, and importantly, not cells derived from tendon perivascular cells) give rise to mesenchymal cells [8,17,18,19]. It is shown herein that tendon perivascular cells display mesenchymal stem cell-like features.
Furthermore, it was surprisingly found that pluripotent cells comprised in the tenosphere "spontaneously" express mRNA encoding for insulin and Insulin/Proinsulin protein, even after 15 passages.
The capacity of pluripotent cells derived from tendon perivascular cells and/or ligament perivascular cells to differentiate into cells associated with all three embryonic germ layers indicates that pluripotent cells and, potentially, tendon perivascular cells and/or ligament perivascular cells represent a more undifferentiated cell type than MSCs. Thus, the present invention also provides the proof of principle that the pluripotent cells provided herein can be differentiated into cells of all three lineages/ into all three germ layers and are, indeed pluripotent cells, i.e. have pluripotent capacity. It is clear that the pluripotent-like cells provided herein are, therefore, highly advantageous in various applications, in particular in medicine, tissue engineering, biotechnology and pharma development (for example in high throuput screening programs).
Taken together, it has been found herein that stem cells reside in the tendon perivascular area, express ES-cell associated markers, have a very broad differentiation capacity, and are likely non-tumorigenic. Therefore, such cells as well as pluripotent cells derived therefrom as described herein may, therefore, be a valuable source for a number of applications in the field of cell based therapy and tissue engineering.

The present invention and its various advantageous applications will now be described in more detail.
As mentioned above, the present invention relates to a method for the in vitro generation of pluripotent cells (Oct4+; nanog+; Sox2+; Klf4+; c-myc+), said method comprising
a) providing a population of tendon perivascular cells (TPC) and/or ligament perivascular cells;
b) cultivating the tendon perivascular cells and/or ligament perivascular cells of a) in a medium for a time sufficient for a tenosphere to form;
c) recovering pluripotent cells (Oct4+; nanog+; Sox2+; Klf4+; c-myc+) from the tenosphere.

In one aspect, the present invention relates, accordingly, to a method for the in vitro generation of pluripotent cells (Oct4+; nanog+; Sox2+; Klf4+; c-myc+), said method comprising
a) providing a population of tendon perivascular cells (TPC);
b) cultivating the tendon perivascular cells of a) in a medium for a time sufficient for a tenosphere to form;
c) recovering pluripotent cells (Oct4+; nanog+; Sox2+; Klf4+; c-myc+) from the tenosphere.

In one aspect, the present invention relates to a method for the in vitro generation of pluripotent cells (Oct4+; nanog+; Sox2+; Klf4+; c-myc+), said method comprising
a) providing a population of ligament perivascular cells;
b) cultivating the ligament perivascular cells of a) in a medium for a time sufficient for a tenosphere to form;
c) recovering pluripotent cells (Oct4+; nanog+; Sox2+; Klf4+; c-myc+) from the tenosphere.

In one aspect, the present invention relates to a method for the in vitro generation of pluripotent cells (Oct4+; nanog+; Sox2+; Klf4+; c-myc+), said method comprising
a) providing a population of tendon perivascular cells (TPC) and of ligament perivascular cells;
b) cultivating the tendon perivascular cells and ligament perivascular cells of a) in a medium for a time sufficient for a tenosphere to form;
c) recovering pluripotent cells (Oct4+; nanog+; Sox2+; Klf4+; c-myc+) from the tenosphere.

The term "pluripotent" as used herein in the characterisation of the cells generated by the methods of the present invention refers to the pluripotent characteristics of the cells. The term "pluripotency" or "pluripotent cell" is well known in the art and described, for example, in Jaenisch (2008 Feb 22) Cell, 132(4), 567-82. "Pluripotency" denotes the ability of a cell to give rise to all cells of the embryo. For example, cells of the inner cell mass (ICM, i.e. cells of the blastocyst embryo that appear transiently during development and give rise to the three germ layers of the developing embryo) and its derivative, embryonic stem (ES) cells are pluripotent. Generally, pluripotent stem cells are capable of forming chimera after in vitro implantation of the pluripotent stem cells in a blastocytes. Such pluripotent stem cells are introduced into all three germ layers. After differentiation of cells of the three germ layers viable chimera form which comprise a mixture of cells derived from the introduced pluripotent stem cells and "natural" blastocyte cells. The term "embryonic stem cells" refers to pluripotent cell lines derived from the ICM upon explantation in culture which can differentiate in vitro into many different lineages and cell types and, upon injection into blastocysts, can give rise to all tissues including the germline.

Accordingly, it is one characteristic feature of the pluripotent cells provided and generated herein that these cells are capable of differentiating into cells of all three germ layers. In other words, the pluripotent cells are capable of differentiating into cells which are similar to cells (i.e. which have similar characteristics of cells), which differentiate in a natural environment (e.g. in vivo, like in a mammal body, such as in the human body) from cells of the three germ layers. This capacity is also shown in the appended Examples which demonstrate that the pluripotent cells can differentiate into a cell type of interest. For example, it was found herein that the pluripotent cells are capable of differentiating into a neuronal cell, a mesenchymal cell and an endodermal cell. Thus, the pluripotent cells are capable of differentiating into cells which are similar to, for example, neuronal cells, mesenchymal cells and endodermals cells, which would differentiate in a natural environment from cells of the three germ layers. Because the differentiated cells obtained by methods using the herein provided pluripotent cells, the differentiated cells are likely somewhat different to cells which have differentiated from cells of the three germ layers in a natural environment. Though most characteristics are essentially the same, the differentiated cells provided in accordance with the present invention may, for example, show a somewhat different expression pattern as compared to a corresponding cell (i.e. cell of the same cell type, like oligodendrocyte, astrocyte, neuron, adipocytes, osteoblast) differentiated in a natural environment. Such a different expression pattern, may, for example, be visualized by techniques in the art, like microarrays etc. Accordingly, the differentiated cells provided herein (the "cell type of interest") may be denoted herein as, for example, "neuronal cell-like", "mesenchymal cell-like", "endodermal cell-like", such as "glial cell-like", "oligodendrocyte-like", "astrocyte-like", "neuron-like", "adipocyte-like", "osteoblast-like", "insulin-producing cell-like" and so on. The above terms can be used interchangeably herein with the terms neuronal cell, mesenchymal cell, endodermal cell, glial cell, oligodendrocyte, astrocyte, neuron, adipocyte, osteoblast, insulin-producing cell, respectively.

Further, the herein provided pluripotent cells express well-known pluripotent marker genes Oct4, nanog, Sox2, Klf4 and c-myc as described below in more detail. The expression of these markers is also characteristic for pluripotent stem cells. In accordance with the above, the capacity to differentiate into cells of all three germ layers (or of all three lineages) and/or to express pluripotent marker genes like Oct4, nanog, Sox2, Klf4 and c-myc can be subsumized herein as "pluripotent-like".
In contrast, "multipotency" refers to the ability of a cell to give rise to different cell types of a given cell lineage. These cells include most adult stem cells, such as gut stem cells, skin stem cells, hematopoietic stem cells and neural stem cells.
The term "pluripotent" (which can be used herein interchangeably with "pluripotent-like") has been chosen in context of the present invention to distinguish the cells obtained by the methods of the invention from embryonic stem cells and cells of the embryo, respectively and also from induced pluripotent stem (iPS) cells. It is generally recognized that the term "iPS cells" refers to cells generated by the overexpression of specific transcription factors in mouse or human somatic cells which are molecularly and functionally highly similar to ES cell counterparts. In contrast to known pluripotent cells, the pluripotent cells provided herein are derived from perivascular tendon and/or ligament cells/samples.
As shown herein, the pluripotent cells/pluripotent-like cells of the invention express well-known pluripotent marker genes, i.e. they express markers/proteins which are expressed in known pluripotent cells. Non-limiting examples of such marker genes are Oct4, nanog, Sox2, Klf4 and c-myc. It is preferred that the herein provided and described pluripotent cells express at least one of the above genes, preferably at least Oct4, optionally in combination with at least one of the remaining marker genes. Accordingly, it is preferred that the pluripotent cells express at least Oct4, more preferably at least Oct4 and Sox2, even more preferably at least Oct4, Sox2 and nanog, more preferably at least Oct4, Sox2, nanog, and Klf4, and most preferably, at least Oct4, Sox2, nanog, Klf4 and c-myc. Should Oct4 not be expressed, it is preferred herein that at least one of the following genes is expressed in the pluripotent cells: nanog, Klf4, c-myc, Sox2, (in order of decreasing preference), optionally in combination with each other or in combination with further pluripotent marker genes. In the context of the above described method, it is particularly preferred that the pluripotent cells express the following combinations of marker genes:
Oct4, nanog and Sox2;
Oct4, nanog, Klf4 and Sox2; or
Oct4, nanog, Klf4, c-myc and Sox2.

It is believed that this expression of very specific marker genes is also characteristic for certain tendon perivascular cells (and/or ligament perivascular cells) used as starting material in the above desribed method for generating pluripotent cells. Examplary nucleic acid sequences and amino acid sequences of Oct4, nanog, Klf4, c-myc and Sox2 to be used herein are shown in SEQ ID NO: 55/56 (Oct4), SEQ ID NO: 63/64 (c-myc), SEQ ID NO: 65/66 (Klf-4), SEQ ID NO: 67/68 (Sox-2) and SEQ ID NO: 69/70 (nanog). These sequences and further sequences to be used herein are shown below and may be deduced from the respective databases.

The denomination "+" (e.g. Oct4+) indicates the presence of the respective marker, i.e. indicates an expression of the marker that may be reflected in a detectable expression level (protein, mRNA etc.) of this marker gene. Generally, a cell is classified as "pluripotent" if at least one of the herein described marker genes is present at a detectable expression level. For example, the at least one marker genes may be expressed at a expression level comparable to a positive control, like known pluripotent cells (e.g. MEL-1, human embryonic stem, cell line). Methods for detecting and evaluating expression levels of such genes are well known in the art and are also demonstrated in the appended examples.

For example, the protein expression level can be detected by taking advantage of immunoagglutination, immunoprecipitation (e.g. immunodiffusion, immunelectrophoresis, immune fixation), western blotting techniques, (in situ) immuno histochemistry, (in situ) immuno cytochemistry, affinity chromatography, enzyme immunoassays, and the like. These and other suitable methods of contacting proteins are well known in the art and are, for example, also described in Sambrook and Russell (2001), Molecular Cloning: A Laboratory Manual, CSH Press, Cold Spring Harbor, NY, USA; Burke et al. (1990), Methods in Yeast Genetics, A Laboratory Course Manual, Cold Spring Harbor Laboratory Press, 1990.)

If the gene product is an mRNA, contacting and binding can be performed by taking advantage of Northern blotting techniques or PCR techniques, like in-situ PCR. These and other suitable methods for binding (specific) mRNA are well known in the art and are, for example, described in Sambrook and Russell (2001, loc. cit.).

Quantification of the protein level can be performed by taking advantage of the techniques referred to above, in particular Western blotting techniques. Generally, the skilled person is aware of methods for the quantitation of polypeptides. Amounts of purified polypeptide in solution can be determined by physical methods, e.g. photometry. Methods of quantifying a particular polypeptide in a mixture rely on specific binding, e.g of antibodies. Specific detection and quantitation methods exploiting the specificity of antibodies comprise for example immunohistochemistry (in situ). Western blotting combines separation of a mixture of proteins by electrophoresis and specific detection with antibodies. Electrophoresis may be multidimensional such as 2D electrophoresis. Usually, polypeptides are separated in 2D electrophoresis by their apparent molecular weight along one dimension and by their isoelectric point along the other direction.
Quantification of the level of the gene product, in particular RNA/mRNA can be performed by taking advantage of Northern blotting techniques, hybridization on microarrays or DNA chips equipped with one or more probes or probe sets specific for mRNA transcripts or PCR techniques referred to above, like, for example, quantitative PCR techniques, such as Real Time PCR. A skilled person is capable of determining the amount of the component, in particular said gene products, by taking advantage of a correlation, preferably a linear correlation, between the intensity of a signal and the amount of the component to be determined.
Non-limiting examples of gene products the amount of which is to be determined are the herein described markers like Oct4, Sox2, nanog, Klf4 and c-myc. These markers are well known in the art. The nucleic acid and amino acid sequences of Oct4, Sox2, nanog, Klf4 and c-myc are provided herein below and also the accession numbers of the respective database is given.
A person skilled in the art is easily in the position to determine the expression level of the herein described marker genes based on his common general knowledge and the teaching provided herein. In particular, a skilled person can readily decide whether a certain marker is or is not expressed in a cell, tissue or the herein described tenospheres.

The herein disclosed method may comprise a step of providing a population of tendon perivascular cells and/or ligament perivascular cells.
The term "perivascular cell(s)" refers generally to (a) cell(s) that locate close to all kinds of blood and lymph vessels (e.g. capillaries, arterioles, artieries, venoles, venes). The perivascular cell(s) can either have direct contact with the endothelium or be separated by one or more layers of basal lamina, or by layers of vessel associated cells like smooth muscle cells. The perivascular cell(s) can originate from endothelial as well as other cell types.
Accordingly, the terms "tendon perivascular cell(s)" and "ligament perivascular cells", respectively, refer to "perivascular cell(s)" that locate close to all kinds of blood and lymph vessels as defined above in the vicinity of tendons or ligaments, respectively. Such "tendon perivascular cell(s)" and/or "ligament perivascular cells" can be easily identified and purified from other cell types (e.g. vascular cells) that may be found in a tendon sample or tissue (or ligament sample or tissue) in accordance with the herein provided protocols and methods. Preferably, the tendon perivascular cells (TPC) show a similar or even identical expression pattern as the pluripotent cells (Oct4+; nanog+; Sox2+; Klf4+; c-myc+) to be generated; this is explained in more detail further below. In accordance with the above, any type of starting material may be used in the place of the population of tendon perivascular cells (TPC), provided that said population of cells used as starting material shows a similar or even identical expression pattern as the tendon perivascular cells (TPC) and/or the pluripotent cells (Oct4+; nanog+; Sox2+; Klf4+; c-myc+) to be generated. For example, a suitable cell type may be found in the vicinity of other tissues than tendon, such as ligament, preferably human ligament such asanterior cruciate ligament.
(A) corresponding cell(s)/population of cells may also be obtained from human dental pulp. For example, individual dental pulps from human third molars ("wisdom teeths") may be used as
suitable "cell population" and spheres may be obtained by cultivating said "cell population" in accordance with the teaching of the present invention. In particular, the method for the in vitro generation of pluripotent cells employing a population of tendon perivascular cells may be used, mutatis mutandis, in this context in order to generate spheres and to revocer pluripotent cells from these spheres. Such spheres may be termed "dental pulp spheres"/"dental pulp derived spheres". Such spheres have similar characteristics as the herein described and in particular they show a similar expression of pluripotent marker genes such as Oct4, nanog, Sox2, Klf4 and/or c-myc. Though the prior art has described cells with certain pluripotent characteristics isolated from human third molar dental pulp, a method for generating pluripotent cells using such dental pulps as provided herein has not been proposed; see Atari (2011) Histol Histopathol 26, 1057-1070.
All explanations, definitions and embodiments provided herein in context of this in vitro generation of pluripotent cells using a population of tendon perivascular cells, tenospheres (and methods for formation of tenospheres), cell cultures, methods of differentiating pluripotent cells and the like apply, mutatis mutandis, in respect of the use of other cell types/other starting material like ligament, preferably human ligament such as anterior cruciate ligament, or human dental pulp (especially of wisdom teeth). Again, such cells can be easily identified and purified from other cell types (e.g. vascular cells) that may be found in such a ligament or dental pulp sample/tissue.

As mentioned above, it has been found herein that a population of tendon perivascular cells (and/or a population of other suitable cells as defined above, like ligament perivascular cells) can be used as "starting material" in the methods of the invention. It is explained herein below how such a "population of cells", preferably a population of tendon perivascular cells (or other suitable cells as defined above, like ligament perivascular cells) can be obtained or provided and how such a cell population can be cultivated in order to generate pluripotent cells.
Without deferring from the gist of the invention, the step of "providing a population of tendon perivascular cells (or of other cells, like ligament perivascular cells)" can, accordingly, be omitted in the methods of the present invention provided that a given/a provided cell population is cultivated as described and defined herein.

Generally, it is preferred herein that the "population of tendon perivascular cells" (preferably a "population of human tendon perivascular cells" is obtained from a tendon sample (preferably a human tendon sample). Any tendon sample may be used, such as a sample of biceps tendons, supraspinatus tendons, subscapularis tendons, semitendinosus tendons, palmaris longus tendons and/or achilles tendons. As mentioned, also a sample of other tissue(s)/organ(s) etc. may be used, provided that such a sample comprises cells as defined above which are useful as starting material in the methods of this invention. Non-limiting examples of such samples are a sample of ligament (preferably human ligament, like anterior cruciate ligament) or a sample of dental pulp, preferably human dental pulp. The explanations and definitions given herein in context of obtaining a "population of tendon perivascular cells" from a "tendon sample" apply, mutatis mutandis, to any other population of suitable cells as defined herein from other samples, in particular as ligament (preferably human ligament) and also to dental pulp, in particular human dental pulp.
Preferably, the sample, in particular the tendon sample or ligament sample, is of human origin, i.e. the sample is obtained from a human being. The tendon sample is preferably a biceps tendon sample, in particular a human biceps tendon sample. Such sample (preferably a tendon or ligament sample) can, for example, be obtained during surgical interventions. Of course, the tendon or ligament sample may be obtained from other sources or animals, preferably from a mammal. Non-limiting examples for mammals are even-toed ungulates such as sheep, cattle and pig, odd-toed angulates such as horses as well as carnivors such as cats and dogs. In the context of this invention, it is particularly envisaged that tissues or samples are obtained from organisms that are economically, agronomically or scientifically important. Scientifically or experimentally important organisms include, but are not limited to, mice, rats, rabbits, guinea pigs and pigs. The sample/tissue/biopsy may also be obtained from primates, which comprise lemurs, monkeys and apes.

Preferably, a population of tendon perivascular cells can be obtained as follows (the explanation applies mutatis mutandis to other populations of suitable cells, such as ligament perivascular cells):
In order to prepare a population of tendon perivascular cells, the above tendon sample/tendon tissue may be first mechanically disrupted/disintegrated, for example by cutting into small pieces (e.g. about 1 mm to about 3 mm in diameter). This mechanical disintegration may be followed or complemented by a digestion. Alternatively, the mechanical disruption is not performed and the sample/tissue is first digested. Such a digestion can be performed employing routine techniques as described in Salingcarnboriboon (2003) Exp Cell Res. 287(2):289-300. In contrast to the present invention, Salingcarnboriboon discloses merely mesenchymal stem cells which do not express the pluripotent markers of the herein provided cells.

It is advantagoues that the collagen of a given sample is removed (preferably completely removed, i.e. no longer detectable by routine methods), as such processed samples give rise to more tenospheres. For example, the sample can be digested with an enzyme such as collagenase. The tendon sample may be digested for a certain period of time (preferably at least one hour, at least 2 hours, at least 5 hours, at least 8 hours and more preferably at least about 12 hourse (e.g. over night)) in an appropriate medium under suitable conditions. Although various types of media may be employed (such as DMEM, MEM or NB), the use of medium supplemented with serum is preferred herein. It has been observed herein that the use of a population of cells obtained by digestion in serum-supplemented medium promotes the formation of tenospheres, i.e. more tenospheres are formed and/or tenospheres of "better quality"are formed. In the absence of serum supplement, primarily mesenchymal stem cells but few or no tenospheres are found after cultivation of the population of tendon perivascular cells. Further, tenospheres formed in the absence of serum are predominantly adherent, i.e. are not free floating in the medium.

Accordingly, digestion of the tendon sample/tissue is preferably performed in the presence of serum, such as fetal calf serum (FCS) and/or autologous serum. Any mammalian (preferably human) serum or serum replacement can be used. For example, a conventional medium used for digestion as described herein, such as DMEM, MEM or NB may be supplemented with serum. Preferably, a conventional medium is supplemented with 2 to 25 % serum (i.e. the final medium contains 98 to 75 % conventional medium and 2 to 25 % serum, respectively). More preferably, the conventional medium is supplemented with 5 to 20 % serum, more preferably with 7 to 15 % serum, even more preferably with 8 to 12 % serum and most preferably with 10 % serum. The term "(digestion) in the presence of medium supplemented with serum" is also referred to herein shortly as "(digestion) in the presence of serum".

The medium (preferably supplemented with serum) contains an appropriate amount of enzyme (such as collagenase) and the digestion is then performed under suitable conditions. For example, the digestion may be performed using a medium containing 1.5U/ml Collagenase type 2 at 37°C in a humidified atmosphere with 5% CO2. One unit (U) of the enzyme (such as collagenase) is generally defined as the amount of the enzyme that catalyzes the conversion of 1 micro mole of substrate per minute. Methods for digestion of such samples e.g. with collagenase are well known in the art and can be employed in context of the present invention. Generally, the higher the concentration of the enzyme (e.g. collagenase) in a digestion assay, the shorter is the time needed for digestion and vice versa. For example, the enzyme may be used at a concentration of between 0.2 U/ml and 15 U/ml. At a concentration of about 0.2 U/ml the digestion would take about 24 hours; At a concentration of about 15 U/ml the digestion would take about 4 to 6 hours. The individual parameter will have to be adjusted depending on the invidial circumstances; for example, the time needed for digestion will be shorter, if samples of a smaller size are used.

Alternatively, the tendon sample/tissue may be digested for at least about 12 hours (e.g.overnight) in e.g. Neurobasal (NB) media containing 15mg/ml Collagenase type 2 at 37°C in a humidified atmosphere with 5% CO₂.
The digestion is typically performed at about 37 °C, preferably of from at least 35 °C up to 39 °C, more preferably of from at least 36 °C up to 38 °C and most preferably of from 36.5 °C to 37.5 °C. Preferably the digestion is performed are cultivated in a 5 % CO₂/97 % air humidified environment. The term "97 % air" is well known in the art and also refers to atmospheric O₂ (about "20% O₂"). These terms are well known in the art.
Furthermore, it is preferred that the tendon sample/tendon tissue/tendon biopsy etc. is "fresh", i.e. the digestion is preferably performed shortly after the sample/tissue/biopsy has been obtained from a donor, so that storage of the sample etc. is avoided.

Other suitable reaction conditions are readily available for a person skilled in the art. Preferably, the digestion is performed for a time sufficient for a suspension to form containing single cells and vessel fragments. The digestion is, preferably, preceded by a step of manually cutting the tendon tissues/samples into small pieces in order to accelerate the creation of the suspension of single cells/vessel fragments. Small pieces may have a size of from 5 mm³ to 0.1 mm³, and are preferably smaller than 4 mm³, 3 mm³, 2 mm³ and most preferably smaller than about 1 mm³. The digestion of the mixture of single cells and vessel fragments (or the vessel fragments alone) may be continued or (a) further digestion step(s) be performed, whereby such a digestion is preferably performed for a time sufficient for a suspension to form containing single cells. For example, the vessel fragments may be manually isolated and may be further digested with 2U/ml Accutase in DMEM for 15 min at RT as described in Reference [8]. An obtained suspension of single vascular and perivascular cells may be diluted (e.g. in cold PBS) so as to allow manual isolation of single cells using a pipette. In particular such single cells are referred to herein as "tendon perivascular cells". These single cells may be further analyzed by (single cell PCR or other methods disclosed herein (see also reference [22]) or they may be cultivated as explained in more detail herein further below.
It is of note that the isolation of single tendon perivascular cells for further cultivation is not essential for obtaining "tenospheres" and/or pluripotent cells in accordance with the present invention. In a simpler protocol whole tendon digests as described above may be cultivated under conditions defined further below without prior isolation of vessels. Also the use of such "unpurified" digests gives rise to tenospheres and/or pluripotent cells as also demonstrated in the appended Examples.

The herein provided pluripotent cells retain their pluripotent capacity/characteristics, if cultivated under appropriate conditions, like conditions described below in context of the formation of tenospheres. The pluripotent cells can be passaged more than 20 times and still retain their pluripotent capacity. "Passaging" means that a single pluripotent cell is taken from a tenosphere which has been produced in accordance with the present invention and this single cell is cultivated under conditions described herein which allow the formation of a tenosphere. The tenosphere which is formed that way from a single pluripotent cell contains pluripotent cells as defined herein. This procedure can be performed many times in order to maintain a pluripotent cell population for a long period of time. This procedure can also be used to produce Pluripotent cells in accordance with the present invention using "tenospheres" or single cells derived therefrom as "starting material". It is envisaged herein that also more than one pluripotent cell of a tenosphere may be used for passaging as described above. For example, 2, 3, 4, 5 or more cells may be used. As described elsewhere herein, the tenospheres comprise a mixture of cells (i.e. besides pluripotent cells also mesenchymal stem cells and/or neural cells can be found). It is believed that cells taken from tenospheres which are not pluripotent cells.(like mesenchymal stem cells and/or neural cells) will not give rise to tenospheres throught passaging as described above, so that pluripotent cells taken from a tenosphere will automatically give rise to a tenosphere if cultivated as described above without the need of further sorting or enriching for pluripotent cells. However, the herein provided methods for identifying and/or enriching pluripotent cells can be advantageously used in this context.

The one or more pluripotent cells taken from a tenosphere can be "passaged" as defined herein (i.e. cultivated in order to form a tenosphere) in the presence of any given medium known in the art. Suitable media are, for example, described in Chapter nineteen of the standard textbook "Protocols for Neural Cell Culture", 3rd Ed., Ed. S. Fedoroffan A. Richardson, Humana Press, Inc. Totowa, NJ, U.S.A.. For example, the following media can be used: MEM + 6 g glucose, N2 supplement and the like. Preferably, Neurobasal medium (NB), a medium designed for neural cell culture, or Dulbeccos Modified Eagle Medium (DMEM) is used. The use of Neurobasal medium (NB) is particularly preferred, since the highest proliferation rate is achieved. Also DMEM can be used to generate a high number of cells. It has been observed herein that the use of DMEM may lead to the formation of smaller tenospheres of about 50 to 100 cells on average. Exemplary compositions of Neurobasal medium, B27 supplement/B27 medium and N2 supplement as disclosed in "Protocols for Neural Cell Culture" (loc. cit.) are provided further below.

An exemplary further cultivation after digestions is described below:
An obtained "population of tendon perivascular cells" (e.g suspension of single vascular and tendon perivascular cells (TPCs)) can be cultivated for example in T-75 flask (75 cm²) containing NB media (Gibco BRL, Germany) supplemented with B27 (Gibco BRL, Germany) (NB/B27), 2 mM L-glutamine (PAN, Germany), 100U/ml penicillin/ 100 µg/ml streptomycin (PAN, Germany), 2 µg/ml heparin (Sigma, Germany), human recombinant 20 ng/ml bFGF-2 (R&D Systems GmbH, Wiesbaden-Nordenstadt, Germany) and human recombinant 20 ng/ml EGF (R&D Systems GmbH, Wiesbaden-Nordenstadt, Germany). Media change may be performed every two weeks. After 4 to 8 weeks in culture, the cells may generate floating spheres with a diameter up to 200 µm (tenospheres). A more frequent change of media may lead to a different phenotype of adherent, spindle shaped cells of so far unknown type. The phenotypic and biological characteristics of tenospheres generated in accordance with the present invention are described in detail herein.

As described above, the term "tendon perivascular cell" refers to cells that locate close to all kinds of blood and lymph vessels as defined above in the vicinity of tendons. The explanations and definitions below apply, mutatis mutandis, to other suitable cell types, like "ligament perivascular cells". A tendon (or sinew) is a tough band of fibrous connective tissue that usually connects muscle to bone and is capable of withstanding tension. Tendons are similar to ligaments and fasciae as they are all made of collagen except that ligaments join one bone to another bone, and fasciae connect muscles to other muscles. Tendon, ligament and fasciae samples, more particularly, the perivascular cells of tendon, ligament and/or fasciae, may be used in accordance with the present invention, whereby the use of tendon and/or ligament perivascular cells is preferred since these cells can be easily obtained by biopsy of tendon and/or ligament samples and subsequent processing of the sample as described herein.
Preferably, the "population of tendon perivascular cells"(or "ligament perivascular cells") sample comprises predominantly tendon perivascular cells (or ligament perivascular cells, respectively) as defined herein. For example, the "population of tendon perivascular cells" (or "population of ligament perivascular cells", respectively) contains preferably at least 90 %, 95 %, 96 %, 97 %, 98 %, more preferably at least 99 % tendon perivascular cells (or ligament perivascular cells", respectively). However, the "population of tendon perivascular cells" (or "population of ligament perivascular cells", respectively) may comprise (an) other cell type(s) as minor constituent(s). Such "minor constituents" are, in particular, non-tendon perivascular cells (or non-ligament perivascular cells, respectively). The "population of tendon perivascular cells" (or "population of ligament perivascular cells", respectively) may, for example, comprise preferably up to 10 %, 5 %, 4 %, 3 %, 2 % or 1 % "tendon vascular cell(s) (or ligament vascular cells, respectively)". Tendon vascular cells or ligament vascular cells are endothelial cells of venes, venoles, arteries, arterioles and capillaries. These cells are commonly characterized by the expression of endothelial cell markers such as Tie2 or vanWillebrand Factor.

A cell population comprising at least 99 % tendon perivascular cells (or at least 99 % ligament perivascular cells, respectively) is considered herein as a cell population essentially consisting of tendon perivascular cells (or ligament perivascular cells, respectively). It is preferred herein that the cell of population tendon (or ligament) perivascular cells does not contain other cell types, i.e. consists of tendon (or ligament) perivascular cells. A skilled person is easily in the position to determine the relative portion of tendon (or ligament) perivascular cells in the above population, for example by phenotypically (e.g. visually) determining tendon (or ligament) perivascular cells and other cell types, counting the number of tendon (or ligament) perivascular cells and other cell types and determining the percentage of tendon (or ligament) perivascular cells (i.e. the relative portion of tendon (or ligament) perivascular cells of the total number of cells in the cell population). This may, inter alia, be done by determining the number of tendon (or ligament) perivascular cells of a small, representative part of the entire cell population by a differential cell counter for cell samples. Alternatively, the expression pattern of a small, representative part of the population may be determined and the percentage of tendon (or ligament) perivascular cells expressing at least one of Oct4, nanog, Sox2, Klf4 and c-myc be determined (i.e. the relative portion of tendon (or ligament) perivascular cells of the total number of cells in the cell population be determined). Other methods are described herein below in context of "compositions enriched in tendon perivascular cells".

Envisaged herein is also the use of cell populations which comprise both tendon perivascular cells and ligament perivascular cells, i.e. which contain a mixed cell population. Thus, a cell population comprising tendon perivascular cells as described above, may also comprise ligament perivascular cells and vice versa.
Such a cell population may, for example, comprise at least 1 %, 5 %, 10 %, 15 %, 20 %, 25 %, 30 %, 35 %, 40 %, 45 %, preferably at least 50 %, 55 %, 60 %, 65 %, 70 %, more preferably at least 75 %, 80 %, 85 %, 90 %, 95 %, 96 %, 97 %, 98 %, more preferably at least 99 % tendon perivascular cells. It is preferred that the cell population comprises predominantly tendon perivascular cells as defined herein above. Correspondingly, said mixed cell population may comprise at most 99 %, 95 %, 90 %, 85 %, 80 %, 75 %, 70 %, 65 %, 60 %, 55 %, preferably at most 50 %, 45 %, 40 %, 35 %, 30 %, more preferably at most 25 %, 20 %, 15 %, 10 %, 5 %, 4 %, 3 %, 2 %, more preferably at most 1 % ligament perivascular cells.
Such a mixed "population of tendon perivascular cells and ligament perivascular cells" may, in addition, comprise (an) other cell type(s) as minor constituent(s). Such "minor constituents" are, in particular, non-tendon perivascular cells and/or non-ligament perivascular cells. The mixed population of tendon and ligament perivascular cells may, for example, comprise preferably up to 10 %, 5 %, 4 %, 3 %, 2 % or 1 % "tendon vascular cell(s) (or ligament vascular cells, respectively)". For example, such a mixed cell population may comprise 95 % tendon perivascular cells, 4 % ligament perivascular cells and 1 % other cells (like vascular cells). Mixed populations of tendon perivascular cells and ligament perivascular cells may, for example, be prepared by simultaneously using a tendon sample and a ligament sample (e.g. both a tendon sample and a ligament sample can be obtained of a patient and both can be processed as described herein, for example digested with collagenase and cultivated in order to form (a) tenosphere(s). Any definitions and explanations given herein in context of the use of a "population of tendon perivascular cells" or "population of ligament perivascular cells" in accordance with the present invention apply mutatis mutandis to the use of such a mixed population comprising tendon perivascular cells and ligament perivascular cells.

The "population of tendon perivascular cells" (or "population of ligament perivascular cells") may consist of a minimum of one tendon perivascular cell (or one ligament perivascular cell, respectively) (for example if a single cell is used as starting material). Yet, the "population of tendon perivascular cells" (or "population of ligament perivascular cells") may, of course, comprise more cells, for example at least 2 tendon perivascular cells (or ligament perivascular cells, respectively) and up to 10⁸ tendon perivascular cells (or ligament perivascular cells, respectively). The number of the "population of tendon perivascular cells" (or "population of ligament perivascular cells") primarily depends on the amount of the sample used - generally, the more sample is used, the more perivascular tendon cells can be obtained and form accordingly the "population of tendon perivascular cells" (or "population of ligament perivascular cells") to be used herein.
The above explanations given for "tendon perivascular cells" (or "population of ligament perivascular cells") apply, mutatis mutandis, for any other cell type that is suitable as starting material as described herein. As mentioned above, even a relatively raw tendon (and similarly ligament) digest is suitable as starting material and also covered by the term "population of tendon perivascular cells". Even the use of a (tendon) sample/tissue which is not processed may be a valuable source and used herein as starting material. Accordingly, the "population of tendon perivascular cells" refers also to a tendon sample/tissue/biopsy per se, since such a tendon sample/tissue/biopsy comprises at least one "tendon perivascular cells" and, therefore, also a population of tendon perivascular cells, i.e. at least one tendon perivascular cell. This applies, mutatis mutandis, to "population of ligament perivascular cells".

The methods of the present invention comprise a step of cultivating the tendon perivascular cells (and/or ligament perivascular cells) in a medium for a time sufficient for a tenosphere to form. As explained above, it has surprisingly been found herein that tendon perivascular cells (or ligament perivascular cells) give rise to pluripotent cells if cultivated under the herein provided culture conditions. These pluripotent cells are comprised in unique compartments denoted herein "tenosphere".

In context of the present invention the term "tenosphere" refers to a group/cluster of free floating cells derived from tendon or from tendon-like tissues, such as ligament, whereby this group/cluster forms regular as well as irregular sphere structures that can be mechanically or enzymatically dissociated into single cells. The term "free floating" refers to the "tenosphere" (not to the cells within the tenosphere) and reflects the fact that the tenosphere dos not adhere to the surface of the culture vessel.
Preferably, the tenosphere is floating in the medium, i.e. the tenosphere is "non-adherent". The term "non-adherent" relates to the fact that the "tenosphere" does not adhere/stick/attach to the culture dish/culture vessel (and the like) at all. The "non-adherent" tenosphere(s) float around in the medium and can easily be removed e.g. with a pipette and the like. In other words, no particular means are necessary to remove the "non-adherent" tenosphere(s) from the surface of the culture dish etc.

The "tenosphere(s)" that form(s) under the cultivation conditions of the above provided method may be described as follows: Within about several days of cultivation the formation of spheroid bodies (denoted herein as "tenospheres") is observed. It has been demonstrated in the appended Examples that on average 3 % of manually isolated tendon perivascular cells generated such clonal spheres/spheroid bodies/tenospheres.
The term "tenosphere" refers to a cell cluster (a cellular aggregate).
This cell cluster/cellular aggregate comprises phenotypically heterogenous cell types. The cell cluster/cellular preferably comprises or consists of at least 3, 5, 10, 20, 30, 40, 50, 70, 90, more preferably at least 100 and preferably up to 800, more preferably up to 900, 1000, 1100, 1200, 1300, 1400 or 1500, even more preferably up to 2000, 3000, 4000, 5000, 6000, 7000, 8000, 9000, 10.000, 11.000, 12.000, 13.000, 14.000, 15.000, 16.000, 17.000, 18.000, 19.000 and up to 20.000 cells. Many of these cells comprised in the tenosphere are pluripotent cells as defined herein. It has been observed that the tenospheres may comprise about 20 to 30 % and higher percentages of 70 % and more pluripotent cells; see Example 8. As mentioned, the tenospheres comprise or consist essentially of pluripotent cells. Preferably, at least 10 %, 15 %, 17 %, 19 %, 20 %, 21 %, 22 %, 23 %, 24 %, 25 %, 26%, 27 %, 28 %, 29 %, 30 %, 31 % 32 %, 33 %, 34 %, 35 %, 40 %, 45 %, 50 %, 55 %, 60 %, 65 %, more preferably at least 70 %, 72 %, 73 %, 74 %, 75 %, 76 %, 77 % ore more, like at least 80 %, 85 %, 90 %, 95 % or at least 99 % of the tenosphere cells are pluripotent cells.

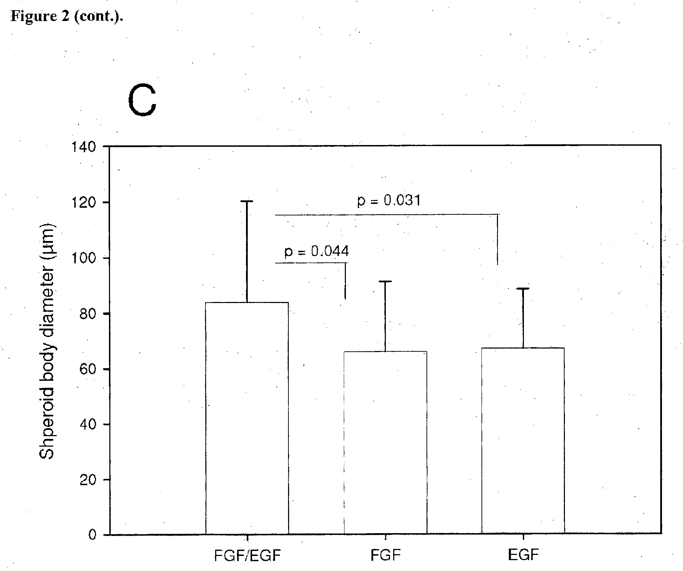

The herein provided tenosphere preferably has an average diameter of from 30 µm and up to 400 µm, preferably up to 200 µm. The tenosphere may have an average diameter of from about 20 to 140 µm, preferably of from about 40 to 120 µm or of from about 60 to 90 µm. For example, tenospheres having an average diameter of about 60 µm (of from about 40 to 120 µm) form when the population of tendon and/or ligament perivascular cells is cultivated in the presence of FGF or EGF. Tenospheres with an average diameter of about 80 µm (of from about 40 to 120 µm) form when the population of tendon and/or ligament perivascular cells is cultivated in the presence of FGF and EGF; see Figure 2C.
Typically, the tenosphere forms within several days under the herein described culture conditions. Usually, the formation of a tenosphere can be observed within about 2 days to about 10 days, for example within 2 days, 3 days, 4 days, 5 days, 6 days, 7 days, 8 days, 9 days or 10 days. Accordingly, such exemplary periods of 3 days, 4 days, 5 days, 6 days, 7 days, 8 days , 9 days or 10 days of cultivation may be considered as "time sufficient for a tenosphere to form". However, the formation of the tenosphere depends on the specific culture conditions used and the "time sufficient for a tenosphere to form" may, therefore, vary and may also deviate from the above given exemplary values, i.e. the time may also be lower than 3 days or higher than 10 days, like 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or even longer. In the appended examples, it is shown that a cell suspension obtained from tenospheres contained 77.3% ±17.8% of pluripotent cells after 1 week of cultivation in accordance with the herein provided methods. Longer culture periods (longer than e.g. 1 week) are advantageous in that a higher number of total cells can be obtained. If the pluripotent cells are cultivated longer than about 30 days, the percentage of pluripotent cells may drop. For example, it was observed that the percentage of pluripotent cells dropped to 41%±22% after 4 weeks cultivation.A person skilled in the art can readily determine whether "tenospheres," form under the herein provided culture conditions. Accordingly he/she will be easily in the position to determine whether a certain time is sufficient for a tenosphere to form and/or which time is needed for a tenosphere to form. What is important is the fact that at the end of the cultivation step a tenosphere will have formed. This tenosphere is used to recover pluripotent cells in accordance with step (c) of the herein provided methods.

The population of tendon perivascular cells (and/or ligament perivascular cells) as defined herein can be cultivated in the presence of any given medium known in the art. Suitable media are, for example, described in Chapter nineteen of the standard textbook "Protocols for Neural Cell Culture", 3rd Ed., Ed. S. Fedoroff an A. Richardson, Humana Press, Inc. Totowa, NJ, U.S.A.. For example, the following media can be used: MEM + 6 g glucose, N2 supplement and the like. Preferably, Neurobasal medium (NB), a medium designed for neural cell culture, or Dulbeccos Modified Eagle Medium (DMEM) is used. The use of Neurobasal medium (NB) is particularly preferred, since the highest proliferation rate is achieved. Also DMEM can be used to generate a high number of cells. It has been observed herein that the use of DMEM may lead to the formation of smaller tenospheres of about 50 to 100 cells on average.
Exemplary compositions of Neurobasal medium, B27 supplement/B27 medium and N2 supplement as disclosed in "Protocols for Neural Cell Culture" (loc. cit.) are provided herein below:

**Table 1. Composition of Neurobasal Medium**

| Component | Final mg/L |
|---|---|
| Inorganic salts | |
| CaCl₂ (anhydrous) | 200.0 |
| Fe(NO₃)₃.9H₂O | 0.1 |
| KCl | 400.0 |
| MgCl₂ (anhydrous)*^{b}* | 77.3 |
| NaCl*^{b}* | 3000.0 |
| NaHCO₃*^{b}* | 2200.0 |
| NaH₂PO₄.H₂O | 125.0 |
| ZnSO₄.7H₂O | 0.19 |

| Other components | |
|---|---|
| D-Glucose | 4500.0 |
| HEPES*^{b}* | 2600.0 |
| Phenol red*^{b}* | 8.1 |
| Na pyruvate*^{b}* | 25.0 |

| Amino acids | |
|---|---|
| L-Alanine*^{b}* | 2.0 |
| L-Arginine.HCl | 84.0 |
| L-Asparagine.H₂O*^{b}* | 0.83 |
| L-Cysteine*^{b}* | 1.21 |
| L-Glutamine^{*a*,*b*} | 73.5 |
| L-Glutamate*^{c}* | |
| Glycine | 30.0 |
| L-Histidine HCl.H₂O | 42.0 |
| L-Isoleucine | 105.0 |
| L-Leucine | 105.0 |
| L-Lysine HCl | 146.0 |
| L-Methionine | 30.0 |
| L-Phenylalanine | 66.0 |
| L-Proline*^{b}* | 7.76 |
| L-Serine | 42.0 |
| L-Threonine | 95.0 |
| L-Tryptophan | 16.0 |
| L-Tyrosine | 72.0 |
| L-Valine | 94.0 |

| Vitamins | |
|---|---|
| D-Calcium pantothenate | 4.0 |
| Choline chloride | 4.0 |
| Folic acid | 4.0 |
| *i*-Inositol | 7.2 |
| Niacinamide | 4.0 |
| Pyridoxal HCl | 4.0 |
| Riboflavin | 0.4 |
| Thiamine HCl | 4.0 |
| Vitamin B₁₂ | 0.34 |
| | |
| *^{a}*Not supplied in liquid medium; needs to be added. | |
| *^{b}*Changed from DMEM. | |
| *^{c}*Glutamate at 3.7 µg/mL (25 µ*M*) should be added to start primary embryonic HC neurons. | |

**Table 2. Composition of B 18 Supplement and Ingredients of B27**

| Component | Final mg/L |
|---|---|
| Amino acids | |
| L-Alanine^{*a*,*b*} | 2.0 |
| L-Glutamate*^{b}* | 3.7 |
| L-Glutamine*^{b}* | 441.0 |
| L-Proline^{*a*,*b*} | 7.76 |
| Vitamins | |
| Biotin*^{b}* | 0.10 |
| Vitamin B₁₂^{*a*,*b*} | 0.34 |

| Hormones | |
|---|---|
| Corticosterone*^{c}* | 0.02 |
| Progesterone*^{c}* | 0.0063 |
| Retinol, all trans (vit.A) | 0.1 |
| Retinol, acetate*^{c}* | 0.1 |
| Insulin^{*a*,*c*} | 4.0 |
| T₃ (triodo-L-thyronine)*^{c}* | 0.002 |

| Antioxidants | |
|---|---|
| Na pyruvate^{*a*,*b*} | 25.0 |
| Lipoic acid (thioctic acid)*^{a}* | 0.047 |
| D,L-α-Tocopherol (vit. E)*^{c}* | 1.0 |
| D,L-α-Tocopherol acetate*^{c}* | 1.0 |
| Catalase^{*a*,*c*} | 2.5 |
| Glutathione (reduced)*^{c}* | 1.0 |
| Superoxide dismutase^{*a*,*c*} | 2.5 |

| Other | |
|---|---|
| L-Carnitine*^{c}* | 2.0 |
| Ethanolamine*^{c}* | 1.0 |
| D(+)-Galactose*^{c}* | 15.0 |
| HEPES^{*a*,*b*} | 2600.0 |
| Putrescine*^{c}* | 16.1 |
| Penicillin | 50 IU/mL |
| Streptomycin | 0.5 |
| Selenium^{*a*,*c*} | 0.016 |
| Zinc sulfate^{*a*,*b*} | 0.194 |
| Linoleic acid*^{c}* | 1.0 |
| Linolenic acid*^{c}* | 1.0 |
| Albumin, bovine*^{c}* | 2500.0 |
| Transferrin^{*a*,*c*} | 5.0 |
| | |
| *^{a}*Changed from the original formulation of Romijn et al. (1984). | |
| *^{b}*Not needed when used with Neurobasal. | |
| *^{c}*Included in B27, but concentrations optimized for 4 d survival of hippocampal | |

**Table 3. Composition of the N2 Supplement**

| Component | Final mg/L |
|---|---|
| Insulin (bovine) | 5 mg/L |
| Progesterone | 20 n*M* |
| Putrescine | 100 µ*M* |
| Na selenite | 30 n*M* |
| Transferrin (human) | 100 mg/L |

In a preferred embodiment of the present invention, the medium comprises B27 supplement. As mentioned, an exemplary composition of B27 supplement is given above. Thus, in one embodiment the medium to be used comprises B27 supplement/B27 medium. For example, NB or DMEM supplemented with B27 medium (or other suitable media) may be used. Such a supplemented medium may be prepared according to routine practice, whereby preferably the ingredients in the medium have a final concentration as indicated in the Tables herein above.B27 supplement is commercially available for example from Gibco under catalogue number 17504-044. Though the use of the above mentioned B27 supplement is particularly preferred in context of the present invention, also the use of further adapted B27 supplements is envisaged herein. Such adapted B27 supplements are, for example, B27 serum-free supplement, B27 supplement minus AO (i.e. antioxidant components, vitamin E, vitamin E acetate, superoxide dismutase, catalase, and glutathione being removed), B27 supplement minus Vitamin A, B27 supplement minus insulin and the like. Accordingly adapted B27 supplements are also commercially available or can be adapted by a person skilled in the art starting from the known and also herein disclosed B27 supplement. A person skilled in the art is also readily in the position to further adapt the herein disclosed media, in particular the various B27 supplement formulations when carrying out the methods of the invention. For example, progesterone may be omitted from the medium, in particular the B27 supplement to be used herein.

Also the use of other supplements such as the N2 supplement in the place of or in addition to the herein provided B27 supplements is envisaged in context of the present invention.

As mentioned, use of a medium (e.g. Neurobasal medium (NB), Dulbeccos Modified Eagle Medium (DMEM) or MEM + 6 g glucose) supplemented with B27 supplement or N2 supplement is envisaged in context of the above method. The term "(cultivation) in the presence of medium supplemented with B27 (or N2 and the like)" is also referred to herein shortly as "(cultivation) in the presence of B27 supplement (or N2 and the like)". These terms may also be used interchangeably with the term "cultivation in a medium which comprises or consists of B27 supplement".
In a further preferred embodiment of the present invention, the medium comprises Fibroblast-Growth-Factor (FGF) and/or Epidermal-Growth-Factor (EGF). Whereas the presence of the B27 supplement appears to be advantageous for the formation of tenospheres, FGF and/or EGF appear to be capable of increasing the size of tenospheres. Preferably, the medium comprises FGF in a concentration of from 1 ng/ml to 1 mg/ml and/or EGF in a concentration or from 1 ng/ml to 1 mg/ml. More preferably, the medium comprises FGF in a concentration of from 5 ng/ml to 100 ng/ml and/or EGF in a concentration or from 5 ng/ml to 100 ng/ml. Even more preferably, the medium comprises FGF in a concentration of from 10 ng/ml, 11, 12, 13, 14, more preferably of from 15, 16, 17, 18 or 19 to 50, 45, more preferably 40, 35, 30, 25, 24, 23, 22, or 21 ng/ml and/or EGF in a concentration or from 10 ng/ml, 11, 12, 13, 14, more preferably of from 15, 16, 17, 18 or 19 up to 50, 45, more preferably 40, 35, 30, 25, 24, 23, 22, or 21 ng/ml. Most preferably, the medium comprises FGF in a concentration of 20 ng/ml and/or EGF in a concentration of 20 ng/ml. For example, the medium comprises FGF in a concentration of 20 ng/ml. Alternatively, the medium comprises FGF in a concentration of 20 ng/ml. Particularly and most preferred, the medium comprises FGF in a concentration of 20 ng/ml and EGF in a concentration of 20 ng/ml.

In one embodiment, the population of tendon perivascular cells (and/or of ligament perivascular cells) are cultivated in DMEM containing B27 supplement, 20ng/ml FGF and 20ng/ml EGF. Alternatively, the population of tendon perivascular cells (and/or of ligament perivascular cells) are cultivated in NB) containing B27 supplement, 20ng/ml FGF and 20ng/ml EGF. The terms "NB" "NB-A", "Neurobasal A" refer to the same medium and can be used interchangeably herein. In case of single tendon (or ligament) perivascular cells to be cultivated well plates (e.g. 96 well plates) may be used (1 cell/well).
In a further embodiment, whole tendon (and/or ligament) collagenase digests can be cultured in DMEM containing B27 supplement, 20ng/ml FGF and 20ng/ml EGF without prior isolation of vessels.
Alternatively, the tendon sample/tissue may be digested for at least about 12 hours (e.g.overnight) in e.g. Neurobasal (NB) media containing 15mg/ml Collagenase type 2 at 37°C in a humidified atmosphere with 5% CO₂.

In a further preferred embodiment of the herein provided methods, the tendon perivascular cells (and/or ligament perivascular cells) are cultivated under high-density conditions. This means in this context that tendon perivascular cells (and/or ligament perivascular cells) are seeded at a cell density of at least 2.5 × 10⁶ cells/100 µl. Preferably, tendon perivascular cells (and/or ligament perivascular cells) are seeded at a cell density of at least 3.0 × 10⁶ cells/100 µl, more preferably at least 3.5 × 10⁶ cells/100 µl, more preferably at least 4.0 × 10⁶ cells/100 µl, even more preferably at least 4.5 × 10⁶ cells/100 µl, 4.6 × 10⁶ cells/100 µl, 4.7 × 10⁶ cells/100 µl, 4.8 × 10⁶ cells/100 µl, or at least 4,9 × 10⁶ cells/100 µl and up to 7. 5 × 10⁶ cells/100 µl. Most preferably, the "high-density conditions" refer to a cultivation at a cell density of 5.0 × 10⁶ cells/100 µl.
Also preferred in the herein provided methods is a change of the cultivation medium at relatively low intervals. For example, the medium may be changed at most every week, every 10 days, every, 11 days, every 12 days, or every 13 days or at even further extended intervals. Preferably, the medium is changed at most every 14 days or at most every two weeks.
As described below, the pluripotent cells can easily be isolated from the culture/from the tenospheres. Isolation (i.e. recovery) of the pluripotent cells from culture/from the tenospheres may involve the steps of enzymatic isolation of all cells from the tenospheres, followed by the isolation of the pluripotent cells.
The cells can be isolated by enzymatic treatment e.g. with Accutase or other suitable available enzymes like Trypsin (.e.g Trypsin 0.2 % w/v). The tenospheres are typically enzymatically treated at about 37 °C, preferably at least 35 °C, more preferably at least 36°C. The enzymatic treatment with an appropriate amount of enzyme(s) is made for a time sufficient to dissolve the tenospheres to a large extent, e.g. at least 50 %, 60 %, 70 %, 80 %, 90 %, 95 % or 99 % of the tenospheres are dissolved after completetion of enzymatic treatment. The "sufficient time" and the "appropriate amount of enzyme" can easily be determined. Usually, the "sufficient time" is from 5 to 30 minutes, like about 10 minutes, about 15 minutes or about 20 minutes. The term "dissolved" as used in this context means that the "tenospheres" as defined herein are no longer present in that the cells forming the tenosphere/contained in the tenosphere form a suspension of cells/cell suspension. In other words, the tenosphere culture comprises after enzymatic treatment at least 50 %, 60 %, 70 %, 80 %, 90 %, 95 % or 99 % single cells (% single cells compared to the overall content of particles (a particle being, for example, a single cell or a cell cluster of one or more cells (like a tenosphere)). Thus, the suspension of cells/cell suspension may comprise at least 50 %, 60 %, 70 %, 80 %, 90 %, 95 % or 99 % single cells (% single cells compared to the overall content of particles (a particle being, for example, a single cell or a cell cluster of one or more cells (like a tenosphere)).

The enzymatic isolated may be proceded or followed by a mechanical step of disrupting the formed tenosphere. For example a cell strainer (e.g. with of from 50 to 120 µm, like with 70 µm or 100 µm pore size) may be used in such a context. The cell strainer may be used to enrich for single cells in a cell suspension, whereby the suspension of cells/cell suspension may comprise at least 50 %, 60 %, 70 %, 80 %, 90 %, 95 % or 99 % single cells (% single cells compared to the overall content of particles (a particle being, for example, a single cell or a cell cluster of one or more cells (like a tenosphere)). For example, the tenospheres may, after enzymatic treatment/digestion, be filtered by a cell strainer to enrich for single cells in the cell suspension. Preferably, single cells are isolated/recovered from the culture/from the tenosphere. More preferably, these single cells are pluripotent cells as defined herein.

As demonstrated in Example 8, a (further) enrichment (e.g. provision of a suspension of cells/cell suspension further enriched in pluripotent cells) can be made by taking advantage of reporter genes under control of a promoter of the herein described pluripotent marker genes (like Oct4, nanog, Sox2, Klf4, or c-myc). The expression of these reporter gene(s) is switched on in pluripotent cells provided in accordance with the present invention, because the promoter of the herein described pluripotent marker genes (like Oct4, nanog, Sox2, Klf4, or c-myc) is activated in pluripotent cells. Im other words, the expression of pluripotent marker genes (like Oct4, nanog, Sox2, Klf4, or c-myc) and, simultaneously, the expression of the reporter gene(s) under control of the same promoter is switched on in pluripotent cells. An exemplary promoter to be used is the Oct4-promoter (i.e. the promoter that regulates expression of the Oct4 gene in (known) pluripotent cells. The expression of the reporter gene can easily be monitored e.g. in case of GFP (and the like) by measuring the fluorescence. The expression of the reporter gene can, thus, be used to screen for, identify and recover/isolate pluripotent cells, allowing enriching for pluripotent cells. The expression of reporter gene(s) and/or of pluripotent marker genes (like Oct4, nanog, Sox2, Klf4, or c-myc) can be determined by routine methods (e.g. protein or mRNA quantification methods as described elsewhere herein, like single cell PCR).

A (further) enrichment (e.g. provision of a suspension of cells/cell suspension further enriched in pluripotent cells) may be made by taking advantage of fusion proteins. Such fusion proteins may comprise a reporter protein (like GFP etc.) and a protein characteristic of the pluripotent cells provided herein (like Oct4, nanog, Klf4 and/or c-myc). As explained above, the expression of these marker gene(s) is switched on in pluripotent cells provided in accordance with the present invention. The expression of fusion genes (i.e. genes allowing the expression of the above fusion proteins) is switched on in pluripotent cells (because the expression of the pluripotent marker genes is switched on). By taking advantage of cell sorting methods (like FACS), the pluripotent cells expressing the fusion genes can be isolated.

The suspension of cells/cell suspension as defined and explained above can be considered as pluripotent cells recovered from the tenosphere. Alternatively, the suspension of cells/cell suspension as defined and explained above can be considered as a cell population comprising pluripotent cells recovered from the tenosphere. This suspension of cells/cell suspension provided in accordance with the present invention, may be used in methods of differentiating a pluripotent cell into a cell type of interest. Accordingly, the step of "providing the pluripotent cells" in the method of differentiating provided herein may encompass the provision of the herein above explained suspension of cells/cell suspension (the cell suspension comprising pluripotent cells of the invention, as described above).

It is known in the art that Oct4 has two isoforms, namely Oct4-A and Oct4-B, both of which can be expressed in the herein provided pluripotent cells. However, it is preferred that predominantly or only the expression of the isoform Oct4A is increased in the pluripotent cells generated in accordance with the present invention.

In the following, a preferred procedure for the cultivation of a population of tendon perivascular cells (and/or ligament perivascular cells) is described.

A population of tendon perivascular cells (and/or ligament perivascular cells) obtained as described above is cultured for typically seven days under high density conditions as follows.

For example, tendon perivascular cells (and/or ligament perivascular cells) from volunteers can be obtained as described above. These tendon (and/or ligament) perivascular cells can be seeded/embedded at high cell density (e.g 5 × 10⁶ cells/100 µl in cell culture as defined and described above) and are cultivated under culture conditions as described above. The term "high cell density" as used herein refers to the cell density at the beginning of the cell culture, i.e. the cell density of the seeded cells. It is thought that the initial cell density further increases during cultivation due to proliferation. For example, cell density may increase to e.g 5.5 × 10⁶ cells/100 µl. However, the initial cell density (i.e. the density the cells are seeded) is more crucial in the methods of the present invention than the development of the cell density during cultivation. Tendon (and/or ligament) perivascular cells and/or the formed tenospheres are typically cultured at about 37 °C, preferably at least 35 °C, more preferably at least 36 °C. Preferably the tendon (and/or ligament) perivascular cells and/or the formed tenospheres are cultivated in a 5 % CO₂/97 % air humidified environment. The term "97 % air" is well known in the art and also refers to atmospheric O₂ (about "20% O₂"). These terms are well known in the art. Generally, cells are grown and maintained in cell culture at an appropriate temperature and gas mixture (typically, 37°C, 5% CO2 for mammalian cells) in a cell incubator.

The use of NB medium optionally supplemented with B27 as defined and described herein is preferred for the cultivation in accordance with the present invention.
Aside from temperature and gas mixture, the most commonly varied factor in culture systems is the growth medium. Recipes for growth media can vary in pH, glucose concentration, growth factors, and the presence of other nutrients. The growth factors used to supplement media are often derived from animal blood, such as (calf) serum. The use of corresponding serum/sera is also envisaged herein.
A person skilled in the art is easily in the position to adapt the cultivation conditions according to the needs of the present invention in order to obtain the pluripotent cells and/or the tenospheres generated in accordance with the present invention. Culture techniques are, for example, described in standard textbooks like Ian Freshney, Culture of Animal cells Wiley & Sons 2010 th ed.

In accordance with the above, the disclosure further relates to pluripotent cells (Oct4+; nanog+; Sox2+; Klf4+; c-myc+) as defined herein which are obtained or obtainable by the herein provided and described methods. Preferably, the pluripotent cells are also Glut2+ (i.e. express the cell surface marker Glut2).

In a further embodiment, the present disclosure relates to a kit for detecting one or more of the marker genes provided in the table below comprising a primer pair (denoted "sense and antisense" in the table).
For example, a kit is provided for detecting human Oct4 comprising a primer having the sequence ACCTGGCTAAGCTTCCAAGGCC (SEQ ID NO: 1) and a primer having the sequence TCGTTTGGCTGAATACCTTCCC (SEQ ID NO: 2).
Furthermore, provided is a kit for detecting human Sox2 comprising a primer having the sequence AAGATGCACAACTCGGAGATC - (SEQ ID NO: 3) and a primer having the sequence TATTTATAATCCGGGTGCTCC (SEQ ID NO: 4). Also a kit is provided for detecting human Nanog comprising a primer having the sequence TCAGGACAGCCCTGATTCTTCC - (SEQ ID NO: 5) and a primer having the sequence TCTTGCATCTGCTGGAGG (SEQ ID NO: 6). A further exemplary kit is for detecting human Klf-4 comprising a primer having the sequence AGACCGAGGAGTTCAACGATC - (SEQ ID NO: 7) and a primer having the sequence TTGATGTCCGCCAGGTTGAAG (SEQ ID NO: 8). Further provided is a kit is for detecting human cMyc comprising a primer having the sequence ACAGGAACTATGACCTCGAC - (SEQ ID NO: 9) and a primer having the sequence TACAGTCCTGGATGATGATG - (SEQ ID NO: 10).

The following tables show primer sequences to be used in accordance with the herein provided means and methods:

| **human** | **sense** | **antisense** | **Acc. No.** |
|---|---|---|---|
| hOct4 | | | NM_002701 |
| Sox2 | | | NM_003106 |
| hNanog | | 5'-TCTTGCATCTGCTGGAGG-3' (SEQ ID NO. 6) | NM_024865 |
| Klf-4 | | | NM_004235 |
| cMyc | | | NM_002467 |
| hNestin | | | NM_006617 |
| Col1A1 | | | NM_000088 |
| Col3A1 | | | NM_000090 |
| hGalc | | | NM_000153 |
| hSCX | | | XM_926116 |
| hSOX9 | | | NM_000346 |
| INS1 | | | NM_000207 |

| **rat** | **sense** | **antisense** | **Acc**. **No**. |
|---|---|---|---|
| ratOct4 | | | NM_001009178 |
| Sox2 | | | NM_001109181 |
| ratNanog | | | NM_001100781 |
| Klf-4 | | | NM_053713 |
| cMyc | | | NM_012603 |
| rat_Nestin | | | NM_012987 |
| Col1A1 | | | NM_053304 |
| Col3A1 | | | NM_032085 |
| ratGalc | | | NM_001005888 |
| SCX | | | NM_001130508 |
| SOX9 | | | XM_343981 |
| INS1 | | | NM_019129.2 |

In a particularly preferred embodiment of the present disclose the kit (to be prepared in context) of this disclosure or the methods and uses of the disclosure may further comprise or be provided with (an) instruction manual(s). For example, said instruction manual(s) may guide the skilled person (how) to detect one or more of the marker genes in accordance with the present disclosure. Particularly, said instruction manual(s) may comprise guidance to use or apply the herein provided methods or uses.
The kit (to be prepared in context) of this disclosure may further comprise substances/chemicals and/or equipment suitable/required for carrying out the methods and uses of this disclosure. For example, such substances/chemicals and/or equipment are solvents, diluents and/or buffers for stabilizing and/or storing (a) compound(s) required for specifically determining the expression level of one or more of the marker genes as defined herein.

In a further embodiment, the present disclosure relates to a method for the in vitro formation of a tenosphere from a population of tendon perivascular cells (and/or ligament perivascular cells), wherein said tendon perivascular cells (and/or ligament perivascular cells) express at least one of Oct4, nanog, Sox2, Klf4 and c-myc, said method comprising
a) providing a population of said tendon perivascular cells (and/or ligament perivascular cells);
b) cultivating the tendon perivascular cells (TPC) (and/or ligament perivascular cells) of a) in a medium under high cell density conditions for a time sufficient for a tenosphere to form.
The method for the in vitro formation of a tenosphere may comprise an optional step c) of recovering (or isolating) the tenosphere.
Alle the definitions and explanations given herein above in context of the method for the in vitro generation of pluripotent cells apply, mutatis mutandis, also to this method for the in vitro formation of a tenosphere. In particular, conditions and media which allow the formation of tenospheres have been described above and can be used in this context.
As mentioned above in context of "passaging" of tenospheres/pluripotent cells, also pluripotent cell(s) taken/obtained from a tenosphere provided in accordance with the present disclosure may be used to generate (a) tenosphere(s) and/or (a) pluripotent cell(s).
Accordingly, the present disclosure relates in a further embodiment to a method for the in vitro formation of a tenospheres from one or more pluripotent cells as defined herein, said method comprising
a) providing one or more pluripotent cells;
b) cultivating one or more pluripotent cells for a time sufficient for a tenosphere to form.

Also envisaged herein is a method for the in vitro generation of pluripotent cells (Oct4+; nanog+; Sox2+; Klf4+; c-myc+), said method comprising
a) providing one or more pluripotent cells;
b) cultivating the one or more pluripotent cells of a) in a medium for a time sufficient for a tenosphere to form;
c) recovering pluripotent cells (Oct4+; nanog+; Sox2+; Klf4+; c-myc+) from the tenosphere.

In accordance with the above, the present disclosure also relates to a tenosphere obtainable or obtained by the above provided methods. As mentioned, the cells of said tenosphere preferably comprise/consist of pluripotent cells (Oct4+; nanog+; Sox2+; Klf4+; c-myc+) as defined herein. Furthermore, also a cell culture comprising the pluripotent cells (Oct4+; nanog+; Sox2+; Klf4+; c-myc+) as defined herein and/or a cell culture comprising the tenosphere as defined herein is subject of the present disclosure.

As mentioned above, the present disclosure does not only provide for pluripotent cells and tenospheres; as proof of principle it has also been demonstrated that such pluripotent cells can be differentiated.
Accordingly, the present disclosure relates to a method of differentiating a pluripotent cell into a cell type of interest, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into the cell type of interest..
The method of differentiating a pluripotent cell may comprise an optional step c) of recovering (or isolating) the differentiated cell(s). Accordingly, the present invention relates to (a) differentiated cell(s) obtained or obtainable by the herein provided methods of differentiating a pluripotent cell.

The provision of pluripotent cells (Oct4+; nanog+; Sox2+; Klf4+; c=myc+) and/or the tenosphere has been described in detail herein above. All explanations and definitions given herein in this context, also apply, mutatis mutandis, in the context of the differentiating method. Also methods for recovering pluripotent cells have been described herein above.
The method may also be employed in the differentiation of "tendon perivascular cells" / as "population of tendon perivascular cells" ((and/or ligament perivascular cells / a "population of tendon perivascular cells" and/or a mixture of tendon and perivascular cells / a mixed population of tendon and perivascular cells), as desribed and defined herein. Accordingly, the present disclosure relates also to a method of differentiating a "tendon perivascular cells" / a "population of perivascular cells" into a cell type of interest, the method comprising
(a) providing the "tendon perivascular cells" / "population of tendon perivascular cells" as defined herein; and
(b) cultivating the "tendon perivascular cells" / "population of tendon perivascular cells" of a) under conditions allowing the differentiation of the pluripotent-like cells into the cell type of interest.
Similarly, the present disclosure relates, also to a method of differentiating a "ligament perivascular cells" / a "population of ligament perivascular cells" (or a mixture of tendon and ligament and cells or a mixed population of tendon and ligament perivascular cells) into a cell type of interest, the method comprising
(a) providing the "ligament perivascular cells" / "population of ligament perivascular cells" (and/or a mixture of tendon and perivascular cells / a mixed population of tendon and perivascular cells), as defined herein; and
(b) cultivating the "ligament perivascular cells" / "population of ligament perivascular cells" (and/or a mixture of tendon and perivascular cells / a mixed population of tendon and perivascular cells), of a) under conditions allowing the differentiation of the pluripotent cells into the cell type of interest.

All explanations and definitions given herein in the context of differentiating (a) pluripotent cell(s) and/or (a) tenosphere(s), also apply, mutatis mutandis, in the context of differentiating "tendon perivascular cells" / "population of tendon perivascular cells" / "ligament perivascular cells" / "population of ligament perivascular cells" (or a mixture of tendon and perivascular cells / a mixed population of tendon and perivascular cells).
Therefore, only exemplary conditions allowing the differentiation of the pluripotent cells/tenosphere/tendon perivascular cells / population of tendon perivascular cells and the like into a cell type of interest are described in the following. Protocols for differentiating pluripotent cells are well known in the art and may be employed in context of the present invention. Such protocols are, for example, desribed in Human Embryonic and Induced Pluripotent Stem Cells Lineage-Specific Differentiation Protocols Series: Springer Protocols Handbooks Ye, Kaiming; Jin, Sha (Eds.) 1st Edition., 2011, 480 p. 96 illus., 53 in color.A Humana Press product ISBN 978-1-61779-266-3.

Once pluripotent cells, the tenosphere and/or tendon perivascular cells / population of perivascular cells and the like are generated, the cells therein can be differentiated in vitro by culturing the cells under appropriate conditions, as described in the following.

In the methods for differentiating the pluripotent cells, the pluripotent cells can be cultivated of from 35 to 39 °C, preferably of from 36 °C to 38 °C, like about 37 °C. The pluripotent can be cultivated in a 5 % CO₂/97 % air humidified environment. The term "97 % air" is well known in the art and also refers to atmospheric O₂ (about "20% O₂"). These terms are well known in the art. Generally, cells are grown and maintained in cell culture at an appropriate temperature and gas mixture (typically, 37°C, 5% CO2 for mammalian cells) in a cell incubator.

The pluripotent cells can be cultivated in appropriate vessels/culture dishes, like T25 flasks, T75 flasks, 6 well, 12 well, 24 well, 48 well, 96 well, roller bottles and so on. The vessels may be uncoated or coated (for example coated with laminin) The laminin coat is advantageous, because, laminin is known to be a factor inducing neural differentiation. Further, it was observed that the survival of neurons on laminin coated vessels is longer. It was also observed that adherence is better. For example, vessels/culture dishes which are coated with poly-L-ornithin and/or laminin (e.g. coated with poly-L-ornithin and laminin) may be used. Exemplary vessels to be used may be coated with 100 µg/ml poly-L-ornithin and/or 5 µg/ml laminin (e.g. coated with 100 µg/ml poly-L-ornitliin and of from 50ng/ ml to 5 mg/ml laminin, like of from 5 µg/ml to 5 mg/ml laminin).

Typically, the pluripotent cells differentiate into a cell type of interest within a short time, usually within one or several days under the herein described culture conditions. Usually, the pluripotent cells differentiate within about 2 days to about 8 weeks days, for example within 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or even longer, like about 4 weeks, 5 weeks, 6 weeks, 7 weeks or 8 weeks (or longer). The time needed for differentiation into a cell type of interest may vary depending on the cell type. For example, it was observed herein that the pluripotent cells may differentiate into neuronal cells within 1 to 2 days, whereas the pluripotent cells differentiate into adipocytes or osteoblasts within up to 6 weeks.

Media change may be performed, for example, one, twice, three times or more often. The media change may be made after 12 hours, 24 hours, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or even longer, like about 4 weeks, 5 weeks, 6 weeks, 7 weeks or 8 weeks of cultivation of the pluripotent cells.

The cultivated pluripotent cells may be tested for expression of marker genes of the cell type of interest in order to assess the amount/percentage of differentiated cells. As shown herein, the differentiated cells provided in accordance with the present invention show characteristic features. For example, differentiated cell express well-known marker genes, i.e. they express markers/proteins which are expressed in known differentiated cells. Non-limiting examples of such marker genes are GalC, GFAP, β-III-Tubulin and GAP43. For example, differentiated cells may stain positive for the 04 antigen; or may contain characteristic components like triglycerides or mineralizations like hydroxyapatite.
For example, oligodendrocytes may express GaIC and/or stain positive for the 04 antigen (e.g they may express GalC and stain positive for the 04 antigen - the 04 antigen is well known in the art and, for example described in Poduslo SE, Curbeam R, Miller K, Reier P (1985) J Neurosci Res. ;14(4):43347); astrocytes may express GFAP; neurons may express β-III-Tubulin and/or GAP43 the above genes (e.g. they may express β-III-Tubulin and GAP43); adipocytes may stain positive for triglycerides (e.g positive Oil Red O staining); osteoblasts may show a positive Alizarin Red S reaction (e.g. positive for mineralization like hydroxyapatite).
If the cell type of interest into which the pluripotent cells differentiate are oligodendrocytes, the oligodendrocytes can be marked as "oligodendroncyte (GalC+; O4+)". If the cell type of interest into which the pluripotent cells differentiate are astrocytes, the astrocytes can be marked as "astrocyte (GFAP+)". If the cell type of interest into which the pluripotent cells differentiate are neurons, the neurons can be marked as "neuron β-III-Tubulin+; GAP43+). If the cell type of interest into which the pluripotent cells differentiate are adipocytes, the adipocytes may be marked as "adipocyte (triglyceride+)". If the cell type of interest into which the pluripotent cells differentiate are osteoblasts, the osteoblasts may be marked as "osteoblast (hydroxyapatite+)". Osteoblasts produce pericellular deposits stained with Alizarin S. Oil Red O is a lipophilic dye, staining any intracellular lipid droplets, in particular those containing triglycerides.

The denomination "+" (e.g. GalC+) indicates the presence of the respective marker, for example, indicates an expression of the marker that may be reflected in a detectable expression level (protein, mRNA etc.) of any of the marker gene, or indicates the presence of the respective antigen (e.g. 04) or indicates the presence of the cellular compoment (e.g triglycerides). Generally, a cell is classified as "differentiated" (e.g. differentiate into oligodendroncytes, astrocyte, neuron, adipocyte, osteoblast) if at least one of the herein described marker(s) (genes) is present at a detectable (expression) level. The (expression) level may be comparable/identical to the (expression) level of positive control cells (e.g. differentiated cells obtained from cell culture/biopsy etc., like cells differentiated in a natural environment as explained above from one of the three germ layers). Methods for detecting and evaluating (expression) levels of such marker (genes) are well known in the art and are also demonstrated in the appended examples.
Examplary nucleic acid sequences and amino acid sequences of e.g. GalC, GFAP, β-III-Tubulin, GAP43 to be used herein are shown in SEQ ID NO: 57/58 (GalC), SEQ ID NO: 105/106 (β-III-Tubulin), SEQ ID NO: 109/110 (GAP43 isoform 2), SEQ ID NO: 111/11 (GAP43 isoform1), SEQ ID NO: 115/116 (GFAP). These sequences and further sequences to be used herein are shown below and may be deduced from the respective databases.

Methods for detecting the expression level at the protein level or the mRNA level are known and have been described above in context of "pluripotent cell". The corresponding explanations and definitions apply, mutatis mutandis, in context of the differentiated cells.

A person skilled in the art is easily in the position to determine the expression level of the herein described marker genes based on his common general knowledge and the teaching provided herein. In particular, a skilled person can readily decide whether a certain marker is or is not expressed/present in a cell or cell population and whether the cell/cell population qualify as "differentiated cell" / cell population comprising "differentiated cell(s)".

It is one advantageous property of the herein provided pluripotent cell(s)/cell population comprising pluripotent cell(s) that the cells can be easily differentiated into a cell type of interest, like neuronal cells, mesenchymal cells or endodermal cells. Thus, the pluripotent cell(s) can be easily differentiated without the need of, for example, compounds inducing or enhancing the differentiation of the cells. Accordingly, the pluripotent cells may differentiate "spontaneously" into various cell types, for example, neuronal cells like neurons, astrocytes and oligodendrocytes; see Example 2 and Example 8.

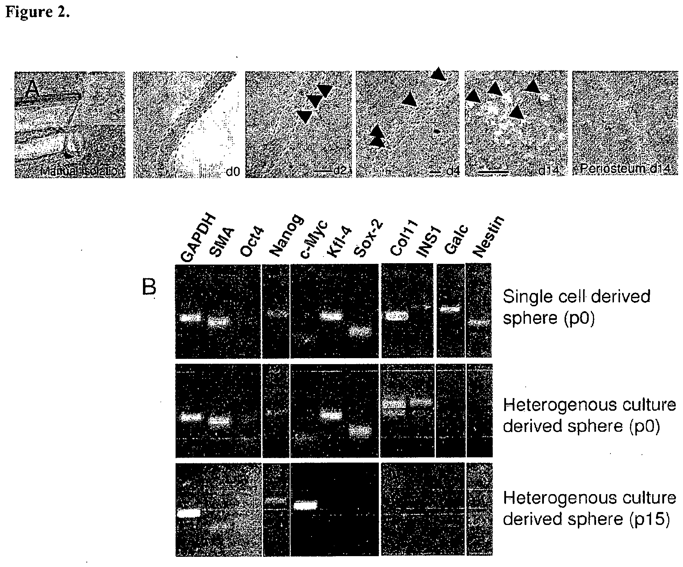

The capacity of the pluripotent cells provided herein to differentiate "spontaneously" is illustrated by the finding that the pluripotent cells have a cell intrinsic differentiation bias towards various cell types, such as neurons, astrocytes and oligodendrocytes. This is hallmarked by two observations:
- These 3 cell types were detected in sectioned spheres, that were grown in Neurobasal medium and the like as described above, as shown for example by the detection of the oligodendrocyte marker protein GalC in Fig. 2b.
- In Example 8, using oct4-egfp-expressing mouse derived, pluripotent cells, neurons β-III Tubulin+; GAP43+) (<15%), oligodendrocytes (O4+; GalC+) (<15%) and astrocytes (GFAP+) (<15%) were obtained without applying any factor inducing differentiation. In other words, the pluripotent cells differentiated in a completely unsupplemented medium (DMEM-KO) on uncoated plastic dishes within 2 days.

A simple protocol for differentiating pluripotent cells into neuronal cells/neural cell types may, accordingly, comprise the provision of the pluripotent cells (e.g. recovered pluripotent cells like the cell suspension described above) and cultivating the pluripotent cells in the presence of unsupplemented medium, like DMEM-Knockout medium (the terms "DMEM-Knockout medium" and "DMEM-KO medium" are used interchangeably herein), DMEM, MEM, NB, DMEM/F12, Hams-F 12 or RPMI medium

DMEM KO medium is different from DMEM medium, in that DMEM KO can be supplemented with 20% Knockout serum replacement, and is commonly used in the art for embryonic stem cell culture.

### Formulation of DMEM:

Inorganic Salts (g/liter)
CaCl2 (anhydrous) 0.20000
Fe(NO3)3·9H2O0.00010
MgSO₄ (anhydrous) 0.09770
KCl 0.40000
NaHCO3 1.50000
NaCl 6.40000
NaH2PO4·H2O 0.12500
Amino Acids (g/liter)
L-Arginine-HCl 0.08400
L-Cystine·2HCl 0.06260
L-Glutamine 0.58400
Glycine 0.03000
L-Histidine·HCl·H2O 0.04200
L-Isoleucine 0.10500
L-Leucine 0.10500
L-Lysine·HCl 0.14600
L-Methionine 0.03000
L-Phenylalanine 0.06600
L-Serine 0.04200
L-Threonine 0.09500
L-Tryptophan 0.01600
L-Tyrosine·2Na·2H2O 0.10379
L-Valine 0.09400

Formulation of DMEM KO medium and Knockout serum replacement is commercially available from e.g. Invitrogen (http://products.invitrogen.com/ivgn/product/10829018).

Following this protocol, neuronal cells, such as glial cells (like oligodendrocytes (O4+; GalC+) and astrocytes (GFAP+)) and neurons (β-III Tubulin+; GAP43+) can be easily obtained. The pluripotent cells can be cultivated under appropriate conditions (exemplary parameters like gas mixture, temperature, duration, vessels are described above).

Accordingly, the present disclosure relates to a method of differentiating a pluripotent cell into a neuronal cell, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into the neuronal cell.

The neuronal cell may be glial cell (like oligodendrocytes (O4+; GalC+) and astrocytes (GFAP+)) or a neuron (β-III Tubulin+; GAP43+). The conditions allowing the differentiation may comprise cultivating the pluripotent cells in the presence of unsupplemented medium, like DMEM-Knockout medium, DMEM, MEM, NB, DMEM/F12, Hams-F12 or RPMI medium.

For example, the pluripotent cells and/or tenospheres may be cultivated in the presence of DMEM-Knockout medium for e.g .2 days on uncoated glass cover slites and the like. Alternatively, the pluripotent cells and/or tenospheres may be cultivated in the presence of DMEM-Knockout 20 % serum replacement medium for e.g .7 days on uncoated glass cover slites and the like.

A more sophisticated protocol allowing for the generation of a cell population enriched in neuronal cells is provided further below.

Differentiation of the pluripotent cells and/or the tenosphere(s) can be induced by cultivation under routine conditions as described, for example, in context of the formation of tenospheres or "passaging", if this cultivation comprises cultivation in the presence of serum, in particular human serum or FCS (fetal calf serum) or FBS (fetal bovine serum). Under such conditions, the pluripotent cells and/or the tenosphere(s) adhere (become adherent, no longer free floating) and form a proliferating cell culture of GFAP positive cells (<20%) (like astrocytes) and cells with the potential to differentiate into osteoblasts and adipocytes, like mesenchymal stem cells. These mesenchymal stem cells are capable of differentiating to desired cell types, such as osteoblasts and/or adipocytes as shown hererein; see Figure 5. Under these conditions, few cells (<5%) differentiate into adipocytes and osteoblasts without, any further stimulus within 2 weeks. This protocoll may, therefore, be considered as a simple protocol for the differentiation into adipocytes and osteoblasts on the one hand or GFAP positive cell (like astrocytes) on the other hand; see Example 8.
Accordingly, the present disclosure relates to a method of differentiating a pluripotent cell into a mesenchymal cell or an astrocyte cell, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into a mesenchymal cell or an astrocyte cell (GFAP+).

The mesenchymal cell may be an adipocyte (triglyceride+) or an osteoblast (hydroxyapatite+). The pluripotent cells can be cultivated under appropriate conditions (exemplary parameters like gas mixture, temperature, duration, vessels are described above). The conditions allowing the differentiation may comprise cultivating the pluripotent cells in the presence of medium supplemented with serum, like DMEM-Knockout medium, DMEM, MEM, NB, DMEM/F12, Hams-F12 or RPMI medium supplemented with serum.

For example, any of the conventional medium mentioned above may be supplemented with 1 to 40 % serum (i.e. the final medium contains 99 to 60 % conventional medium and 1 to 40 % serum, respectively), 2 to 39 % serum, 3 to 38 % serum, 4 to 37 % serum, 5 to 36 % serum, 6 to 35 % serum, 7 to 34 % serum, or 8 to 35 % serum. For example, the conventional medium may be supplemented with 5 to 20 % serum, more preferably with 7 to 15 % serum, even more preferably with 8 to 12 % serum and most preferably with 10 % serum.
The serum may be human serum or FCS (fetal calf serum) or FBS (fetal bovine serum). For example, the pluripotent cells and/or tenospheres may be cultivated in the presence of αMEM medium medium supplemented with serum like FBS (e.g 10 % FBS) for e.g. 2 weeks:

The percentage/amount of a differentiated cell type of interest can be increased (i.e. the differentiated cell type can be "enriched") by taking advantage of the following, more sophisticated protocols:
If an enrichment of adipocytes or osteoblasts is desired, the pluripotent cells can be cultivated in the presence of Bone Morphogenetic Protein 2 (BMP-2) and Bone Morphogenetic Protein 4 (BMP-4). Bone Morphogenetic Protein 2 (BMP-2) and Bone Morphogenetic Protein 4 (BMP-4) are commercially available e.g. from Sigma Aldrich, BMP2 #B3555-10UG, BMP4 #SRP3016-10UG. It has been found herein that in case the pluripotent cells are stimulated with BMP 2/4 for 7 days, the cells differentiate into adipocytes (>30%), osteoblasts (>30%) and GFAP⁺ cells (>10%) (like astrocytes).

Accordingly, the present disclosure relates to a method of differentiating a pluripotent cell into a mesenchymal cell or an astrocyte cell, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into a mesenchymal cell or an astrocyte cell (GFAP+), comprising the cultivating in the presence of Bone Morphogenetic Protein 2 (BMP-2) and/or Bone Morphogenetic Protein 4 (BMP-4) (like in the presence of Bone Morphogenetic Protein 2 (BMP-2) and of Bone Morphogenetic Protein 4 (BMP-4)).

The mesenchymal cell may be an adipocyte (triglyceride+) or an osteoblast (hydroxyapatite+). The pluripotent cells can be cultivated under appropriate conditions (exemplary parameters like gas mixture, temperature, duration, vessels are described above). The conditions allowing the differentiation may comprise cultivating the pluripotent cells in the presence of medium described above, optionally supplemented with serum, like DMEM-Knockout medium, DMEM, MEM, NB, DMEM/F12, Hams-F12 or RPMI medium optionally supplemented with serum.

The medium used for cultivation may comprise BMP-2 in a concentration of from 1 ng/ml to 1 mg/ml and/or BMP-4 in a concentration or from 1 ng/ml to 1 mg/ml. More preferably, the medium comprises BMP-2 in a concentration of from 5 ng/ml to 100 ng/ml and/or BMP-4 in a concentration of from 5 ng/ml to 100 ng/ml. For example, the medium may comprise BMP-2 in a concentration of from 5 ng/ml, 6 ng/ml, 7 ng/ml, 8 ng/ml or 9 ng/ml to 20 ng/ml, 30 ng/ml, 40 ng/ml, 50 ng/ml, 60 ng/ml, 70 ng/ml, 80 ng/ml or 90 ng/ml and/or BMP-4 in a concentration of from 5 ng/ml, 6 ng/ml, 7 ng/ml, 8 ng/ml or 9 ng/ml to 20 ng/ml, 30 ng/ml, 40 ng/ml, 50 ng/ml, 60 ng/ml, 70 ng/ml, 80 ng/ml or 90 ng/ml. For example, the medium may comprise BMP-2 in a concentration of 10 ng/ml and/or BMP-4 in a concentration of 10 ng/ml (like BMP-2 in a concentration of 10 ng/ml and BMP-4 in a concentration of 10 ng/ml).

For example, the pluripotent cells and/or tenospheres may be cultivated in the presence of DMEM-Knockout medium 20 % serum replacement medium for e.g .12 days. The pluripotent cells and/or tenospheres may be cultivated in vessels as describe above, e.g. vessels (like glass cover slites) which are coated with poly-L-ornithin and/or laminin (e.g. coated with poly-L-ornithin and laminin) may be used. Exemplary vessels to be used may be coated with 100 µg/ml poly-L-ornithin and/or 5 µg/ml laminin (e.g. coated with coated with 100 µg/ml poly-L-ornithin and 5 µg/ml laminin). After 12 hours the medium may be exchanged. The medium for further cultivation for e.g 7 days may comprise BMP-2 in a concentration of 10 ng/ml and/or BMP-4 in a concentration of 10 ng/ml (like BMP-2 in a concentration of 10 ng/ml and BMP-4 in a concentration of 10 ng/ml); see Example 1 and 2.

If an enrichment of adipocytes or osteoblasts is desired, the pluripotent cells can be cultivated according to the following protocols:
Enrichment with adipocytes and osteoblasts can also be achieved by 4 weeks of culture in the following, differentiation media, as exemplified in Example 1:
   - adipocytes: αMEM-10%FBS supplemented with 1 µM dexamethasone, 10 µg/ml insulin (Sigma, Germany), 0.5 mM 3-isobutyl-1-methylxanthine (Calbiochem, Germany) and 100 µM indomethacin (Sigma, Germany);
   - osteoblasts: αMEM-10%FBS supplemented with 0.1 µM dexamethasone, 10 mM β-glycerophosphate, and 50 µM L-ascorbic acid-2-phosphate (all from Sigma, Germany).
This way of culturing the cells yields >90% adipocytes or osteoblasts, as monitored by either red oil O- staining (adipocytes) or staining of either alkaline phosphatise or mineralisation (visualized by alizarin s); see Example 1.

Accordingly, the present disclosure relates to a method of differentiating a pluripotent cell into an adipocyte, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into an adipocyte, comprising the cultivating in the presence of a medium supplemented with serum, dexamethasone, insulin, 3-isobutyl-1-methylxanthine and indomethacin.

In one aspect, the present disclosure relates to a method of differentiating a pluripotent cell into a adipocytes, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into an adipocyte, comprising the cultivating in the presence of αMEM-10%FBS supplemented with 1 µM dexamethasone, 10 µg/ml insulin, 0.5 mM 3-isobutyl-1-methylxanthine and 100 µM indomethacin.

Accordingly, the present disclosure relates to a method of differentiating a pluripotent cell into an osteoblast, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into a osteoblast, comprising the cultivating in the presence of a medium supplemented with serum, dexamethasone, β-glycerophosphate, and L-ascorbic acid-2-phosphate.

In one aspect, the present disclosure relates to a method of differentiating a pluripotent cell into an osteoblast, the method comprising
(a) providing the pluripotent cells (Oct4+; nanog+; Sox2+; Klf4+; c-myc+) as defined herein and/or the tenosphere as defined herein; and
(b) cultivating the pluripotent cells (Oct4+; nanog+; Sox2+; Klf4+; c-myc+) and/or the tenosphere of a) under conditions allowing the differentiation of the pluripotent-like cells into a osteoblast, comprising the cultivating in the presence of αMEM-10%FBS supplemented with 0.1 µM dexamethasone, 10 mM β-glycerophosphate, and 50 µM L-ascorbic acid-2-phosphate.

The adipocyte may be an adipocyte (triglyceride+). The osteoblast may be an osteoblast (hydroxyapatite +):

The pluripotent cells can be cultivated under appropriate conditions (exemplary parameters like gas mixture, temperature, duration, vessels are described above). The conditions allowing the differentiation may comprise cultivating the pluripotent cells in the presence of medium described above, supplemented with serum, like DMEM-Knockout medium, NB, DMEM/F12, Hams-F12 or RPMI medium supplemented with serum.

The method of differentiating a pluripotent cell may omprise a first step of cultivating in the presence of medium which is only supplemented with serum. For example, the pluripotent cells and/or tenospheres may, in a first step, be cultivated in the presence of medium which is only supplemented with serum (like FBS, e.g. 10 % FBS, for example DMEM supplemented with 10 % FBS). For example, the pluripotent cells and/or tenospheres may be cultivated in the presence of αMEM-medium supplemented with e.g 10 % FBS for e.g two weeks.
The pluripotent cells and/or tenospheres may be cultivated in vessels as describe above, e.g. vessels (like glass cover slites) which are coated with poly-L-ornithin and/or laminin (e.g. coated with poly-L-ornithin and laminin) may be used. Exemplary vessels to be used may be coated with 100 µg/ml poly-L-ornithin and/or 5 µg/ml laminin (e.g. coated with coated with 100 µg/ml poly-L-ornithin and 5 µg/ml laminin).
After 12 hours the medium used for cultivation in a first step may be exchanged. The medium for further cultivation for e.g. up to 4 weeks may (if a differentiation into adipocytes is desired) comprise medium supplemented with serum, dexamethasone, insulin, 3-isobutyl-1-methylxanthine and indomethacin. or it may (if a differentiation into osteoblasts is desired) comprise medium supplemented with serum, dexamethasone, β-glycerophosphate, and L-ascorbic acid-2-phosphate.

Tenosphere adipogenic differentiation can, for example, be induced as follows: Tenosphere adipogenic differentiation can be induced with αMEM-10%FBS supplemented with 1 µM dexamethasone, 10 µg/ml insulin (Sigma, Germany), 0.5 mM 3-isobutyl-1-methylxanthine (Calbiochem, Germany) and 100 µM indomethacin (Sigma, Germany).

Tenosphere osteogenic differentiation can be achieved by 4 weeks of culture in µMEM-10% FBS supplemented with 0.1 µM dexamethasone, 10 mM β-glycerophosphate, and 50 µM Lascorbic acid-2-phosphate (all from Sigma-Aldrich, Taufkirchen, Germany).

Tenosphere chondrocyte differentiation can be induced with αMEM-10% FBS supplemented with 6.0 µg/ml human insulin, 200 µM L-ascorbic acid-2-phosphate and 1ng/ml of TGF β1 (RD Systems, Minneapolis, USA) for 4 weeks.

Contrary to conventional mesenchymal stem cells, a much higher percentage (about 95 %) of the thus provided mesenchymal stem cells are capable of differentiating, whereas only 40 to 60 % of conventational mesenchymal stem cells differentiate under comparable conditions.

Accordingly, differentiation of the pluripotent cells and/or the tenosphere(s) is preferably performed in the presence of serum, such as fetal calf serum (FCS) and/or autologous serum. Any mammalian (preferably human) serum or serum replacement can be used. For example, a conventional medium such as DMEM, MEM, NB, DMEM/F12, Hams-F12 or RPMI may be supplemented with serum. Preferably, a conventional medium is supplemented with 2 to 25 % serum (i.e. the final medium contains 98 to 75 % conventional medium and 2 to 25 % serum, respectively). More preferably, the conventional medium is supplemented with 5 to 20 % serum, more preferably with 7 to 15 % serum, even more preferably with 8 to 12 % serum and most preferably with 10 % serum. The term "(differentiation) in the presence of medium supplemented with serum" is also referred to herein shortly as "(differentiation) in the presence of serum".

The above procedure may also be employed to induce differentiation of tendon/ligament perivascular cells / population of tendon / ligament perivascular cells.

The method may comprise cultivating the pluripotent cells (Oct4+; nanog+; Sox2+; Klf4+; c-myc+) the tenosphere and/or tendon perivascular cells / population of perivascular cells and the like in a culture medium comprising a differentiation-inducing amount of one or more factors as described above that induce differentiation of the pluripotent cells (Oct4+; nanog+; Sox2+; Klf4+; c-myc+), the tenosphere and/or tendon perivascular cells / population of perivascular cells and the like or derivatives thereof into the cell type of interest until the cell type of interest appears in the in vitro culture. Thus, the conditions allowing differentiation encompass the cultivation of pluripotent cells in the presence of one or more factors inducing or enhancing differentiation of the pluripotent cells, whereby the cultivation of pluripotent cells is in the presence of an differentiation-inducing (or differentiation-enhancing) amount of the one or more factors. In other words, the one or more factors are present in an amount sufficient to induce or enhance differentiation. Examplary factors described above which induce or enhance differentiation are Bone-Morphogenetic Protein 2 (BMP-2), Bone Morphogenetic Protein 4 (BMP-4), mesenchymal stem cell conditioned medium (MSC-CM), estrogen, insulin, dexamethasone, serum, 3-isobutyl-1-methylxanthine, indomethacin, β-glycerophosphate, or L-ascorbic acid-2-phosphate.

As used herein, the phrase "differentiation-inducing amount" refers to an amount of a growth factor or other activator that when present within an in vitro differentiation medium, causes a pluripotent cells, tenosphere and/or tendon perivascular cells / population of perivascular cells cell and the like or derivative thereof to differentiate into a cell type of interest. In some embodiments, the growth factor or other activator includes, Bone-Morphogenetic Protein 2 (BMP-2), Bone Morphogenetic Protein 4 (BMP-4), mesenchymal stem cell conditioned medium (MX-CM/MSC-CM), estrogen, insulin, dexamethasone, altered medium osmolarity, ascorbic acid or others known in the art.

The choice of growth factors and/or other supplements can depend on the cell type desired into which the pluripotent cells, the tenosphere and/or tendon perivascular cells / population of perivascular cells and the like are to differentiate. In some embodiments, the pluripotent cells, the tenosphere and/or tendon perivascular cells / population of tendon perivascular cells and the like can be differentiated into neuronal derivatives including, but not limited to neurons, oligodendrocytes, astrocytes, and glial cells. As disclosed in Example 2, pluripotent cells/tenospheres can be differentiated into neuronal derivatives (astrogenic response) by culturing them in medium comprising DMEM Knockout-20 % SR (Gibco Invitrogen) followed by cultivating in medium comprising DMEM Knockout-20 % SR (Gibco Invitrogen) supplemented with BMP-2 and BMP-4. Example 2 also demonstrates a oligodendrogenic response of pluripotent cells/tenospheres in differentiation experiments. It is shown that pluripotent cells/tenospheres can be differentiated by plating tehm on 100 µg/ml poly-ornithine (Sigma-Aldrich) and 5 µg/ml laminin coated (Sigma-Aldrich) glass coverslips into well plates in MEM 10 % fetal bovine serum (Gibco Invitrogen), followed by incubation in the presence of MSC-CM medium.

If differentiation into a neuronal cell type is desired, the pluripotent cells (Oct4+; nanog+; Sox2+; Klf4+; c-myc+) and/or the tenosphere may be cultivated in the presence of Bone Morphogenetic Protein 2 (BMP-2) and/or Bone Morphogenetic Protein 4 (BMP-4).

An exemplary more detailed protocol is provided below:
To assess the astrogenic potential and the response to bone morphogenetic proteins (BMP), spheres can be dissociated and 2.5 x 104 (i.e. 2.5 x 10⁴) cells/well can be plated on 100 µg/ml poly-L-ornithine (Sigma-Aldrich, Tauflcirchen,Germany) and 5 µg/ml laminin-coated (Sigma-Aldrich, Tauflcirchen, Germany) glass coverslips. The coverslips can be placed in 24-well plates and cells can be grown in DMEM Knockout-20% serum replacement (SR) (Gibco Invitrogen, Karlsruhe, Germany). After 12 hours, the medium can be changed to DMEM Knockout- 20% SR supplemented with 10 ng/ml BMP-2 and BMP-4 (R&D Systems GmbH, Wiesbaden-Nordenstadt, Germany). After 7 days, the cells can be fixed for 30 minutes with 4% paraformaldehyde and processed for immunofluorescence staining. Following BMP treatment, TPCs can be tested for their mesenchymal differentiation potential using Alkaline phosphatase and red oil O staining.

An alternative detailed protocol is provided below:
To assess astrogenic potential of tenospheres, 2-5 x 104 (10⁴) (e.g. 2.5 x 10⁴) cells/well can be plated on 100 µg/ml poly-L-ornithine (Sigma-Aldrich, Taufkirchen, Germany) and 5 µg/ml laminin-coated (Sigma-Aldrich, Taufkirchen, Germany) glass coverslips into 24-well plates in DMEM Knockout-20% SR (Gibco Invitrogen, Karlsruhe, Germany). After 12 hours, the media can be refreshed with DMEM Knockout-20% SR, supplemented with 10 ng/ml BMP-2 and BMP-4 (R&D Systems GmbH, Wiesbaden-Nordenstadt, Germany) to evaluate astrogenic response. Finally, in order to analyze tenosphere oligodendrogenic response, 2-5 x 104 (10⁴) (e.g. 2.5 x 10⁴) cells/well can be plated on 100 µg/ml poly-L-ornithine (Sigma-Aldrich, Taufkirchen, Germany) and 5 µg/ml laminin-coated (Sigma-Aldrich, Taufkirchen, Germany) glass coverslips into 24-well plates in (□) αMEM 10% fetal bovine serum (Gibco Invitrogen, Karlsruhe, Germany). After 12 hours, cells can be incubated in the presence of MSC-CM. In all cases, media may be refreshed every third day. After 7 days, the cells can be fixed for 30 minutes with 4% paraformaldehyde and processed for immunofluorescence staining.

For differentiation into an oligodendrocyte cell type, the pluripotent cells (Oct4+; nanog+; Sox2+; Klf4+; c-myc+) and/or the tenosphere may be cultivated in the presence of mesenchymal stem cell conditioned medium (MSC-CM). The presence of mesenchymal stem cells (MSC) is believed to be beneficial in the desired differentiation of pluripotent cells into a oligodendrocyte cell type cell type since MSCs release soluble factor(s) able to induce oligodendrogenesis in tenospheres derived cells/pluripotent cells; see Example 2.

An exemplary protocol is provided below:
MSCs can be incubated in alpha-MEM 10%FBS (fetal bovine serum) and after 3 days incubation conditioned media may be filtered with a 0,22 um pore size filter. The resulting media is denoted herein "mesenchymal stem cell conditioned medium" or, short, "MSC-CM". It has been found that this medium induces oligodendrocyte differentiation in adult derived neural progenitor cells (Rivera, et al 2006, Stem Cells).

Herein below the preparation of the MSCs and MSC-CM is described in detail:

### MSCs culture

Bone marrow plugs can be harvested from femurs and tibias of 2-4 month-old female Fisher-344 rats (Charles River Deutschland GmbH, Germany). Plugs can be mechanically dissociated in αMEM (Gibco Invitrogen, Karlsruhe, Germany) and recovered by centrifugation. Cell pellets can be resuspended in αMEM-10% FBS and seeded at 1x106 cells/cm2. After 3 days, media may be changed and non-adherent cells may be removed. Adherent cells can be incubated in fresh αMEM-10% FBS until a confluent layer of cells is achieved. Cells can be trypsinized using 0.25% Trypsin (Gibco Invitrogen, Karlsruhe, Germany) and seeded in αMEM-10% FBS at 8,000 cells/cm2. After 3-5 days of culture, the resulting monolayer of cells, hereafter named rat bone marrow derived mesenchymal stem cells (MSCs), can be trypsinized and further cultured for experiments or frozen for later use. This cell culture preparation is highly enriched in multipotent MSCs with virtually no hematopoietic contamination.

### Mesenchymal stem cell conditioned media preparation

MSCs can be plated at 12,000 cells/cm2 and incubated in αMEM-10% FBS. After 3 days, the conditioned medium can be collected and filtered using a 0.22 µm-pore filter and used as a tenosphere oligodendrogenic stimulus.

### Formation of MSC like cells

The formation of MSC like cells can be induced by cultivating dissociated spheres (5xl04 (i.e. 5x 10⁴) cells/well) in DMEM supplemented with 10% FBS for 2 weeks.

Osteogenic differentiation can be observed following 4 weeks of culture in αMEM-10%FBS supplemented with 0.1 µM dexamethasone, 10 mM β-glycerophosphate, and 50 µM L-ascorbic acid-2-phosphate (all from Sigma, Germany). Osteogenic differentiation may be revealed by deposits in the extracellular matrix of mineralized hydroxyapatite, the latter may be further stained with 40 mM Alizarin Red S (Sigma, Germany). MSC adipogenic differentiation can be induced with αMEM-10%FBS supplemented with 1 µM dexamethasone, 10 µg/ml insulin (Sigma, Germany), 0.5 mM 3-isobutyl-1-methylxanthine (Calbiochem, Germany) and 100 µM indomethacin (Sigma, Germany). After one week of culture in these conditions, adipogenic differentiation can be confirmed by staining the cytoplasmic lipid droplets with saturated Oil Red O (Merck, Germany).

The cultivation of tenospheres and/or pluripent cells under conditions allowing the formation of tenospheres, is also advantageous for the generation of differentiated cells, in particular oligodendrocyte precursor cells. For example, "passaging" as described above, results in the maintenance of pluriptent cell cultures / tenospheres containing pluripotent cells. The same process and the same conditions described for generation of pluripotent cells/ tenospheres can be used for convenient and reliable provision of differentiated cells. Also "passaging" can be used. As mentioned, tenospheres provided herein contain pluripotent cells, but also other, more differentiated cell types, such as mesenchymal stem cells and neural cells. The presence of mesenchynal stem cells in the tenospheres induces the neural cells to differentiate into oligodend rocyte precursor cells, if the tenospheres are cultivated under conditions described herein, in particular conditions allowing the formation of tenospheres. This generation oligodendrocyte precursor cells is particularly advantageous as further component promoting the differentiation of neural cells do not have to be added. The tenosphere can be cultivated in the presence of any given medium known in the art, for example, MEM + 6 g glucose, N2 supplement and the like. Preferably, Neurobasal medium (NB), a medium designed for neural cell culture, or Dulbeccos Modified Eagle Medium (DMEM). Suitable conditions are described herein in context of passaging or formation of tenospheres.

The herein above defined "the cell type of interest" may be a neuronal cell, a mesenchymal cell and an endodermal cell. The neuronal cell may be an oligodendrocyte, an astrocyte, a glial cell and a neuron. The mesenchymal cell may be an adipocyte or an osteoblast. The endodermal cell may be an insulin-producing cell.

As explained above, it has surprisingly been found herein that tendon perivascular cells show pluripotent characteristics, and preferably show a similar or even identical expression pattern as the pluripotent cells (Oct4+; nanog+; Sox2+; Klf4+; c-myc+) to be generated in accordance with the present invention. The present invention provides, therefore, for means and methods of isolating and/or enriching such tendon perivascular cells which may also be considered to be "pluripotent" as defined herein.

Accordingly, the present disclosure relates in a further embodiment to a composition enriched in the pluripotent cells, the tenosphere, and/or tendon (and/or ligament) perivascular cells, in particular of tendon perivascular cells and/or pluripotent cells to be generated as described herein. Also envisaged herein is a composition enriched in other suitable cells as defined herein, for example a suitable cell found in the vicinity of other tissues than tendon, such as ligament, preferably human ligament likeanterior cruciate ligament. In the context of the present disclosure, also a composition enriched in tendon vascular cells as defined herein is envisaged. (A) corresponding cell(s)/population of cells may also be obtained from human dental pulp or ligament as described above. The explanations in context of "compositions enriched in pluripotent cells, tenosphere(s), and/or tendon perivascular cells" apply, mutatis mutandis, also to such other cells/cell types.

The composition of pluripotent cells, tenosphere(s), and/or tendon perivascular cells may be any suitable composition of such cells provided that the composition is enriched compared to a natural source of said cells. The natural source of tendon perivascular cells is, as described above, tendon tissue. The natural source of other suitable cell types to be used in accordance with the present invention is, for example, human ligament (anterior cruciate ligament) or human dental pulp.

The composition containing pluripotent cells, tenosphere(s), and/or tendon perivascular cells for use in the invention may be enriched compared to the natural sources by any suitable method, typically involving cell fractionation and concentration methods. Suitable methods are well known in the art and include the Ficoll-Paque methodology. Other suitable methods include concentration of pluripotent cells, tenosphere(s), and/or tendon perivascular cells using antibodies directed to cell markers which are immobilised, for example in an affinity chromatography column or to a substrate in a "panning" scheme.

In particular it has been found herein that pluripotent cells, tenosphere(s) comprising same, and/or tendon perivascular cells are positive for Oct4, Nanog, Sox2, c-myc and/or Klf4 and Glut2 (i.e. Oct4+, Nanog+, Sox2+, c-myc+ and/or Klf4+ and, in addition, Glut2+) in both tendons and tenospheres; see Example 7. Glut2 is a glucose transporter molecule, responsible for glucose sensing in pancreatic b-cells (e.g. Thorens B. (2001) GLUT2 in pancreatic and extra-pancreatic gluco-detection. Mol Membr Biol. 2001 Oct-Dec;18(4):265-73.). As the pluripotent cells as defined herein express Glut2 at the cell surface, the expression of the cell surface protein Glut2 allows convenient isolation of pluripotent cells, tendon perivascular cells, ligament perivascular cells and/or tenospheres expressing Glut2 from cells lacking this protein by either FACS- or MACS- sorting. The expression of pluripotent markers Oct4, nanog, Sox2, Klf4 and/or c-myc as described herein may be used to verify that the Glut2 positive cells are indeed the pluripotent-like cells provided herein.

An exemplary protocol is provided below.

For isolating a population of cells expressing Glut2 a population of tendon perivascular cells is provided as described herein. The cells are then incubated with an antibody directed against Glut2 (for example: Glut2- antibody, Chemicon, #AB1342). Cells binding this antibody can then be separated by fluorescence-activated cell sorting or magnetic activated cell sorting (MACS). The Glut2-positive cells can, for example, be purified by incubation with magnetic Goat Anti-Rabbit IgG MicroBeads and selection on a Mini-MACS apparatus (Miltenyi Biotec, Bergisch-Gladbach, Germany) following the protocol recommended by the manufacturer.

Enrichment can also be achieved by culturing the pluripotent cells, tenosphere(s), tendon perivascular cells and/or ligament perivascular cells and expanding the cells under conditions which retains their character as a pluripotent cells, tenosphere(s), tendon perivascular cells and/or ligament perivascular cells. Such methods are described herein. For example, the methods for generating pluripotent cells and/or for formation of tenospheres may be considered as methods allowing the enrichment of such cells/tenospheres. Corresponding markers have been described herein above and relate, in particular, to Oct4, nanog, Sox2, Klf4 and/or c-myc. Pluripotent cells, tenosphere(s), and/or tendon perivascular cells and/or cell types derived therefrom may be identified using these or other appropriate markers.

Typically, the enrichment of pluripotent cells, tenosphere(s), tendon perivascular cells and/or ligament perivascular cells in the composition is at least 2-fold over the said cell content in the natural source from which they are enriched. Preferably, the enrichment is at least 3-fold, 4-fold, 5- fold, 10-fold, 20-fold or more preferably at least 30 or 40 or 50 or 100-fold. Preferably, it is at least 1000-fold or 10⁴-fold or 10⁵ fold. Typically, the enriched composition contains at least 10% of its cells as pluripotent cells, tenosphere(s), tendon perivascular cells and/or ligament perivascular cells, respectively, and preferably at least 50% or 60% or 70% or more. It may be advantageous for at least 90% or at least 95% or 99% of the cells in the composition to be pluripotent cells, tenosphere(s tendon perivascular cells and/or ligament perivascular cells.

The presently disclosed subject matter provides methods of isolating a subpopulation of pluripotent cells, tenosphere(s), tendon perivascular cells and/or ligament perivascular cell from a population of cells. The method may comprise the steps of (a) providing a population of cells suspected of comprising pluripotent cells, tenosphere(s), tendon perivascular cells and/or ligament perivascular cells; (b) contacting the population of cells with an antibody that is specific for Glut2 under conditions sufficient to allow binding of the antibody to its target, if present, preferably on each cell of the population of cells; (c) selecting subpopulation of cells that are positive for the Glut2 marker.

An exemplary antibody directed against Glut2 which is to be used in this context is commercially available from Chemicon #AB1342. However, the use of other suitable antibodies is envisaged in this context. Such antibodies are commercially available or can be generated by routine techniques. As mentioned above, the selection/separation of the subpopulation may be performed by fluorescence-activated cell sorting or magnetic activated cell sorting (MACS). The Glut2-positive cells/tenospheres can, for example, be purified by incubation with magnetic Goat Anti-Rabbit IgG MicroBeads. This may be followed by selection on a Mini-MACS apparatus (for example available from Miltenyi Biotec, Bergisch-Gladbach, Germany) following the protocol recommended by the manufacturer.

The method may comprise additional steps of enriching/isolating the pluripotent cells. For example, the step of contacting the cells/tenospheres with a Glut2 specific antibody and the selection/separation of Glut2 positive cells may be repeated one time or several times in order to obtain a more pure cell and/or tenosphere population.

In addition, or in the alternative, the method may comprise steps of contacting the cells/tenospheres with antibodies specific for further cell surface markers different from Glut2. For example, the method may comprise a step (d) contacting the subpopulation of cells positive for Glut2 with one or more antibodies that are specific for one or more cell surface markers (different from Glut2) under conditions sufficient to allow binding of each antibody to its target, if present, preferably on each cell of the subpopulation of cells; (e) removing from the subpopulation of cells those cells that bind to at least one of the antibodies of step (d); and (f) collecting a second subpopulation of cells, wherein these cells are preferably Oct4+, nanog+, Sox2+, Klf4+, and/or c-myc+ and, in addition, Glut2+. The separation step can be performed in a stepwise manner as a series of steps or concurrently. For example, the presence or absence of each marker can be assessed individually, producing two subpopulations at each step based on whether the individual marker is present. Thereafter, the subpopulation of interest can be selected and further divided based on the presence or absence of the next marker. Alternatively, the subpopulation can be generated by separating out only those cells that have a particular marker profile, wherein the phrase "marker profile" refers to a summary of the presence or absence of two or more markers (i.e. Glut2 and one or more further markers). For example, a mixed population of cells can contain both Glut2+ cells and cells positive for one or more further markers. Each of these individual combinations of markers represents a different marker profile. As additional markers are added, the profiles can become more complex and correspond to a smaller and smaller percentage of the original mixed population of cells.

A plurality of antibodies, antibody derivatives, and/or antibody fragments with different specificities for cell surface markers may beemployed. Each antibody, or fragment or derivative thereof, may comprise a detectable label. Different antibodies, or fragments or derivatives thereof, which bind to different markers can comprise different detectable labels or can employ the same detectable label.

A variety of detectable labels are known to the skilled artisan, as are methods for conjugating the detectable labels to biomolecules such as antibodies and fragments and/or derivatives thereof. As used herein, the phrase "detectable label" refers to any moiety that can be added to an antibody, or a fragment or derivative thereof, that allows for the detection of the antibody.

Representative detectable moieties include, but are not limited to, covalently attached chromophores, fluorescent moieties, enzymes, antigens, groups with specific reactivity, chemiluminescent moieties, and electrochemically detectable moieties, etc. In some embodiments, the antibodies are biotinylated. In some embodiments, the biotinylated antibodies are detected using a secondary antibody that comprises an avidin or streptavidin group and is also conjugated to a fluorescent label including, but not limited to Cy3, Cy5, and Cy7. In some embodiments, the antibody, fragment, or derivative thereof is directly labeled with a fluorescent label such as Cy3, Cy5, or Cy7. In some embodiments, the antibody, fragment, or derivative thereof is directly labeled with a fluorescent label and cells that bind to the antibody are separated by fluorescence-activated cell sorting. Additional detection strategies are known to the skilled artisan.

While FACS scanning is a convenient method for purifying subpopulations of cells, it is understood that other methods can also be employed. An exemplary method that can be used is to employ antibodies that specifically bind to Glut2 (and/or, optionally, to one or more further cell surface markers) with the antibodies comprising a moiety (e.g., biotin) for which a high affinity binding reagent is available (e.g., avidin or streptavidin). For example, a biotin moiety could be attached to antibodies for each marker for which the presence on the cell surface is desirable (e.g., Glut2 and/or one or more further markers), and the cell population with bound antibodies could be contacted with an affinity reagent comprising an avidin or streptavidin moiety (e.g., a column comprising avidin or streptavidin). Those cells that bound to the column would be recovered and further fractionated as desired.

Alternatively, the antibodies that bind to markers present on those cells in the population that are to be removed can be labeled with biotin, and the cells that do not bind to the affinity reagent can be recovered and purified further.

It is also understood that different separation techniques (e.g., affinity purification and FACS) can be employed together at one or more steps of the purification process.

Generally, the enrichment procedures preferably include sorting the cells by size and/or cellular markers.

In one aspect of the invention, the cells collected by the methods of the invention may be sorted into at least two subpopulations which may be cryopreserved separately or together {e.g., in the same vial). The at least two subpopulations of cells may be two subpopulation of pluripotent cells, tenosphere(s), and/or tendon perivascular cells

However, the at least two subpopulation of cells may be (1) a population enriched for pluripotent cells, tenosphere(s), and/or tendon perivascular cells and (2) a population depleted for pluripotent cells, tenosphere(s), and/or tendon perivascular cells. Furthermore, it is also envisioned that the two subpopulations (i.e., (1) and (2) above) may be cryopreserved together.

Pluripotent cells, tenosphere(s), and/or tendon perivascular cells may be sorted according to cell surface markers that are associated with pluripotent cells, tenosphere(s), and/or tendon perivascular cells. Since it is one embodiment of the invention to enrich for pluripotent cells, tenosphere(s), and/or tendon perivascular cells, useful markers for cell sorting need not be exclusively expressed in pluripotent cells, tenosphere(s), and/or tendon perivascular cells. A cell marker which is not exclusively expressed in stem cell will nevertheless have utility in enriching for pluripotent cells, tenosphere(s), and/or tendon perivascular cells.

It should noted also that markers of differentiated cells are also useful in the methods of the invention because these markers may be used, for example, to selectively remove differentiated cells and thus enrich pluripotent cells, tenosphere(s), and/or tendon perivascular cells in the remaining cell population.

The pattern of markers express by pluripotent cells, tenosphere(s), and/or tendon perivascular cells may also be used to sort and categorize pluripotent cells, tenosphere(s), and/or tendon perivascular cells with greater accuracy. Any means of characterizing, including the detection of markers or array of markers, may be used to characterized and/or identify the cells obtained through the embodiments disclosed herein. For example, certain cell types are known to express a certain pattern of markers, and the cells collected by the processes described herein may be sorted on the basis of these known patterns. Multiparameter sorting may also be employed.

The size of the pluripotent cells, tenosphere(s), and/or tendon perivascular cells may also form a basis to devise a sorting strategy to prepare an enriched population of pluripotent cells, tenosphere(s), and/or tendon perivascular cells. A combination of cellular markers and size patterns may be used to sort and categorize pluripotent cells, tenosphere(s), and/or tendon perivascular cells with greater accuracy. For example, an enriched population of tenosphere(s) is prepared by sorting for a size between 30 µm and 400 µm, preferably between 30 µm and 200 µm. Generally, an enriched population of pluripotent cells and/or tendon perivascular cells is prepared by sorting for a size between 2 to 15µm.

Furthermore, the pluripotent cells, tenosphere(s) particularly the differentiated cells (such as the neural stem cells and/or oligodendrocytes/oligodendrocyte precursor cells) derived therefrom are useful in medicine, in particular in the treatment and prevention of neural disorders/diseases and neurodegenerative disorders and diseases. Also tendon perivascular cells, and/or ligament perivascular cells are useful in this context.

The present invention is further described by reference to the following non-limiting figures and examples.

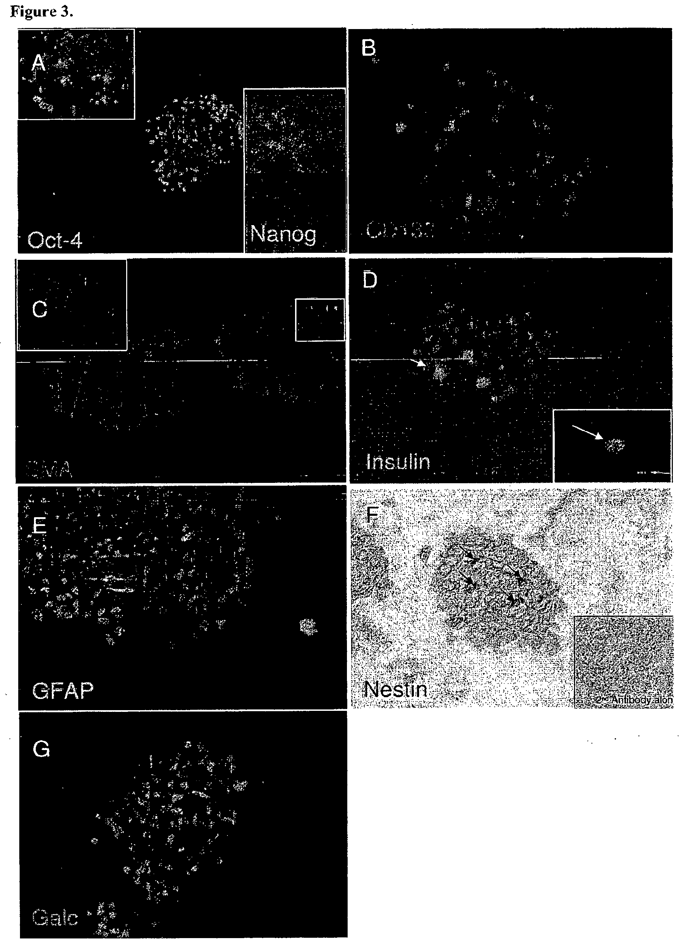

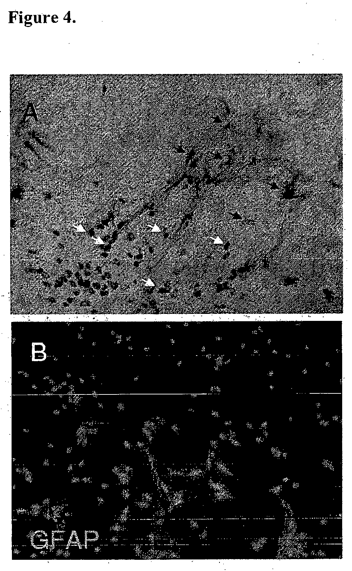

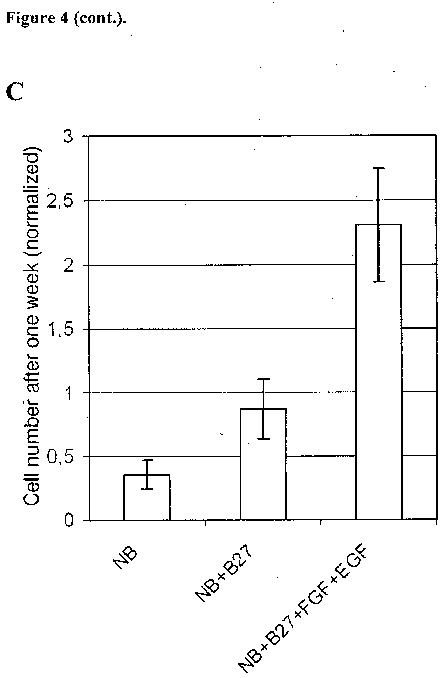

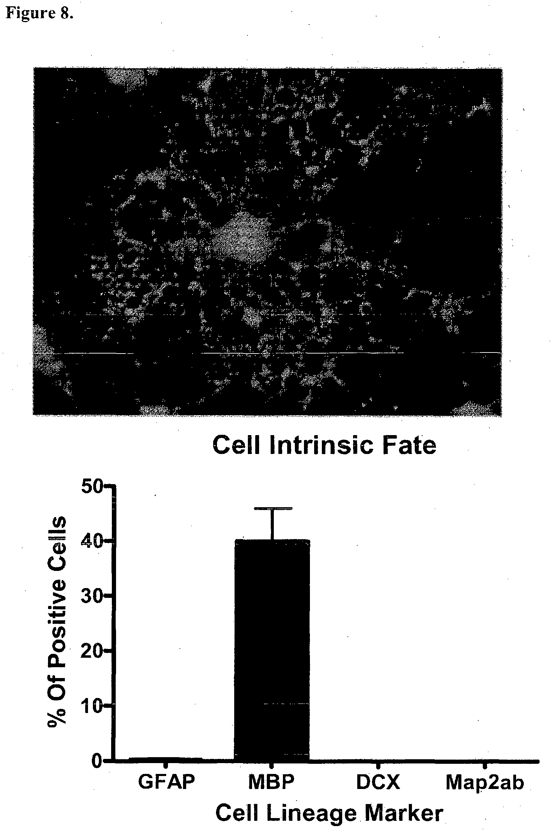

The Figures show:

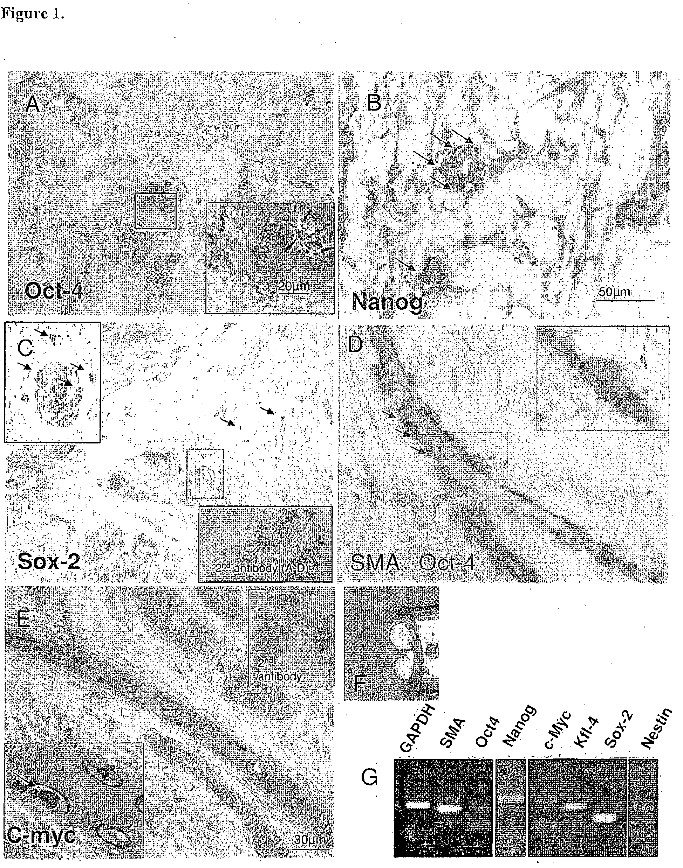

**Figure 1****. Tendon perivascular cells express pluripotent marker genes.**
   Immunohistochemical analyses of 7µm sections of human biceps tendons from 2 male donors aged 62 (A, B, C) and 21 (A, D, E) show the expression of Oct 4 and Nanog being restricted to the perivascular area (A, B D); Sox 2 is mainly expressed in perivascular cells and in few cells located in the dense collagenous tissue (C). Oct-4 positive cells in the perivascular area also express smooth muscle actin (SMA) (D) A longitudinal section of a tendon vessel shows nuclear c-myc expression in vessel associated cells. Single cell analysis of an isolated vessel associated cell (F) shows the expression ES-cell associated markers (G).
**Figure 2****. Tenospheres obtained after cultivation of tendon perivascular cells contain pluripotent cells.**
   A vessel fragment isolated from a biceps tendon specimen of a female, 70 year old donor desintegrates within 4 days in culture, only several spheres (black arrows) remain and grow up to a size of 200µm within 14 days, periosteum derived vessels do not give rise to spheres (a).). RT-PCR analyses of single cell and heterogenous culture derived spheres shows the expression of transcripts of Smooth muscle actin (SMA), Oct 4, Nanog, C-myc, Klf4, Sox2, Collagen type 1 (Col1), Insulin (Ins1), Galactosyl ceramidase (GalC only in single cell derived spheres) and Nestin, at passage 0, at passage 15 the spheres express Oct-4, c.myc, Insulin and Nestin. **(B).** Addition of FGF and EGF to the culture medium increase sphere size, the lack of one of these factors signifacantly reduces sphere growth of p0 spheres within one week (total n=90) (**C**). B27 supplement is a crucial factor for the formation of spheres, the omittance causes cells to attach within 24h. Tendon mircovessel cells cultured on Matrigel in the presence of DMEM-KO supplemented with bFGF and ß-Mercaptoethnol give rise to small cell aggregates (arrow) and attached cells expressing DCX (**D**). Scale bars: 200µm
**Figure 3****. Cells within tenospheres express pluripotent marker genes.**
   Immunohistochemistry on 7µm cryostat sections of p0 spheres after 2 weeks in culture derived from a biceps tendon specimen of a 70 year old female donor shows the expression of Oct-4 and Nanog (**A**), CD133 (**B**), smooth muscle actin (SMA) (**C**), Insulin (**D**), glial fibrilary acidic protein (GFAP) (**E**), Nestin (**F**) and Galactosylceramidase (Galc) (**G**). Scale bar: 50µm
**Figure 4****. Differentiation of pluripotent cells in the presence of BMP.**
   In the presence of 10 ng/ml BMP-2 and BMP-4 cells derived from spheres (p3) differentiate into osteoblasts (brown colour by alkaline phosphatase staining, black arrows) and into adipocytes (Orange colour by Red Oil O staining, white arrows) (**A**). Immunocytochemistry using an antibody against glial fibrillary acidic protein indicates differentiation into glial cells induced by the same treatment (**B**). When cultured in DMEM with 10% serum, the same cells express Collagen type 1 (**C**). The growth factors FGF and EGF are advantageous for TPC proliferation, withdrawal causes a stop of proliferation within one week (**E**). Scale bar: 50µm
**Figure 5****. Differentiation of pluripotent cells.**
   TPCs cultured in DMEM +10% FBS respond to adipogenic stimuli (**A**, visualized by Red oil O staining) as well as to osteogenic (**B**, Alkaline Phosphatase, **C** mineralization stained with Alizarin S). No response to chondrogenic stimuli could be detected (D). Cells referred to as "controls" were cultured in DMEM+10% FBS without differentiation factors. Scale bar: 50µm
**Figure 6****. TPCs grown as spheres (tenospheres) and single cells.**
   The upper panel of Figure 6 shows TPCs grown under proliferative conditions as tenospheres. The lower panel of Figure 6 shows dissociated tenospheres displaying singe TPC morphology.
**Figure 7****. TPCs marker expression profile.**
   The upper panel of Figure 7 shows 04-expressing TPCs. The lower panel of Figure 7 shows a quantification of TPCs neural stem/progenitor markers expression (Nestin as neural stem cell marker, GFAP as astrocyte marker, 04 as OPCs marker and DCX as neuronal progenitor marker).
**Figure 8****. TPCs intrinsic fate analysis.**
   The upper panel of Figure 8 shows MBP-expressing TPCs. The lower panel of Figure 8 shows a quantification of TPCs neural marker expression after growth factor withdrawal (GFAP as astrocyte marker, MBP as mature oligodendrocyte marker, DCX as a neuronal progenitor marker and Map2ab as a mature neuron marker).

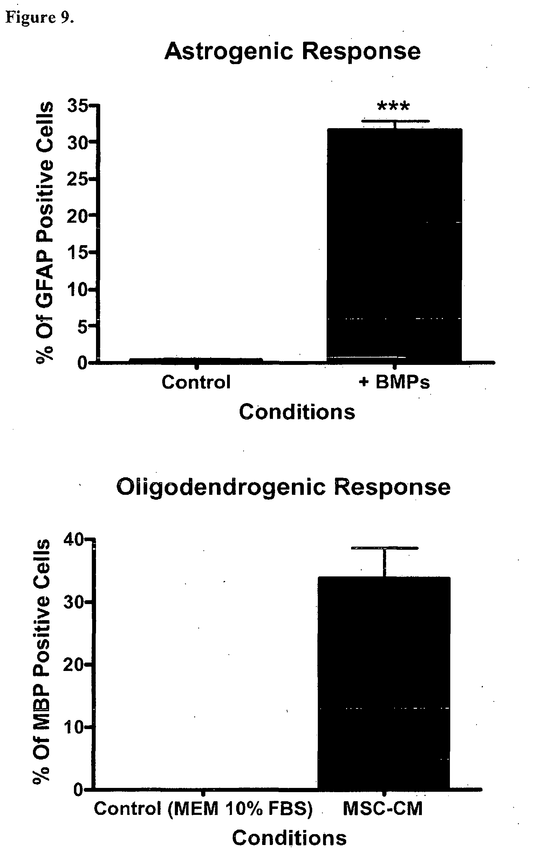

**Figure 9****. TPCs differentiation potential.**
   Figure 9 shows that OPCs derived TPCs respond to astrogenic as well as oligodendrogenic stimuli. Abbreviations: BMPs, bone morphogenetic protein 2 and 4; MSC-CM, mesenchymal stem cell conditioned media; FBS, fetal bovine serum.
**Figure 10****. Cultivation of human buffy-coats does not give rise to spheres.**
   A human Buffy coat cultured in Neurobasal medium supplemented with B27, FGF and EGF does not contain spheres after 2 weeks, only few groups of three cells or less (arrowhead)(A). Human biceps tendon cells cultured in the same medium form spheres within 2 weeks (B).

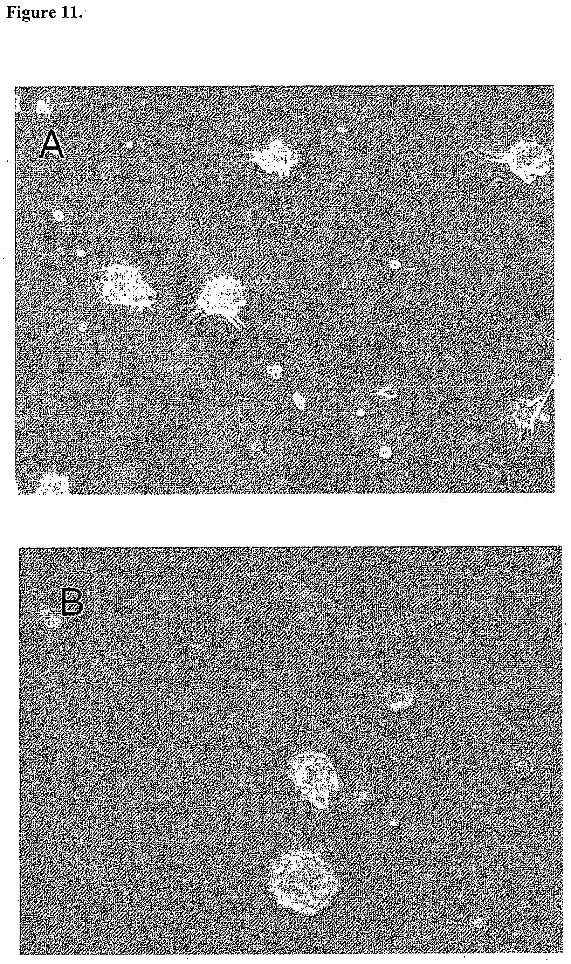

**Figure 11****. Digestion of a tendon sample in the presence of serum improves formation of tenospheres.**
   Spheres derived from a tendon sample that was digested without serum are smaller ad adhere to the plate (A), whereas spheres derived from a tendon sample that was digested with serum are free floating (B).
**Figure 12****. Generation of tenospheres after cultivation of a ligament sample.**
   Anterior cruciate ligament vascular/ perivascular cells form spheres within 2 weeks in culture (A). RT-PCR analysis of single spheres reveal expression of stem cell related markers as well as various differentiation related markers.
**Figure 13****. Pluripotent cells recovered from tenospheres do not induce formation of a tumor in an animal model.**
   Injection of 3.106 human tenosphere derived cells into immunodeficient mice (n=5) does not cause tumor formation after 8 weeks, whereas the same number of NTERA-2 cells lead to formation of a local tumor (black arrowhead).

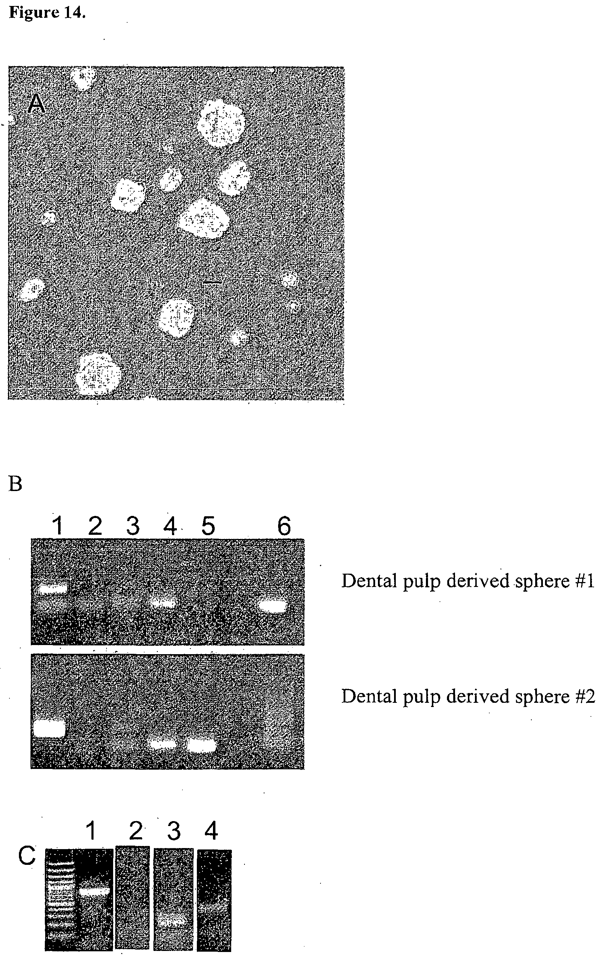

**Figure 14****. Spheres derived from dental pulp.**
   Human dental pulp derived cells form spheres after 2 weeks in culture (A). RT-PCR profile of 2 isolated human dental pulp derived spheres (p0, after 2 weeks in culture) in respect of expression of GAPDH (1), Oct 4 (2), nanog (3), c-myc (4), Sox2 (5) and insulin (6) (from left to right) showing one sphere expressing Nanog, SOX2 and Insulin related mRNA (#1) and one sphere expressing only Nanog and Insulin mRNA (#2). RNA anaysis of human dental pulp in respect of expression of GAPDH (1), Nestin (2), SMA (3) and Oct 4 (4) (from left to right) reveals expression of the stem cell markers Nestin and OCT4 and smooth muscle actin (SMA) (scale bar: 30µm).

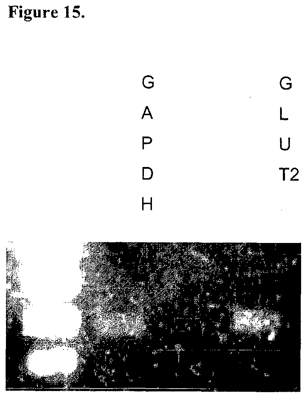

**Figure 15****. Glut2 expression in a tendon sample.**
   RT-PCR analysis of a female Lewis rat achilles tendon showing expression of GLUT2.

The present invention is additionally described by way of the following illustrative non-limiting examples that provide a better understanding of the present invention and of its many advantages.
Unless otherwise indicated, established methods of recombinant gene technology were used as described, for example, in Sambrook, Russell "Molecular Cloning, A Laboratory Manual", Cold Spring Harbor Laboratory, N.Y. (2001).

The Examples illustrate the invention.

### Example 1. Generation of tendon perivascular cell derived tenospheres comprising pluripotent cells

Herein below a detailed molecular and cellular characterization of human tendon perivascular cells (TPCs) as well as an anylsis of their fate and potential after isolation from their natural tissue environment is presented. As discussed above,it was surprisingly found that tendon perivascular cells are a good candidate for a cell type encompassed with pluripotency without the need of genetic or epigenetic manipulations. In particular, it was unexpectedly found that tendon perivascular cells express embryonic/pluripotent marker genes and give, after isolation from the natural environment and under appropriate culture conditions rise to pluripotent cells and tenospheres comprising such cells.

### 1. Maternal and Methods

### Tissue sampling and cell culture

Tissue samples from 5 human biceps tendons (donors: 3 males aged 43, 66 and 71; and 2 females aged 65 and 71) were obtained during surgical interventions with patients' informed consent. Fresh tendon tissues were cut into small pieces (ca. 1mm3) and digested over night in DMEM containing 1.5U/ml Collagenase type 2 at 37°C in a humidified atmosphere with 5% CO2. This digest resulted in a suspension containing single cells and vessel fragments. The latter were manually isolated and further digested with 2U/ml Accutasetm in DMEM for 15 min at RT [8]. The obtained suspension of single vascular and perivascular cells was highly diluted in cold PBS, allowing manual isolation of single cells using a pipette. Single cells were either immediately analyzed by single cell PCR [22] or cultivated in 96 well plates (1 cell/well) in DMEM containing B27 supplement, 20ng/ml FGF and 20ng/ml EGF. To see whether single TPCs are capable of forming spheres, we isolated 100 single TPCs by micropipette suction, and transferred the cells to a microtiterplate (1 cell/well). The spheres formed by 3 of 100 plated cells were either directly analyzed by IHC or PCR, or were passaged to examine phenotypic changes. The generation of single cell derived spheres following micropipette suction and the analysis of single cells are elaborate and serve only as a tool for identification and characterization of multipotent cells and their differentiation potential. For generating a large number of spheres for passaging, differentiation experiments or implantation, whole tendon collagenase digests were cultured in DMEM containing B27 supplement, 20ng/ml FGF and 20ng/ml EGF without prior isolation of vessels. Spheres generated this way will be referred to as "heterogenous culture derived spheres". For examining the reaction of TPCs on culture under es-cell conditions, cells from isolated tendon microvessels were plated on dishes coated with matrigel in DMEM-Serum Knockout supplemented with 8ng FGF/ml and 0.1mM ß-mercaptoethanol [12,13].

### Differentiation experiments

Heterogeneous culture derived spheres were removed from the medium using a cell strainer with 70µm pore size followed by a 10' digest with Accutasetm. The resulting sphere derived single cell suspension was cultivated for 7 days on uncoated cell culture dishes for RT-PCR analysis, and on glass cover slips for immunocytochemistry.

To assess the astrogenic potential and the response to bone morphogenetic proteins (BMP), spheres were dissociated and 2.5 x 104 (i.e. 2.5 x 10⁴)cells/well were plated on 100 µg/ml poly-L-ornithine (Sigma-Aldrich, Taufkirchen,Germany) and 5 µg/ml laminin-coated (Sigma-Aldrich, Taufkirchen, Germany) glass coverslips. The coverslips were placed in 24-well plates and cells were grown in DMEM Knockout-20% serum replacement (SR) (Gibco Invitrogen, Karlsruhe, Germany). After 12 hours, the medium was changed to DMEM Knockout- 20% SR supplemented with 10 ng/ml BMP-2 and BMP-4 (R&D Systems GmbH, Wiesbaden-Nordenstadt, Germany). After 7 days, the cells were fixed for 30 minutes with 4% paraformaldehyde and processed for immunofluorescence staining. Following BMP treatment, TPCs were tested for their mesenchymal differentiation potential using Alkaline phosphatase and red oil O staining.

The formation of MSC like cells was induced by cultivating dissociated spheres (5x104 cells/well, i.e. 5 x 10⁴)) in DMEM supplemented with 10% FBS for 2 weeks.

Osteogenic differentiation was observed following 4 weeks of culture in αMEM-10%FBS supplemented with 0.1 µM dexamethasone, 10 mM β-glycerophosphate, and 50 µM L-ascorbic acid-2-phosphate (all from Sigma, Germany). Osteogenic differentiation was revealed by deposits in the extracellular matrix of mineralized hydroxyapatite, the latter was further stained with 40 mM Alizarin Red S (Sigma, Germany). MSC adipogenic differentiation was induced with αMEM-10%FBS supplemented with 1 µM dexamethasone, 10 µg/ml insulin (Sigma, Germany), 0.5 mM 3-isobutyl-1-methylxanthine (Calbiochem, Germany) and 100 µM indomethacin (Sigma, Germany). After one week of culture in these conditions, adipogenic differentiation was confirmed by staining the cytoplasmic lipid droplets with saturated Oil Red O (Merck, Germany)

### Immunocytochemistry

1 week old heterogeneous culture derived spheres were fixed in 4%PFA washed in PBS and processed either for paraffin- or cryosectioning. Immunohistochemical stainings were performed using standard protocols with minor modifications [8]. Type and dilution of antibodies used are listed in Table 1.

For doubleimmunolabeling on paraffin sections we used the MultiVision Polymer Detection System: MultiVision anti-rabbit/HRP + anti-mouse/AP polymers (Thermo Fisher Scientific, Fremont, USA) according to the manufacturer's instructions.

### In vivo and in vitro assessment of tumorgenic potential

Heterogenous culture derived spheres were genererated from female fisher rat Achilles tendons (n=3) as described above and passaged 3 times. Passage 3 spheres were digested with Accutase in order to obtain a single cell suspension. 106 cells were injected subcutaneously to 3 female fisher rats. 6 weeks after injection, the rats were sacrificed and examined for teratoma formation.

For the assessment of the role of medium growth factors for TPC proliferation, heterogenous culture derived spheres (passage 3) were dissociated and plated at a density of 5.103 cells/ml. The cells were cultured in NeurobasalTM either supplemented with B27 alone or supplemented with B27, FGF and EGF, or without any supplement. After 1 week, the number of viable cells was determined.

### Karyotyping

Heterogenous culture derived spheres at passage 3 were treated with 10 µg/mL of Colcemid for 3 hours, diccociated with Accutasetm, treated briefly with 0.075 M hypotonic KCl solution, and fixed with Methanol: glacial acetic acid 3:1 over night at 4°C. Cells were then dropped on a microscope glass slide and dried. Metaphase cells were stained with Giemsa dye. At least 20 metaphases per sample were karyotyped.

### Statistical analysis

In all graphs, means and standard deviations are given. Student's t-test was used to test for effects of FGF and EGF on sphere growth treatment, and the significance level was set at p = 0.05.

### 2. Results

### Tendon perivascular cells express ESC specific markers

The immunohistological characterisation of human biceps tendons revealed that TPCs are immunoreactive for Oct-4, Nanog, Sox-2 and, c-myc (Fig. 1A-E)). Oct-4 positive TPCs co-localized with SMA suggesting a pericyte identity of the cells (Fig. 1 D). Unfortunately, the high collagen content associated autofluorescence of the tendon tissue did not allow a detailed immunofluorescence confocal analysis of marker co-localization. As an alternative approach, we performed single cell RT-PCR on isolated TPCs. In support of co-localization of ESC markers in TPCs, a subpopulation of TPCs co-expressed mRNA encoding Oct-4, Nanog, Sox2, C-myc, Klf4, Nestin and SMA (Fig. 1G). The co-expression of ESC-associated markers in perivascular cells was specific for tendon tissue, since we did not detect it in perivascular cells of rat brain, human subcutaneous adipose tissue and human periosteumeum.

### TPCs generate multilineage spheroid bodies in vitro

Explanted tendon microvessels human readily disintegrated within 4 days following isolation. This was paralleled by spheroid body formation (Fig. 2A), especially when cells were grown in DMEM supplemented with B27 in presence of 40 ng/ml FGF and 20 ng/ml EGF (Figure 2A). In average, 3% of manually isolated TPCs (n=150 from 3 different donors generated clonal spheres within 10 days. Active proliferation of cells in spheres was demonstrated by quantitative Ki67-immunoreactivity in passage three spheres, which demonstrated that 56 ± 26% of the cells were ki67 positive indicating that these cells were in the cell cycle. Perivascular cells derived from periosteumeum or adipose tissue did not give rise to spheres despite the fact that they were cultured under the exact same condition. RT-PCR analyses of single TPC- derived primary spheres indicated the expression of ESC associated markers Oct-4, Nanog, Sox2, Klf4 and c-myc (Fig 2B),. In addition, the TPC derived spheres expressed the neuroectodermal genes GFAP, Nestin and GalC, the mesenchymal genes Collagen type I and smooth muscle actin, as well as the endodermal gene insulin, indicating the pluripotency of the TPCs. (Fig. 2B). TPC-derived spheres expressed the same of genes tested regardless of whether they were generated under clonal or non-clonal conditions. (Fig. 2B). The pluripotency of TPC derived spheres was further demonstrated by immunocytochemistry. All spheres tested (N = 31) contained cells expressing the ESC markers Oct-4, Nanog, and Sox2, the neuroectodermal markers Nestin, GFAP, and GalC, and the mesenchymal marker SMA. (Fig. 3, A-F). 21 out of 31 spheres analyzed contained cells immunoreactive for insulin, and 16 out of 31 spheres harboured CD133 expressing cells. Upon cultivation with DMEM supplemented with 10% FBS, the cells adhered and aquired MSCs like phenotype. Over 95% of all cells responded to adipogenic and osteogenic stimuli (Fig 5).

11%±7 of the sphere derived TPCs were found to express GFAP after incubation with BMP2 and BMP4, and to also differentiate into adipocytes and osteoblasts under these conditions (Figure 4a and b).

Both FGF and EGF appear to independently increase sphere size (Fig. 2C) Nanog and oct-4 expression depend on the presence of FGF.

The sphere forming capacity of TPC seems to critically depend on the presence of B27 supplement. Absence of B27 supplement from the medium caused the primary cells to attach to the plastic surface and inhibited sphere formation (Fig. 2D). Moreover, and surprisingly, TPCs did not generate floating spheres when grown in typical ESC media such as DMEM serum knockout, Under such conditions, tendon microvessel associated cells readily adhere to the plastic dish and express the neural markers doublecortin and GFAP one week after seeding. Some of the tendon microvessel derived cells clustered in small aggregates, however, they newever formed floating spheres (Fig 2D). RT-PCR analysis of single aggregates revealed expression of c-myc in all aggregates analyzed (N = 10), while Sox2 expression was found in one out of 10 aggregates analyzed (data not shown). Neither expression of the stem cell markers Oct-4, Nanog and KLF4, nor of the above mentioned lineage markers could be detected.

Upon growth factor withdrawal in vitro, TPCs react by cessation of proliferation (Fig 4D).

### TPCs do not form tumors following implantation

All described experiments were also performed with tendons from rat and mouse, yielding similar results (data not shown).
Following subcutaneous implantation of rat TPCs, no tumors were found 6 weeks after implantation.These experiments were confirmed in nude athymic mice (N=3) with human cells; also here no tumours were found.
Injection of 3.106 human tenosphere derived cells into immunodeficient mice (n=5) does not cause tumor formation after 8 weeks, whereas the same number of NTERA-2 cells lead to formation of a local tumor (black arrowhead); see **Figure 13****.**

To further support these observations, chromosome stability of TPCs at passage 3 was determined (n=3). The results showed a normal karyotype of 46 XY chromosomes without any numerical and/or structural abnormalities.

### Example 2. Generation of glial progenitor / precursor cells (GPCs) from adult non-nervous system derived tissue (tendon perivascular cells)

### 1. Tissue sampling and cell culture for the induction of tendon derived OPCs (oligodendritic precursor cells)

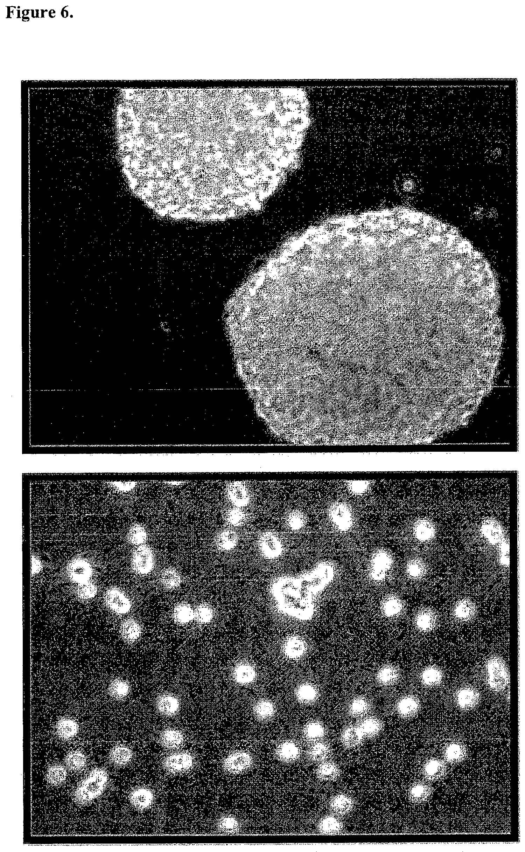

Human biceps tendon biopsies (>100 µg) are obtained during surgical interventions on the rotator cuff. Fresh tendon biopsies are cut into small pieces (ca. 1 mm³) and digested overnight in Neurobasal (NB) media containing 15mg/ml Collagenase type 2 at 37°C in a humidified atmosphere with 5% CO₂. This digest results in a suspension of single cells and vessel fragments. The obtained suspension of single vascular and tendon perivascular cells (TPCs) is thereafter cultivated in T-75 flask (75 cm²) containing NB media (Gibco BRL, Germany) supplemented with B27 (Gibco BRL, Germany) (NB/B27), 2 mM L-glutamine (PAN, Germany), 100U/mL penicillin/ 100 µg/ml streptomycin (PAN, Germany), 2 µg/ml heparin (Sigma, Germany), human recombinant 20 ng/ml bFGF-2 (R&D Systems GmbH, Wiesbaden-Nordenstadt, Germany) and human recombinant 20 ng/ml EGF (R&D Systems GmbH, Wiesbaden-Nordenstadt, Germany). Media change is performed every two weeks. After 4 to 8 weeks in culture, the cells generate floating spheres with a diameter up to 200 µm (tenospheres) (**Figure** 6 upper panel). More frequent change of media leads to a different phenotype of adherent, spindle shaped cells of so far unknown type. The phenotypic and biological characteristics of tenospheres generated in accordance with the present invention have been described in detail in **Example 1.** These explanations apply similarly to the tenospheres obtained in **Example 2.**

### 2. Induction of OPC phenotype from sphere forming TPCs

### 1. Cell-lineage marker expression profile analysis

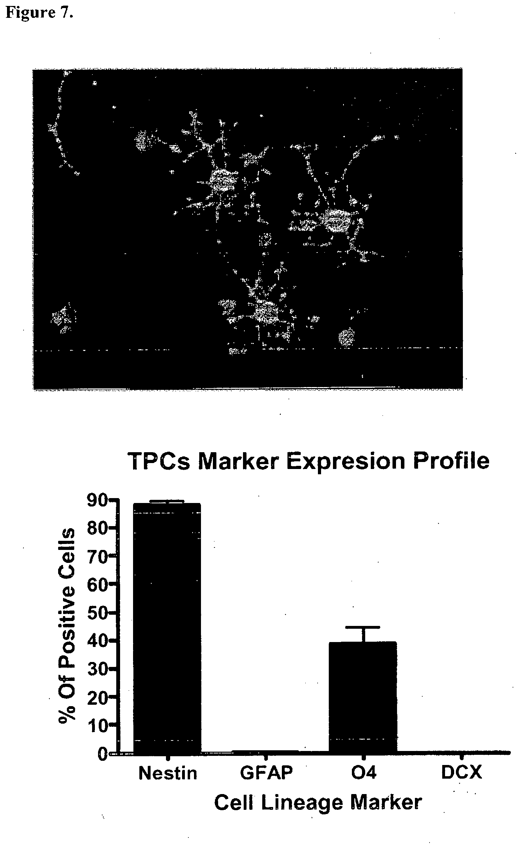

TPCs derived tenospheres (passage 3 and higher) were dissociated with Accutase (Innovative Cell Technologies Inc., distributed by PAA) and plated on 100 µg/ml poly-L-ornithine (Sigma-Aldrich, Taufkirchen, Germany) and 5 µg/ml laminin-coated (Sigma-Aldrich, Taufkirchen, Germany) glass coverslips at a density of 2.5 x 10⁴ cells/cm² in DMEM Knockout-20% Serum Replacement supplement (SR) (Gibco Invitrogen, Karlsruhe, Germany) for 12 hours. This serum-free media is commonly used for embryonic stem cell maintenance and does not actively induce differentiation. Under these conditions, dissociated sphere derived cells attach to the plate (**Figure 6**, lower panel) and express neural stem cell as well as OPCs markers (Nestin and 04, respectively) (**Figure 7**).

### 2. Cell intrinsic fate analysis: growth factor withdrawal and serum-free conditions response

To determine the cell intrinsic fate of TPCs, growth factor withdrawal (GFW) response was analyzed. Proliferating TSph in the presence of FGF-2 and EGF were dissociated with Accutase and plated as described in the previous section. Cells were incubated in DMEM Knockout-20% SR (Gibco Invitrogen, Karlsruhe, Germany) without growth factors and in the absence of serum. After 7 days, cells were fixed for 30 minutes with 4% paraformaldehyde and processed for immunofluorescence to detect the expression of the different cell specific markers tested. TPCs give rise mainly to MBP-expressing oligodendrocytes rather to other neural cell types, suggesting an oligodendrogenic cell intrinsic fat (**Figure 8**).

### 3. Differentiation assay: astrogenic and oligodendrogenic response

To assess astrogenic potential of TSph, 2-5 x 10⁴ cells/well were plated on 100 µg/ml poly-L-ornithine (Sigma-Aldrich, Taufkirchen, Germany) and 5 µg/ml laminin-coated (Sigma-Aldrich, Taufkirchen, Germany) glass coverslips into 24-well plates in DMEM Knockout-20% SR (Gibco Invitrogen, Karlsruhe, Germany). After 12 hours, the media was refreshed with DMEM Knockout-20% SR, supplemented with 10 ng/ml BMP-2 and BMP-4 (R&D Systems GmbH, Wiesbaden-Nordenstadt, Germany) to evaluate astrogenic response. Finally, in order to analyze TSphs oligodendrogenic response, 2-5 x 10⁴ cells/well were plated on 100 µg/ml poly-L-ornithine (Sigma-Aldrich, Taufkirchen, Germany) and 5 µg/ml laminin-coated (Sigma-Aldrich, Taufkirchen, Germany) glass coverslips into 24-well plates in Alpha-MEM 10% fetal bovine serum (Gibco Invitrogen, Karlsruhe, Germany). After 12 hours, cells were incubated in the presence of MSC-CM. In all cases, media were refreshed every third day. After 7 days, the cells were fixed for 30 minutes with 4% paraformaldehyde and processed for immunofluorescence staining. TPCs display astrogenic as well as oligodendrogenic potentials (**Figure 9**).

### Supplementary Methodology

### MSCs culture

Bone marrow plugs were harvested from femurs and tibias of 2-4 month-old female Fisher-344 rats (Charles River Deutschland GmbH, Germany). Plugs were mechanically dissociated in Alpha-MEM (Gibco Invitrogen, Karlsruhe, Germany) and recovered by centrifugation. Cell pellets were resuspended in Alpha- MEM-10% FBS and seeded at 1x106 cells/cm2. After 3 days, media was changed and non-adherent cells were removed. Adherent cells were incubated in fresh Alpha- MEM-10% FBS until a confluent layer of cells was achieved. Cells were trypsinized using 0.25% Trypsin (Gibco Invitrogen, Karlsruhe, Germany) and seeded in Alpha-MEM-10% FBS at 8,000 cells/cm². After 3-5 days of culture, the resulting monolayer of cells, hereafter named rat bone marrow derived mesenchymal stem cells (MSCs), was trypsinized and further cultured for experiments or frozen for later use. As demonstrated in our previous work, this cell culture preparation is highly enriched in multipotent MSCs with virtually no hematopoietic contamination.

### Mesenchymal stem cell conditioned media preparation

MSCs were plated at 12,000 cells/cm² and incubated in Alpha-MEM-10% FBS. After 3 days, the conditioned medium was collected and filtered using a 0.22 µm-pore filter and used as a TSphs oligodendrogenic stimulus.

### Immunofluorescence

Fixed cells were washed in TBS (0.15 M NaCl, 0.1 M Tris-HCl, pH 7.5), then blocked with solution composed of TBS; 0.1% Triton-X100 (only for intracellular antigens); 1% bovine serum albumin (BSA) and 0.2% Teleostean gelatin (Sigma, Germany) (fish gelatin buffer, FGB). The same solution was used during the incubations with antibodies. Primary antibodies were applied overnight at 4°C. Fluorochrome-conjugated species-specific secondary antibodies were used for immunodetection. The following antibodies and final dilutions were used. Primary antibodies: rabbit anti-GFAP 1:1000 (Dako, Denmark); mouse anti-ratNestin 1:500 (Pharmingen, Heidelberg, Germany); rabbit anti-NG2 proteoglican (Millipore); IgM mouse anti-O4 1:100 (Chemicon, USA); mouse anti-Map 2a+2b 1:250 (Sigma, Germany); rabbit anti-doublecortin (DCX) 1:500 (NEB, Frankfurt, Germany); mouse anti-Myelin Basic Protein (MBP) 1:750 (SMI-94, Sternberger Monoclonals Incorporated, U.S.A.). Secondary antibodies: donkey anti-mouse, rabbit conjugated with Alexa Fluor 488 (Molecular Probes, Eugene, OR, USA), rhodamine X 1:1000 (Dianova, Hamburg, Germany). In cases of detergent-sensitive antigens (i.e. 04, NG2), Triton X-100 was omitted from FGB buffer. Nuclear counterstaining was performed with 4', 6'-diamidino-2-phenylindole dihydrochloride hydrate at 0.25 µg/µl (DAPI; Sigma, Germany). Specimens were mounted on microscope slides using in Prolong Antifade kit (Molecular Probes, U.S.A.). Epifluorescence observation and photo-documentation were realized using a Olympus microscope equipped with a digital camera. For each culture condition, 5 randomly selected observation fields, containing in total 300-500 cells, were photographed for cell fate analysis. Expression frequency of selected cell type markers was determined.

### Example 3. Cultivation of human Buffy-coats does not give rise to spheres.

Human Buffy coats were isolated as follows: from 5 heathy human volunteers (3 males aged 25, 36 and 63; 2 females aged 37 and 52) 9ml venous blood were obtained and stored in a K-EDTA vial (S-Monovette, #046379, Sarstedt, Germany). The blood was then centrifuged at 3200g for 10'at 4°C, leading to a seperation of the blood in a plasma phase, the buffy coat and an erythrocyte phase. After removal of the plasma phase the Buffy coat (0.5ml) was transferred to a culture dish containing 12ml Neurobasal medium supplemented with B27, FGF and EGF and cultured for 2 weeks. After 2 weeks, mainly single cells were observed, less than 1% of the cells were in groups of 2 or 3 cells. Bigger cell aggregates were not observed; see **Figure 10****.**

### Example 4. The digestion of a tendon sample with medium containing serum is advantageous.

In order to determine the role of serum added to the digestion medium, a human biceps tendon sample was divided in 2 equal parts of which one was digested in DMEM containing 1.5 U/ml Collagenase, the other in DMEM containing 1.5 U/ml Collagenase and 10% fetal calf serum for 12h. The cells of both samples were further cultivated in Neurobasal medium containing B27-supplement, 20ng/ml FGF and 20ng/ml EGF. After 2 weeks, the developed spheres were analyzed. Spheres from the serum free digest were smaller and adhered to the surface of the plate (Figure 11). Other than the free floating spheres from the serum containing digest, they did not have the capacity to give rise to OPCs (oligodendritic precursor cells) within 3 passages.

### Example 5. Generation of tenospheres and pluripotent cells from a ligament sample.

A sample of an anterior cruciate ligament was obtained from a 54 year old male donor. The sample had a size of 0.25cm³ and was treated like the tendon samples in **Example 1.** Briefly, the tissue was cut into small pieces and digested in DMEM containing 10% fetal bovine serum and 1.5 U/ml Collagenase for 12h. Vessel fragments were manually isolated from the resulting suspension of singe cells and vessel fragments and cultivated in Neurobasal medium containing B27- supplement, 20ng/ml FGF and 20ng/ml EGF. After 2 weeks, single spheres (see **Figure 12** **A**) were manually isolated and analyzed by RT-PCR. RT-PCR analysis revealed that marker expression of ligament derived tenospheres was similar to tendon derived tenospheres; see **Figure 12B**

### Example 6. Spheres can be obtained from dental pulp.

Two3^{rd} molar teeth were obtained from a healthy male donor with informed consent. The pulps were isolated by mechanical dissociation/ destruction of adamantine and dentine. The pulps were then transferred to a cell culture dish containing DMEM supplemented with 10% FBS and 1.5 U/ml Collagenase and digested for 12h at 37°C, 95% humidity and 5%CO2. This digest lead to a suspension containing single cells and vessel fragments. The cells and the vessels were the transferred to a cell culture dish containing 12ml Neurobasal medium, supplemented with B27 supplement, 20ng/ml FGF and 20ng/ml EGF and cultured for 2 weeks. After that period of time, spheres with a diameter of 75µm ±28µm were observed. These sphere were analyzed by RT-PCR, revealing expression of Nanog, SOX2 and Insulin; see **Figure 14****.**

### Example 7. Protocol for isolation of Glut2+ pluripotent cells or Glut2+ perivascular tendon cells by MACS-sorting

We observe a population of cells positive for Oct4, Nanog, Sox2, Cmyc and Glut2 in both tendons and tenospheres; see **Figure 15****.** Glut2 is a glucose transporter molecule, responsible for glucose sensing in pancreatic b-cells (e.g. Thorens B. (2001) GLUT2 in pancreatic and extra-pancreatic gluco-detection. Mol Membr Biol. 2001 Oct-Dec; 18(4):265-73.).

The expression of the cell surface protein Glut2 allows isolation of cells expressing Glut2 from cells lacking this protein by either FACS- or MACS- sorting.

For isolating a population of cells expressing Glut2 from human tendon, a solution of tendon perivascular cells needs to be prepared as described in **Example 1.** The cells are then incubated with an antibody directed against Glut2 (for example: Glut2- antibody, Chemicon, #AB1342), cells binding this antibody can then be separated by fluorescence-activated cell sorting or magnetic activated cell sorting (MACS). The Glut2-positive cells can, for example, be purified by incubation with magnetic Goat Anti-Rabbit IgG MicroBeads and selection on a Mini-MACS apparatus (Miltenyi Biotec, Bergisch-Gladbach, Germany) following the protocol recommended by the manufacturer.

### Example 8. Differentiation of pluripotent Oct4 positive cells isolated from a tenosphere

### Methods

Tenospheres were generated from 10 female Oct4-GFP mice (Jackson Laboratory # B6;12954-Pou5fltm2Jae/J) as described in Example 1. In Example 8 example mouse tissue is used instead of human. Therefore, the starting material are 20 achilles tendons isolated fom freshly killed mice (viable cells can be obtained up to 48h post mortem). The digestion is performed as described in Example 1. The spheres were cultivated for one week in Neurobasal A, supplemented with B27 supplement, 20ng/ml FGF and 20ng/ml EGF at 37°C and 5%CO2.

The spheres were then dissociated in Accutase™ for 15 min at 37°C. The resulting suspension of single cells and cell aggregates was filtered with a cell strainer (100µm) and sorted with a FACS-sorter (Becton Dickinson, BD FACSAria III), isolating GFP- positive cells. gfp is expressed, if the Oct4 promoter is active. All cells having an active Oct4 promoter produce GFP; however, GFP is not directly fused to the Oct4 protein. In single cell PCT both the mRNA of gfp and the mRNA of oct4 was detected. The model may therefore be adressed as a "Oct4 reporter animal" model.

The GFP- positive fraction was divided and either subcultured in unsupplemented DMEM- KO medium on uncoated plastic for 2 days or cultured in MEM supplemented with 10% FBS for 2 weeks.

The cells cultured in DMEM-KO were fixed in 4% PFA and stained with antibody specific for the neuronal markers ß-III Tubulin and GAP43, for the astrocyte specific marker GFAP and for the oligodendrocyte markers 04 and Galactosylcerebroside (GalC).

The cells cultured in MEM+10% FBS were differentiated to adipocytes and osteoblasts as follows. The Oct4+ cell obtained by FACS sorting were immediately transferred to 6 well plates at a density of 10³ cells/ml and cultured in MEM supplemented with 10% FBS and cultured for 2 weeks. The cell were then cultivated in αMEM-10%FBS supplemented with 1 µM dexamethasone, 10 µg/ml insulin (Sigma, Germany), 0.5 mM 3-isobutyl-1-methylxanthine (Calbiochem, Germany) and 100 µM indomethacin (Sigma, Germany) for 3 weeks to obtain adipocyte differentiation, or in αMEM-10%FBS supplemented with 0.1 µM dexamethasone, 10 mM β-glycerophosphate, and 50 µM L-ascorbic acid-2-phosphate (all from Sigma, Germany) to obtain osteoblasts. Adipocyte differentiation was visualized by red oil o- staining, osteoblasts by Alizarin S.

### Results

*Portion of Oct4- positive cells* The described method yields a total number of 207333 ±6429 cells, of which 77.3% ±17.8% are considered as Oct4 positive.
*Differentiation capacity* The cells cultured in DMEM KO spontaneously differentiate to Neuron-like cells positive for both ß-III tubulin and GAP 43 (23%), to oligodendrocyte like cells positive for 04 and GalC (13%) and to astrocyte like cells positive for GFAP (17%).
The cells cultured in MEM+10%FBS could be differentiated to Adipocytes (>35%) and osteoblasts (>50%).

The present invention refers to the following nucleotide and amino acid sequences:
The sequences provided herein are available in the NCBI database and can be retrieved from www.ncbi.nlm.nih.gov/sites/entrez?db=gene; Theses sequences also relate to annotated and modified sequences. The present invention also provides techniques and methods wherein homologous sequences, and variants of the concise sequences provided herein are used.

SEQ ID No. 49:
   Nucleotide sequence encoding Homo sapiens Nestin (NM_006617)
   The coding region ranges from nucleotide 134 to nucleotide 4999.
SEQ ID No. 50:
   Amino acid sequence of Homo sapiens Nestin (NP_006608.1)
SEQ ID No. 51:
   Nucleotide sequence encoding Homo sapiens Col1A1 (NM_000088)
   The coding region ranges from nucleotide 126 to nucleotide 4522.
SEQ ID No. 52:
   Amino acid sequence of Homo sapiens Col1A1 (NP_000079.2)
SEQ ID no. 53:
   Nucleotide sequence encoding Homo sapiens Col3A1 (NM_000090)
   The coding region ranges from nucleotide 117 to nucleotide 4518.
SEQ ID No. 54:
   Amino acid sequence of Homo sapiens Col3A1 (NP_000081.2)
SEQ ID No. 55:
   Nucleotide sequence of Homo sapiens Oct4/POU5F1 (NM_002701)
   The coding region ranges from nucleotide 55 to nucleotide 1137.
SEQ ID No. 56:
   Amino acid sequence of Homo sapiens Oct4/POU5F1 (NP_002692.2)
SEQ ID No. 57:
   Nucleotide sequence encoding Homo sapiens Galc (NM_000153)
   The coding region ranges from nucleotide 107 to nucleotide 2390.
SEQ ID No. 58:
   Amino acid sequence of Homo sapiens Galc (NM_000144.2)
SEQ ID No. 59:
   Nucleotide sequence encoding for Homo sapiens Scleraxis A (NM_001008271.1) and Scleraxis B (NM_001080515)
   The coding region ranges from nucleotide 1 to 606.
SEQ ID No. 60:
   Amino acid sequence for Homo sapiens Scleraxis A (NP_001008272.1) and B (NP_001073983.1)
SEQ ID No. 61:
   Nucleotide sequence encoding Homo sapiens Sox-9 (NM_000346)
   The coding region ranges from nucleotide 372 to 1902.
SEQ ID No. 62:
   Amino acid sequence for Homo sapiens Sox-9 (NP_000337.1)
SEQ ID No. 63: Nucleotide sequence encoding Homo sapiens c-Myc (NM_002467) The coding region ranges from nucleotide 526 to nucleotide 1890.
SEQ No. 64:
   Amino acid sequence of Homo sapiens c-Myc (NP_002458.2)
SEQ ID No. 65:
   Nucleotide sequence encoding Homo sapiens Klf-4 (NM_004235)
   The coding region ranges from nucleotide 595 to nucleotide 2034.
SEQ ID No. 66:
   Amino acid sequence of Homo sapiens Klf-4 (NP_004226.3)
SEQ ID No. 67: Nucleotide sequence encoding Homo sapiens Sox-2 (NM_003106) The coding region ranges from nucleotide 438 to nucleotide 1391.
SEQ ID No. 68:
   Amino acid sequence of Homo sapiens Sox-2 (NP_003097.1)
SEQ ID No. 69:
   Nucleotide sequence encoding Homo sapiens NANOG (NM_024865)
   The coding region ranges from nucleotide 216 to nucleotide 1134.
SEQ ID No. 70:
   Amino acid sequence for Homo sapiens NANOG (NP_079141.2)
SEQ ID No. 71:
   The nucleotide sequence encoding Homo sapiens Insulin (NM_000207)
   The coding sequence ranges from nucleotide 60 to nucleotide 392.
SEQ ID No. 72:
   Amino acid sequence for Homo sapiens Insulin
SEQ ID No. 73:
   Nucleotide sequence encodes for Homo sapiens GAPDH (NM_002046)
   The coding region ranges from nucleotide 102 to nucleotide 1110.
SEQ ID No. 74:
   Amino acid sequence for Homo sapiens GAPDH (NP_002037.2)
SEQ ID No. 75:
   The nucleotide sequence encoding Rattus norvegicus Nestin (NM_012987)
   The coding region ranges from nucleotide 128 to nucleotide 5545.
SEQ ID No. 76:
   Amino acid sequence for Rattus norvegicus Nestin (NP_037119.1)
SEQ ID No. 77:
   The nucleotide sequence encoding Rattus norvegicus Col1A1 (NM_053304)
   The coding region ranges from nucleotide 112 to nucleotide 4473.
SEQ ID No. 78:
   Amino acid sequence of Rattus norvegicus Col1A1 (NP_445756.1)
SEQ ID No. 79:
   Nucleotide sequence encoding Rattus norvegicus Col3A1 (NM_032085)
   The coding region ranges from nucleotide 104 to nucleotide 4496.
SEQ ID No. 80:
   Amino acid sequence for Rattus norvegicus Col3A1 (NP_114447.1)
SEQ ID No. 81:
   Nucleotide sequence encoding Rattus norvegicus Oct-4/POU5F1 (NM_001009178)
   The coding region ranges from nucleotide 81 to nucleotide 1139.
SEQ ID No. 82:
   Amino acid sequence of Rattus norvegicus Oct-4 (NP_001009178.1)
SEQ ID No. 83:
   Nucleotide sequence encoding Rattus norvegicus Galc (NM_001005888)
   The coding region ranges from nucleotide 1 to nucleotide 2053.
SEQ ID No. 84:
   Amino acid sequence for Rattus norvegicus Galc (NP_001005888.1)
SEQ ID No. 85:
   Nucleotide sequences encoding Rattus norvegicus Scleraxis (NM_001130508)
   The coding region ranges from nucleotide 166 to nucleotide 795.
SEQ ID No. 86:
   Amino acid sequence of Rattus norvegicus Scleraxis (NP_00123980.1)
SEQ ID No. 87:
   Nucleotide sequence encoding Rattus norvegicus Sox-9 (XM_343981)
   The coding region ranges from nucleotide 1 to nucleotide 885.
SEQ ID No. 88:
   Amino acid sequence for Rattus norvegicus Sox-9 (XP_343982.2)
SEQ ID No. 89:
   Nucleotide sequence encoding Rattus norvegicus c-Myc (NM_012603)
   The coding region ranges from nucleotide 537 to nucleotide 1898.
SEQ ID No. 90:
   Amino acid sequence for Rattus norvegicus c-Myc (NP_036735.2)
SEQ ID No. 91:
   Nucleotide sequence encoding Rattus norvegicus Klf-4 (NM_053713)
   The coding region ranges from nucleotide 77 to nucleotide 1525.
SEQ ID No. 92:
   Amino acid sequence for Rattus norvegicus Klf-4 (NP_446165.1)
SEQ ID No. 93:
   Nucleotide sequence encoding Rattus norvegicus Sox-2 (NM_001109181)
   The coding region ranges from nucleotide 364 to nucleotide 1323.
SEQ ID No. 94:
   Amino acid sequence Rattus norvegicus Sox-2 (NP_001102651.1)
SEQ ID No. 95:
   Nucleotide sequence encoding for Rattus norvegicus NANOG (NM_001100781)
   The coding region ranges from nucleotide 204 to nucleotide 1142.
SEQ ID No. 96:
   Amino acid sequence for Rattus norvegicus NANOG (NP_001094251.1)
SEQ ID No. 97:
   Nucleotide sequence encoding Rattus norvegicus Insulin (NM_019129.2)
   The coding region ranges from nucleotide 58 to nucleotide 390.
SEQ ID No. 98:
   Amino acid sequence for Rattus norvegicus Insulin (NP_062002.1)
SEQ ID No. 99:
   Nucleotide sequence encoding for Rattus norvegicus GAPDH (NM_017008)
   The coding region ranges from nucleotide 77 to nucleotide 1078.
SEQ ID No. 100:
   Amino acid sequence for Rattus norvegicus GAPDH (NP_58704.1)
SEQ ID No. 101:
   Nucleotide sequence encoding for Homo sapiens Glut-2 (SLC2A2) (NM_000340.1)
   The coding region ranges from nucleotide 1 to nucleotide 1575.
SEQ ID No. 102:
   Amino acid sequence of Homo sapiens Glut-2 (SLC2A2) (NP_000331.1)
SEQ ID No. 103:
   Nucleotide sequence encoding for Rattus norvegicus Glut-2 (SLC2A2) (NM_012879)
   The coding region ranges from nucleotide 1 to nucleotide 1569.
SEQ ID No. 104:
   Amino acid sequence of Rattus norvegicus (SLC2A2)(NP_037011.2)
SEQ ID No. 105:
   Nucleotide sequence encoding Homo sapiens beta-III-tubulin (TUBB3) NM_006086.3)
   The coding region ranges from nucleotide 124 to nucleotide 1476.
SEQ ID No. 106:
   Amino acid sequence of Homo sapiens beta-III-tubulin (TUBB3) (NP_006077.2)
SEQ ID No. 107:
   Nucleotide sequence encoding Rattus norvegicus beta-III-tubulin (TUBB3)
   The coding region ranges from nucleotide 57 to nucleotide 1409.
SEQ ID No. 108:
   Amino acid sequence of Amino acid sequence coding for Rattus norvegicus bet-III-tubulin (TUBB3)(NP_640347.2)
SEQ ID No. 109:
   Nucleotide sequence encoding Homo sapiens GAP43 isoform 2 (neuomodulin isoform 2)(NM_002045.3)
   The coding region ranges from nucleotide 367 to nucleotide 1083.
SEQ ID No. 110:
   Amino acid sequence of Homo sapiens GAP43 isoform 2 (neuromodulin isoform 2)(NP_002036.1)
SEQ ID No. 111:
   Nucleotide sequence encoding Homo sapiens GAP43 isoform 1 (neuromodulin isoform 1)( NM_001130064.1)
   The coding region ranges from nucleotide 469 to nucleotide 1294.
SEQ ID No. 112:
   Amino acid sequence of Homo sapiens GAP 43 isoform 1 (neuromodulin isoform 1) (NP_001123536.1)
SEQ ID No. 113:
   Nucleotide sequence encoding Rattus norvegicus GAP43 (NM_017195.3)
   The coding region ranges from nucleotide 217 to nucleotide 897.
SEQ ID No. 114:
   Amino acid sequence of Rattus norvegicus GAP43 (neuromodulin) (NP_058891.1)
SEQ ID No. 115:
   Nucleotide sequence encoding Homo sapiens GFAP (S40719.1)
   The coding region ranges from nucleotide 61 to nucleotide 1359.
SEQ ID No. 116:
   Amino acid sequence of Homo sapiens GFAP (AAB22581.1)
SEQ ID No. 117:
   Nucleotide sequence encoding Rattus norvegicus GFAP (BC088851.1)
   The coding region ranges from nucleotide 26 to nucleotide 1318.
SEQ ID No. 118:
   Amino acid sequence of Rattus norvegicus GFAP (AAH88851.1)

### Reference List

1 Li M, Chen M, Han W, Fu X. 2010 How far are induced pluripotent stem cells from the clinic? Ageing Res Rev;9(3):257-64. Epub 2010 Apr 1.
2 Kucia M, Reca R, Campbell FR, Zuba-Surma E, Majka M, Ratajczak J, Ratajczak MZ. 2006 A population of very small embryonic-like (VSEL) CXCR4(+)SSEA-1(+)Oct-4+ stem cells identified in adult bone marrow. Leukemia. 20(5):857-69.
3 Conrad S, Renninger M, Hennenlotter J, Wiesner T, Just L, Bonin M, Aicher W, Bühring HJ, Mattheus U, Mack A, Wagner HJ, Minger S, Matzkies M, Reppel M, Hescheler J, Sievert KD, Stenzl A, Skutella T. 2008 Generation of pluripotent stem cells from adult human testis. Nature. 456(7220):344-9.
4 Lindroos B, Mäenpää K, Ylikomi T, Oja H, Suuronen R, Miettinen S. Characterisation of human dental stem cells and buccal mucosa fibroblasts. Biochem Biophys Res Commun. 368(2):329-35
5 Crisan, M., Yap, S., Casteilla, L., et al. 2008 A Perivascular Origin for Mesenchymal Stem Cells in Multiple Human Organs. Cell Stem Cell, 3, 301-313.
6 Tavazoie, M., Van der Veken, L., Silva-Vargas, V., et al. 2008. A specialized vascular niche for adult neural stem cells. Cell Stem Cell, 3, 279-288.
7 Caplan, A. I. 2008. All MSCs are pericytes? Cell Stem Cell, 3, 229-230.
8 Tempfer H, Wagner A, Gehwolf R, Lehner C, Tauber M, Resch H, Bauer HC 2009 Perivascular cells of the supraspinatus tendon express both tendon- and stem cell-related markers. Histochem Cell Biol; 131(6):733-41
9 Forsgren S, Grimsholm O, Jönsson M, Alfredson H, Danielson P. 2009 New insight into the non-neuronal cholinergic system via studies on chronically painful tendons and inflammatory situations. Life Sci. 19;84(25-26):865-70.
10 Andersson G, Danielson P, Alfredson H, Forsgren S. 2008 Presence of substance P and the neurokinin-1 receptor in tenocytes of the human Achilles tendon. Regul Pept.150(1-3):81-7.
11 Violini S, Ramelli P, Pisani LF, Gorni C, Mariani P. 2009 Horse bone marrow mesenchymal stem cells express embryo stem cell markers and show the ability for tenogenic differentiation by in vitro exposure to BMP-12. BMC Cell Biol. 10:29.
12 Xu C, Inokuma MS, Denham J, Golds K, Kundu P, Gold JD, Carpenter MK. 2001 Feeder-free growth of undifferentiated human embryonic stem cells.Nat Biotechnol. 19(10):971-4.
13 Nagase T, Ueno M, Matsumura M, Muguruma K, Ohgushi M, Kondo N, Kanematsu D, Kanemura Y, Sasai Y. 2009 Pericellular matrix of decidua-derived mesenchymal cells: a potent human-derived substrate for the maintenance culture of human ES cells. Dev Dyn.;238(5):1118-30.
14 Feng J, Mantesso A, Sharpe PT. 2010, Perivascular cells as mesenchymal stem cells. Expert Opin Biol Ther.;10(10):141-51.
15 Díaz-Flores L, Gutiérrez R, Madrid JF, Varela H, Valladares F, Acosta E, Martin-Vasallo P, Díaz-Flores L Jr. 2009 Pericytes. Morphofunction, interactions and pathology in a quiescent and activated mesenchymal cell niche. Histol Histopathol. 24(7):909-69.
16 Corselli M, Chen CW, Crisan M, Lazzari L, Péault B. 2010 Perivascular Ancestors of Adult Multipotent Stem Cells. Arterioscler Thromb Vasc Biol. 30(6):1104-9
17 Salingcarnboriboon R, Yoshitake H, Tsuji K, Obinata M, Amagasa T, Nifuji A, Noda M. 2003. Establishment of tendon-derived cell lines exhibiting pluripotent mesenchymal stem cell-like property. Exp. Cell Res. 287, 289-300
18 Bi Y, Ehirchiou D, Kilts TM, Inkson CA, Embree MC, Sonoyama W, Li L, Leet AI, Seo BM, Zhang L, Shi S, Young MF 2007 .Identification of tendon stem/progenitor cells and the role of the extracellular matrix in their niche. Nat Med. 13(10):1219-27.
19 Rui YF, Lui PP, Li G, Fu SC, Lee YW, Chan KM. Isolation and characterization of multipotent rat tendon-derived stem cells. Tissue Eng Part A. 2010 May;16(5):1549-58.
20 Romagnani P, Annunziato F, Liotta F, Lazzeri E, Mazzinghi B, Frosali F, Cosmi L, Maggi L, Lasagni L, Scheffold A, Kruger M, Dimmeler S, Marra F, Gensini G, Maggi E, Romagnani S. 2005 CD14+CD34low cells with stem cell phenotypic and functional features are the major source of circulating endothelial progenitors. Circ Res. 19;97(4):314-22.
21 Li HY, Chen YJ, Chen SJ, Kao CL, Tseng LM, Lo WL, Chang CM, Yang DM, Ku HH, Twu NF, Liao CY, Chiou SH, Chang YL 2010 Induction of Insulin-Producing Cells Derived from Endometrial Mesenchymal Stem-like Cells. J Pharmacol Exp Ther. 335(3):817-29.
22 Gehwolf R, Griessner M, Pertl H, Obermeyer G 2002, First patch, then catch: measuring the activity and the mRNA transcripts of a proton pump in individual Lilium pollen protoplasts. FEBS Lett;512(1-3):152-6.
23 Shin DM, Zuba-Surma EK, Wu W, Ratajczak J, Wysoczynski M, Ratajczak MZ, Kucia M. 2009 Novel epigenetic mechanisms that control pluripotency and quiescence of adult bone marrow-derived Oct4(+) very small embryonic-like stem cells. Leukemia.;23(11):2042-51.

## Claims

1. Method for the in vitro generation of pluripotent cells (Oct4+; nanog+; Sox2+; Klf4+; c-myc+), said method comprising
a) providing a population of tendon perivascular cells and/or ligament perivascular cells;
b) cultivating the tendon perivascular cells (TPC) and/or ligament perivascular cells of a) in a medium for a time sufficient for a tenosphere to form;
c) recovering pluripotent cells (Oct4+; nanog+; Sox2+; Klf4+; c-myc+) from the tenosphere.

2. The method of claim 1, wherein said population of tendon perivascular cells is obtained from a tendon sample.

3. The method of claim 2, wherein said tendon sample is a sample of biceps tendons, supraspinatus tendons, subscapularis tendons, semitendinosus tendons, palmaris longus tendons or achilles tendons.

4. The method of claim 2 or 3, wherein said tendon is a human tendon, such as a human biceps tendon.

5. The method of any of claims 1 to 4, wherein said population of ligament perivascular cells is obtained from a ligament sample.

6. The method of any of claim 2 to 5, wherein said tendon sample and/or said ligament sample is digested in the presence of serum.

7. The method of any one of claims 1 to 6, wherein the medium comprises Neurobasal medium (NB) or Dulbeccos Modified Eagle Medium (DMEM).

8. The method of any one of claims 1 to 7, wherein the medium comprises B27 supplement.

9. The method of any one of claims 1 to 8, wherein the medium comprises, optionally, FGF and/or EGF.

10. The method of claim 9, wherein the medium comprises FGF in a concentration of from 1 ng/ml to 1 mg/ml and/or EGF in a concentration of from 1 ng/ml to 1 mg/ml.

11. The method of claim 10, wherein the medium comprises FGF in a concentration of 20 ng/ml and/or EGF in a concentration of 20 ng/ml.

12. The method of any one of claims 1 to 11, wherein the tendon perivascular cells and/or the ligament perivascular cells are cultivated under high cell density conditions, such as a cell density of at least 2.5 × 10⁶ cells/100 µl.

13. The method of any one of claims 1 to 12, wherein said medium is changed at most every two weeks.

14. The method of any one of claims 1 to 13, wherein said tenosphere comprises at least 3 and up to 10.000 cells, and/or wherein said tenosphere is floating in the medium.

15. The method of any one of claims 1 to 14, wherein said tenosphere has an average diameter of from 30 µm and up to 400 µm.

16. The method of any one of claims 1 to 15, wherein said pluripotent (reprogrammed) cells are capable of differentiating into cells of all three germ layers.

## Patentansprüche

1. Verfahren zur *in* vitro-Herstellung von pluripotenten Zellen (Oct4+; nanog+; Sox2+; Klf4+; c-myc+), wobei das Verfahren umfasst:
a) Bereitstellen einer Population von perivaskulären Sehnenzellen und/oder perivaskulären Ligamentzellen;
b) Züchten der perivaskulären Sehnenzellen (tendon perivascular cells; TPC) und/oder der perivaskulären Ligamentzellen aus a) in einem Medium über einen Zeitraum, der ausreicht, damit sich eine Tenosphäre (tenosphere) bildet;
c) Gewinnen pluripotenter Zellen (Oct4+; nanog+; Sox2+; Klf4+; c-myc+) aus der Tenosphäre.

2. Verfahren nach Anspruch 1, wobei die Population von perivaskulären Sehnenzellen aus einer Sehnenprobe stammt.

3. Verfahren nach Anspruch 2, wobei die Sehnenprobe eine Probe aus Bizepssehnen, Supraspinatussehnen, Subscapularissehnen, Semitendinosussehnen, Palmaris longus-Sehnen oder Achillessehnen ist.

4. Verfahren nach einem der Ansprüche 2 oder 3, wobei die Sehne eine menschliche Sehne, wie zum Beispiel eine Bizepssehne des Menschen, ist.

5. Verfahren nach einem der Ansprüche 1 bis 4, wobei die Poplulation von perivaskulären Ligamentzellen aus einer Ligamentprobe erhalten wird.

6. Verfahren nach einem der Ansprüche 2 bis 5, wobei die Sehnenprobe und/oder die Ligamentprobe in Gegenwart von Serum verdaut wird.

7. Verfahren nach einem der Ansprüche 1 bis 6, wobei das Medium Neurobasalmedium (NB) oder Dulbeccos Modified Eagle Medium (DMEM) umfasst.

8. Verfahren nach einem der Ansprüche 1 bis 7, wobei das Medium B27-Supplement umfasst.

9. Verfahren nach einem der Ansprüche 1 bis 8, wobei das Medium gegebenenfalls FGF und/oder EGF umfasst.

10. Verfahren nach Anspruch 9, wobei das Medium FGF in einer Konzentration von 1 ng/ml bis 1 mg/ml und/oder EGF in einer Konzentration von 1 ng/ml bis 1 mg/ml umfasst.

11. Verfahren nach Anspruch 10, wobei das Medium FGF in einer Konzentration von 20 ng/ml und/oder EGF in einer Konzentration von 20 ng/ml umfasst.

12. Verfahren nach einem der Ansprüche 1 bis 11, wobei die perivaskulären Sehnenzellen und/oder die perivaskulären Ligamentzellen unter Bedingungen hoher Zelldichte gezüchtet werden, wie einer Zelldichte von mindestens 2,5 x 10⁶ Zellen/100 µl.

13. Verfahren nach einem der Ansprüche 1 bis 12, wobei das Medium höchstens alle zwei Wochen gewechselt wird.

14. Verfahren nach einem der Ansprüche 1 bis 13, wobei die Tenosphäre mindestens 3 und bis zu 10.000 Zellen umfasst und/oder die Tenosphäre im Medium schwimmt.

15. Verfahren nach einem der Ansprüche 1 bis 14, wobei die Tenosphäre einen mittleren Durchmesser von 30 µm und bis zu 400 µm hat.

16. Verfahren nach einem der Ansprüche 1 bis 15, wobei die pluripotenten (reprogrammierten) Zellen in der Lage sind, in Zellen aller drei Keimblätter zu differenzieren.

## Revendications

1. Procédé pour la génération *in vitro* de cellules pluripotentes (Oct4+ ; nanog+ ; Sox2+ ; Klf4+ ; c-myc+), ledit procédé comprenant
a) la mise à disposition d'une population de cellules périvasculaires de tendon et/ou cellules périvasculaires de ligament ;
b) la mise en culture des cellules périvasculaires de tendon (TPC) et/ou cellules périvasculaires de ligament de a) dans un milieu pendant une durée suffisante pour permettre la formation d'une ténosphère ;
c) la récupération des cellules pluripotentes (Oct4+ ; nanog+ ; Sox2+ ; Klf4+ ; c-myc+) à partir de la ténosphère.

2. Procédé selon la revendication 1, dans lequel ladite population de cellules périvasculaires de tendon est obtenue à partir d'un échantillon de tendon.

3. Procédé selon la revendication 2, dans lequel ledit échantillon de tendon est un échantillon de tendons du biceps, tendons du sus-épineux, tendons du sous-scapulaire, tendons du semi-tendineux, tendons du long palmaire ou tendons d'Achille.

4. Procédé selon la revendication 2 ou 3, dans lequel ledit tendon est un tendon humain, comme un tendon de biceps humain.

5. Procédé selon l'une quelconque des revendications 1 à 4, dans lequel ladite population de cellules périvasculaires de ligament est obtenue à partir d'un échantillon de ligament.

6. Procédé selon l'une quelconque des revendications 2 à 5, dans lequel ledit échantillon de tendon et/ou ledit échantillon de ligament est digéré en présence de sérum.

7. Procédé selon l'une quelconque des revendications 1 à 6, dans lequel le milieu comprend un milieu Neurobasal (NB) ou un milieu de Eagle modifié de Dulbecco (DMEM).

8. Procédé selon l'une quelconque des revendications 1 à 7, dans lequel le milieu comprend un supplément B27.

9. Procédé selon l'une quelconque des revendications 1 à 8, dans lequel le milieu comprend, facultativement, le FGF et/ou l'EGF.

10. Procédé selon la revendication 9, dans lequel le milieu comprend le FGF en une concentration comprise entre 1 ng/ml et 1 mg/ml et/ou l'EGF en une concentration comprise entre 1 ng/ml et 1 mg/ml.

11. Procédé selon la revendication 10, dans lequel le milieu comprend le FGF en une concentration de 20 ng/ml et/ou l'EGF en une concentration de 20 ng/ml.

12. Procédé selon l'une quelconque des revendications 1 à 11, dans lequel les cellules périvasculaires de tendon et/ou les cellules périvasculaires de ligament sont mises en culture dans des conditions de haute densité cellulaire, comme une densité cellulaire d'au moins 2,5 x 10⁶ cellules/100 µl.

13. Procédé selon l'une quelconque des revendications 1 à 12, dans lequel ledit milieu est remplacé au maximum toutes les deux semaines.

14. Procédé selon l'une quelconque des revendications 1 à 13, dans lequel ladite ténosphère comprend au moins 3 et jusqu'à 10 000 cellules, et/ou dans lequel ladite ténosphère flotte dans le milieu.

15. Procédé selon l'une quelconque des revendications 1 à 14, dans lequel ladite ténosphère possède un diamètre moyen compris entre 30 µm et jusqu'à 400 µm.

16. Procédé selon l'une quelconque des revendications 1 à 15, dans lequel lesdites cellules pluripotentes (reprogrammées) sont capables de se différencier en des cellules de l'ensemble des trois couches germinales.
